# EUROPÄISCHE PATENTSCHRIFT

(11) **EP 2 555 774 B1**
(45) Veröffentlichungstag und Bekanntmachung des Hinweises auf die Patenterteilung: **21.10.2015**
(21) Anmeldenummer: 11712243.2
(22) Anmeldetag: 01.04.2011
(51) Int. Cl.: A61K 31/437, A61K 31/44, A61K 31/498, A61P 11/00, A61K 45/06, A61K 31/519, A61K 31/4745

(54) **ARNZEIMITTELKOMBINATIONEN ENTHALTEND PDE4-INHIBITOREN UND EP4-REZEPTOR-ANTAGONISTEN**
Medicine combinations containing pde4 inhibitors and ep4 receptor antagonists
Combinaisons de médicaments comprenant des inhibiteurs de pde4 et antagonistes des récepteurs d'ep4

(30) Priorität: 08.04.2010 EP 10159390
(43) Veröffentlichungstag der Anmeldung: 13.02.2013
(73) Patentinhaber: Boehringer Ingelheim International GmbH, 55216 Ingelheim am Rhein (DE)
(72) Erfinder: NICKOLAUS, Peter, 55216 Ingelheim Am Rhein (DE)
(74) Vertreter: Simon, Elke Anna Maria
(86) Internationale Anmeldenummer: PCT/EP2011/055074
(87) Internationale Veröffentlichungsnummer: WO 2011/124525

(56) Entgegenhaltungen:
- WO-A1-02/092097
- WO-A1-2006/122403
- WO-A1-2009/050248
- US-A1- 2004 142 969
- US-A1- 2004 254 233
- HERTZ ANGIE L ET AL: "Elevated cyclic AMP and PDE4 inhibition induce chemokine expression in human monocyte-derived macrophages", PROCEEDINGS OF THE NATIONAL ACADEMY OF SCIENCES OF THE UNITED STATES OF AMERICA, Bd. 106, Nr. 51, Dezember 2009 (2009-12), Seiten 21978-21983, XP002589903, ISSN: 0027-8424
- SASTRE B ET AL: "Prostaglandin E2 may decrease airway inflammation and muscular hyperplasia in eosinophilic bronchitis patients through prostanoid receptors EP2 and EP4", ALLERGY (OXFORD), Bd. 64, Nr. Suppl. 90, 2009, Seiten 135-136, XP009135577, & 28TH CONGRESS OF THE EUROPEAN-ACADEMY-OF-ALLERGY-AND-CLINICAL-I MMUNO LOGY; WARSAW, POLAND; JUNE 06 -10, 2009 ISSN: 0105-4538
- REHAL SONIA ET AL: "Characterization of biosynthesis and modes of action of prostaglandin E-2 and prostacyclin in guinea pig mesenteric lymphatic vessels", BRITISH JOURNAL OF PHARMACOLOGY, Bd. 158, Nr. 8, Dezember 2009 (2009-12), Seiten 1961-1970, XP002589905,
- BENYAHIA C ET AL: "PGE2 receptor (EP4) agonists: Potent dilators of human bronchi and future asthma therapy?", PULMORNARY PHARMACOLOGY & THERAPEUTICS, ACADEMIC PRESS, GB , vol. 25, no. 1 1 February 2012 (2012-02-01), pages 115-118, XP008154291, ISSN: 1094-5539, DOI: 10.1016/J.PUPT.2011.12.012 Retrieved from the Internet: URL:http://sciencedirect.com/science/journ al/10945539 [retrieved on 2012-01-05]
- J. Buckley ET AL: "EP4 receptor as a new target for bronchodilator therapy", Thorax, vol. 66, no. 12, 23 May 2011 (2011-05-23), pages 1029-1035, XP055121416, ISSN: 0040-6376, DOI: 10.1136/thx.2010.158568
- OKUMURA T ET AL: "Effects of the selective EP4 antagonist CJ-023,423 on chronic inflammation and bone destruction in rat adjuvant-induced arthritis", JOURNAL OF PHARMACY AND PHARMACOLOGY, JOHN WILEY & SONS LTD, LONDON; GB, vol. 60, no. 6, 1 June 2008 (2008-06-01), pages 723-730, XP002712071, ISSN: 0022-3573, DOI: 10.1211/JPP.60.6.0007
- XINRONG MA ET AL: "Prostaglandin E receptor EP4 antagonism inhibits breast cancer metastasis", CANCER RESEARCH, AMERICAN ASSOCIATION FOR CANCER RESEARCH, US , vol. 66, no. 6 15 March 2006 (2006-03-15), pages 2923-2927, XP002695036, ISSN: 0008-5472, DOI: 10.1158/0008-5472.CAN-05-4348 Retrieved from the Internet: URL:http://cancerres.aacrjournals.org/cont ent/66/6/2923

## Beschreibung

Die Erfindung betrifft Arzneimittelkombinationen, die neben einem oder mehreren, bevorzugt einem PDE4-Inhibitor der allgemeinen Formel **1** 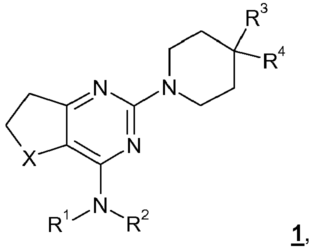 worin
**X** SO oder SO₂, vorzugsweise jedoch SO ist, und worin R¹, R², R³ und R⁴ die in Anspruch 1 genannten Bedeutungen haben, wenigstens einen EP4-Rezeptor-Antagonisten (**2**) umfassen, deren Herstellung und deren Verwendung zur Therapie von insbesondere Atemwegserkrankungen wie beispielsweise COPD, chronische Sinusitis und Asthma.

### STAND DER TECHNIK

Die WO2009050248 offenbart Piperidino-Dihydrothienopyrimidine der Formel **1** als PDE4-Inhibitoren, deren Herstellung sowie deren Verwendung zur Therapie von Atemwegserkrankungen.
Weiterhin ist bekannt, dass viele PDE4-Inhibitoren der "1. Generation" wie beispielsweise Rolipram zu unerwünschten Nebenwirkungen führen. Es war folglich Aufgabe der vorliegenden Erfindung, ein Arzneimittel bzw. eine Arzneimittelkombination enthaltend einen PDE4-Inhibitor bereitzustellen, welches über ein geringes Nebenwirkungsprofil verfügt. Überraschenderweise könnte gefunden werden, dass ein EP4-Rezeptor-Antagonist, der gleichzeitig mit einem PDE4-lnhibitor oder um wenige Stunden (maximal 6 Stunden) zeitlich versetzt mit einem PDE4-Inhibitor verabreicht wird, die typischen Nebenwirkungen eines PDE4-Inhibitors stark verringert, ohne jedoch selbst nennenswerte Nebenwirkungen bei einer Dauertherapie aufzuweisen.

EP4-Rezeptor-Antagonisten wie beispielsweise [N-{[4-(5,9-Diethoxy-6-oxo-6,8-dihydro-7H-pyrrolo[3,4-g]chinolin-7yl)-3-methylbenzyl]sulfonyl}-2-(2-methoxyphenyl)acetamid] (auch MF498 genannt) waren zwar z.B. aus Clark et al; The Journal of Pharmacology and Experimental Therapeutics; Vol. 325, Nr. 2; Seiten 425-434 bereits bekannt, dennoch war nicht bekannt, dass typische PDE4-vermittelte Nebenwirkungen durch solche EP4-Rezeptor-Antagonisten deutlich verringert werden.

Die vorliegende Erfindung betrifft daher eine neuartige Arzneimittelkombination, die neben einem oder mehreren PDE4-Inhibitoren (**1**) wenigstens einen EP4-Rezeptor-Antagonisten (**2**) umfasst.
Die vorliegende Erfindung betrifft vorzugsweise solche Arzneimittelkombinationen, die neben einem oder mehreren PDE4-Inhibitoren (**1**) wenigstens einen EP4-Rezeptor-Antagonisten (**2**) umfassen und bei denen der wenigstens eine EP4-Rezeptor-Antagonist (**2**) ein EP4-spezifischer Antagonist ist.

Eine bevorzugte Ausführungsform der vorliegenden Erfindung betrifft eine der oben genannten Arzneimittelkombinationen, bei der der wenigstens eine EP4-Rezeptor-Antagonist ausgewählt ist aus der Gruppe bestehend aus
[N-{[4-(5,9-Diethoxy-6-oxo-6,8-dihydro-7H-pyrrolo[3,4-g] chinolin-7yl)-3-methylbenzyl]sulfonyl}-2-(2-methoxyphenyl)acetamid] (**2.1**);
5-Butyl-2,4-dihydro-4-[[2'-[N-(3-methyl-2-thiophen-carbonyl)sulfamoyl]biphenyl-4-yl]methyl]-2-[(2-trifluoromethyl)phenyl]-1, 2, 4-triazol-3-on (**2.2**);
(4-{(1S)-1-[({5-chloro-2-[(4-fluorophenyl)oxy]phenyl}carbonyl)amino]ethyl}benzoesäure (**2.3**); N-[({2-[4-(2-ethyl-4,6-dimethyl-1 H-imidazo [4,5-c]pyridin-1-yl)phenyl]ethyl}amino)carbonyl]-4-methylbenzol sulfonamid (**2.4**);
4-[[4-(5-methoxy-2-pyridinyl)phenoxy]methyl]-5-methyl-N-[(2-methylphenyl)sulfonyl]-2-Furancarboxamid (**2.5**);
11alpha, 15alpha-Dihydroxy-16-(3-methoxymethylphenyl)-9-oxo-17,18,19, 20-tetranor-5-thia-13(E) Prostansäuremethylester (**2.6**);
4-cyano-2-[[2-(4-fluoro-1-naphthalenyl)-1-oxopropyl]amino]-Benzolbutansäure (**2.7**) und N-{2-[4-(4,9-diethoxy-1-oxo-1,3-dihydro-2H-benzo[f]isoindol-2-yl)phenyl]acetyl}benzene sulphonamide (**2.8**).

Eine besonders bevorzugte Ausführungsform der vorliegenden Erfindung betrifft eine der oben genannten Arzneimittelkombinationen, die neben einem oder mehreren, bevorzugt einem PDE4-Inhibitor (**1**) der allgemeinen Formel **1** 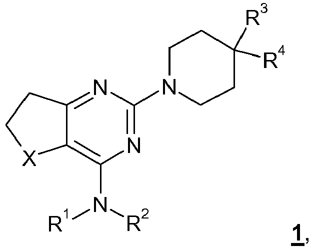 worin
- **X**: SO oder SO₂,
- **R¹**: H, C₁₋₆-Alkyl,
- **R²**: H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, C₆₋₁₀-Aryl, -Het, Hetaryl, einem mono- oder bicyclischem -C₃₋₁₀-cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
- **Het**: ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
- **Hetaryl**: ein fünf- bis zehngliedriges, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
- **Cycloalkyl**: gesättigt oder teilweise gesättigt sein kann,
- wobei **R^{2.1}**: H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀-Aryl-C₁₋₆-alkylen, Hetaryl-C₁₋₆-alkylen-, Het-C₁₋₆-alkylen-, C₃₋₁₀-Cycloalkyl-C₁₋₆-alkylen-, ein mono- oder bicyclisches C₆₋₁₀-Aryl, Heteroaryl und ein -Het,
der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann,
- wobei **R^{2.2}** und **R^{2.3}**: unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀-Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃, - CO-N(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
oder
- **R²**: ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, -SO₂-NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -NH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl), -N(C₁₋₃-Alkyl)-CO-(C₁₋₆-Alkyl), -N(C₁₋₃-Alkyl)-CO-O-(C₆₋₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀-Aryl), - N(C₁₋₃-Alkyl)-CO-(C₁₋₃-Alkylen)-(C₆₋₁₀-Aryl), C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, Het, C₁₋₆-Alkyl, C₁₋₃-Fluoroalkyl, CF₃, CHF₂, CH₂F, C₆₋₁₀-Aryl-C₁₋₆-alkylen,
Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
- **NR¹R²**: gemeinsam einen heterocyclischen vier- bis siebengliedrigen Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
- **R³**: ein C₆₋₁₀-Aryl ist,
welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het, - CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁₀-Aryl-C₁₋₂-alkylen, Het-C₁₋₂-alkylen, -Het, -CO-Het, , CO-N(CH₃)-C₃₋₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist,
wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
- **R³**: ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-Aryl, Het, C₃₋₇-Cycloalkyl und Hetaryl substituiert sein kann,
der wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
oder worin
- **R³**: -O-R^{3.1},
- wobei **R^{3.1}**: ein Rest ausgewählt aus der Gruppe bestehend aus -C₁₋₆-Alkyl, -C₆₋₁₀-Aryl, - C₁₋₃-Alkylen-C₆₋₁₀-Aryl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁₋₆-Alkylen)-Hetaryl, -CO-N(C₁₋₃-Alkyl)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, C₆₋₁₀-Aryl, C₆₋₁₀-Aryl-C₁₋₂-alkylen, Het-C₁₋₂-alkylen, -CO-Het, Het, -CO-C₃₋₇-Cycloalkyl, -CO-N(C₁₋₃-Alkyl)-C₃₋₇-Cycloalkyl C₃₋₇-Cycloalkyl, C₃₋₇-Cycloalkyl-C₁₋₂-alkylen, Hetaryl-C₁₋₂-alkylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann.
und worin
- **R⁴**: H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(C₁₋₃-Alkyl)-SO₂-(C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl), -C₁₋₃-Alkylen-NH-CO-(C₁₋₃-Alkylen)-O-(C₁₋₃-Alkyl), -CO-(C₆₋₁₀-Aryl), -(C₁₋₂-Alkylen)-N(C₁₋₃-Alkyl)-CO-(C₁₋₂-Alkylen)-O-(C₁₋₃-Alkyl),
wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann,
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆-Alkyl, -O-R^{2.1}, - COOR^{2.1}, SO-R^{2.1}, SO₂-R^{2.1}, -C₁₋₃-alkylen-NR^{2.2}R^{2.3}, -NR^{2.2}R^{2.3}, C₆₋₁₀-Aryl, C₃₋₇-Cycloalkyl, Het und Hetaryl substituiert sein kann,
bedeuten,
wenigstens einen EP4-Rezeptor-Antagonisten (**2**) umfassen.

Bevorzugt sind weiterhin die oben genannten Arzneimittelkombinationen, die neben einem oder mehreren, bevorzugt einem PDE4-Inhibitor (**1**) der allgemeinen Formel **1**,
worin
- **X**: SO,
- **R¹**: H
- **R²**: H ist oder C₁₋₆-Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
- **Het**: ein drei- bis siebengliedriger, monocyclischer, gesättigter oder teilweise gesättigter Heterocyclus ist oder ein sieben- bis elfgliedriger, bicyclischer, gesättigter oder teilweise gesättigter Heterocyclus ist, der 1, 2 oder 3 Heteroatome unabhängig voneinander ausgewählt aus der Gruppe bestehend aus N, S oder O enthält, und wobei
- **Hetaryl**: ein fünf- bis sechsgliedriges, monocyclisches, aromatisches Heteroaryl ist, oder ein sieben- bis elfgliedriges, bicyclisches, aromatisches Heteroaryl ist, das 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
wobei **R^{2.1}** H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂-alkylen, -Het-C₁₋₂-alkylen, C₃₋₇-Cycloalkyl-C₁₋₂-alkylen, Phenyl, Hetaryl und ein Het, der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Alkyl), CO-R^{2.1} und COOR^{2.1},
der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
- **R²**: ein monocyclisches C₃₋₇ Cycloalkyl, das gegebenenfalls mit einem Rest ausgewählt aus der Gruppe bestehend aus C₁₋₂-Alkanol, C₁₋₃-Fluoroalkyl, C₁₋₃-alkylen-OR^{2.1}, OR^{2.1}, COOR^{2.1}, SO₂-NR^{2.2}R^{2.3}, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
- **R²**: ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
- **R²**: ein Rest ausgewählt aus einer Gruppe bestehend aus Het und Hetaryl, der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂-alkylen, -Het, -Hetaryl und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Butyl, Isobutyl, Phenyl und NR^{2.2}R^{2.3} substituiert sein kann,
und worin
- **R³**: ein Naphthalin oder Phenyl ist,
welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃; SO₂-CH₃, SO-CH₃, COOCH₃, COOCH₂CH₃, -CO-NH-(Methylen)-Hetaryl, -CO-NH-(Ethylen)-Hetaryl, -CO-NH-Hetaryl, -CO-N(CH₃)-Het, - CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het, -NH-CO-Methyl , NCH₃-CO-Methyl, -NH-CO-Ethyl , NCH₃-CO-Ethyl, -NH-CO-Propyl, NCH₃-CO-Propyl, -NH-CO-Isopropyl, NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO-N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₃) und -N(CH₃)₂ substituiert sein kann,
wobei dieser Rest gegebenenfalls durch einen oder mehrere Reste ausgewählt aus der Gruppe bestehend aus OH, F, Cl, -CF₃, CHF₂, CH₂F, Oxo, Methyl und Phenyl substituiert sein kann
oder worin
- **R³**: ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl, - COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₃)₂, -NH₂, - NH(CH₃), -N(CH₃)₂, Het und Hetaryl substituiert sein kann,
der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
oder worin
- **R³**: -O-R^{3.1},
- wobei **R^{3.1}**: ein Rest ausgewählt aus der Gruppe bestehend aus -C₁₋₃-Alkyl, -Phenyl, - C₁₋₃-Alkylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N(C₅₋₇-Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂, -Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl, , SO₂-Methyl, -SO₂-Ethyl, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇-Cycloalkyl, -CO-N(CH₃)-Cyclopropyl,
C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
- **R⁴**: H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Me -(Ethylen)-NH-SO₂-(E -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇-Cycloalkyl, Het und Hetaryl substituiert sein kann,
bedeuten,
wenigstens einen EP4-Rezeptor-Antagonisten (**2**) umfassen.

Einen weiteren besonders bevorzugten Gegenstand der Erfindung stellen solche der oben genannten Arzneimittelkombinationen dar, die neben einem oder mehreren, bevorzugt einem PDE4-lnhibitor der allgemeinen Formel **1**, worin
- **R²**: ein Rest nach Formel **3** 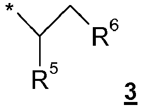
ist,
- worin **R⁶**: OH oder NH₂ ist und
- worin **R⁵**: einen Rest ausgewählt aus der Gruppe bestehend aus C₁₋₄-Alkyl, einem fünfbis sechsgliedrigen Heteroaryl mit 1, 2 oder 3 Heteroatomen aus der Gruppe S, O und N und Phenyl, welcher gegebenenfalls mit einem oder mehreren Resten ausgewählt aus der Gruppe bestehend aus OH, F, Br, OR^{2.1}, Oxo, Methyl, Ethyl, Methanol, Ethanol, Phenyl, COOR^{2.1}, CH₂-NR^{2.2}R^{2.3} und NR^{2.2}R^{2.3} substituiert sein kann,
bedeutet,
wenigstens einen EP4-Rezeptor-Antagonisten (**2**) umfassen.

In einer weiteren besonders bevorzugten Ausführungsform betrifft die vorliegende Erfindung solche der oben genannten Arzneimittelkombinationen, die neben einem oder mehreren, bevorzugt einem PDE4-Inhibitor der allgemeinen Formel **1**, worin
- **R²**: ein Rest ist nach Formel **3** 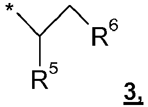
- worin **R⁶**: OH oder NH₂ ist und
- worin **R⁵**: Methyl, Ethyl, Propyl oder Isopropyl
bedeutet,
wenigstens einen EP4-Rezeptor-Antagonisten (**2**) umfassen.

Die vorliegende Erfindung betrifft weiterhin vorzugsweise solche der obigen Arzneimittelkombinationen, die neben einem oder mehreren, bevorzugt einem PDE4-Inhibitor der allgemeinen Formel **1**, worin
- **R²**: ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, und wobei
R^{2.1} ausgewählt ist aus der Gruppe bestehend aus Methyl, Ethyl, Propyl, Isopropyl, Butyl, Isobutyl,
wenigstens einen EP4-Rezeptor-Antagonisten (**2**) umfassen.

In einer weiteren besonders bevorzugten Ausführungsform betrifft die vorliegende Erfindung solche der oben genannten Arzneimittelkombinationen, die neben einem oder mehreren, bevorzugt einem PDE4-Inhibitor der allgemeinen Formel **1**, worin
- **R²**: ein Cyclopropyl ist, das gegebenenfalls mit einem weiteren Rest ausgewählt aus der Gruppe bestehend aus -NH₂, CH2- NH₂, -NH(CH₃), -N(CH₃)₂, Methyl, Ethyl, Propyl, Isopropyl, -NH-CO-(tert-Butyl), -NH-CO-O-(tert-Butyl), -N(CH₃)-CO-(tert-Butyl), -N(CH₃)-CO-O-(tert-Butyl), -CF₃, -CHF₂, CH₂F, F, Cl und Br substituiert sein kann,
wenigstens einen EP4-Rezeptor-Antagonisten (**2**) umfassen.

Einen weiteren bevorzugten Gegenstand der vorliegenden Erfindung stellen solche der oben genannten Arzneimittelkombinationen dar, die neben einem oder mehreren, bevorzugt einem PDE4-Inhibitor der allgemeinen Formel **1**, worin
- **R²**: ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei R^{2.1} H, Methyl oder Ethyl sein kann,
wenigstens einen EP4-Rezeptor-Antagonisten (**2**) umfassen.

Im Rahmen der vorliegenden Erfindung ist eine solche der obigen Arzneimittelkombinationen weiterhin besonders bevorzugt, die neben einem oder mehreren, bevorzugt einem PDE4-Inhibitor der allgemeinen Formel **1**, worin
- **R²**: ein Rest ist ausgewählt aus einer Gruppe bestehend aus einem monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, CF₃, CHF₂, CH₂F, OH und Oxo oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
wobei **R^{2.1}**, **R^{2.2}** und **R^{2.3}** wie in Anspruch 1 definiert sind,
wenigstens einen EP4-Rezeptor-Antagonisten (**2**) umfasst.

Ein weiterer besonders bevorzugter Gegenstand der Erfindung betrifft eine Arzneimittelkombination, die neben einem oder mehreren, bevorzugt einem PDE4-Inhibitor der allgemeinen Formel **1**, worin
- **R²**: einen Rest ausgewählt aus einer Gruppe bestehend aus einem monocyclischen, gesättigten sechsgliedrigem Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃, N(CH₃)₂, Methyl, Ethyl, Propyl, Isopropyl, Cyclopropyl, Methoxy und Ethoxy substituiert sein kann,
wenigstens einen EP4-Rezeptor-Antagonisten (**2**) umfasst.

Weiterhin besonders bevorzugt ist eine solche der obigen Arzneimittelkombinationen, die neben einem oder mehreren, bevorzugt einem PDE4-Inhibitor der allgemeinen Formel **1**, worin
- **R²**: einen Rest ausgewählt aus einer Gruppe bestehend aus Piperidin oder Tetrahydropyran bedeutet, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
wenigstens einen EP4-Rezeptor-Antagonisten (**2**) umfasst.

Ein weiterer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **R³**: ein Naphthalin oder Phenyl ist,
welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, - OCH₃, OCH₂CH₃; SO₂-CH₃, SO₂-CH₂CH₃, COOCH₃ und CO-O-CH₂CH₃ substituiert sein kann,
wenigstens einen EP4-Rezeptor-Antagonisten (**2**) umfasst.

Weiterhin besonders bevorzugt ist eine solche der obigen Arzneimittelkombinationen, die neben einem oder mehreren, bevorzugt einem PDE4-Inhibitor der allgemeinen Formel **1**, worin
- **R³**: ein Rest ausgewählt aus der Gruppe bestehend aus Het und Hetaryl ist, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₅₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl, -COO-Propyl, -COO-Isopropyl und O-Methyl, O-Ethyl, O-Propyl und O-Isopropyl substituiert sein kann,
und worin
- **R⁴**: H, CN, OH, CF₃, CHF₂, CH₂F, F, Methyl, Ethyl, O-Methyl oder O-Ethyl, und
wobei
- **Het**: ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
bedeuten,
wenigstens einen EP4-Rezeptor-Antagonisten (**2**) umfasst.

Ein weiterer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **R³**: ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl, - COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann,
der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, - COO-Ethyl und O-Methyl, O-Ethyl substituiert sein kann,
wenigstens einen EP4-Rezeptor-Antagonisten (**2**) umfasst.

Ein anderer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **R³**: ein Rest ausgewählt aus einem monocyclischen, gesättigten oder teilweise gesättigten, drei- bis siebengliedrigem Heterocyclus oder einem monocyclischen fünfbis sechsgliedrigem Heteroaryl ist,
der ausgewählt ist aus der Gruppe bestehend aus Imidazol, Dihydroimidazol, Oxadiazol, Oxadiazolidin, Pyrazol, Pyridin und Dihydropyrazol,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, --O-Methyl, -O-Ethyl, -O-Propyl, -O-Isopropyl, - COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
wenigstens einen EP4-Rezeptor-Antagonisten (**2**) umfasst.

Weiterhin besonders bevorzugt ist eine solche der obigen Arzneimittelkombinationen, die neben einem oder mehreren, bevorzugt einem PDE4-Inhibitor der allgemeinen Formel **1**, worin
- **R³** und **R⁴**: gemeinsam einen mono- oder bicyclischen, ungesättigten oder teilweise gesättigten, drei- bis elfgliedrigen Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CHrCH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
wenigstens einen EP4-Rezeptor-Antagonisten (**2**) umfassen.

Ein weiterer besonders bevorzugter Gegenstand der Erfindung betrifft eine der oben genannten Arzneimittelkombinationen, die neben einem oder mehreren, bevorzugt einem PDE4-lnhibitor der allgemeinen Formel **1**, worin
- **R³** und **R⁴**: gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigten, fünf- oder sechsgliedrigem Heterocyclus und einem fünf- oder sechsgliedrigem Heteroaryl substituiert sein kann,
wenigstens einen EP4-Rezeptor-Antagonisten (**2**) umfassen.

Weiterhin besonders bevorzugt ist eine solche der obigen Arzneimittelkombinationen, die neben einem oder mehreren, bevorzugt einem PDE4-Inhibitor der allgemeinen Formel **1**, worin
- **R³**: -O-R^{3.1} ist,
- **R^{3.1}**: ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, -CF₃, CHF₂, CH₂F, CO-(Methyl), CO-(Ethyl), CO-(Propyl), CO-(Isopropyl), CO-(Butyl), CO-(Isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NHCH₃, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇-Cycloalkyl, -CO-Cyclopropyl, -CO-N(CH₃)-Cyclopropyl, -CO-N(CH₃)-C₄₋₇-Cycloalkyl, C₄₋₇-Cycloalkyl, Cyclopropyl, C₄₋₇-Cycloalkyl-Methylen-, Cyclopropyl-Methylen-, C₄₋₇-Cycloalkyl-Ethylen-, Cyclopropyl-Ethylen-, Hetaryl-Methylen-, Hetaryl-Ethylen- und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O- Methyl, Ethyl, O-Ethyl, OH, Oxo und CF₃ substituiert sein kann,
wenigstens einen EP4-Rezeptor-Antagonisten (**2**) umfasst.

Ein weiterer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **R⁴**: ausgewählt ist aus der Gruppe bestehend aus
H, CN, OH, CF₃, CHF₂, CH₂F, F, Methyl, Ethyl, O-Methyl, O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl,
und wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann,
wenigstens einen EP4-Rezeptor-Antagonisten (**2**) umfasst.

Ein anderer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **R³**: ein Rest ist ausgewählt aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl, O-Methyl, O-Ethyl, O-Propyl, O-Isopropyl, OH, F, Cl, Br, CF₃, Phenyl, Hetaryl und C₃₋₆-Cycloalkyl substituiert sein kann,
wenigstens einen EP4-Rezeptor-Antagonisten (**2**) umfasst.

Ein weiterer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1**, worin
- **X**: SO₂ ist,
wenigstens einen EP4-Rezeptor-Antagonisten (**2**) umfasst.

Ein weiterer besonders bevorzugter Gegenstand der Erfindung betrifft eine der oben genannten Arzneimittelkombinationen, die neben einem oder mehreren, bevorzugt einem PDE4-Inhibitor der allgemeinen Formel **1** ausgewählt aus der Gruppe bestehend aus:
1.1. (R)-2-{2-[4-(4-Chlorphenyl)-piperidin-1-yl]-5-oxo-6,7-dihydro-5*H*-5λ⁴-thieno[3,2-*d*]pyrimidin-4-ylamino}-3-methylbutan-1-ol
1.2. (1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-methanol
1.3. (R)-2-{2-[4-(4-Chlorphenyl)-piperidin-1-yl]-5-oxo-6,7-dihydro-5H-5λ⁴-thieno[3,2-*d*]pyrimidin-4-ylamino}-pentan-1-ol
1.4. (R)-1-{2-[4-(4-Chlorphenyl)-piperidin-1-yl]-5-oxo-6,7-dihydro-5*H*-5λ⁴-thieno[3,2-*d*]pyrimidin-4-ylamino}-1-(4-fluorphenyl)-2-methylpropan-2-ol
1.5. (S)-5-{2-[4-(4-Chlorphenyl)-piperidin-1-yl]-5-oxo-6,7-dihydro-5*H*-5λ4-thieno[3,2-*d*]pyrimidin-4-ylamino}-1-methylpiperidin-2-on
1.6. {2-[4-(4-Chlorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.7. 1-(4-(1-Hydroxymethylcyclopropylamino)-5-oxo-6,7-dihydro-5*H*-5λ⁴-thieno[3,2-*d*]pyrimidin-2-yl)-3'-methyl-1'*H*-spiro[piperidin-4,4'-chinazolin]-2'(3'*H*)-on
1.8. {1-[2-(4-Benzo[*d*]isoxazol-3-yl-piperidin-1-yl)-5-oxo-6,7-dihydro-5*H*-5λ⁴-thieno[3,2-*d*]pyrimidin-4-ylamino]-cyclopropyl}-methanol
1.9. (1-{2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-ylamino}-cyclopropyl)-methanol
1.10. 1-[4-((S)-1-Methyl-6-oxopiperidin-3-ylamino)-5-oxo-6,7-dihydro-5*H*-5λ⁴-thieno[3,2-*d*]pyrimidin-2-yl]-4-phenylpiperidin-4-carbonitril
1.11. 3'-methyl-1-(4-(tetrahydro-2*H*-pyran-4-ylamino)-5-oxo-6,7-dihydro-5*H*-5λ⁴-thieno[3,2-*d*]pyrimidin-2-yl)-1'*H*-spiro[piperidin-4,4'-chinazolin]-2'(3'*H*)-on
1.12. (3-Fluorphenyl)-[5-oxo-2-(3,4,5,6-tetrahydro-2*H*-[4,4']bipyridinyl-1-yl)-6,7-dihydro-5*H-*5λ⁴-thieno[3,2-*d*]pyrimidin-4-yl]-amin
1.13. {2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-yl}-(3-fluorphenyl)-amin
1.14. (1-{2-[4-(2,4-Difluorphenyl)-piperidin-1-yl]-5-oxo-6,7-dihydro-5*H*-5λ⁴-thieno[3,2-*d*]pyrimidin-4-ylamino}-cyclopropyl)-methanol
1.15. {2-[4-(2,4-Difluorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.16. (S)-5-[2-(4-Benzoxazol-2-yl-piperidin-1-yl)-5-oxo-6,7-dihydro-5*H*-5λ⁴-thieno[3,2-*d*]pyrimidin-4-ylamino]-1-methylpiperidin-2-on
1.17. (1-{2-[4-(6-Fluorbenzo[*d*]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.18. (1-{2-[4-(5-Fluorbenzo[*d*]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.19. {2-[4-(5-Furan-2-yl-2*H*-pyrazol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.20. (3-Fluorphenyl)-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-yl}-amin
1.21. (R)-3-Methyl-2-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-ylamino}-butan-1-ol
1.22. (S)-5-{2-[4-(4-Fluorphenoxy)-piperidin-1-yl]-5-oxo-6,7-dihydro-5*H*-5λ⁴-thieno[3,2-*d*]pyrimidin-4-ylamino}-1-methylpiperidin-2-on
1.23. (2-{4-[4-(4,5-Dihydrooxazol-2-yl)-phenoxy]-piperidin-1-yl}-5-oxo-6,7-dihydro-5H-5λ⁴-thieno[3,2-d]pyrimidin-4-yl)-(tetrahydropyran-4-yl)-amin
1.24. 4-{1-[5-Oxo-4-(tetrahydropyran-4-ylamino)-6,7-dihydro-5H-5λ⁴-thieno[3,2-*d*]pyrimidin-2-yl]-piperidin-4-yloxy}-benzoesäure
1.25. 2-(1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-propan-2-ol
1.26. {2-[4-(5-*tert*-Butyl-1-methyl-1H-indol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.27. 2-[4-(5-Furan-2-yl-1-methyl-1*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.28. (S)-5-(2-{4-[4-(4,5-Dihydrooxazol-2-yl)-phenoxy]-piperidin-1-yl}-5-oxo-6,7-dihydro-5*H*-5λ⁴-thieno[3,2-*d*]pyrimidin-4-ylamino)-1-methylpiperidin-2-on
1.29. {2-[4-(5-Furan-2-yl-2-methyl-2*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.30. {2-[4-(1-Methyl-1*H*-imidazo[4,5-*c*]pyridin-2-yl)-piperidin-1-yl]-5-oxo-6,7-dihydro-5*H-*5λ⁴-thieno[3,2-*d*]pyrimidin-4-yl}-(tetrahydropyran-4-yl)-amin
1.31. 2-Methoxy-*N*-{1-[5-oxo-4-(tetrahydropyran-4-ylamino)-6,7-dihydro-5*H*-5λ⁴-thieno[3,2-*d*]pyrimidin-2-yl]-4-phenylpiperidin-4-ylmethyl}-acetamid
1.32. N-Cyclopropyl-N-methyl-4-{1-[5-oxo-4-(tetrahydropyran-4-ylamino)-6,7-dihydro-5*H-*5λ⁴-thieno[3,2-*d*]pyrimidin-2-yl]-piperidin-4-yl}-benzamid
1.33. *N*-Cyclopropyl-N-methyl-4-{1-[5-oxo-4-(tetrahydropyran-4-ylamino)-6,7-dihydro-5*H-*5λ⁴-thieno[3,2-*d*]pyrimidin-2-yl]-piperidin-4-yloxy}-benzamid
1.34. {5-Oxo-2-[4-(pyridin-4-yloxy)-piperidin-1-yl]-6,7-dihydro-5*H*-5λ⁴-thieno[3,2-*d*]pyrimidin-4-yl}-(tetrahydropyran-4-yl)-amin
1.35. {2-[4-(4-Chlorphenoxy)-piperidin-1-yl]-5-oxo-6,7-dihydro-5*H*-5λ⁴-thieno[3,2-*d*]pyrimidin-4-yl}-(tetrahydropyran-4-yl)-amin
1.36. (S)-1-Methyl-5-{2-[4-(5-methyl-4-phenyloxazol-2-yl)-piperidin-1-yl]-5-oxo-6,7-dihydro-5*H*-5λ⁴-thieno[3,2-*d*]pyrimidin-4-ylamino}-piperidin-2-on
1.37. (1-{2-[4-(5-Methyl-4-phenyloxazol-2-yl)-piperidin-1-yl]-5-oxo-6,7-dihydro-5*H*-5λ⁴-thieno[3,2-*d*]pyrimidin-4-ylamino}-cyclopropyl)-methanol
1.38. (S)-5-{2-[4-(4,5-Diphenyloxazol-2-yl)-piperidin-1-yl]-5-oxo-6,7-dihydro-5*H*-5λ⁴-thieno[3,2-*d*]pyrimidin-4-ylamino}-1-methylpiperidin-2-on
1.39. {4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-yl}-methanol
1.40. [1-(2-{4-[5-(4-Chlorphenyl)-4-methyloxazol-2-yl]-piperidin-1-yl}-5-oxo-6,7-dihydro-5H-5λ⁴-thieno[3,2-*d*]pyrimidin-4-ylamino)-cyclopropyl]-methanol
1.41. 4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-ol
1.42. {2-[4-(4-Chlorphenyl)-4-methoxypiperidin-1-yl]-5-oxo-6,7-dihydro-5*H*-5λ⁴-thieno[3,2-*d*]pyrimidin-4-yl}-(tetrahydropyran-4-yl)-amin
1.43. 4-{1-[4-(1-Hydroxymethylcyclopropylamino)-5-oxo-6,7-dihydro-5*H*-5λ⁴-thieno[3,2-*d*]pyrimidin-2-yl]-piperidin-4-yloxy}-benzonitril
1.44. 5-Oxo-2-[4-(4,5,6,7-tetrahydrobenzoxazol-2-yl)-piperidin-1-yl]-6,7-dihydro-5H-5λ⁴-thieno[3,2-*d*]pyrimidin-4-yl}-(tetrahydropyran-4-yl)-amin
1.45. (S)-5-{2-[4-(4-Chlorphenyl)-piperidin-1-yl]-5,5-dioxo-6,7-dihydro-5H-5λ⁶-thieno[3,2-*d*]pyrimidin-4-ylamino}-1-methylpiperidin-2-on.
wenigstens einen EP4-Rezeptor-Antagonisten (**2**) umfasst.

Die oben genannten Verbindungen der Formel **1** werden wie in den Synthesevorschriften im Detail beschrieben hergestellt.

Besonders bevorzugt sind im Rahmen der vorliegenden Erfindung solche der oben genannten Arzneimittelkombinationen, die ausgewählt sind aus
**2.1** und **1.1; 2.1** und **1.2; 2.1** und **1.3, 2.1** und **1.4; 2.1** und **1.5; 2.1** und **1.6; 2.1** und **1.7; 2.1** und **1.8, 2.1** und **1.9; 2.1** und **1.10; 2.1** und **1.11; 2.1** und **1.12; 2.1** und **1.13, 2.1** und **1.14; 2.1** und **1.15; 2.1** und **1.16; 2.1** und **1.17; 2.1** und **1.18, 2.1** und **1.19; 2.1** und **1.20; 2.1** und **1.21; 2.1** und **1.22; 2.1** und **1.23, 2.1** und **1.24; 2.1** und **1.25; 2.1** und **1.26; 2.1** und **1.27; 2.1** und **1.28, 2.1** und **1.29; 2.1** und **1.30; 2.1** und **1.31; 2.1** und **1.32; 2.1** und **1.33, 2.1** und **1.34; 2.1** und **1.35; 2.1** und **1.36; 2.1** und **1.37, 2.1** und **1.38; 2.1** und **1.39; 2.1** und **1.40; 2.1** und **1.41; 2.1** und **1.42, 2.1** und **1.43**; **2.1** und **1.44; 2.1** und **1.45;**
**2.2** und **1.1; 2.2** und **1.2; 2.2** und **1.3**, **2.2** und **1.4; 2.2** und **1.5; 2.2** und **1.6; 2.2** und **1.7; 2.2** und **1.8, 2.2** und **1.9; 2.2** und **1.10; 2.2** und **1.11; 2.2** und **1.12; 2.2** und **1.13, 2.2** und **1.14; 2.2** und **1.15; 2.2** und **1.16; 2.2** und **1.17; 2.2** und **1.18, 2.2** und **1.19; 2.2** und **1.20; 2.2** und **1.21; 2.2** und **1.22; 2.2** und **1.23, 2.2** und **1.24; 2.2** und **1.25; 2.2** und **1.26; 2.2** und **1.27; 2.2** und **1.28, 2.2** und **1.29; 2.2** und **1.30; 2.2** und **1.31; 2.2** und **1.32; 2.2** und **1.33, 2.2** und **1.34; 2.2** und **1.35; 2.2** und **1.36; 2.2** und **1.37, 2.2** und **1.38**; **2.2** und **1.39; 2.2** und **1.40; 2.2** und **1.41; 2.2** und **1.42, 2.2** und **1.43; 2.2** und **1.44; 2.2** und **1.45;**
**2.3** und **1.1; 2.1** und **1.3; 2.1** und **1.3, 2.3** und **1.4; 2.3** und **1.5; 2.3** und **1.6; 2.3** und **1.7; 2.3** und **1.8, 2.3** und **1.9; 2.3** und **1.10**; **2.3** und **1.11; 2.3** und **1.12; 2.3** und **1.13, 2.3** und **1.14; 2.3** und **1.15; 2.3** und **1.16; 2.3** und **1.17; 2.3** und **1.18, 2.3** und **1.19; 2.3** und **1.20; 2.3** und **1.21; 2.3** und **1.22; 2.3** und **1.23**, **2.3** und **1.24; 2.3** und **1.25; 2.3** und **1.26; 2.3** und **1.27**; **2.3** und **1.28**, **2.3** und **1.29; 2.3** und **1.30; 2.3** und **1.31; 2.3** und **1.32; 2.3** und **1.33, 2.3** und **1.34; 2.3** und **1.35; 2.3** und **1.36; 2.3** und **1.37, 2.3** und **1.38; 2.3** und **1.39; 2.3** und **1.40; 2.3** und **1.41; 2.3** und **1.42, 2.3** und **1.43; 2.3** und **1.44; 2.3** und **1.45;**
**2.4** und **1.1; 2.4** und **1.2; 2.4** und **1.3, 2.4** und **1.4; 2.4** und **1.5; 2.4** und **1.6; 2.4** und **1.7; 2.4** und **1.8, 2.4** und **1.9; 2.4** und **1.10; 2.4** und **1.11; 2.4** und **1.12; 2.4** und **1.13, 2.4** und **1.14; 2.4** und **1.15; 2.4** und **1.16; 2.4** und **1.17; 2.4** und **1.18, 2.4** und **1.19; 2.4** und **1.20; 2.4** und **1.21; 2.4** und **1.22; 2.4** und **1.23, 2.4** und **1.24; 2.4** und **1.25; 2.4** und **1.26; 2.4** und **1.27; 2.4** und **1.28, 2.4** und **1.29; 2.4** und **1.30; 2.4** und **1.31; 2.4** und **1.32; 2.4** und **1.33, 2.4** und **1.34; 2.4** und **1.35; 2.4** und **1.36; 2.4** und **1.37, 2.4** und **1.38; 2.4** und **1.39; 2.4** und **1.40; 2.4** und **1.41; 2.4** und **1.42, 2.4** und **1.43; 2.4** und **1.44; 2.4** und **1.45;**
**2.5** und **1.1; 2.5** und **1.2; 2.5** und **1.3, 2.5** und **1.4; 2.5** und **1.5; 2.5** und **1.6; 2.5** und **1.7; 2.5** und **1.8, 2.5** und **1.9; 2.5** und **1.10; 2.5** und **1.11; 2.5** und **1.12; 2.5** und **1.13, 2.5** und **1.14; 2.5** und **1.15; 2.5** und **1.16; 2.5** und **1.17; 2.5** und **1.18, 2.5** und **1.19; 2.5** und **1.20; 2.5** und **1.21; 2.5** und **1.22; 2.5** und **1.23, 2.5** und **1.24; 2.5** und **1.25; 2.5** und **1.26; 2.5** und **1.27; 2.5** und **1.28, 2.5** und **1.29; 2.5** und **1.30; 2.5** und **1.31; 2.5** und **1.32; 2.5** und **1.33, 2.5** und **1.34; 2.5** und **1.35; 2.5** und **1.36; 2.5** und **1.37, 2.5** und **1.38; 2.5** und **1.39; 2.5** und **1.40; 2.5** und **1.41; 2.5** und **1.42, 2.5** und **1.43; 2.5** und **1.44; 2.5** und **1.45;**
**2.6** und **1.1; 2.6** und **1.2; 2.6** und **1.3, 2.6** und **1.4; 2.6** und **1.5; 2.6** und **1.6; 2.6** und **1.7; 2.6** und **1.8, 2.6** und **1.9; 2.6** und **1.10; 2.6** und **1.11; 2.6** und **1.12; 2.6** und **1.13, 2.6** und **1.14; 2.6** und **1.15; 2.6** und **1.16; 2.6** und **1.17; 2.6** und **1.18, 2.6** und **1.19; 2.6** und **1.20**; **2.6** und **1.21; 2.6** und **1.22; 2.6** und **1.23, 2.6** und **1.24; 2.6** und **1.25; 2.6** und **1.26; 2.6** und **1.27; 2.6** und **1.28, 2.6** und **1.29; 2.6** und **1.30; 2.6** und **1.31; 2.6** und **1.32; 2.6** und **1.33, 2.6** und **1.34; 2.6** und **1.35; 2.6** und **1.36**; **2.6** und **1.37**, **2.6** und **1.38**; **2.6** und **1.39; 2.6** und **1.40; 2.6** und **1.41; 2.6** und **1.42, 2.6** und **1.43; 2.6** und **1.44; 2.6** und **1.45;**
**2.7** und **1.1; 2.7** und **1.2; 2.7** und **1.3, 2.7** und **1.4; 2.7** und **1.5; 2.7** und **1.6; 2.7** und **1.7; 2.7** und **1.8, 2.7** und **1.9; 2.7** und **1.10; 2.7** und **1.11; 2.7** und **1.12; 2.7** und **1.13, 2.7** und **1.14; 2.7** und **1.15; 2.7** und **1.16; 2.7** und **1.17; 2.7** und **1.18, 2.7** und **1.19; 2.7** und **1.20; 2.7** und **1.21; 2.7** und **1.22; 2.7** und **1.23, 2.7** und **1.24; 2.7** und **1.25; 2.7** und **1.26; 2.7** und **1.27; 2.7** und **1.28, 2.7** und **1.29; 2.7** und **1.30; 2.7** und **1.31; 2.7** und **1.32; 2.7** und **1.33, 2.7** und **1.34; 2.7** und **1.35; 2.7** und **1.36; 2.7** und **1.37**, **2.7** und **1.38**; **2.7** und **1.39**; **2.7** und **1.40; 2.7** und **1.41; 2.7** und **1.42**, **2.7** und **1.43**; **2.7** und **1.44**; **2.7** und **1.45;**
**2.8** und **1.1; 2.8** und **1.2; 2.8** und **1.3, 2.8** und **1.4; 2.8** und **1.5**; **2.8** und **1.6**; **2.8** und **1.7**; **2.8** und **1.8, 2.8** und **1.9; 2.8** und **1.10; 2.8** und **1.11; 2.8** und **1.12;** 2.8 und **1.13, 2.8** und **1.14; 2.8** und **1.15; 2.8** und **1.16; 2.8** und **1.17; 2.8** und **1.18, 2.8** und **1.19; 2.8** und **1.20; 2.8** und **1.21; 2.8** und **1.22; 2.8** und **1.23, 2.8** und **1.24**; **2.8** und **1.25; 2.8** und **1.26**; **2.8** und **1.27; 2.8** und **1.28, 2.8** und **1.29; 2.8** und **1.30; 2.8** und **1.31; 2.8** und **1.32; 2.8** und **1.33, 2.8** und **1.34; 2.8** und **1.35; 2.8** und **1.36; 2.8** und **1.37, 2.8** und **1.38; 2.8** und **1.39; 2.8** und **1.40; 2.8** und **1.41; 2.8** und **1.42, 2.8** und **1.43**; **2.8** und **1.44; 2.8** und **1.45.**

Weiterhin besonders bevorzugt ist eine solche der obigen Arzneimittelkombinationen, bei der der PDE4-Inhibitor (**1**) in einer Tagesdosis von 0,01 mg bis 50 mg, vorzugsweise von 0,1 mg bis 10 mg umfasst ist.

Ein weiterer besonders bevorzugter Gegenstand der Erfindung betrifft eine der oben genannten Arzneimittelkombinationen, bei der der EP4-Rezeptor-Antagonist (**2**) in einer Tagesdosis von 0,001 bis 100 mg/kg Körpergewicht, vorzugsweise von 0,01 bis 50 mg/kg Körpergewicht, stärker bevorzugt von 0,1 bis 10 mg/kg Körpergewicht eingesetzt wird.

Weiterhin besonders bevorzugt ist eine solche der obigen Arzneimittelkombinationen, bei der der EP4-Rezeptor-Antagonist (**2**) und der PDE4-Inhibitor (**1**) in einem Gewichtsverhältnis von 1:1 bis 200:1, vorzugsweise in einem Gewichtsverhältnis von 10:1 bis 150:1, besonders bevorzugt in einem Gewichtsverhältnis von 30:1 bis 100:1 eingesetzt werden.

Ein weiterer Gegenstand der vorliegenden Erfindung ist die Verwendung eines EP4-Rezeptor-Antagonisten (**2**) zur Reduzierung der Nebenwirkungen eines oder mehrerer PDE4-Inhibitoren bei der Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

Die vorliegende Erfindung betrifft weiterhin die Verwendung einer Kombination enthaltend einen oder mehrere PDE4-Inhibitoren (**1**) und wenigstens einen EP4-Rezeptor-Antagonisten (**2**) zur Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

Ein weiterer bevorzugter Gegenstand der vorliegenden Erfindung ist eine der oben genannten Verwendungen, wobei der eine oder die mehreren PDE4-Inhibitoren eine Verbindung der allgemeinen Formel **1** ist/sind, 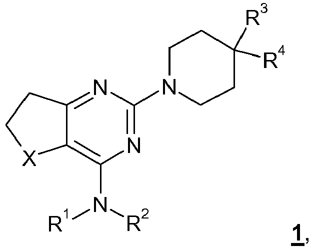 worin X, R¹, R², R³ und R⁴ wie vorstehend definiert sind.

Die vorliegende Erfindung betrifft weiterhin die oben genannten Verwendungen eines EP4-Rezeptor-Antagonisten (**2**) bzw. einer Kombination enthaltend einen oder mehrere PDE4-Inhibitoren (**1**) und wenigstens einen EP4-Rezeptor-Antagonisten (**2**) zur Behandlung einer Erkrankung ausgewählt aus COPD, chronischer Sinusitis, Asthma, Morbus Crohn und Colitis ulcerosa.

In einer Variante der vorstehend genannten Verwendungen wird der PDE4-Inhibitor (**1**) und der wenigstens eine EP4-Rezeptor-Antagonist (**2**) in einer einzigen gemeinsamen Formulierung gleichzeitig verabreicht.

In einer anderen Variante der vorstehend genannten Verwendungen kann der PDE4-Inhibitor (**1**) und der wenigstens eine EP4-Rezeptor-Antagonist (**2**) jedoch auch in zwei separaten Formulierungen innerhalb eines zeitlichen Abstandes von 0 bis 6 Stunden voneinander versetzt verabreicht werden.

Bei dieser getrennten Applikation in zwei separaten Formulierungen kann die Formulierung enthaltend den PDE4-Inhibitor - insbesondere den PDE4-Inhibitor der Formel **1** - eine orale oder inhalative Formulierung sein, ist aber vorzugsweise eine orale Formulierung, und die Formulierung enthaltend den wenigstens einen EP4-Rezeptor-Antagonisten (**2**) ist vorzugsweise eine orale Formulierung.

Weiterhin wird bei der Verwendung der Kombination in getrennten Formulierungen zur Herstellung einer Arzneimittelkombination zur Behandlung der oben genannten Erkrankungen die Formulierung enthaltend den PDE4-Inhibitor - insbesondere des PDE4-Inhibitors der Formel **1** - vorzugsweise einmal täglich und die Formulierung enthaltend den wenigstens einen EP4-Rezeptor-Antagonisten (**2**) vorzugsweise entweder einmal oder zweimal täglich verabreicht.

Insbesondere sind bei den oben genannten Verwendungen die PDE4-Inhibitoren (**1**) der Formel **1** ausgewählt aus:
1.1. (R)-2-{2-[4-(4-Chlorphenyl)-piperidin-1-yl]-5-oxo-6,7-dihydro-5*H*-5λ⁴-thieno[3,2-*d*]pyrimidin -4-ylamino}-3-methylbutan-1-ol
1.2. (1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-methanol
1.3. (R)-2-{2-[4-(4-Chlorphenyl)-piperidin-1-yl]-5-oxo-6,7-dihydro-5*H*-5λ⁴-thieno[3,2-*d*]pyrimidin-4-ylamino}-pentan-1-ol
1.4. (R)-1-{2-[4-(4-Chlorphenyl)-piperidin-1-yl]-5-oxo-6,7-dihydro-5*H*-5λ⁴-thieno[3,2-*d*]pyrimidin-4-ylamino}-1-(4-fluorphenyl)-2-methylpropan-2-ol
1.5. (S)-5-{2-[4-(4-Chlorphenyl)-piperidin-1-yl]-5-oxo-6,7-dihydro-5*H*-5λ⁴-thieno[3,2-*d*]pyrimidin-4-ylamino}-1-methylpiperidin-2-on
1.6. {2-[4-(4-Chlorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.7. 1-(4-(1-Hydroxymethylcyclopropylamino)-5-oxo-6,7-dihydro-5*H*-5λ⁴-thieno[3,2-*d*]pyrimidin-2-yl)-3'-methyl-1'*H*-spiro[piperidin-4,4'-chinazolin]-2'(3'*H*)-on
1.8. {1-[2-(4-Benzo[*d*]isoxazol-3-yl-piperidin-1-yl)-5-oxo-6,7-dihydro-5*H*-5λ⁴-thieno[3,2-*d*]pyrimidin-4-ylamino]-cyclopropyl}-methanol
1.9. (1-{2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-ylamino}-cyclopropyl)-methanol
1.10. 1-[4-((S)-1-Methyl-6-oxopiperidin-3-ylamino)-5-oxo-6,7-dihydro-5*H*-5λ⁴-thieno[3,2-*d*]pyrimidin-2-yl]-4-phenylpiperidin-4-carbonitril
1.11. 3'-methyl-1-(4-(tetrahydro-2*H*-pyran-4-ylamino)-5-oxo-6,7-dihydro-5*H*-5λ⁴-thieno[3,2-*d*]pyrimidin-2-yl)-1'*H*-spiro[piperidin-4,4'-chinazolin]-2'(3'*H*)-on
1.12. (3-Fluorphenyl)-[5-oxo-2-(3,4,5,6-tetrahydro-2*H*-[4,4']bipyridinyl-1-yl)-6,7-dihydro-5*H-*5λ⁴-thieno[3,2-*d*]pyrimidin-4-yl]-amin
1.13. {2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-yl}-(3-fluorphenyl)-amin
1.14. (1-{2-[4-(2,4-Difluorphenyl)-piperidin-1-yl]-5-oxo-6,7-dihydro-5*H*-5λ⁴-thieno[3,2-*d*]pyrimidin-4-ylamino}-cyclopropyl)-methanol
1.15. {2-[4-(2,4-Difluorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.16. (S)-5-[2-(4-Benzoxazol-2-yl-piperidin-1-yl)-5-oxo-6,7-dihydro-5*H*-5λ⁴-thieno[3,2-*d*]pyrimidin-4-ylamino]-1-methylpiperidin-2-on
1.17. (1-{2-[4-(6-Fluorbenzo[*d*]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.18. (1-{2-[4-(5-Fluorbenzo[*d*]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.19. {2-[4-(5-Furan-2-yl-2*H*-pyrazol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.20. (3-Fluorphenyl)-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-yl}-amin
1.21. (R)-3-Methyl-2-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-ylamino}-butan-1-ol
1.22. (S)-5-{2-[4-(4-Fluorphenoxy)-piperidin-1-yl]-5-oxo-6,7-dihydro-5*H*-5λ⁴-thieno[3,2-*d*]pyrimidin-4-ylamino}-1-methylpiperidin-2-on
1.23. (2-{4-[4-(4,5-Dihydrooxazol-2-yl)-phenoxy]-piperidin-1-yl}-5-oxo-6,7-dihydro-5*H*-5λ⁴-thieno[3,2-*d*]pyrimidin-4-yl)-(tetrahydropyran-4-yl)-amin
1.24. 4-{1-[5-Oxo-4-(tetrahydropyran-4-ylamino)-6,7-dihydro-5H-5λ⁴-thieno[3,2-*d*]pyrimidin-2-yl]-piperidin-4-yloxy}-benzoesäure
1.25. 2-(1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-propan-2-ol
1.26. {2-[4-(5-*tert*-Butyl-1-methyl-1*H*-indol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.27. 2-[4-(5-Furan-2-yl-1-methyl-1*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.28. (S)-5-(2-{4-[4-(4,5-Dihydrooxazol-2-yl)-phenoxy]-piperidin-1-yl}-5-oxo-6,7-dihydro-5*H*-5λ⁴-thieno[3,2-*d*]pyrimidin-4-ylamino)-1-methylpiperidin-2-on
1.29. {2-[4-(5-Furan-2-yl-2-methyl-2*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.30. {2-[4-(1-Methyl-1*H*-imidazo[4,5-*c*]pyridin-2-yl)-piperidin-1-yl]-5-oxo-6,7-dihydro-5*H-*5λ⁴-thieno[3,2-*d*]pyrimidin-4-yl}-(tetrahydropyran-4-yl)-amin
1.31. 2-Methoxy-*N*-{1-[5-oxo-4-(tetrahydropyran-4-ylamino)-6,7-dihydro-5*H*-5λ⁴-thieno[3,2-*d*]pyrimidin-2-yl]-4-phenylpiperidin-4-ylmethyl}-acetamid
1.32. N-Cyclopropyl-N-methyl-4-{1-[5-oxo-4-(tetrahydropyran-4-ylamino)-6,7-dihydro-5*H-*5λ⁴-thieno[3,2-*d*]pyrimidin-2-yl]-piperidin-4-yl}-benzamid
1.33. N-Cyclopropyl-N-methyl-4-{1-[5-oxo-4-(tetrahydropyran-4-ylamino)-6,7-dihydro-5H-5λ⁴-thieno[3,2-*d*]pyrimidin-2-yl]-piperidin-4-yloxy}-benzamid
1.34. {5-Oxo-2-[4-(pyridin-4-yloxy)-piperidin-1-yl]-6,7-dihydro-5*H*-5λ⁴-thieno[3,2-*d*]pyrimidin-4-yl}-(tetrahydropyran-4-yl)-amin
1.35. {2-[4-(4-Chlorphenoxy)-piperidin-1-yl]-5-oxo-6,7-dihydro-5*H*-5λ⁴-thieno[3,2-*d*]pyrimidin-4-yl}-(tetrahydropyran-4-yl)-amin
1.36. (S)-1-Methyl-5-{2-[4-(5-methyl-4-phenyloxazol-2-yl)-piperidin-1-yl]-5-oxo-6,7-dihydro-5*H*-5λ⁴-thieno[3,2-*d*]pyrimidin-4-ylamino}-piperidin-2-on
1.37. (1-{2-[4-(5-Methyl-4-phenyloxazol-2-yl)-piperidin-1-yl]-5-oxo-6,7-dihydro-5*H*-5λ⁴-thieno[3,2-*d*]pyrimidin-4-ylamino}-cyclopropyl)-methanol
1.38. (S)-5-{2-[4-(4,5-Diphenyloxazol-2-yl)-piperidin-1-yl]-5-oxo-6,7-dihydro-5H-5λ⁴-thieno[3,2-*d*]pyrimidin-4-ylamino}-1-methylpiperidin-2-on
1.39. {4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-yl}-methanol
1.40. [1-(2-{4-[5-(4-Chlorphenyl)-4-methyloxazol-2-yl]-piperidin-1-yl}-5-oxo-6,7-dihydro-5H-5λ⁴-thieno[3,2-*d*]pyrimidin-4-ylamino)-cyclopropyl]-methanol
1.41. 4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-ol
1.42. {2-[4-(4-Chlorphenyl)-4-methoxypiperidin-1-yl]-5-oxo-6,7-dihydro-5*H*-5λ⁴-thieno[3,2-*d*]pyrimidin-4-yl}-(tetrahydropyran-4-yl)-amin
1.43. 4-{1-[4-(1-Hydroxymethylcyclopropylamino)-5-oxo-6,7-dihydro-5*H*-5λ⁴-thieno[3,2-*d*]pyrimidin-2-yl]-piperidin-4-yloxy}-benzonitril
1.44. 5-Oxo-2-[4-(4,5,6,7-tetrahydrobenzoxazol-2-yl)-piperidin-1-yl]-6,7-dihydro-5H-5λ⁴-thieno[3,2-*d*]pyrimidin-4-yl}-(tetrahydropyran-4-yl)-amin
1.45. (S)-5-{2-[4-(4-Chlorphenyl)-piperidin-1-yl]-5,5-dioxo-6,7-dihydro-5H-5λ⁶-thieno[3,2-*d*]pyrimidin-4-ylamino}-1-methylpiperidin-2-on.

Bei diesen oben genannten Verwendungen sind die EP4-Rezeptor-Antagonisten (**2**) vorzugsweise ausgewählt aus der Gruppe bestehend aus:
[N-{[4-(5,9-Diethoxy-6-oxo-6,8-dihydro-7H-pyrrolo[3,4-g] chinolin-7yl)-3-methylbenzyl]sulfonyl}-2-(2-methoxyphenyl)acetamid] (**2.1**);
5-Butyl-2,4-dihydro-4-[[2'-[N-(3-methyl-2-thiophen-carbonyl)sulfamoyl]biphenyl-4-yl]methyl]-2-[(2-trifluoromethyl)phenyl]-1, 2, 4-triazol-3-on (**2.2**);
(4-{(1S)-1-[({5-chloro-2-[(4-fluorophenyl)oxy]phenyl}carbonyl)amino]ethyl}benzoesäure (2.3);
N-[({2-[4-(2-ethyl-4,6-dimethyl-1H-imidazo [4,5-c]pyridin-1-yl)phenyl]ethyl}amino)carbonyl]-4-methylbenzol sulfonamid (**2.4**);
4-[[4-(5-methoxy-2-pyridinyl)phenoxy]methyl]-5-methyl-N-[(2-methylphenyl)sulfonyl]-2-Furancarboxamid (**2.5**);
11alpha, 15alpha-Dihydroxy-16-(3-methoxymethylphenyl)-9-oxo-17,18,19, 20-tetranor-5-thia-13(E) Prostansäuremethylester (**2.6**);
4-cyano-2-[[2-(4-fluoro-1-naphthalenyl)-1-oxopropyl]amino]-Benzolbutansäure (**2.7**) und N-{2-[4-(4,9-diethoxy-1-oxo-1,3-dihydro-2H-benzo[f]isoindol-2-yl)phenyl]acetyl}benzene sulphonamide (**2.8**).

In einer besonders bevorzugten Ausführungsform der Erfindung wird bei diesen oben genannten Verwendungen der PDE4-Inhibitor (**1**) der allgemeinen Formel **1** in einer Tagesdosis von 0,01 mg bis 50 mg eingesetzt wird.

In einer weiteren besonders bevorzugten Ausführungsform der Erfindung wird bei diesen oben genannten Verwendungen der EP4-Rezeptor-Antagonist (**2**) in einer Tagesdosis von 0,001 bis 100 mg/kg Körpergewicht eingesetzt, vorzugsweise in einer Tagesdosis von 0,01 bis 50 mg/kg Körpergewicht, stärker bevorzugt in einer Tagesdosis von 0,1 bis 10 mg/kg Körpergewicht eingesetzt.

In einer weiteren besonders bevorzugten Ausführungsform der Erfindung werden bei diesen oben genannten Verwendungen der EP4-Rezeptor-Antagonist (**2**) und der PDE4-Inhibitor (**1**) in einem Gewichtsverhältnis von 1:1 bis 200:1, vorzugsweise in einem Gewichtsverhältnis von 10:1 bis 150:1, besonders bevorzugt in einem Gewichtsverhältnis von 30:1 bis 100:1 eingesetzt.

Insbesondere betrifft die Erfindung die oben genannten Verwendungen, bei denen die oder zumindest eine oder mehrere der PDE4-Inhibitor-vermittelten Nebenwirkungen in erheblichem Ausmaß reduziert oder völlig vermieden wird im Vergleich zu einer alleinigen Applikation des in der Arzneimittelkombination verwendeten PDE4-Inhibitors.

Insbesondere betrifft die Erfindung weiterhin die Verwendung von EP4-Rezeptor-Antagonisten, vorzugsweise wie vorstehend definiert und bevorzugt definiert, zur Reduktion oder Vermeidung einer oder mehrerer PDE4-Inhibitor-vermittelter Nebenwirkungen.

Diese PDE4-Inhibitor-vermittelten Nebenwirkungen sind vorzugsweise ausgewählt aus Körpergewichtsverlust, Milzgewichtsverlust Leukozytose, Neutrophilie, Übelkeit, Erbrechen, Diarrhoe und mesenterer Vaskulitis Diese PDE4-Inhibitor-vermittelten Nebenwirkungen sind stärker bevorzugt ausgewählt aus Körpergewichtsverlust, Milzgewichtsverlust, Leukozytose, Neutrophilie und mesenterer Vaskulitis.

### SYNTHESEVORSCHRIFTEN

Die Verbindungen der allgemeinen Formel (I) können nach folgendem allgemeinen Syntheseschema hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken.

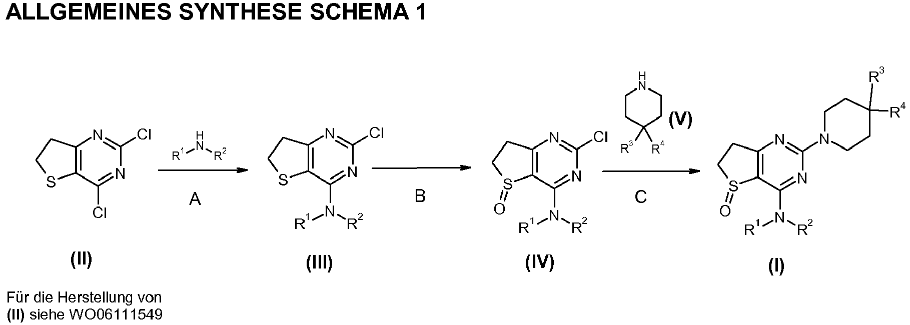

### 1. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN -4-YLAMINO}-3-METHYLBUTAN-1-OL (BEISPIEL 1.1)

### 1.1 (R)-2-(2-Chlor-6,7-dihydrothieno[3,2-d]pyrimidin-4-ylamino)-3-methylbutan-1-ol (III-1):

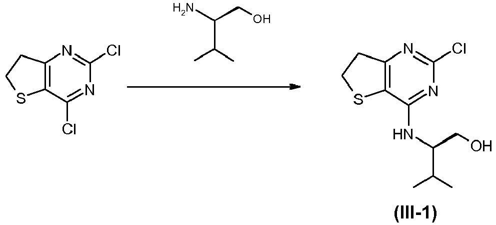

7,2 g 2,4-Dichlor-6,7-dihydrothieno[3,2-*d*]pyrimidin **(II)** werden in 36 ml Dioxan vorgelegt, erst werden 18 ml Diisopropylethylamin, dann 6,1 g (R)-(-)-2-Amino-3-methyl-1-butanol zugegeben. Das Reaktionsgemisch wird bei 100°C, bis keine weitere Umsetzung erfolgt, erhitzt und nach Abkühlen eingedampft. Der Rückstand wird mit Petrolether/Essigester (9:1) im Ultraschallbad behandelt und der Feststoff abgesaugt und getrocknet. 8,3 g **(III-1)** werden als Feststoff erhalten. Analytische HPLC (Methode A): RT = 2,75 min

### 1.2 (R)-2-(2-Chlor-5-oxo-6,7-dihydro-5H-5λ⁴-thieno[3,2-d]pyrimidin-4-ylamino)-3-methylbutan-1-ol (IV-1):

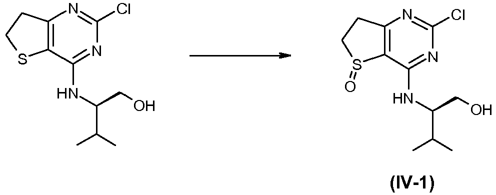

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschließend wird eine Suspension von 4,1 g **(III-1)** in 107 ml Dichlormethan zugegeben. Das Reaktionsgemisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 86/14) gereinigt. 2,45 g **(IV-1)** werden als Feststoff erhalten. Analytische HPLC (Methode A): RT = 2,37 min

### 1.3 (R)-2-{2-[4-(4-Chlorphenyl)-piperidin-1-yl]-5-oxo-6,7-dihydro-5H-5λ⁴-thieno[3,2-d]pyrimidin -4-ylamino}-3-methylbutan-1-ol (Beispiel 1.1)

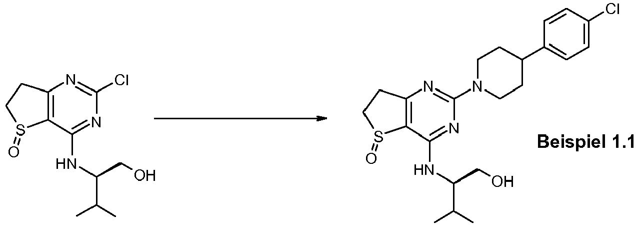

0,2 g **(IV-1)** wird in 3 ml Dioxan und 360 µl Diisopropylethylamin vorgelegt, mit 0,16 g 4-(4-Chlorphenyl)-piperidin versetzt und bei 120°C, bis keine weitere Umsetzung erfolgt, in der Mikrowelle erhitzt. Das Reaktionsgemisch wird mit Wasser versetzt, mit Dichlormethan extrahiert und das Produkt chromatographisch (Kieselgel, Dichlormethan/Methanol 100/0 bis 92/8) gereinigt. 0,33 g **Beispiel 1.1** werden als Feststoff erhalten. Analytische HPLC-MS (Methode A): RT = 1,24 min.

### 2. SYNTHESE VON (1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.2)

### 2.1 (1-Hydroxymethylcyclopropyl)-carbamidsäure tert-butylester:

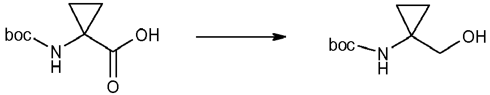

1 g 1-(BOC-amino)-cyclopropancarbonsäure wird in 20 ml Dimethoxyethan gelöst und auf - 70°C abgekühlt. Dann werden 0,65 ml N-Methylmorpholin zugegeben und 0,71 ml Isobutylchloroformiat in 5 ml Dimethoxyethan zugetropft. Das Reaktionsgemisch wird auf - 5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 2.2 1-Aminocyclopropanmethanol:

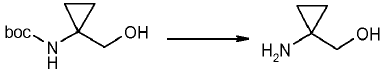

1,04 g (1-Hydroxymethylcyclopropyl)-carbamidsäure *tert*-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1H, t); 0,91 (2H, t); 0,71 (2H, t).

### 2.3 [1-(2-Chlor-6,7-dihydrothieno[3,2-d]pyrimidin-4-ylamino)-cyclopropyl]-methanol (III-2):

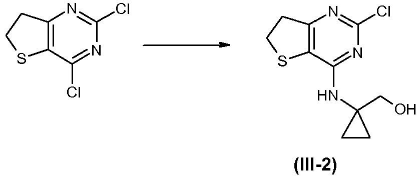

1,4 g **(II)** werden in 10 ml Dioxan vorgelegt, anschliessend werden 3,6 ml Diisopropylethylamin und dann 1 g 1-Aminocyclopropanmethanol (siehe 2.2) zugegeben. Das Reaktionsgemisch wird bei 160°C, bis keine weitere Umsetzung erfolgt, erhitzt und nach Abkühlen eingedampft. Der Rückstand wird mit Cyclohexan/Essigester (4:1) im Ultraschallbad behandelt, der Feststoff abgesaugt und getrocknet. 1,24 g **(III-2)** werden als Feststoff erhalten. Analytische HPLC-MS (Methode A): RT = 1,01 min.

### 2.4 [1-(2-Chlor-5-oxo-6,7-dihydro-5H-5λ⁴-thieno[3,2-d]pyrimidin-4-ylamino)-cyclopropyl]-methanol (IV-2):

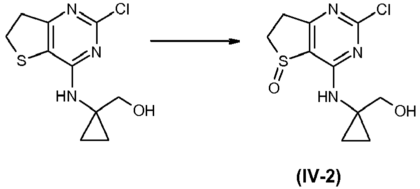

0,28 g S-(-)-1,1'-Bi-2-naphtol werden in 20 ml Chloroform unter Argon vorgelegt, dann 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g **(III-2)** in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Die Wasserphase wird mit Dichlormethan gewaschen und gefriergetrocknet. 1 g **(IV-2)** wird als Feststoff erhalten. Analytische HPLC-MS (Methode A) RT = 0,85 min

### 2.5 (1-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-methanol (Beispiel 1.2)

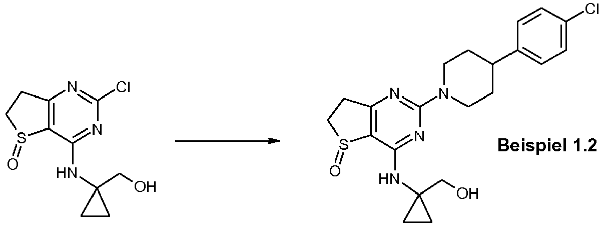

Ausgehend von 0,17 g **(IV-2)** und 0,15 g 4-(4-Chlorphenyl)-piperidin werden 0,14 g **Beispiel 1.2** analog zu Beispiel 1.1 (siehe 1.3) hergestellt und aufgereinigt. Analytische HPLC-MS (Methode B): RT = 1,32 min.

### 3. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-PENTAN-1-OL (BEISPIEL 1.3)

### 3.1 (R)-2-(2-Chlor-6,7-dihydrothieno[3,2-d]pyrimidin-4-ylamino)-pentan-1-ol (III-3):

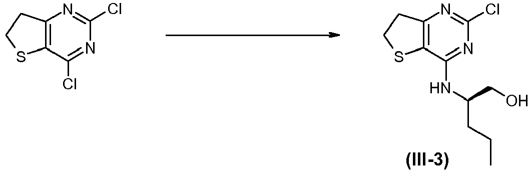

1,4 g 2,4-Dichlor-6,7-dihydrothieno[3,2-*d*]pyrimidin **(II)** werden in 9 ml Dioxan vorgelegt, erst werden 3,5 ml Diisopropylethylamin, dann 0,9 g D-norvalinol zugegeben. Das Reaktionsgemisch wird bei 120°C, bis keine weitere Umsetzung erfolgt, in der Mikrowelle erhitzt und nach Abkühlen eingedampft. Der Rückstand wird mit Petrolether/Essigester 9:1 im Ultraschallbad behandelt, der Feststoff wird abgesaugt und getrocknet. 1,5 g **(III-3)** werden als Feststoff erhalten. ¹H NMR (400 MHz, DMSO): 4,67 (1H, t); 0,86 (3H, t).

### 3.2 (R)-2-(2-Chlor-5-oxo-6,7-dihydro-5H-5λ⁴-thieno[3,2-d]pyrimidin-4-ylamino)-pentan-1-ol (IV-3):

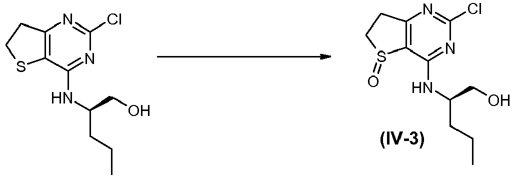

0,3 g S-(-)-1,1'-Bi-2-naphtol werden in 5 ml Chloroform unter Argon vorgelegt, dann 0,15 ml Titan(IV)-isopropylat und 0,19 ml Wasser zugegeben. Das Reaktionsgemisch wird 1 Stunde bei Raumtemperatur gerührt. Anschließend wird eine Suspension von 1,4 g **(III-3)** in 20 ml Dichlormethan zugegeben. Das Reaktionsgemisch wird auf -5°C abgekühlt und nach 30 Minuten werden 0,95 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird mit Dichlormethan extrahiert und chromatographisch (Essigester/Methanol 100/0 bis 80/20) gereinigt. 1,17 g **(IV-3)** werden als Feststoff erhalten. Analytische HPLC (Methode A): RT = 2,41 min

### 3.3 (R)-2-{2-[4-(4-Chlorphenyl)-piperidin-1-yl]-5-oxo-6,7-dihydro-5H-5λ⁴-thieno[3,2-d]pyrimidin-4-ylamino}-pentan-1-ol (Beispiel 1.3)

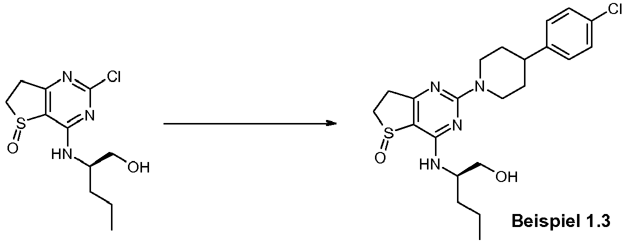

0,2 g **(IV-3)** werden in 4 ml Dioxan und 237 µl Diisopropylethylamin vorgelegt, mit 0,149 g 4-(4-Chlorphenyl)-piperidin versetzt und 30 min bei 130°C in der Mikrowelle erhitzt. Das Reaktionsgemisch wird mit Wasser versetzt und das Produkt mit Dichlormethan extrahiert. Der Rückstand wird mit Acetonitril im Ultraschallbad behandelt und der Feststoff abgesaugt. 0,104 g **Beispiel 1.3** werden als Feststoff erhalten. Analytische HPLC-MS (Methode A): RT = 1,29 min.

### 4. SYNTHESE VON (R)-1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-(4-FLUORPHENYL)-2-METHYLPROPAN-2-OL (BEISPIEL 1.4)

### 4.1 (R)-Amino-(4-fluorphenyl)-essigsäuremethylester:

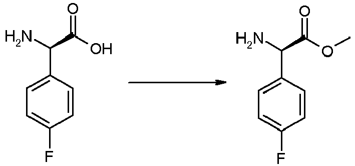

4 g (R)-4-fluorphenylglycin werden in 80 ml Methanol suspendiert. Unter Eisbadkühlung werden 3,28 ml Thionylchlorid langsam zugetropft, so dass die Temperatur zwischen 15°C und 20°C gehalten wird. Das Reaktionsgemisch wird 12 Stunden bei Raumtemperatur gerührt und anschließend zur Trockne eingedampft. 5,1 g des Produkts werden als Hydrochlorid erhalten. Analytische HPLC-MS (Methode A): RT = 0,8 min.

### 4.2 (R)-(4-Fluorphenyl)-(2,2,2-trifluoracetylamino)-essigsäuremethylester:

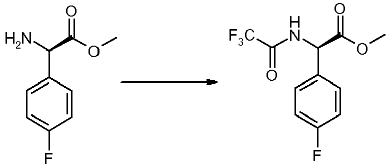

5,1 g (R)-Amino-(4-fluorphenyl)-essigsäuremethylester werden in 36,5 ml abs. Tetrahydrofuran vorgelegt, dann 3,9 ml Triethylamin zugegeben. Das Reaktionsgemisch wird auf -70°C abgekühlt. 3,9 ml Trifluoressiganhydrid werden dann langsam zugetropft, so dass die Temperatur nicht -60°C übersteigt. Das Reaktionsgemisch wird 12 Stunden bei Raumtemperatur gerührt und anschließend mit Wasser versetzt. Dann wird Kaliumhydrogencarbonat, bis keine Schaumbildung mehr zu beobachten ist, zugegeben und das Produkt mit Essigester extrahiert. 6,2 g des Produkts werden als Öl erhalten. Analytische HPLC-MS (Methode A): RT = 1,28 min.

### 4.3 2,2,2-Trifluor-N-[(R)-1-(4-fluorphenyl)-2-hydroxy-2-methylpropyl]-acetamid:

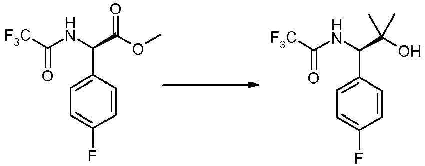

6,2 g (R)-(4-Fluorphenyl)-(2,2,2-trifluoracetylamino)-essigsäuremethylester werden in 195 ml abs. Tetrahydrofuran vorgelegt und das Reaktionsgemisch auf +3°C gekühlt. 37,2 ml einer Methylmagnesiumiodid Lösung (3 M) werden langsam zugetropft, so dass die Temperatur nicht +10°C übersteigt. Das Reaktionsgemisch wird 12 Stunden bei Raumtemperatur gerührt und anschließend in Eiswasser gerührt. Ammoniumchlorid wird, bis der Niederschlag gelöst ist, zugegeben und das Produkt mit Essigester extrahiert. 5,6 g des Produkts werden als Öl erhalten. Analytische HPLC-MS (Methode A): RT = 1,19 min

### 4.4 (R)-1-Amino-1-(4-fluorphenyl)-2-methylpropan-2-ol:

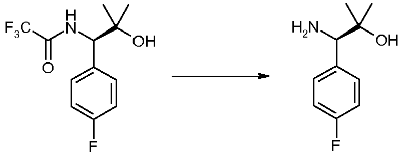

5,6 g 2,2,2-Trifluor-*N*-[(R)-1-(4-fluorphenyl)-2-hydroxy-2-methylpropyl]-acetamid und 2,27 g KOH werden in 60 ml Methanol suspendiert. Das Reaktionsgemisch wird 20 Stunden bei 60°C gerührt, anschließend mit Wasser versetzt und das Produkt mit Dichlormethan extrahiert. 3,2 g Produkt werden als Öl erhalten. Analytische HPLC-MS (Methode A): RT = 0,79 min.

### 4.5 (R)-1-(2-Chlor-6,7-dihydrothieno[3,2-d]pyrimidin-4-ylamino)-1-(4-fluorphenyl)-2-methylpropan-2-ol (III-4):

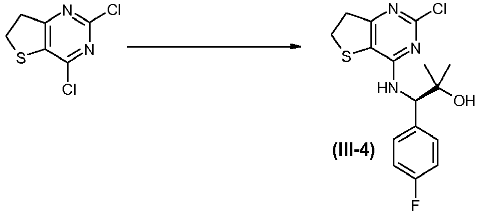

0,533 g **(II)**, 0,850 g (R)-1-Amino-1-(4-fluorphenyl)-2-methylpropan-2-ol und 1,3 ml Diisopropylethylamin werden in 9,8 ml Dioxan suspendiert. Das Reaktionsgemisch wird in der Mikrowelle 2 Stunden bei 80°C erhitzt und anschießend zur Trockne eingedampft. Der Rückstand wird mit Wasser versetzt. Der ausgefallene Niederschlag wird abgesaugt und chromatographisch (Kieselgel, Petrolether/Essigester 100/0 bis 60/40) gereinigt. 0,260 g **(III-4)** werden als Feststoff erhalten. Analytische HPLC-MS (Methode A): 1,39 min.

### 4.6 (R)-1-(2-Chlor-5-oxo-6,7-dihydro-5H-5λ⁴-thieno[3,2-d]pyrimidin-4-ylamino)-1-(4-fluorphenyl)-2-methylpropan-2-ol (IV-4):

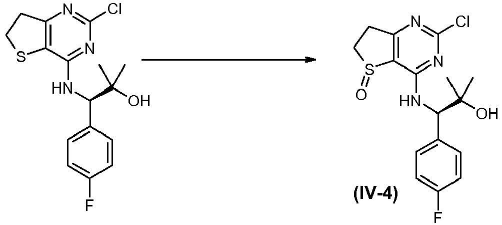

0,24 g S-(-)-1,1'-Bi-2-naphtol werden in 4 ml Chloroform unter Argon vorgelegt, dann 0,125 ml Titan(IV)-isopropylat und 0,15 ml Wasser zugegeben. Das Reaktionsgemisch wird 1 Stunde bei Raumtemperatur gerührt. Anschließend wird eine Suspension von 1,51 g **(III-4)** in 26 ml Chloroform zugegeben. Das Reaktionsgemisch wird auf -6°C abgekühlt und nach 30 Minuten werden 0,78 ml tert-Butylhydroperoxid 5-6 M in Decan zugetropft. Das Reaktionsgemisch wird bei -6 °C, bis keine weitere Umsetzung erfolgt, weitergerührt und mit NH₄OH basisch gestellt. Das Produkt wird mit Dichlormethan extrahiert und chromatographisch (Dichlormethan/Methanol 100/0 bis 95/5) gereinigt. 0,62 g **(IV-4)** werden als Feststoff erhalten. Analytische HPLC-MS (Methode A): RT = 1,19 min.

### 4.7 (R)-1-{2-[4-(4-Chlorphenyl)-piperidin-1-yl]-5-oxo-6,7-dihydro-5H-5λ⁴-thieno[3,2-d]pyrimidin-4-ylamino}-1-(4-fluorphenyl)-2-methylpropan-2-ol (Beispiel 1.4)

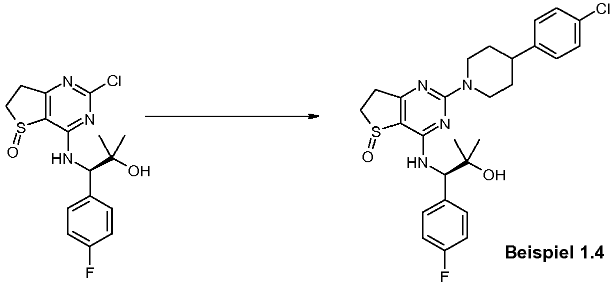

Ausgehend von 0,24 g **(IV-4)** und 0,15 g 4-(4-Chlorphenyl)-piperidin werden 0,19 g **Beispiel 1.4** analog zu Beispiel 1.1 (siehe 1.3) hergestellt. Das Produkt wird chromatographisch (Dichlormethan/Methanol 100/0 bis 96/4) gereinigt. Analytische HPLC-MS (Methode A): RT = 1,36 min.

### 5. SYNTHESE VON (S)-5-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.5)

### 5.1 (S)-5-Dibenzylaminopiperidin-2-on:

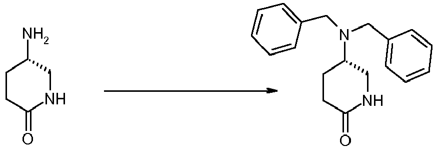

0,600 g 4-(S)-Amino-delta-valerolactam Hydrochlorid, 0,970 ml Benzylbromid und 1,5 g Natriumhydrogencarbonat werden in 30 ml Ethanol suspendiert. Das Reaktionsgemisch wird dann 8 Stunden bei 80°C gerührt und anschließend zur Trockne eingedampft. Der Rückstand wird in Wasser suspendiert und das Produkt mit Dichlormethan extrahiert und chromatographisch (Kieselgel, Dichlormethan/Methanol 100/0 bis 95/5) gereinigt. 0,500 g Produkt werden als Öl erhalten. Analytische HPLC-MS (Methode A): RT = 1,01 min.

### 5.2 (S)-5-Dibenzylamino-1-methylpiperidin-2-on:

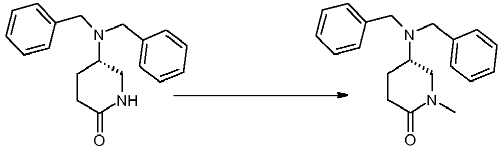

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g Produkt werden als Öl erhalten. Analytische HPLC-MS (Methode A): RT = 1,07 min.

### 5.3 (S)-5-Amino-1-methylpiperidin-2-on:

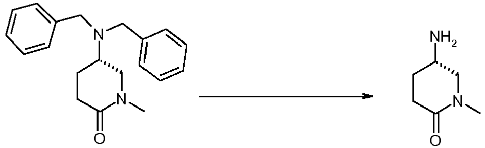

0,450 g (S)-5-Dibenzylamino-1-methylpiperidin-2-on werden in 25 ml Methanol suspendiert und mit 0,150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 5.4 (S)-5-(2-Chlor-6,7-dihydrothieno[3,2-d]pyrimidin-4-ylamino)-1-methylpiperidin-2-on (III-5):

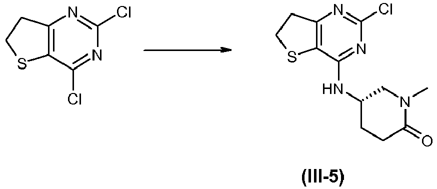

0,27 g **(II)** werden in 3 ml Dioxan vorgelegt, erst werden 0,45 ml Diisopropylethylamin, dann 0,25 g (S)-5-Amino-1-methylpiperidin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode A) gereinigt. 0,26 g **(III-5)** werden als Feststoff erhalten. Analytische HPLC-MS (Methode A): RT = 1,06 min.

### 5.5 (S)-5-(2-Chlor-5-oxo-6,7-dihydro-5H-5λ⁴-thieno[3,2-d]pyrimidin-4-ylamino)-1-methylpiperidin-2-on (IV-5):

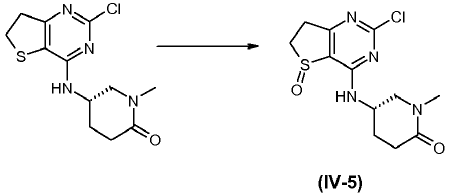

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g **(III-5)** in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird chromatographisch (Kieselgel, Essigester/Methanol 100/0 bis 60/40) gereinigt. 0,09 g **(IV-5)** werden als Feststoff erhalten. Analytische HPLC-MS (Methode A): RT = 0,83 min.

### 5.6 (S)-5-{2-[4-(4-Chlorphenyl)-piperidin-1-yl]-5-oxo-6,7-dihydro-5H-5λ⁴-thieno[3,2-d]pyrimidin-4-ylamino}-1-methylpiperidin-2-on (Beispiel 1.5)

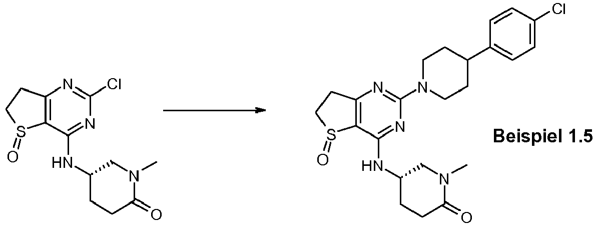

Ausgehend von 0,2 g **(IV-5)** und 0,18 g 4-(4-Chlorphenyl)-piperidin werden 0,17 g Beispiel **1.5** analog zu Beispiel 1.1 (siehe 1.3) hergestellt. Das Produkt wird chromatographisch (präparative HPLC, Methode A) gereinigt. Die Produktfraktionen werden mit Ammoniak basisch gestellt und gefriergetrocknet. Analytische HPLC-MS (Methode A): RT = 1,18 min

### 6. SYNTHESE VON {2-[4-(4-CHLORPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.6)

### 6.1 (2-Chlor-6,7-dihydrothieno[3,2-d]pyrimidin-4-yl)-(tetrahydropyran-4-yl)-amin (III-6):

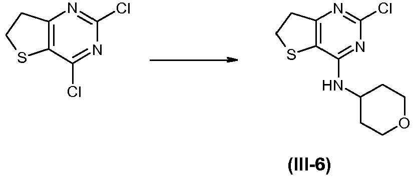

0,68 g **(II)** werden in 6 ml Dioxan vorgelegt, erst werden 1,72 ml Diisopropylethylamin, dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt, dann abgesaugt und getrocknet. 0,66 g **(III-6)** werden als Feststoff erhalten. Analytische HPLC-MS (Methode C): RT = 1,08 min.

### 6.2 (2-Chlor-5-oxo-6,7-dihydro-5H-5λ⁴-thieno[3,2-d]pyrimidin-4-yl)-(tetrahydropyran-4-yl)-amin (IV-6):

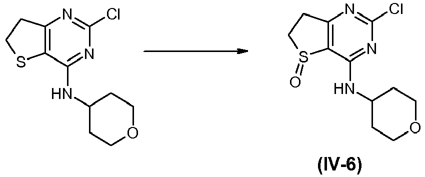

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g **(III-6)** in 25 ml Chloroform zugegeben. Das Reaktionsgemisch wird auf -10°C abgekühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g **(IV-6)** werden als Feststoff erhalten. Analytische HPLC-MS (Methode A): RT = 0,94 min.

### 6.3 {2-[4-(4-Chlorphenyl)-piperidin-1-yl]-5-oxo-6,7-dihydro-5H-5λ⁴-thieno[3,2-d]pyrimidin-4-yl}-(tetrahydropyran-4-yl)-amin (Beispiel 1.6)

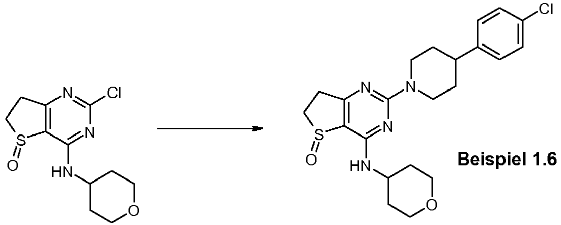

Ausgehend von 0,18 g **(IV-6)** und 0,17 g 4-(4-Chlorphenyl)-piperidin werden 0,23 g **Beispiel 1.6** analog zu Beispiel 1.1 (siehe 1.3) hergestellt. Das Produkt wird mit Wasser im Ultraschallbad behandelt und der Feststoff abgesaugt. Analytische HPLC-MS (Methode A): RT = 1,24 min

### 7. SYNTHESE VON 1-(4-(1-HYDROXYMETHYLCYCLOPROPYLAMINO)-5-OXO-6,7-DIHYDRO-5H-5λ⁴-THIENO[3,2-d]PYRIMIDIN-2-YL)-3'-METHYL-1'H-SPIRO[PIPERIDIN-4,4'-CHINAZOLIN]-2'(3'H)-ON (BEISPIEL 1.7)

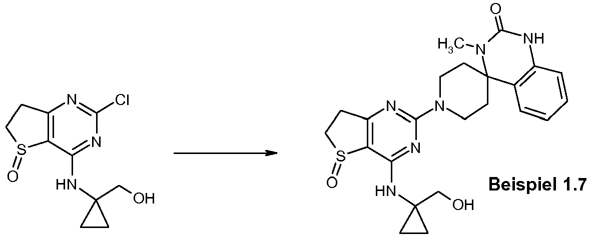

Ausgehend von **(IV-2)** (siehe 2.4) und 3'-methyl-1'*H*-spiro[piperidin-4,4'-chinazolin]-2'(3'*H*)-on (Chem. Pharm. Bull. 1988, 4659) (0,1 mmol) in 400µl NMP versetzt und 30 min bei 120°C in der Mikrowelle erhitzt. Anschliessend werden 600 µL DMF zugesetzt, die Reaktionslösung über präparative HPLC-MS (Methode A) aufgereinigt und die Produktfraktionen gefriergetrocknet. Analytische HPLC-MS (Methode C): RT = 1,52 min.

### 8. SYNTHESE VON {1-[2-(4-BENZO[d]ISOXAZOL-3-YL-PIPERIDIN-1-YL)-5-OXO-6,7-DIHYDRO-5H-5λ⁴-THIENO[3,2-d]PYRIMIDIN-4-YLAMINO]-CYCLOPROPYL}-METHANOL (BEISPIEL 1.8)

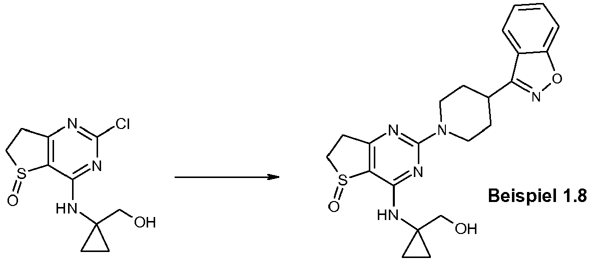

Ausgehend von **(IV-2)** (siehe 2.4) und 3-Piperidin-4-yl-benzo[*d*]isoxazol kann **Beispiel 1.8** analog zu Beispiel 1.7 (siehe 7.) hergestellt und aufgereinigt werden. Analytische HPLC-MS (Methode C): RT = 1,7 min.

### 9. SYNTHESE VON (1-{2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.9)

### 9.1 2-But-1-ynyl-4-fluorphenylamin

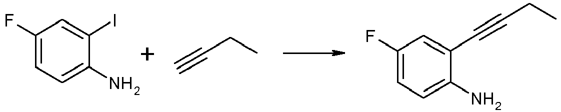

80 ml Tetrahydrofuran wird unter Argon vorgelegt. 5 g 4-Fluor-2-iodphenylamin, 0,74 g Dichlorbis(triphenylphosphin) palladium(II), 0,2 g Kupferiodid und 8,8 ml Triethylamin werden zugegeben. 4 g gasförmiges 1-Butin werden durch die Suspension geleitet. Das Reaktionsgemisch wird unter Argon 15 Stunden bei Raumtemperatur gerührt, dann über Celite filtriert und zur Trockne eingedampft. 3,4 g Produkt werden als Feststoff erhalten. ¹H NMR (400 MHz, DMSO): 2,45 (2H, q); 1,18 (3H, t).

### 9.2 2-Ethyl-5-fluor-1H-indol

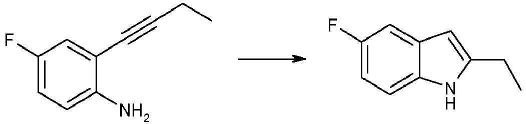

Unter Argon werden 4,9 g Kalium-*tert*-butylat in 25 ml N-Methyl-2-pyrrolidinon suspendiert und eine Suspension von 3,4 g 2-But-1-ynyl-4-fluorphenylamin in 25 ml N-Methyl-2-pyrrolidinon dazu getropft. Das Reaktionsgemisch wird 3 Stunden bei Raumtemperatur gerührt und mit Wasser versetzt. Das Produkt wird mit Diethylether extrahiert und chromatographisch (Kieselgel, Cyclohexan/Essigester 100/0 - 90/10) gereinigt. 2,83 g Produkt werden als Feststoff erhalten. ¹H NMR (400 MHz, DMSO): 2,72 (2H, q); 1,27 (3H, t).

### 9.3 2-Ethyl-5-fluor-3-(1,2,3,6-tetrahydropyridin-4-yl)-1H-indol

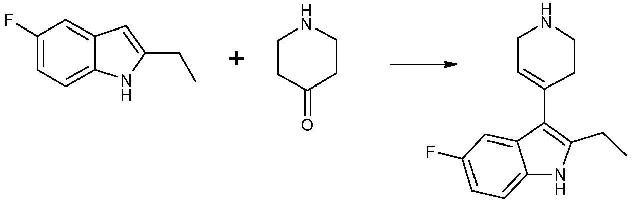

2,83 g 2-Ethyl-5-fluor-1*H*-indol werden in 50 ml Essigsäure suspendiert und auf 90°C erwärmt. Eine Suspension von 6,66 g 4-Piperidon in 15 ml Phosphorsäure 2N wird zugegeben. Das Reaktionsgemisch wird 3 Stunden bei 90°C gerührt, mit Natronlauge versetzt und das Produkt mit Essigester extrahiert. 2,85 g Produkt werden als Feststoff erhalten. ¹H NMR (400 MHz, DMSO): 5,63 (1H, s); 2,73 (2H, q); 1,23 (3H, t).

### 9.4 2-Ethyl-5-fluor-3-piperidin-4-yl-1H-indol (V-1)

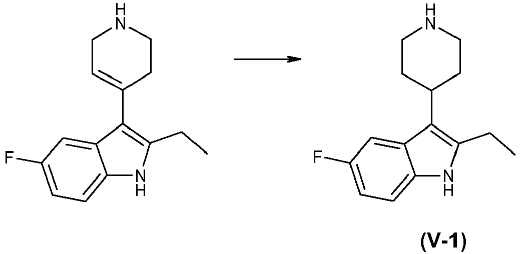

2,83 g 2-Ethyl-5-fluor-3-(1,2,3,6-tetrahydropyridin-4-yl)-1*H*-indol werden in 50 ml Methanol suspendiert und mit 0,3 g Pd/C 10% bei Normaldruck und Raumtemperatur hydriert. Der Katalysator wird abgesaugt und das Filtrat zur Trockne eingedampft. 2,3 g **(V-1)** werden als Feststoff erhalten. ¹H NMR (400 MHz, DMSO): 2,70 (2H, q); 1,19 (3H, t).

### 9.5 (1-{2-[4-(2-Ethyl-5-fluor-1H-indol-3-yl)-piperidin-1-yl]-5-oxo-6,7-dihydro-5H-5λ⁴-thieno[3,2-d]pyrimidin-4-ylamino}-cyclopropyl)-methanol (Beispiel 1.9)

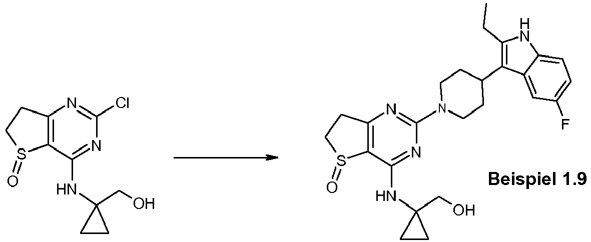

Ausgehend von **(IV-2)** (siehe 2.4) und **(V-1)** kann **Beispiel 1.9** analog zu Beispiel 1.7 (siehe 7.) hergestellt und aufgereinigt werden. Analytische HPLC-MS (Methode C): RT = 1,78 min.

### 10. SYNTHESE VON 1-[4-((S)-1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-CARBONITRIL (BEISPIEL 1.10)

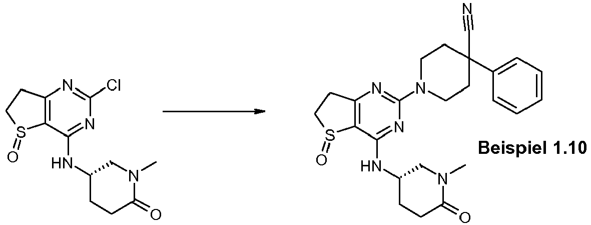

Ausgehend von **(IV-5)** (siehe 5.5) und 4-Phenylpiperidin-4-carbonitril
kann **Beispiel 1.10** analog zu Beispiel 1.7 (siehe 7) hergestellt und aufgereinigt werden. Analytische HPLC-MS (Methode C): RT = 1,71 min.

### 11. SYNTHESE VON 3'-METHYL-1-(4-(TETRAHYDRO-2H-PYRAN-4-YLAMINO)-5-OXO-6,7-DIHYDRO-5H-5λ⁴-THIENO[3,2-d]PYRIMIDIN-2-YL)-1'H-SPIRO[PIPERIDIN-4,4'-CHINAZOLIN]-2'(3'H)-ON (BEISPIEL 1.11)

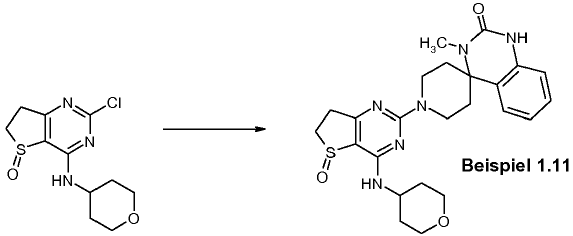

Ausgehend von **(IV-6)** (siehe 6.2) und 3'-methyl-1'*H*-spiro[piperidin-4,4'-chinazolin]-2'(3'*H*)-on (Chem. Pharm. Bull. 1988, 4659) kann **Beispiel 1.11** analog zu Beispiel 1.7 (siehe 7.) hergestellt und aufgereinigt werden. Analytische HPLC-MS (Methode C): RT = 1,56 min.

### 12. SYNTHESE VON (3-FLUORPHENYL)-[5-OXO-2-(3,4,5,6-TETRAHYDRO-2H-[4,4']BIPYRIDINYL-1-YL)-6,7-DIHYDRO-5H-5λ⁴-THIENO[3,2-d]PYRIMIDIN-4-YL]-AMIN (BEISPIEL 1.12)

### 12.1 (2-Chlor-6,7-dihydrothieno[3,2-d]pyrimidin-4-yl)-(3-fluorphenyl)-amin (III-7):

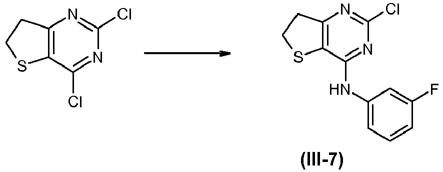

4 g **(II)** werden in 15 ml Dimethylformamid vorgelegt, anschliessend werden 4,5 ml Diisopropylethylamin und dann 2,5 ml 3-Fluorphenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g **(III-7)** werden als Feststoff erhalten. Analytische HPLC (Methode A): RT = 3,27 min

### 12.2 2-Chlor-5-oxo-6,7-dihydro-5H-5λ⁴-thieno[3,2-d]pyrimidin-4-yl)-(3-fluorphenyl)-amin (IV-7):

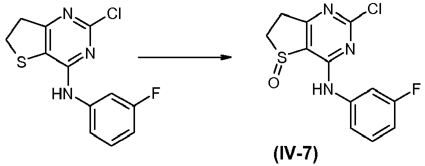

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g **(III-7)** in 25 ml Chloroform zugegeben. Das Reaktionsgemisch wird auf -2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g **(IV-7)** werden als Feststoff erhalten. Analytische HPLC-MS (Methode A): RT = 1,15 min.

### 12.3 (3-Fluorphenyl)-[5-oxo-2-(3,4,5,6-tetrahydro-2H-[4,4']bipyridinyl-1-yl)-6,7-dihydro-5H-5λ⁴-thieno[3,2-d]pyrimidin-4-yl]-amin (Beispiel 1.12)

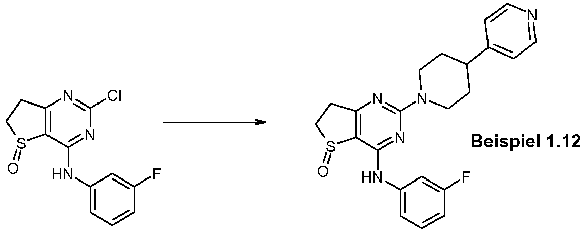

Ausgehend von **(IV-7)** (siehe 12.2) und 1,2,3,4,5,6-Hexahydro-[4,4']bipyridinyl kann **Beispiel 1.12** als Trifluoracetat analog zu Beispiel 1.7 (siehe 7.) hergestellt und aufgereinigt werden. Analytische HPLC-MS (Methode C): RT = 1,55 min.

### 13. SYNTHESE VON {2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 1.13)

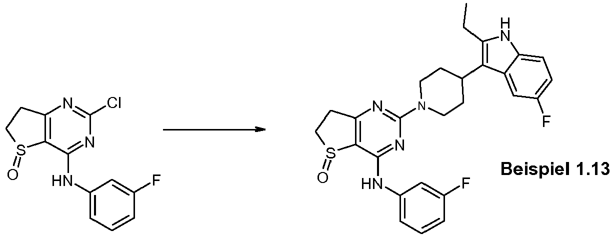

Ausgehend von **(IV-7)** (siehe 12.2) und **(V-1)** (siehe 9.4) kann **Beispiel 1.13** analog zu Beispiel 1.7 (siehe 7.) hergestellt und aufgereinigt werden. Analytische HPLC-MS (Methode C): RT = 2,12 min.

### 14. SYNTHESE VON (1-{2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.14)

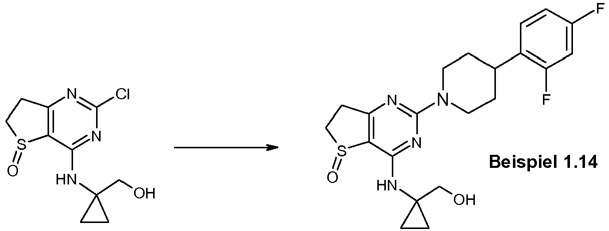

Ausgehend von **(IV-2)** (siehe 2.4) und 4-(2,4-Difluorphenyl)-piperidin kann **Beispiel 1.14** analog zu Beispiel 1.7 (siehe 7.) hergestellt werden. Das Produkt kann chromatographisch (präparative HPLC, Methode B) gereinigt werden. Analytische HPLC-MS (Methode D): RT = 1,18 min.

### 15. SYNTHESE VON {2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.15)

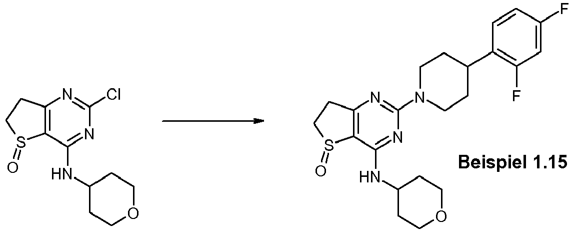

Ausgehend von **(IV-6)** (siehe 6.2) und 4-(2,4-Difluorphenyl)-piperidin kann **Beispiel 1.15** analog zu Beispiel 1.14 (siehe 14.) hergestellt und aufgereinigt werden. Analytische HPLC-MS (Methode D): RT = 1,23 min.

### 16. SYNTHESE VON (S)-5-[2-(4-BENZOXAZOL-2-YL-PIPERIDIN-1-YL)-5-OXO-6,7-DIHYDRO-5H-5λ⁴-THIENO[3,2-d]PYRIMIDIN-4-YLAMINO]-1-METHYLPIPERIDIN-2-ON (BEISPIEL 1.16)

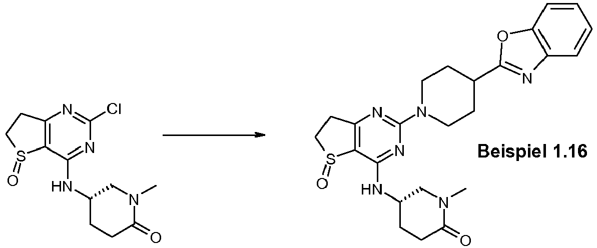

Ausgehend von **(IV-5)** (siehe 5.5) und 2-Piperidin-4-yl-benzoxazol kann **Beispiel 1.16** analog zu Beispiel 1.14 (siehe 14.) hergestellt und aufgereinigt werden. Analytische HPLC-MS (Methode B): RT = 1,18 min.

### 17. SYNTHESE VON (1-{2-[4-(6-FLUORBENZO[d]ISOXAZOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.17)

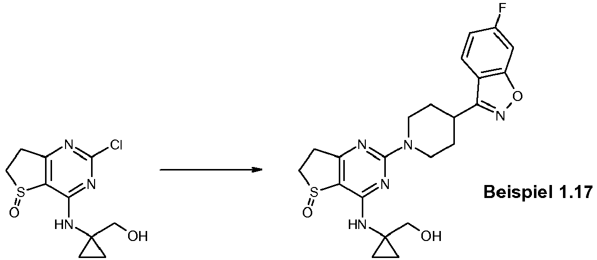

Ausgehend von **(IV-2)** (siehe 2.4) und 6-Fluor-3-piperidin-4-yl-benzo[*d*]isoxazol
kann **Beispiel 1.17** analog zu Beispiel 1.7 (siehe 7.) hergestellt und aufgereinigt werden. Analytische HPLC-MS (Methode C): RT = 1,76 min.

### 18. SYNTHESE VON (1-{2-[4-(5-FLUORBENZO[d]ISOXAZOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.18)

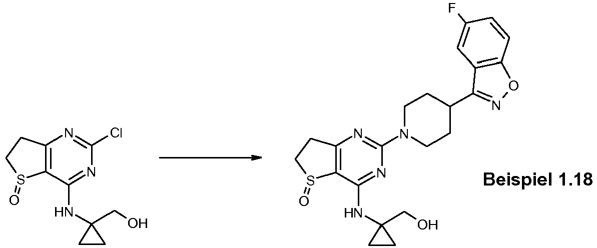

Ausgehend von **(IV-2)** (siehe 2.4) und 5-Fluor-3-piperidin-4-yl-benzo[*d*]isoxazol
kann **Beispiel 1.17** analog zu Beispiel 1.7 (siehe 7.) hergestellt und aufgereinigt werden. Analytische HPLC-MS (Methode C): RT = 1,74 min.

### 19. SYNTHESE VON {2-[4-(5-FURAN-2-YL-2H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.19)

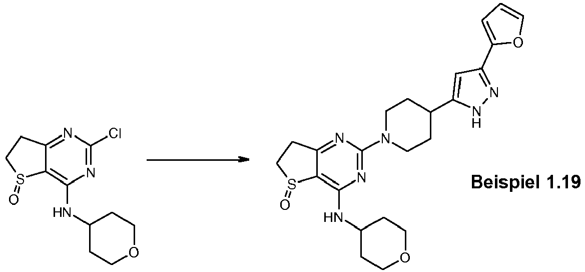

Ausgehend von **(IV-6)** (siehe 6.2) und 4-(5-Furan-2-yl-2H-pyrazol-3-yl)-piperidin kann **Beispiel 1.19** analog zu Beispiel 1.11 (siehe 11.) hergestellt und aufgereinigt werden. Analytische HPLC-MS (Methode C): RT = 1,64 min.

### 20. SYNTHESE VON (3-FLUORPHENYL)-{5-OXO-2-[4-(3-PYRIDIN-4-YL-[1,2,4] OXADIAZOL-5-YL)-PIPERIDIN-1-YL]-6,7-DIHYDRO-5H-5λ⁴-THIENO[3,2-d]PYRIMIDIN-4-YL}-AMIN (BEISPIEL 1.20)

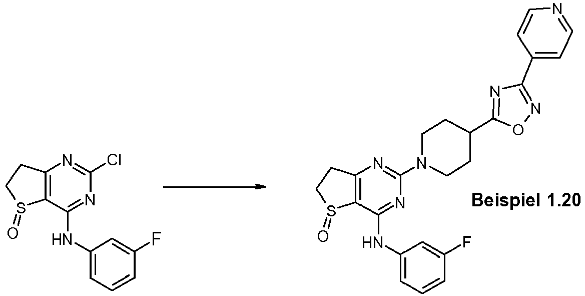

Ausgehend von **(IV-7)** (siehe 12.2) und 4-(5-Piperidin-4-yl-[1,2,4]oxadiazol-3-yl)-pyridin kann **Beispiel 1.20** als Trifluoracetat analog zu Beispiel 1.7 (siehe 7.) hergestellt und aufgereinigt werden. Analytische HPLC-MS (Methode C): RT = 1,72 min.

### 21. SYNTHESE VON (R)-3-METHYL-2-{5-OXO-2-[4-(3-PYRIDIN-4-YL-[1,2,4] OXADIAZOL-5-YL)-PIPERIDIN-1-YL]-6,7-DIHYDRO-5H-5λ⁴-THIENO[3,2-d]PYRIMIDIN-4-YLAMINO}-BUTAN-1-OL (BEISPIEL 1.21)

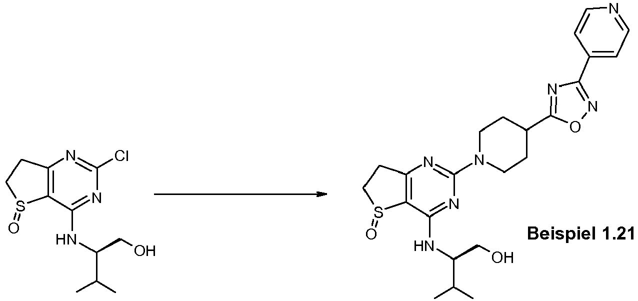

Ausgehend von **(IV-1)** (siehe 1.2) und 4-(5-Piperidin-4-yl-[1,2,4]oxadiazol-3-yl)-pyridin kann **Beispiel 1.21** als Trifluoracetat analog zu Beispiel 1.7 (siehe 7.) hergestellt und aufgereinigt werden. Analytische HPLC-MS (Methode C): RT = 1,48 min.

### 22. SYNTHESE VON: (S)-5-{2-[4-(4-FLUORPHENOXY)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.22)

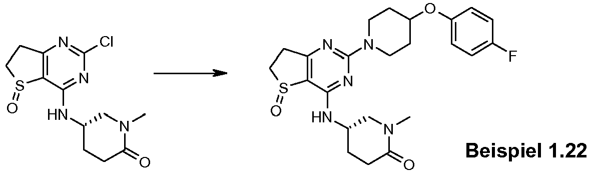

Ausgehend von **(IV-5)** (siehe 5.5) und 4-(4-Fluorphenoxy)-piperidin kann **Beispiel 1.22** analog zu Beispiel 1.16 (siehe 16.) hergestellt und aufgereinigt werden. Analytische HPLC-MS (Methode A): RT = 1,15 min.

### 23. SYNTHESE VON: (2-{4-[4-(4,5-DIHYDROOXAZOL-2-YL)-PHENOXY]-PIPERIDIN-1-YL}-5-OXO-6,7-DIHYDRO-5H-5λ⁴-THIENO[3,2-d]PYRIMIDIN-4-YL)-(TETRAHYDRO PYRAN-4-YL)-AMIN (BEISPIEL 1.23)

### 23.1 4-(Toluol-4-sulfonyloxy)-piperidin-1-carbonsäure-tert-butylester:

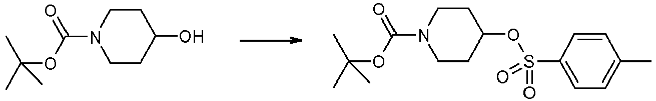

5 g 4-Hydroxypiperidin-1-carbonsäure-*tert*-butylester werden in 15 ml Pyridin vorgelegt, dann 4,7 g *p*-Toluolsulfonylchlorid portionsweise zugegeben. Das Reaktionsgemisch wird bei Raumtemperatur gerührt, nach 12 Stunden auf Eiswasser gegossen und die erhaltene Mischung eine weitere Stunde bei Raumtemperatur gerührt. Der ausgefallene Feststoff wird abgesaugt und getrocknet. 7,5 g Produkt werden erhalten.

### 23.2 4-[4-(4,5-Dihydrooxazol-2-yl)-phenoxy]-piperidin-1-carbonsäure-tert-butylester:

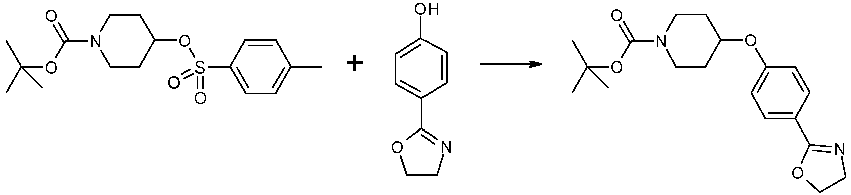

2,0 g 4-(4,5-Dihydrooxazol-2-yl)-phenol (siehe US5491201) werden in 30 ml Dimethylformamid vorgelegt, dann 3,3 g Kaliumcarbonat und 4,2 g 4-(Toluol-4-sulfonyloxy)-piperidin-1-carbonsäure-*tert*-butylester zugegeben. Das Reaktionsgemisch wird bei 75°C gerührt, nach 12 Stunden mit Wasser versetzt und der ausgefallene Feststoff abgesaugt und getrocknet. 2,8 g Produkt werden erhalten.

### 23.3 4-[4-(4,5-Dihydrooxazol-2-yl)-phenoxy]-piperidin (V-2):

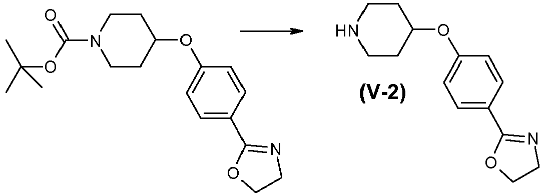

50 mg 4-[4-(4,5-Dihydrooxazol-2-yl)-phenoxy]-piperidin-1-carbonsäure-*tert*-butylester werden vorgelegt und mit 6 ml einer (5/1) Dichlormethan/Trifluoressigsäure Mischung versetzt. Das Reaktionsgemisch wird bei Raumtemperatur gerührt und nach 15 min vorsichtig mit einer gesättigten NaHCO₃ Lösung versetzt. Die organische Phase wird getrocknet und zur Trockne eingedampft. 20 mg **(V-2)** werden erhalten.

### 23.4 (2-{4-[4-(4,5-Dihydrooxazol-2-yl)-phenoxy]-piperidin-1-yl}-5-oxo-6,7-dihydro-5H-5λ⁴-thieno[3,2-d]pyrimidin-4-yl)-(tetrahydropyran-4-yl)-amin (Beispiel 1.23)

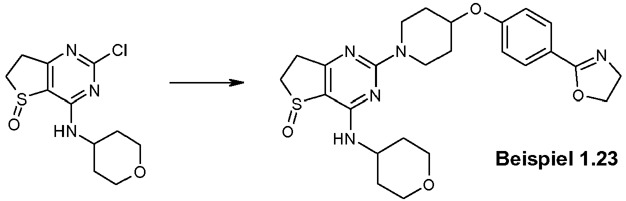

Ausgehend von **(IV-6)** (siehe 6.2) und **(V-2)** kann **Beispiel 1.23** analog zu Beispiel 1.14 (siehe 14.) hergestellt und aufgereinigt werden. Analytische HPLC-MS (Methode A): RT = 0,99 min.

### 24. SYNTHESE VON: 4-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-BENZOESÄURE (BEISPIEL 1.24)

### 24.1 4-{1-[5-Oxo-4-(tetrahydropyran-4-ylamino)-6,7-dihydro-5H-5λ⁴-thieno[3,2-d]pyrimidin-2-yl]-piperidin-4-yloxy}-benzoesäuremethylester

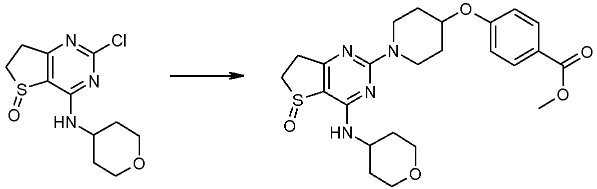

Ausgehend von **(IV-6)** (siehe 6.2) und 4-(Piperidin-4-yloxy)-benzoesäuremethylester (J. Med. Chem. 2002, 3406) kann 4-{1-[5-Oxo-4-(tetrahydropyran-4-ylamino)-6,7-dihydro-5*H*-5λ⁴-thieno[3,2-*d*]pyrimidin-2-yl]-piperidin-4-yloxy}-benzoesäuremethylester analog zu Beispiel 1.15 (siehe 15.) hergestellt und aufgereinigt werden. Analytische HPLC-MS (Methode A): RT = 1.17 min.

### 24.2 4-{1-[5-Oxo-4-(tetrahydropyran-4-ylamino)-6,7-dihydro-5H-5λ⁴-thieno[3,2-d]pyrimidin-2-yl]-piperidin-4-yloxy}-benzoesäure (Beispiel 1.24)

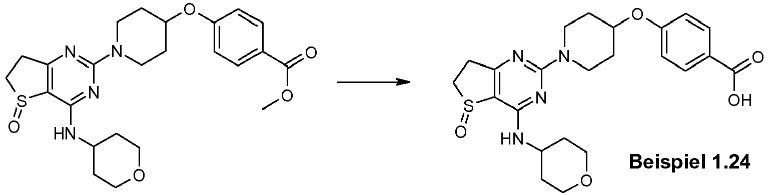

8 0 m g von 4-{1-[5-Oxo-4-(tetrahydropyran-4-ylamino)-6,7-dihydro-5*H*-5λ⁴-thieno[3,2-*d*]pyrimidin-2-yl]-piperidin-4-yloxy}-benzoesäuremethylester werden in 1,5 ml Methanol vorgelegt, dann 560 µl einer 1 N NaOH Lösung zugegebenDas Reaktionsgemisch wird bei 50°C, bis keine weitere Umsetzung erfolgt, gerührt, dann mit einer 1 M HCl Lösung versetzt. Das Produkt wird mit Dichlormethan extrahiert. 77 mg **Beispiel 1.24** werden als Feststoff erhalten. Analytische HPLC-MS (Methode B): RT = 1,19 min.

### 25. SYNTHESE VON 2-(1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-PROPAN-2-OL (BEISPIEL 1.25)

### 25.1 2-[1-(2-Chlor-6,7-dihydrothieno[3,2-d]pyrimidin-4-ylamino)-cyclopropyl]-propan-2-ol (III-8):

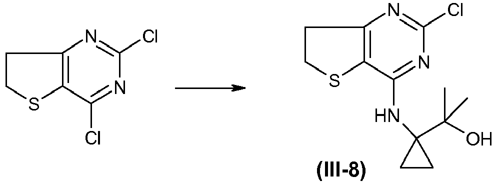

2,7 g **(II)** werden in 30 ml Dioxan vorgelegt, dann 6,8 ml Diisopropyl-ethylamin und 1,8 g 2-(1-Aminocyclopropyl)-propan-2-ol (siehe Liebigs Ann. Chem. 1978,1194) zugegeben. Das Reaktionsgemisch wird, bis keine weitere Umsetzung erfolgt, bei 160°C erhitzt und nach Abkühlen zur Trockne eingedampft. Der Rückstand wird mit Eiswasser versetzt. Das Produkt wird mit Dichlormethan extrahiert und chromatographisch gereinigt. 125 mg **(III-8)** werden als Feststoff erhalten. Analytische HPLC-MS (Methode A): RT = 1,08 min.

### 25.2 2-[1-(2-Chlor-5-oxo-6,7-dihydro-5H-5λ⁴--thieno[3,2-d]pyrimidin-4-ylamino)-cyclopropyl]-propan-2-ol (IV-8):

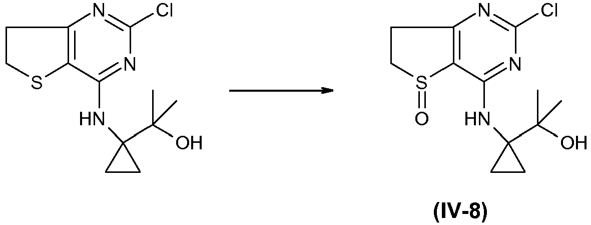

21,6 mg S-(-)-1,1'-Bi-2-naphtol werden in 1 ml Chloroform unter Argon vorgelegt, dann 11 µl Titan(IV)-isopropylat und 14 µl Wasser zugegeben. Das Reaktionsgemisch wird 1 Stunde bei Raumtemperatur gerührt. Anschließend wird eine Mischung von 120 mg **(III-8)** in 4 ml Dichlormethan zugegeben. Das Reaktionsgemisch wird auf -5°C abgekühlt und nach 30 Minuten werden 69,5 µl *tert*-Butylhydroperoxid 5-6 M in Decan zugetropft. Das Reaktionsgemisch wird bei -5 °C gerührt. Nach 2 Tage werden nochmal die gleichen Mengen von S-(-)-1,1'-Bi-2-naphtol, Titan(IV)-isopropylat, Wasser und *tert*-Butylhydroperoxid zugegeben. Das Reaktionsgemisch wird bei -5°C bis 5°C weitergerührt bis keine weitere Umsetzung erfolgt, mit Wasser versetzt und mit NH₄OH basisch gestellt. Die organische Phase wird zur Trockne eingedampft und das Produkt chromatographisch (präparative HPLC, Methode B) gereinigt. 105 mg **(IV-8)** werden als erhalten. Analytische HPLC-MS (Methode A): RT = 0,96 min.

### 25.3 2-(1-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-propan-2-ol (Beispiel 1.25)

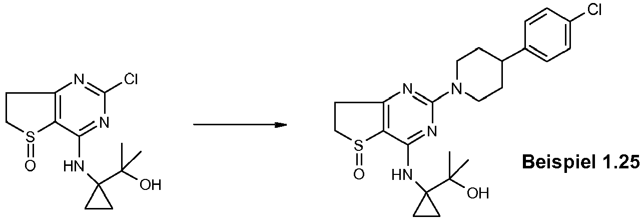

Ausgehend von **(IV-8)** und 4-(4-Chlorphenyl)-piperidin Hydrochlorid kann **Beispiel 1.25** analog zu Beispiel 1.14 (siehe 14.) hergestellt und aufgereinigt werden. Analytische HPLC-MS (Methode B): RT = 1,37 min.

### 26. SYNTHESE VON: {2-[4-(5-tert-BUTYL-1-METHYL-1H-INDOL-3-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 1.26)

### 26.1 4-(1H-Indol-3-yl)-piperidin-1-carbonsäure-tert-butylester:

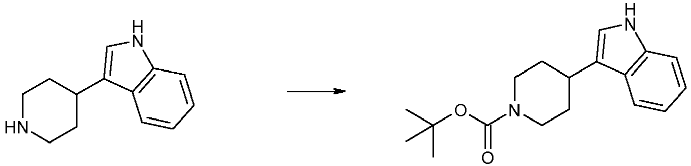

10 g 3-Piperidin-4-yl-1H-indol werden in 300 mL THF vorgelegt und 10,9 g Di-*tert*-butyldicarbonat zugegeben. Das Reaktionsgemisch wird über Nacht bei Raumtemperatur gerührt und zur Trockne eingedampft. Der Rückstand wird mit Wasser versetzt und das Produkt mit Diethylether extrahiert und chromatographisch gereinigt. 9 g des Produkts werden als Feststoff erhalten.

### 26.2 4-(1-Methyl-1H-indol-3-yl)-piperidin-1-carbonsäure-tert-butylester:

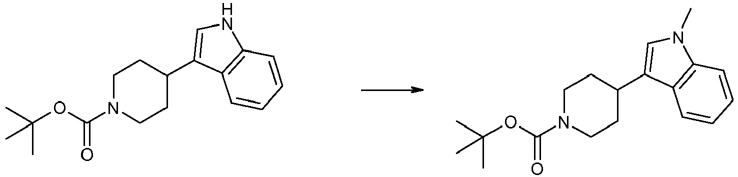

500 mg 4-(1H-Indol-3-yl)-piperidin-1-carbonsäure-*tert*-butylester werden in 8 ml Dimethylformamid vorgelegt und 73,3 mg Natriumhydrid (60% in Mineralöl) zugegeben. Nach 15 min werden 175 µl Methyliodid zugegeben. Das Reaktionsgemisch wird bei Raumtemperatur gerührt. Nach vollständiger Umsetzung wird das Produkt direkt über präparative HPLC (Methode C) gereinigt. 302 mg des Produkts werden als Öl erhalten. Analytische HPLC-MS (Methode A): RT = 1,65 min.

### 26.3 5-tert-Butyl-1-methyl-3-piperidin-4-yl-1H-indol (V-3)

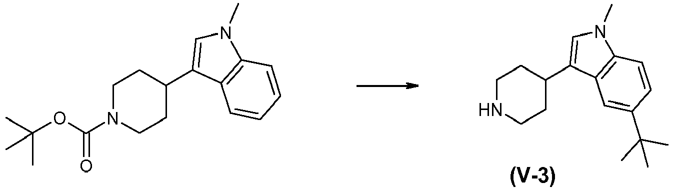

365 mg 4-(1-Methyl-1H-indol-3-yl)-piperidin-1-carbonsäure-*tert*-butylester werden in 1 ml Dichlormethan vorgelegt und mit 1,03 ml Trifluoressigsäure versetzt. Das Reaktionsgemisch wird bei Raumtemperatur gerührt. Nach 12 und 16 h werden nochmal 1,03 ml Trifluoressigsäure zugegeben. Nach weiteren 12 h wird das Reaktionsgemisch zur Trockne eingedampft. Der Rückstand wird mit Toluol versetzt und zur Trockne eingedampft. Der Rückstand wird mit Diethylether verrieben, der Niederschlag abgesaugt und getrocknet. 154 mg **(V-3)** werden als Feststoff erhalten. Analytische HPLC-MS (Methode A): RT = 1,34 min.

### 26.4 {2-[4-(5-tert-Butyl-1-methyl-1H-indol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.26)

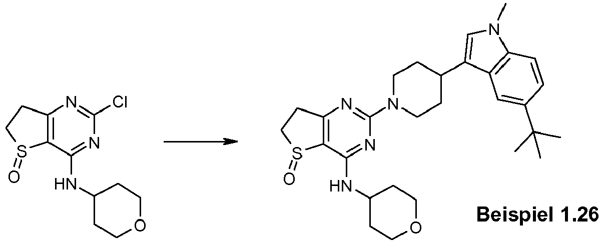

Ausgehend von **(IV-6)** (siehe 6.2) und **(V-3)** kann **Beispiel 1.26** analog zu Beispiel 1.14 (siehe 14.) hergestellt und aufgereinigt werden. Analytische HPLC-MS (Methode B): RT = 1,16 min.

### 27. SYNTHESE VON: {2-[4-(5-FURAN-2-YL-1-METHYL-1H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.27)

### 27.1 14-(5-Furan-2-yl-2H-pyrazol-3-yl)-piperidin-1-carbonsäure-tert-butylester:

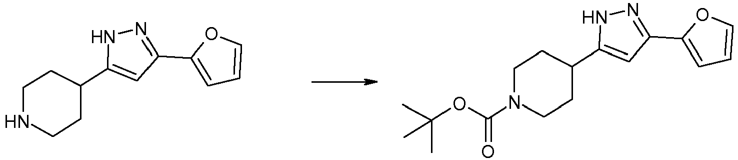

200 mg 4-(5-Furan-2-yl-2H-pyrazol-3-yl)-piperidin werden in 2 ml Dioxan vorgelegt. Anschließend werden 0,34 ml Wasser und 155 mg Natriumcarbonat zugegeben. Das Reaktionsgemisch wird beim Raumtemperatur gerührt. Nach 5 min werden 204 mg Di-tert-butyl-dicarbonat zugegeben. Nach 3 h wird das Reaktionsgemisch mit Wasser versetzt und das Produkt mit Dichlormethan extrahiert. 300 mg Produkt werden als Öl erhalten. Analytische HPLC-MS (Methode B): RT = 1,54 min.

### 27.2 4-(5-Furan-2-yl-2-methyl-2H-pyrazol-3-yl)-piperidin-1-carbonsäure-tert-butylester und 4-(5-Furan-2-yl-1-methyl-1H-pyrazol-3-yl)-piperidin-1-carbonsäure-tert-butylester:

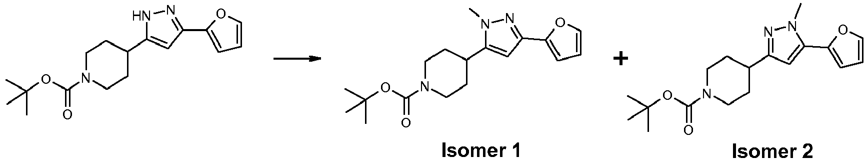

250 mg 14-(5-Furan-2-yl-2H-pyrazol-3-yl)-piperidin-1-carbonsäure-*tert*-butylester werden in 1,5 ml Dimethylformamid vorgelegt. Das Reaktionsgemisch wird im Eisbad abgekühlt und 40 mg Natriumhydrid (60% in Mineralöl) werden zugegeben. Nach 10 min werden 60 µl Methyliodid zugegeben. Das Reaktionsgemisch wird 30 min bei 5°C und anschliessend 4 h bei Raumtemperatur gerührt. Das Produkt wird dann direkt über präparative HPLC (Methode D) aufgereinigt. 90 mg **Isomer 1** und 50 mg **Isomer 2** werden als Feststoff erhalten. Analytische HPLC-MS (Methode D): RT = 1,33 min (Isomer 1); RT = 1,28 (Isomer 2).

### 27.3 4-(5-Furan-2-yl-1-methyl-1 H-pyrazol-3-yl)-piperidin (V-4):

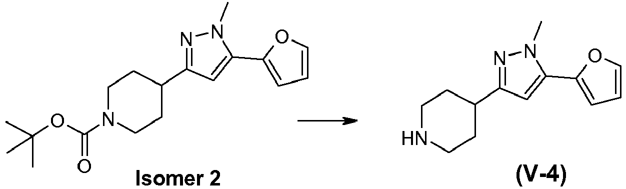

47 mg **Isomer 2** werden in 1 ml Dichlormethan vorgelegt und 120 µl Trifluoressigsäure zugegeben. Das Reaktionsgemisch wird 2h bei Raumtemperatur gerührt, dann zur Trockne eingedampft. Der Rückstand wird mit Toluol versetzt und zur Trockne eingedampft. Der Rückstand wird mit Wasser versetzt, mit konz. Ammoniak basisch gestellt und das Produkt mit Dichlormethan extrahiert. 23 mg **(V-4)** werden als Feststoff erhalten. Analytische HPLC-MS (Methode B): RT = 0,85 min

### 27. 4 {2-[4-(5-Furan-2-yl-1-methyl-1H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.27)

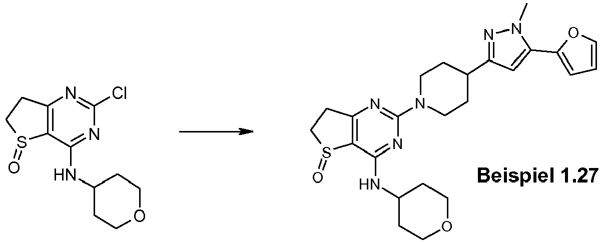

Ausgehend von **(IV-6)** (siehe 6.2) und **(V-4)** kann **Beispiel 1.27** analog zu Beispiel 1.14 (siehe 14) hergestellt und aufgereinigt werden. Analytische HPLC-MS (Methode B): RT = 1,21 min.

### 28. SYNTHESE VON: (S)-5-(2-{4-[4-(4,5-DIHYDROOXAZOL-2-YL)-PHENOXY]-PIPERIDIN-1-YL}-5-OXO-6,7-DIHYDRO-5H-5λ⁴-THIENO[3,2-d]PYRIMIDIN-4-YLAMINO)-1-METHYLPIPERIDIN-2-ON (BEISPIEL 1.28)

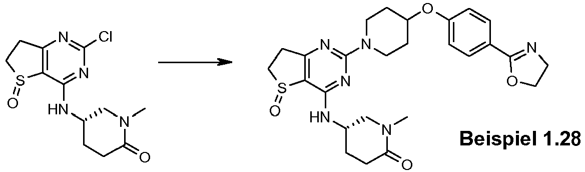

Ausgehend von **(IV-5)** (siehe 5.5) und **(V-2)** (siehe 23.3) kann **Beispiel 1.28** analog zu Beispiel 1.16 (siehe 16.) hergestellt und aufgereinigt werden. Analytische HPLC-MS (Methode B): RT = 1.07 min.

### 29. SYNTHESE VON: {2-[4-(5-FURAN-2-YL-2-METHYL-2H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.29)

### 29.1 4-(5-Furan-2-yl-2-methyl-2H-pyrazol-3-yl)-piperidin (V-5):

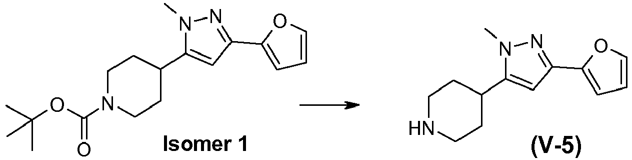

Ausgehend von **Isomer 1** (siehe 27.2), kann **(V-5)** analog zu **(V-4)** (siehe 27.3) hergestellt werden. Analytische HPLC-MS (Methode D): RT = 0,89 min.

### 29.2 Synthese von: {2-[4-(5-Furan-2-yl-2-methyl-2H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.29)

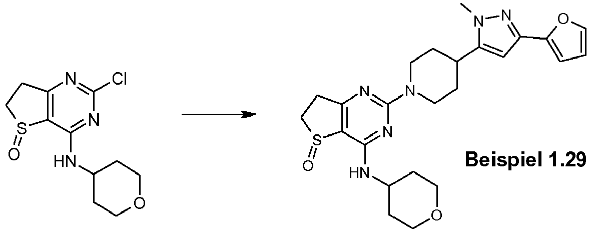

Ausgehend von **(IV-6)** (siehe 6.2) und **(V-5)** kann **Beispiel 1.29** analog zu Beispiel 1.14 (siehe 14.) hergestellt und aufgereinigt werden. Analytische HPLC-MS (Methode B): RT = 1,26 min.

### 30. SYNTHESE VON: {2-[4-(1-METHYL-1H-IMIDAZO[4,5-c]PYRIDIN-2-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 1.30)

### 30.1 Methyl-(3-nitropyridin-4-yl)-amin:

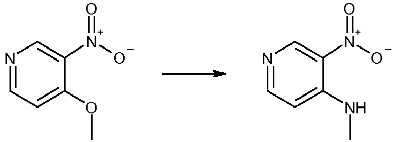

2,36 g 4-Methoxy-3-nitro-pyridin und 2,33 ml Methylamin (40%ig in Wasser) werden in 25 ml Ethanol 3 h unter Rückfluss gekocht. Anschließend wird das Reaktionsgemisch zur Trockne eingedampft. 2,3 g Produkt werden als Feststoff erhalten.

### 30.2 N⁴-Methylpyridin-3,4-diamin:

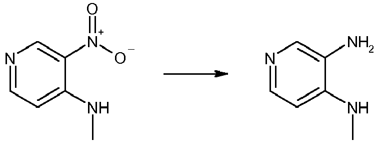

2,3 g Methyl-(3-nitropyridin-4-yl)-amin werden in 50 ml Methanol für und mit 0,8 g Raney-Nickel 2,5 h bei 50°C und 50 psi Wasserstoffdruck hydriert. Der Katalysator wird abfiltriert und das Filtrat zur Trockne eingedampft. Das Produkt wird chromatographisch (Alox, Dichlormethan/Methanol von 99/1 bis 19/1) gereinigt. 1,55 g Produkt werden als Feststoff erhalten. Smp: 163-165°C.

### 30.3 1-Methyl-2-piperidin-4-yl-1 H-imidazo[4,5-c]pyridin (V-6):

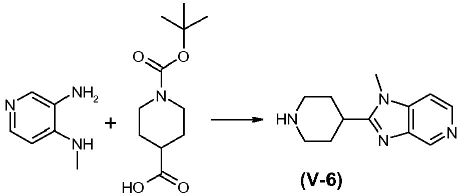

450 mg *N*⁴-Methylpyridin-3,4-diamin und 838 mg Piperidin-1,4-Dicarbonsäure-mono-*tert-*butylester werden in 8,6 g Polyphosphorsäure für 4 h bei 200°C erhitzt. Nach dem Abkühlen wird mit 4 N NaOH basisch gestellt und mit Trifluoressigsäure angesäuert. Das Gemisch wird über präparative HPLC (Methode C) aufgereinigt. 3,37 g (50%ig) **(V-6)** werden als Trifluoracetat erhalten. Analytische HPLC-MS (Methode B): RT = 0,30 min.

### 30.4 {2-[4-(1-Methyl-1H-imidazo[4,5-c]pyridin-2-yl)-piperidin-1-yl]-5-oxo-6,7-dihydro-5H-5λ⁴-thieno[3,2-d]pyrimidin-4-yl}-(tetrahydropyran-4-yl)-amin (Beispiel 1.30)

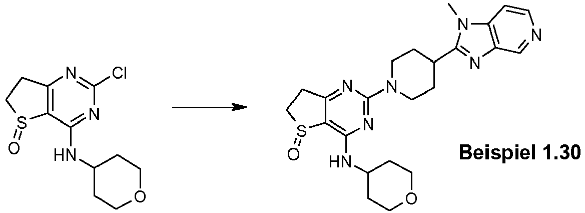

Ausgehend von **(IV-6)** (siehe 6.2) und **(V-6)** kann **Beispiel 1.30** analog zu Beispiel 1.14 (siehe 14) hergestellt und aufgereinigt werden. Analytische HPLC-MS (Methode D): RT = 0,86 min.

### 31. SYNTHESE VON 2-METHOXY-N-{1-[5-OXO-4-(TETRAHYDROPYRAN-4-YLAMINO)-6,7-DIHYDRO-5H-5λ⁴-THIENO[3,2-d]PYRIMIDIN-2-YL]-4-PHENYLPIPERIDIN-4-YLMETHYL}-ACETAMID (BEISPIEL 1.31)

### 31.1 4-[(2-Methoxyacetylamino)-methyl]-4-phenylpiperidin-1-carbonsäure-tert-butylester:

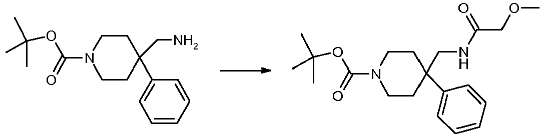

3,7 g des käuflichen 4-Aminomethyl-4-phenyl-piperidin-1-carbonsäure-*tert*-butylester und 3 ml Diisopropylethylamin werden in 30 ml Dichlormethan vorgelegt. Anschließend werden 2,25 ml Methoxyacetylchlorid langsam zugegeben. Das Reaktionsgemisch wird bei Raumtemperatur, bis keine weitere Umsetzung erfolgt, gerührt, dann mit Wasser versetzt. Die organische Phase wird zur Trockne eingedampft. 4,7 g Produkt werden als Öl erhalten.

### 31.2 2-Methoxy-N-(4-phenylpiperidin-4-ylmethyl)-acetamid (V-7):

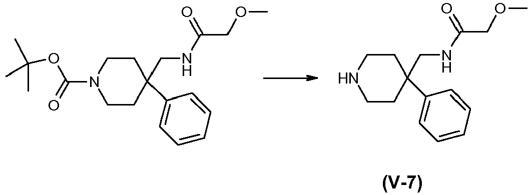

1 g 4-[(2-Methoxyacetylamino)-methyl]-4-phenylpiperidin-1-carbonsäure-*tert*-butylester werden in 4 ml Dichlormethan vorgelegt. Anschließend werden 1,7 ml Trifluoressigsäure zugegeben und über Nacht bei Raumtemperatur gerührt. Das Reaktionsgemisch wird mit Kaliumcarbonat basisch gestellt und die organische Phase zur Trockne eingedampft. 610 mg **(V-7)** werden als Öl erhalten.

### 31.3 Synthese von 2-Methoxy-N-{1-[5-oxo-4-(tetrahydropyran-4-ylamino)-6,7-dihydro-5H-5λ⁴-thieno[3,2-d]pyrimidin-2-yl]-4-phenylpiperidin-4-ylmethyl}-acetamid (Beispiel 1.31)

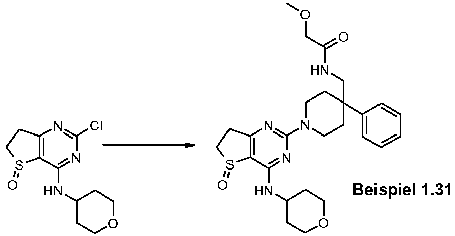

Ausgehend von **(IV-6)** (siehe 6.2) und **(V-7)** kann **Beispiel 1.31** analog zu Beispiel 1.15 (siehe 15.) hergestellt und aufgereinigt werden. Analytische HPLC-MS (Methode B): RT = 1,21 min.

### 32. SYNTHESE VON: N-CYCLOPROPYL-N-METHYL-4-{1-[5-OXO-4-(TETRAHYDRO PYRAN-4-YLAMINO)-6,7-DIHYDRO-5H-5λ⁴-THIENO[3,2-d]PYRIMIDIN-2-YL]-PIPERIDIN-4-YL}-BENZAMID (BEISPIEL 1.32)

### 32.1 4-[4-(Cyclopropylmethylcarbamoyl)-phenyl]-piperidin-1-carbonsäure-tert-butylester:

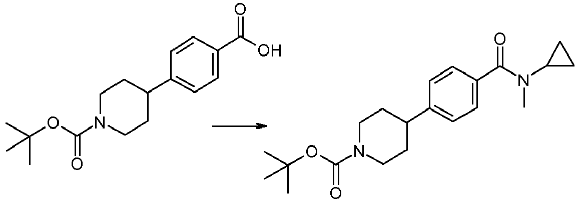

500 mg 4-(4-Carboxyphenyl)-piperidin-1-carbonsäure-*tert*-butylester werden in 28 ml Dimethylformamid vorgelegt, dann 1,14 ml Diisopropylethylamin und 747 mg HATU zugegeben. Das Reaktionsgemisch wird 15 min bei Raumtemperatur gerührt, dann werden 194 mg Cyclopropylmethylamin Hydrochlorid zugegeben. Das Reaktionsgemisch wird über Nacht bei Raumtemperatur gerührt. Anschließend wird das Produkt über präparative HPLC (Methode A) gereinigt. 480 mg Produkt werden als Öl erhalten. Analytische HPLC-MS (Methode B): RT = 1,64 min.

### 32.2 N-Cyclopropyl-N-methyl-4-piperidin-4-yl-benzamid (V-8):

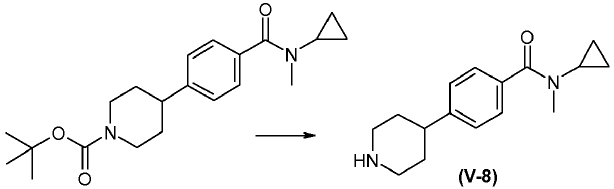

480 mg 4-[4-(Cyclopropylmethylcarbamoyl)-phenyl]-piperidin-1-carbonsäure-*tert*-butylester werden in 7,8 ml Dichlormethan vorgelegt und mit 1,09 ml Trifluoressigsäure versetzt. Das Reaktionsgemisch wird 1,5 h bei Raumtemperatur gerührt und anschließend zur Trockne eingedampft. Der Rückstand wird mit Toluol versetzt und wieder zur Trockne eingedampft. 444 mg **(V-8)** werden als Trifluoracetat erhalten. Analytische HPLC-MS (Methode B): RT = 1,11 min.

### 32.3 N-Cyclopropyl-N-methyl-4-{1-[5-oxo-4-(tetrahydropyran-4-ylamino)-6,7-dihydro-5H-5λ⁴-thieno[3,2-d]pyrimidin-2-yl]-piperidin-4-yl}-benzamid (Beispiel 1.32)

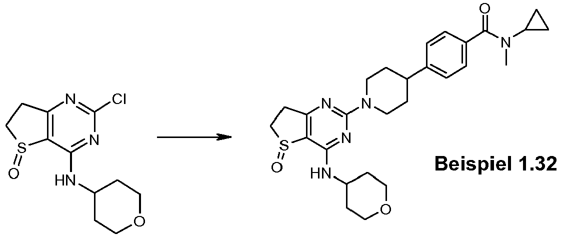

Ausgehend von **(IV-6)** (siehe 6.2) und **(V-8)** kann **Beispiel 1.32** analog zu Beispiel 1.14 (siehe 14.) hergestellt und aufgereinigt werden. Analytische HPLC-MS (Methode D): RT = 1,05 min.

### 33. SYNTHESE VON: N-CYCLOPROPYL-N-METHYL-4-{1-[5-OXO-4-(TETRAHYDRO-.PYRAN-4-YLAMINO)-6,7-DIHYDRO-5H-5λ⁴-THIENO[3,2-d]PYRIMIDIN-2-YL]-PIPERIDIN-4-YLOXY}-BENZAMID (BEISPIEL 1.33)

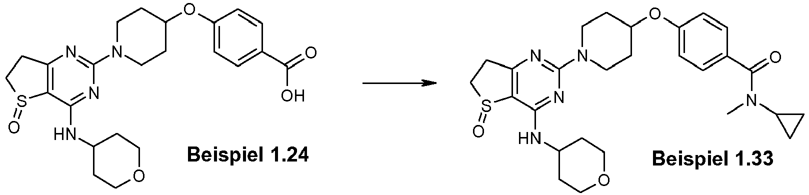

55 mg von **Beispiel 1.24** (siehe 24.2) werden in 2 ml Dimethylformamid vorgelegt, dann 81 µl Diisopropylethylamin und 53,1 mg O-(7-Azabenzotriazol-1-yl-)-N,N,N',N'-tetramethyluroniumhexafluorphosphat (HATU) zugegeben. Nach 15 min werden 13,8 mg cyclopropylmethylamin Hydrochlorid zugegeben. Das Reaktionsgemisch wird bei Raumtemperatur, bis keine weitere Reaktion erfolgt, gerührt und das Produkt direkt über präparative HPLC (Methode B) gereinigt. 30 mg **Beispiel 1.33** werden als Feststoff erhalten. Analytische HPLC-MS (Methode D): RT = 1,03 min.

### 34. SYNTHESE VON: {5-OXO-2-[4-(PYRIDIN-4-YLOXY)-PIPERIDIN-1-YL]-6,7-DIHYDRO-5H-5λ⁴-THIENO[3,2-d]PYRIMIDIN-4-YL}-(TETRAHYDROPYRAN-4-YL)-AMIN (BEISPIEL 1.34)

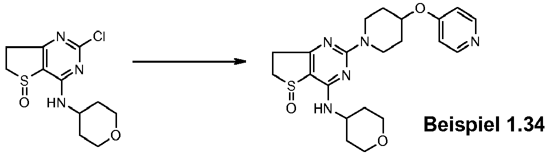

Ausgehend von **(IV-6)** (siehe 6.2) und 4-(Piperidin-4-yloxy)-pyridin kann **Beispiel 1.34** analog zu Beispiel 1.15 (siehe 15.) hergestellt und aufgereinigt werden. Analytische HPLC-MS (Methode B): RT = 0,99 min.

### 35. SYNTHESE VON: {2-[4-(4-CHLORPHENOXY)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.35)

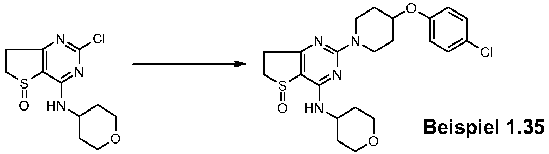

Ausgehend von **(IV-6)** (siehe 6.2) und 4-(4-Chlorphenoxy)-piperidin kann **Beispiel 1.35** analog zu Beispiel 1.15 (siehe 15.) hergestellt und aufgereinigt werden. Analytische HPLC-MS (Methode B): RT = 1,39 min.

### 36. SYNTHESE VON: (S)-1-METHYL-5-{2-[4-(5-METHYL-4-PHENYL-OXAZOL-2-YL)-PIPERIDIN-1-YL]-5-OXO-6,7-DIHYDRO-5H-5λ⁴-THIENO[3,2-d]PYRIMIDIN-4-YLAMINO}-PIPERIDIN-2-ON (BEISPIEL 1.36)

### 36.1 4-(5-Methyl-4-phenyloxazol-2-yl)-piperidin (V-9):

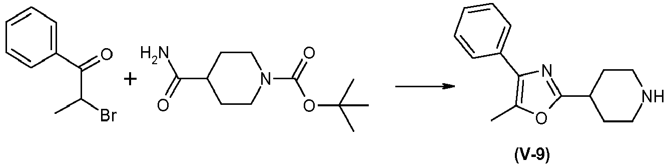

1,75 g 2-Brom-1-phenylpropan-1-on und 1,87 g 4-Carbamoylpiperidin-1-carbonsäure-*tert-*butylester werden in 0,5 ml NMP vorgelegt. Das Reaktionsgemisch wird bei 160°C für 20 min in der Mikrowelle und 35 min im Ölbad erhitzt, dann nach dem Abkühlen in Methanol aufgenommen und zur Trockne eingedampft. Der Rückstand wird mit Wasser versetzt, im Ultraschallbad behandelt und das unlösliche Öl abgesaugt. Die Mutterlauge wird über präparative HPLC (Methode C) gereinigt. 160 mg **(V-9)** werden als Trifluoracetat erhalten. Analytische HPLC-MS (Methode B): RT = 1,24 min.

### 36.2 (S)-1-Methyl-5-{2-[4-(5-methyl-4-phenyloxazol-2-yl)-piperidin-1-yl]-5-oxo-6,7-dihydro-5H-5λ⁴-thieno[3,2-d]pyrimidin-4-ylamino}-piperidin-2-on (Beispiel 1.36)

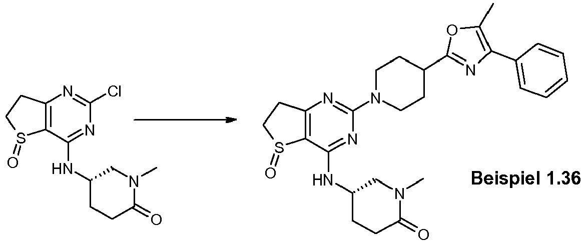

Ausgehend von **(IV-5)** (siehe 5.5) und **(V-9)** kann **Beispiel 1.36** analog zu Beispiel 1.14 (siehe 14.) hergestellt und aufgereinigt werden. Analytische HPLC-MS (Methode D): RT = 1,08 min.

### 37 SYNTHESE VON: (1-{2-[4-(5-METHYL-4-PHENYLOXAZOL-2-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.37)

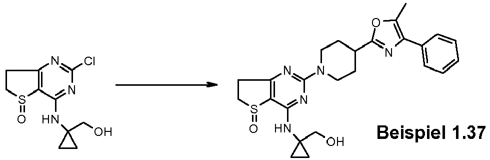

Ausgehend von **(IV-2)** (siehe 2.4) und **(V-9)** (siehe 36.1) kann **Beispiel 1.37** analog zu Beispiel 1.14 (siehe 14.) hergestellt und aufgereinigt werden. Analytische HPLC-MS (Methode B): RT = 1,33 min.

### 38. SYNTHESE VON: (S)-5-{2-[4-(4,5-DIPHENYLOXAZOL-2-YL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 1.38)

### 38.1 4-(4,5-Diphenyloxazol-2-yl)-piperidin-1-carbonsäure-tert-butylester

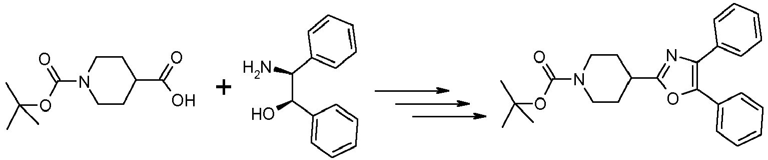

Ausgehend von 1,08 g Piperidin-1,4-dicarbonsäure-mono-*tert*-butylester und 1 g 2-Amino-1,2-diphenyl-ethanol kann das Produkt wie in der Literatur beschrieben (siehe Tet. 2001, 4867) hergestellt werden. Das Produkt wird chromatographisch (Methode B) gereinigt. 560 mg werden als Öl erhalten. Analytische HPLC-MS (Methode A): RT = 1,72 min.

### 38.2 4-(4,5-Diphenyloxazol-2-yl)-piperidin (V-10)

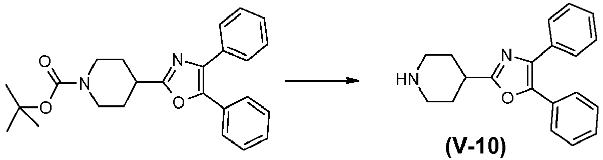

560 mg 4-(4,5-Diphenyloxazol-2-yl)-piperidin-1-carbonsäure-*tert*-butylester werden in 2 ml Dichlormethan vorgelegt, dann 1,1 ml Trifluoressigsäure zugegeben. Das Reaktionsgemisch wird 15 Stunden bei Raumtemperatur gerührt, dann zur Trockne eingedampft. Der Rückstand wird mit Toluol versetzt und wieder zur Trockne eingedampft. Der Rückstand wird mit Diethylether versetzt und der ausgefallene Feststoff abgesaugt und getrocknet. 510 mg **(V-10)** werden erhalten. Analytische HPLC-MS (Methode B): RT = 1,38 min.

### 38.3 (S)-5-{2-[4-(4,5-Diphenyloxazol-2-yl)-piperidin-1-yl]-5-oxo-6,7-dihydro-5H-5λ⁴-thieno[3,2-d]pyrimidin-4-ylamino}-1-methylpiperidin-2-on (Beispiel 1.38)

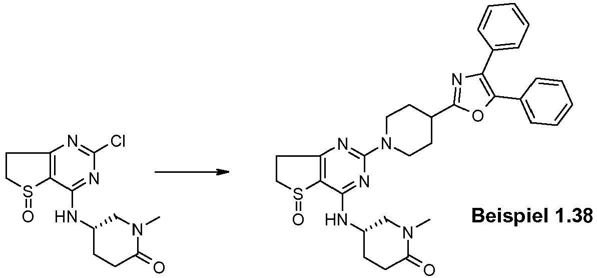

Ausgehend von **(IV-5)** (siehe 5.5) und **(V-10)** kann **Beispiel 1.38** analog zu Beispiel 1.14 (siehe 14.) hergestellt und aufgereinigt werden. Analytische HPLC-MS (Methode B): RT = 1,40 min.

### 39. SYNTHESE VON: {4-(4-CHLORPHENYL)-1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YL}-METHANOL (BEISPIEL 1.39)

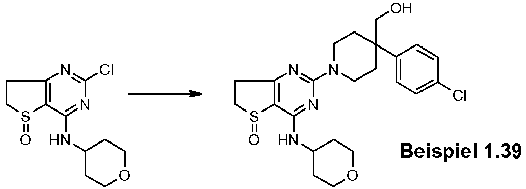

Ausgehend von **(IV-6)** (siehe 6.2) und [4-(4-Chlorphenyl)-piperidin-4-yl]-methanol (J. Med. Chem. 2004, 497) kann **Beispiel 1.39** analog zu Beispiel 1.15 (siehe 15.) hergestellt und aufgereinigt werden. Analytische HPLC-MS (Methode B): RT = 1,24 min.

### 40. SYNTHESE VON: [1-(2-{4-[5-(4-CHLORPHENYL)-4-METHYLOXAZOL-2-YL]-PIPERIDIN-1-YL}-5-OXO-6,7-DIHYDRO-5H-5λ⁴-THIENO[3,2-d]PYRIMIDIN-4-YLAMINO)-CYCLOPROPYL]-METHANOL (BEISPIEL 1.40)

### 40.1 4-[5-(4-Chlorphenyl)-4-methyloxazol-2-yl]-piperidin (V-11):

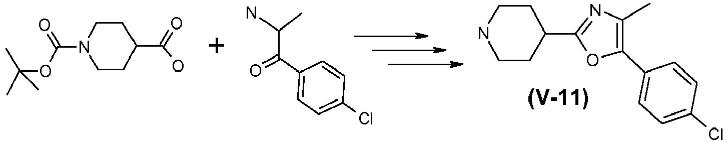

Ausgehend von Piperidin-1,4-dicarbonsäure-mono-*tert*-butylester und 2-Amino-1-(4-chlorphenyl)-propan-1-on (siehe J. Med. Chem. 1974, 416) kann **(V-11)** analog zu (V-10) (siehe 38.2) hergestellt werden. Analytische HPLC-MS (Methode B): RT = 1,30 min.

### 40.2 [1(2-{4-[5-(4-Chlorphenyl)-4-methyloxazol-2-yl]-piperidin-1-yl}-5-oxo-6,7-dihydro-5H-5λ⁴-thieno[3,2-d]pyrimidin-4-ylamino)-cyclopropyl]-methanol (Beispiel 1.40)

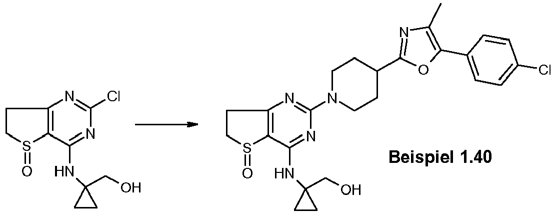

Ausgehend von **(IV-2)** (siehe 2.4) und **(V-11)** kann **Beispiel 1.40** analog zu Beispiel 1.14 (siehe 14.) hergestellt und aufgereinigt werden. Analytische HPLC-MS (Methode B): RT = 1,37 min.

### 41. SYNTHESE VON: 4-(4-CHLORPHENYL)-1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-OL (BEISPIEL 1.41)

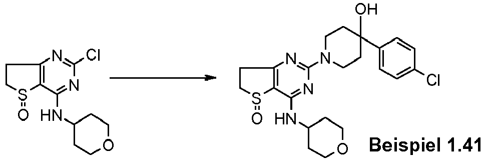

Ausgehend von **(IV-6)** (siehe 6.2) und 4-(4-Chlorphenyl)-piperidin-4-ol kann **Beispiel 1.41** analog zu Beispiel 1.15 (siehe 15.) hergestellt und aufgereinigt werden. Analytische HPLC-MS (Methode B): RT = 1,25 min.

### 42. SYNTHESE VON: {2-[4-(4-CHLORPHENYL)-4-METHOXYPIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.42)

### 42.1 4-(4-Chlorphenyl)-4-hydroxypiperidin-1-carbonsäure-tert-butylester:

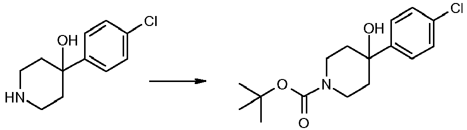

500 mg 4-(4-Chlorphenyl)-piperidin-4-ol werden in 6 ml Dioxan vorgelegt, dann 0,9 ml Wasser und 400 mg Natriumcarbonat zugegeben. Nach 5 min werden 530 mg Di-*tert*-butyldicarbonat zugegeben. Das Reaktionsgemisch wird 12 Stunden bei Raumtemperatur gerührt, anschließend mit Wasser versetzt und das Produkt mit Dichlormethan extrahiert.. 790 mg Produkt werden als Öl erhalten. Analytische HPLC-MS (Methode B): RT = 1,65 min.

### 42.2 4-(4-Chlorphenyl)-4-methoxypiperidin-1-carbonsäure-tert-butylester:

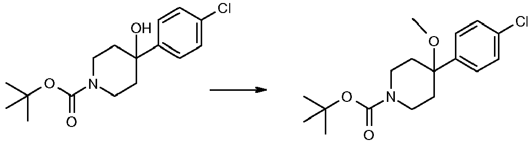

790 mg 4-(4-Chlorphenyl)-4-hydroxypiperidin-1-carbonsäure-*tert*-butylester werden in 5 ml Dimethylformamid vorgelegt und 193 mg Natriumhydrid (60% in Mineralöl) zugegeben. Das Reaktionsgemisch wird 30 min bei Raumtemperatur gerührt, dann 267 µl Methyliodid zugegeben. Nach 1 h wird das Reaktionsgemisch auf Eis gegossen und das Produkt mit Diethylether extrahiert. 650 mg Produkt werden als Öl erhalten. Analytische HPLC-MS (Methode B): RT = 1,88 min.

### 42.3 4-(4-Chlorphenyl)-4-methoxypiperidin (V-12):

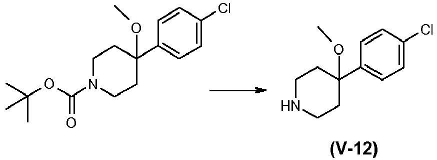

650 mg 4-(4-Chlorphenyl)-4-methoxypiperidin-1-carbonsäure-*tert*-butylester werden in 3 ml Dichlormethan vorgelegt, dann 1,46 ml Trifluoressigsäure zugegeben. Das Reaktionsgemisch wird über Nacht bei Raumtemperatur gerührt und zur Trockne eingedampft. Der Rückstand wird mit Toluol versetzt und wieder zur Trockne eingedampft. Der Rückstand wird mit Diethylether verrieben und der Feststoff abgesaugt. 450 mg **(V-12)** werden als Trifluoracetat erhalten. Analytische HPLC-MS (Methode B): RT = 1,22 min.

### 42.4 {2-[4-(4-Chlorphenyl)-4-methoxypiperidin-1-yl]-5-oxo-6,7-dihydro-5H-5λ⁴-thieno[3,2-d]pyrimidin-4-yl}-(tetrahydropyran-4-yl)-amin (Beispiel 1.42)

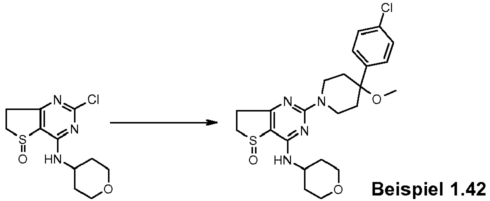

Ausgehend von **(IV-6)** (siehe 6.2) und **(V-12)** kann **Beispiel 1.42** analog zu Beispiel 1.15 (siehe 15.) hergestellt und aufgereinigt werden. Analytische HPLC-MS (Methode B): RT = 1,39 min.

### 43. SYNTHESE VON: 4-{1-[4-(1-HYDROXYMETHYLCYCLOPROPYLAMINO)-5-OXO-6,7-DIHYDRO-5H-5λ⁴-THIENO[3,2-d]PYRIMIDIN-2-YL]-PIPERIDIN-4-YLOXY}-BENZONITRIL (BEISPIEL 1.43)

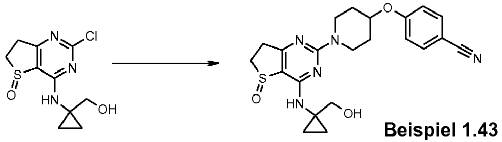

Ausgehend von **(IV-2)** (siehe 2.4) und 4-(Piperidin-4-yloxy)-benzonitril (WO2007/106705) kann **Beispiel 1.43** analog zu Beispiel 1.14 (siehe 14.) hergestellt und aufgereinigt werden. Analytische HPLC-MS (Methode B): RT = 1,24 min.

### 44. SYNTHESE VON: 5-OXO-2-[4-(4,5,6,7-TETRAHYDROBENZOXAZOL-2-YL)-PIPERIDIN-1-YL]-6,7-DIHYDRO-5H-5λ⁴-THIENO[3,2-d]PYRIMIDIN-4-YL}-(TETRAHYDRO PYRAN-4-YL)-AMIN (BEISPIEL 1.44)

### 44.1 2-(1-Benzylpiperidin-4-yl)-4,5,6,7-tetrahydrobenzoxazol:

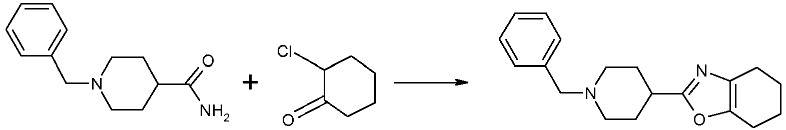

Ein Gemisch von 2,43 g 2-Chlorcyclohexanon und 1g 1-Benzylpiperidin-4-carbonsäureamid (WO005/61483) wird bei 160°C in der Mikrowelle, bis keine weitere Umsetzung erfolg, erhitzt. Das Produkt wird chromatographisch gereinigt. 963 mg des Produkts werden erhalten. Analytische HPLC-MS (Methode B): RT = 1,28 min.

### 44.2 2-Piperidin-4-yl-4,5,6,7-tetrahydrobenzoxazol (V-13):

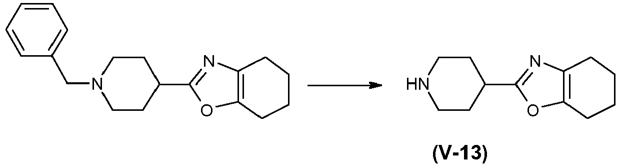

903 mg 2-(1-Benzyl-piperidin-4-yl)-4,5,6,7-tetrahydrobenzoxazol werden in 20 ml Methanol vorgelegt und mit 450 mg Pd/C 10% bei einem Druck von 3 bar und bei Raumtemperatur hydriert. Nach 12 Stunden wird der Katalysator abgesaugt und das Filtrat zur Trockne eingedampft. Das Produkt wird chromatographisch gereinigt. 469 mg **(V-13)** werden als Trifluoracetat erhalten. Analytische HPLC-MS (Methode B): RT = 1,09 min.

### 44.3 5-Oxo-2-[4-(4,5,6,7-tetrahydrobenzoxazol-2-yl)-piperidin-1-yl]-6,7-dihydro-5H-5λ⁴-thieno[3,2-d]pyrimidin-4-yl}-(tetrahydropyran-4-yl)-amin (Beispiel 1.44)

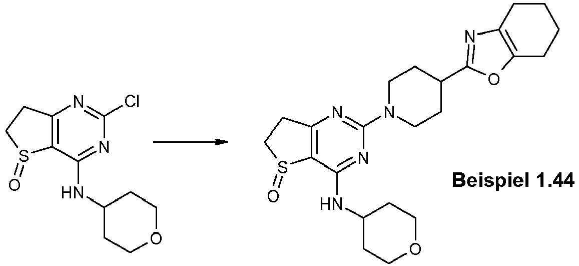

Ausgehend von **(IV-6)** (siehe 6.2) und **(V-13)** kann **Beispiel 1.44** analog zu Beispiel 1.14 (siehe 14.) hergestellt und aufgereinigt werden. Analytische HPLC-MS (Methode B): RT = 1,23 min.

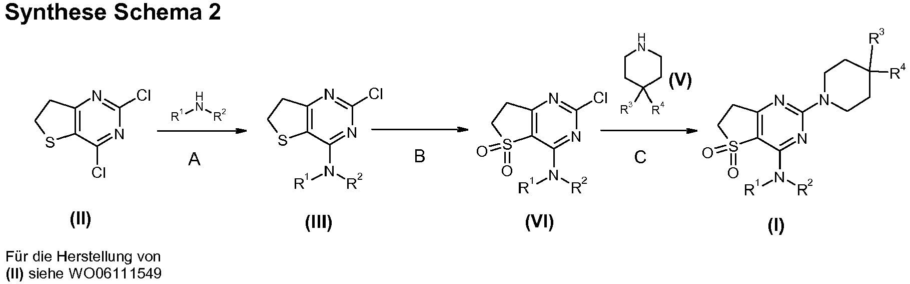

### 45. SYNTHESE VON: (S)-5-{2-[4-(4-CHLORPHENYL)-PIPERIDIN-1-YL]-5,5-DIOXO-6,7-DIHYDRO-5H-5λ⁶-THIENO[3,2-d]PYRIMIDIN-4-YLAMINO}-1-METHYLPIPERIDIN-2-ON (Beispiel 1.45)

### 45.1 5-(2-Chlor-5,5-dioxo-6,7-dihydro-5H-5λ⁶-thieno[3,2-d]pyrimidin-4-ylamino)-1-methylpiperidin-2-on (VI-1):

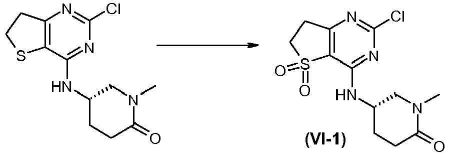

200 mg **(III-5)** (siehe 5.4) werden in 3 ml Trifluoressigsäure vorgelegt, dann 165 µl Wasserstoffperoxid (35%) langsam zugetropft. Eine exotherme Reaktion findet statt. Das Reaktionsgemisch wird 12 Stunden bei Raumtemperatur gerührt, dann mit Eiswasser versetzt und mit NH₄OH basisch gestellt. Das Produkt wird mit Dichlormethan extrahiert. 150 mg **(VI-1)** werden als Feststoff erhalten.

### 45.2 (S)-5-{2-[4-(4-Chlorphenyl)-piperidin-1-yl]-5,5-dioxo-6,7-dihydro-5H-5λ⁶-thieno[3,2-d]pyrimidin-4-ylamino}-1-methylpiperidin-2-on (Beispiel 1.45)

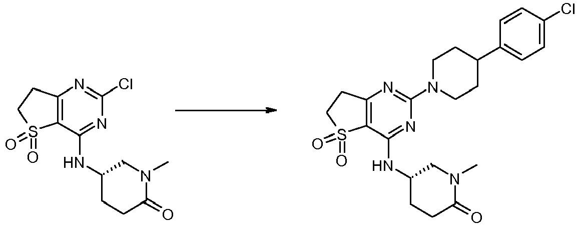

Ausgehend von **(VI-1)** und 4-(4-Chlorphenyl)-piperidin Hydrochlorid kann **Beispiel 1.45** analog zu Beispiel 1.14 (siehe 14.) hergestellt und aufgereinigt werden. Analytische HPLC-MS (Methode B): RT = 1,48 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in der Tabellen B, D und E angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampier, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith^{™} Flash RP-18e, 4.6 mm x 25 mm (Säulentemperatur: konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.80 |
| 0.30 | 95 | 5 | 2.80 |
| 1.60 | 2 | 98 | 2.80 |
| 1.90 | 2 | 98 | 2.80 |
| 2.00 | 95 | 5 | 2.50 |

Als stationäre Phase dient eine Säule Merck Chromolith^{™} Flash RP-18e, 3 mm x 100 mm (Säulentemperatur: konstant bei 25°C).

### Analytische HPLC-MS, Methode C

Waters ZQ2000 Massenspektrometer (positive Ionisation (ESI+)), HP1100 HPLC (DAD, Wellenlängenbereich: 210 to 500 nm), and Gilson 215 Autosampler.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC-MS, Methode D

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur.

### Analytische HPLC-MS, Methode E

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.20 |
| 0.30 | 95 | 5 | 1.20 |
| 9.00 | 2 | 98 | 1.20 |
| 9.40 | 2 | 98 | 1.20 |
| 9.50 | 95 | 5 | 2.80 |
| 9.90 | 95 | 5 | 2.80 |
| 10.00 | 95 | 5 | 0.20 |

Als stationäre Phase dient eine Säule Merck Chromolith^{™} Flash RP-18e, 4.6 mm x 25 mm (Säulentemperatur: konstant bei 25°C).

### Analytische HPLC, Methode A

Agilent 1100 (Diodenarraydetektion, Wellenlängenbereich: 210-380 nm).
A: Wasser mit 0.10% TFA
B: Acetonitril mit 0.13% TFA

| Zeit in min | %A | %B | Flussrate in ml/min |
|---|---|---|---|
| 0.00 | 95 | 5 | 1.50 |
| 0.60 | 95 | 5 | 1.50 |
| 3.40 | 2 | 98 | 1.50 |
| 3.90 | 2 | 98 | 1.50 |
| 4.20 | 95 | 5 | 1.50 |
| 4.90 | 95 | 5 | 1.50 |

Als stationäre Phase dient eine Säule Varian Microsorb, RP C18, 3 µm, 100 A, Raumtemperatur.

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.
Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur.

### Präparative HPLC, Methode B

Gilson HPLC mit Gilson UV-VIS-155 Detektor, Sampling Injektor 231 XL.
Als Wellenlänge wird das substanzspezifische UV-Maximum angegeben.
A: Wasser mit 0.1% Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode C

Gilson HPLC mit Gilson UV-VIS-155 Detektor, Sampling Injektor 231 XL.
Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.50 | 95 | 5 | 180 |

Als stationäre Phase dient eine Säule Microsorb RP 18, 8 µm, 50 X 150 mm, Raumtemperatur.

### Präparative HPLC, Methode D

Gilson HPLC mit Gilson UV-VIS-155 Detektor, Sampling Injektor 231 XL.
Als Wellenlänge wird das substanzspezifische UV-Maximum angegeben.
A: Wasser mit 0.1% Ammoniak 35%ig
B: Acetonitril

| Zeit in min | %A | %B | Flussrate in ml/min |
|---|---|---|---|
| 0.00 | 95 | 5 | 180 |
| 1.10 | 95 | 5 | 180 |
| 9.00 | 2 | 98 | 180 |
| 10.00 | 2 | 98 | 180 |
| 10.50 | 95 | 5 | 180 |
| 12.00 | 95 | 5 | 180 |

Als stationäre Phase dient eine Säule X-Bridge C18, 5 µm, 50 X 65 mm, Raumtemperatur.

### INDIKATIONSGEBIETE

Wie gefunden wurde, zeichnen sich die erfindungsgemäßen Kombinationen enthaltend einen PDE4-Inhibitor, vorzugsweise einen PDE4-Inhibitor der Formel **1** und wenigstens einen EP4-Rezeptor-Antagonisten durch vielfältige Anwendungsmöglichkeiten auf therapeutischem Gebiet aus. Hervorzuheben sind solche Anwendungsmöglichkeiten, für welche die erfindungsgemäßen Kombinationen aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung der erfindungsgemäßen Kombinationen zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der erfindungsgemäßen Kombinationen zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose, Amyotrophe Lateralsklerose (ALS) oder akute und chronische Schmerzzustände sowie Verletzungen des Gehirns hervorgerufen durch Schlaganfall, Hypoxie oder Schädel-Hirn-Trauma.

Am meisten bevorzugt ist die Verwendung der erfindungsgemäßen Kombinationen zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ein herausragender Aspekt der erfindungsgemäßen Formulierungen enhaltend eine Kombination eines (oder mehrerer) PDE4-Inhibitoren, vorzugsweise der PDE4-Inhibitoren der Formel **1**, und wenigstens eines EP4-Rezeptor-Antagonisten ist deren reduziertes Profil an Nebenwirkungen im Vergleich zu Formulierungen, die die gleichen PDE4-Inhibitoren in gleicher Menge in Abwesenheit eines EP4-Rezeptor-Antagonisten enthalten. Nebenwirkungen, die bei der Verabreichung eines PDE4-Inhibitors häufig auftreten, sind unter anderen vorzugsweise Diarrhoe, Übelkeit und Erbrechen. Im Rattenmodell konnten noch weitere Nebenwirkungen nach PDE4-Inhibitor-Verabreichung beobachtet werden wie beispielsweise Körpergewichtsverlust, Verlust des Milzgewichts, Leukozytose und Neutrophilie, Diarrhoe und das Auftreten von mesenterer Vaskulitis.

Unter einem reduzierten Profil an Nebenwirkungen wird im Rahmen der Erfindung insbesondere verstanden, eine therapeutisch wirksame Dosis eines PDE4-Inhibitors in einer der erfindungsgemäßen pharmazeutischen Zusammensetzung verabreichen zu können, ohne beim Patienten die oder zumindest eine der häufig bei der Verabreichung von PDE4-Inhibitoren beobachteten Nebenwirkungen (Diarrhoe, Übelkeit, Erbrechen, Körpergewichtsverlust, Verlust des Milzgewichts, Leukozytose und Neutrophilie und das Auftreten von mesenterer Vaskulitis) in nennenswertem Ausmaß auszulösen. Besonders bevorzugt ist die Verabreichung einer therapeutisch wirksamen Substanzmenge eines PDE4-Inhibitors in der erfindungsgemäßen pharmazeutischen Zusammensetzung in jedem Stadium des Krankheitsverlaufs, ohne die typischen PDE4-Inhibitor-vermittelten Nebenwirkungen der Diarrhoe, des Körpergewichtsverlustes, der Leukozytose, der Neutrophilie oder der mesenteren Vaskulitis auszulösen. Ein besonderer Gegenstand der vorliegenden Erfindung betrifft die Verabreichung einer therapeutisch wirksamen Substanzmenge der erfindungsgemäßen pharmazeutischen Zusammensetzung in jedem Stadium des Krankheitsverlaufs, ohne die typische PDE4-Inhibitor-vermittelte Nebenwirkung der mesenteren Vaskulitis in nennenswertem Ausmaß auszulösen.

Im folgenden konnte experimentell am Rattenmodell gezeigt werden, dass die erfindungsgemäßen pharmazeutischen Zusammensetzungen enthaltend einen PDE4-Inhibitor und wenigstens einen EP4-Rezeptor-Antagonisten viele der Nebenwirkungen, die bei der Verabreichung des entsprechenden PDE4-Inhibitors alleine auftreten, erheblich reduzieren oder sogar völlig vermeiden.

### EXPERIMENTELLE DURCHFÜHRUNG:

### Versuch 1: Diclofenac schützt vor Roflumilast-vermittelten Effekten wie Körperaewichtsverlust, Milzgewichtsverlust, Neutrophilie und mesentere Vaskulitis:

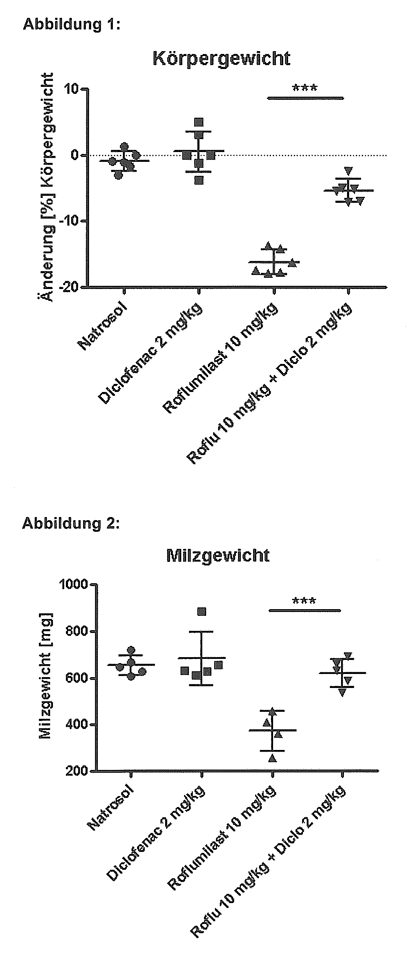

Jeweils sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Diclofenac-Gruppe"): Sechs männliche Wistar-Ratten erhielten eine tägliche Applikation von jeweils 1 mg/kg Diclofenac (NSAID) zu den Zeitpunkten 8 Uhr und 17 Uhr und von 0.5% Natrosol (Placebo) um 13 Uhr.
Gruppe 3 ("Roflumilast-Gruppe"): Sechs männliche Wistar-Ratten erhielten eine tägliche Applikation von 0.5% Natrosol (Placebo) jeweils zu den Zeitpunkten 8 Uhr und 17 Uhr und von 10 mg/kg Roflumilast (PDE4-Inhibitor) um 13 Uhr.
Gruppe 4 ("Roflumilast + Diclofenac-Gruppe"): Sechs männliche Wistar-Ratten erhielten eine tägliche Applikation von jeweils 1 mg/kg Diclofenac (NSAID) zu den Zeitpunkten 8 Uhr und 17 Uhr und von 10 mg/kg Roflumilast (PDE4-Inhibitor) um 13 Uhr.
Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung. Das gleiche traf für eine Ratte aus der Roflumilast-Gruppe zu, die zwischen Versuchstag 4 und 5 verstarb.
Während des Versuchs wurden die Körpergewichte der Tiere bestimmt und die Unterschiede der Körpergewichte der Ratten aus den verschiedenen Gruppen zum Versuchsende als Veränderung in % bezogen auf den 1. Applikationszeitpunkts (= Tag 1, 8 Uhr (= Zeitpunkt t₀)) dargestellt. Der Mittelwert ± Standardabweichung der Körpergewichte zum Zeitpunkt t₀ betrug 355 ± 17 g.
Am Versuchsende (Tag 5, 95 Stunden nach t₀ (t₀ = Zeitpunkt der ersten Applikation am Tag 1, 8 Uhr)) wurden aus dem Blut von 4 bzw. 5 der Ratten aus den einzelnen Gruppen der Anteil der Neutrophilen (in % der Weißen Blutzellen, Abbildung 2) bestimmt. Desweiteren wurden die Milzgewichte der Tiere gemessen und die Mesenterien für eine histopathologische Untersuchung auf Vaskulitis (multifokale perivaskuläre mononukleäre/ polymorphnukleäre Infiltration) präpariert.
- Abbildung 1: zeigt die Änderungen des gemessenen Körpergewichts nach den jeweiligen Applikationen bei der Kontroll (Natrosol-)-Gruppe, der Diclofenac-Gruppe, der Roflumilast-Gruppe und der Roflumilast + Diclofenac-Gruppe (Statistik: Two-way analysis of variance; *** = p < 0.001).
- Abbildung 2: zeigt die Änderungen des gemessenen Milzgewichtes nach den jeweiligen Applikationen bei der Kontroll (Natrosol-)-Gruppe, der Diclofenac-Gruppe, der Roflumilast-Gruppe und der Roflumilast + Diclofenac-Gruppe (Statistik: Two-way analysis of variance; *** = p < 0.001).
- Abbildung 3: zeigt den Prozentsatz der Neutrophilen im Blut nach den jeweiligen Applikationen bei der Kontroll (Natrosol-)-Gruppe, der Diclofenac-Gruppe, der Roflumilast-Gruppe und der Roflumilast + Diclofenac-Gruppe (Statistik: Two-way analysis of variance; *** = p < 0.001).
- Abbildung 4: zeigt die Erhöhung der beobachteten Vaskulitis im Mesenterium nach den jeweiligen Applikationen bei der Kontroll (Natrosol-)-Gruppe, der Diclofenac-Gruppe, der Roflumilast-Gruppe und der Roflumilast + Diclofenac-Gruppe (Statistik: Two-way analysis of variance; *** = p < 0.001).

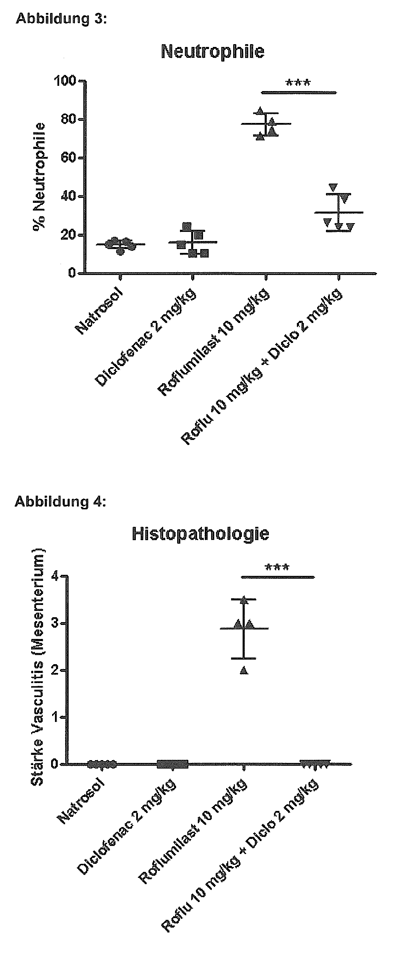

Zusammenfassend lässt sich sagen, dass die in der Roflumilast-Gruppe beobachteteten PDE4-Inhibitor-vermittelten Nebenwirkungen wie Körpergewichtsverlust (Abb. 1), Milzgewichtsverlust (Abb. 2), Neutrophilie (Abb. 3) und Mesentere Vaskulitis (monozytäre / polymorphnukleäre Infiltration im Mesenterium, Abb. 4) erheblich reduziert oder verhindert werden können (häufig sogar Reduktion fast auf den Wert der Kontroll-Gruppe), indem man gleichzeitig bzw. nur um einige Stunden versetzt, ein NSAID wie beispielsweise Diclofenac koappliziert (siehe Roflumilast + Diclofenac-Gruppe). Die gemessenen Parameter nach Applikation von Diclofenac alleine zeigten sich sehr ähnlich zu den Kontrollgruppen.

Einerseits hat bei einer Therapie von COPD- und Asthma-Patienten mit einem PDE4-Inhibitor die gleichzeitige Verabreichung eines NSAIDs wie beispielsweise Diclofenac folglich große Vorteile, da durch das NSAID die PDE4-Rezeptor-vermittelten Nebenwirkungen erheblich reduziert oder sogar verhindert werden können. Andererseits, ist seit langem bekannt, dass NSAIDs wie Diclofenac bei regelmäßiger Einnahme wiederum eigene Nebenwirkungen zeigen, wie beispielsweise starke gastrointestinale Nebenwirkungen, insbesondere die Ausbildung von Magengeschwüren. Eine regelmäßige Einnahme dieser NSAIDs wäre jedoch notwendig zur Reduktion der PDE4-vermittelten Nebenwirkungen, da die Therapie von COPD- und Asthma-Patienten mit PDE4-Inhibitoren in der Regel eine Dauertherapie darstellt. Folglich stellt sich also die Frage nach Alternativen zur PDE4-Inhibitor/ NSAID-Kombinationstherapie, die ein geringeres Nebenwirkungsprofil aufweisen.

### Versuch 2: Der COX-2 selektive Inhibitor Lumiracoxib, aber nicht der COX-1 selektive Inhibitor SC-560, schützt vor Roflumilast-vermittelten Effekten wie Körpergewichtsverlust, Milzgewichtsverlust, Neutrophilie und mesentere Vaskulitis:

Jeweils sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("SC-560-Gruppe"): Sechs männliche Wistar-Ratten erhielten eine tägliche Applikation von jeweils 2 mg/kg SC-560 (NSAID, selektiv für Cox-1) zu den Zeitpunkten 8 Uhr und 17 Uhr und von 0.5% Natrosol um 13 Uhr.
Gruppe 3 ("Lumiracoxib-Gruppe"): Sechs männliche Wistar-Ratten erhielten eine tägliche Applikation von jeweils 2 mg/kg Lumiracoxib (NSAID, selektiv für Cox-2) zu den Zeitpunkten 8 Uhr und 17 Uhr und von 0.5% Natrosol um 13 Uhr.
Gruppe 4 ("Roflumilast-Gruppe"): Sechs männliche Wistar-Ratten erhielten eine tägliche Applikation von 0.5% Natrosol (Placebo) jeweils zu den Zeitpunkten 8 Uhr und 17 Uhr und von 10 mg/kg Roflumilast (PDE4-Inhibitor) um 13 Uhr.
Gruppe 5 ("Roflumilast + SC-560-Gruppe"): Sechs männliche Wistar-Ratten erhielten eine tägliche Applikation von jeweils 2 mg/kg SC-560 (NSAID, selektiv für COX-1) zu den Zeitpunkten 8 Uhr und 17 Uhr und von 10 mg/kg Roflumilast (PDE4-Inhibitor) um 13 Uhr.
Gruppe 6 ("Roflumilast + Lumiracoxib-Gruppe"): Sechs männliche Wistar-Ratten erhielten eine tägliche Applikation von jeweils 2 mg/kg Lumiracoxib (NSAID, selektiv für COX-2) zu den Zeitpunkten 8 Uhr und 17 Uhr und von 10 mg/kg Roflumilast (PDE4-Inhibitor) um 13 Uhr. Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.
Während des Versuchs wurden die Körpergewichte der Tiere bestimmt und die Unterschiede der Körpergewichte der Ratten aus den verschiedenen Gruppen zum Versuchsende als Veränderung in % bezogen auf den 1. Applikationszeitpunkts (= Tag 1, 8 Uhr (= Zeitpunkt t₀)) dargestellt. Der Mittelwert ± Standardabweichung der Körpergewichte zum Zeitpunkt t₀ betrug 306 ± 11 g.
Am Versuchsende (Tag 5, 95 Stunden nach t₀ (t₀ = Zeitpunkt der ersten Applikation am Tag 1, 8 Uhr)) wurden aus dem Blut von 4 bzw. 5 der Ratten aus den einzelnen Gruppen der Anteil der Neutrophilen (in % der Weißen Blutzellen, Abbildung 2) bestimmt. Desweiteren wurden die Milzgewichte der Tiere gemessen und die Mesenterien für eine histopathologische Untersuchung auf Vaskulitis (multifokale perivaskuläre mononukleäre/ polymorphnukleäre Infiltration) präpariert.

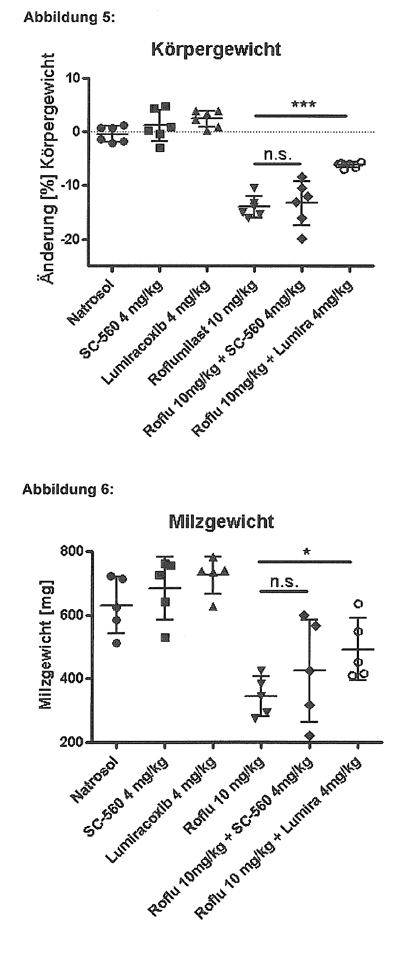

- Abbildung 5: zeigt die Änderung des gemessenen Körpergewichts nach den jeweiligen Applikationen bei der Kontroll (Natrosol-)-Gruppe, der SC 560-Gruppe, der Lumiracoxib-Gruppe, der Roflumilast-Gruppe, der Roflumilast + SC 560-Gruppe und der Roflumilast + Lumiracoxib-Gruppe (Statistik: One-way analysis of variance; ns = nicht signifikant; * = p< 0.05;***= p < 0.001).
- Abbildung 6: zeigt die Änderung des gemessenen Milzgewichts nach den jeweiligen Applikationen bei der Kontroll (Natrosol-)-Gruppe, der SC 560-Gruppe, der Lumiracoxib-Gruppe, der Roflumilast-Gruppe, der Roflumilast + SC 560-Gruppe und der Roflumilast + Lumiracoxib-Gruppe (Statistik: One-way analysis of variance; ns = nicht signifikant; * = p< 0.05; ***= p < 0.001).

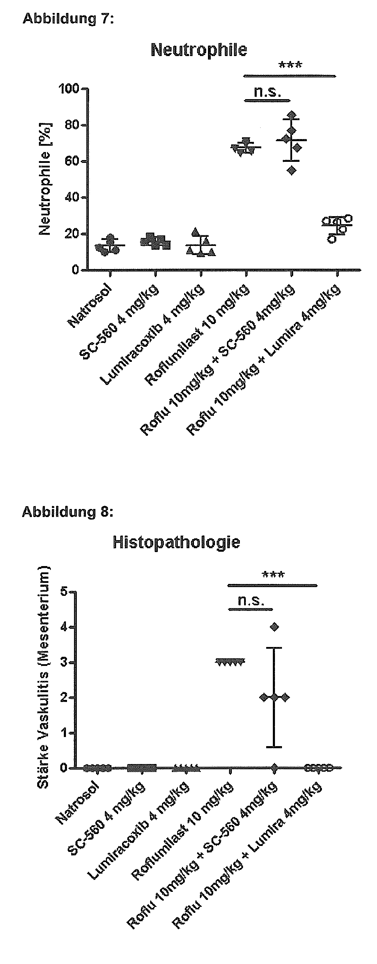

- Abbildung 7: zeigt den Prozentsatz der gemessenen Neutrophile im Blut nach den jeweiligen Applikationen bei der Kontroll (Natrosol-)-Gruppe, der SC 560-Gruppe, der Lumiracoxib-Gruppe, der Roflumilast-Gruppe, der Roflumilast + SC 560-Gruppe und der Roflumilast + Lumiracoxib-Gruppe (Statistik: One-way analysis of variance; ns = nicht signifikant; * = p< 0.05;***= p < 0.001).
- Abbildung 8: zeigt die Erhöhung der beobachteten Vaskulitis im Mesenterium nach den jeweiligen Applikationen bei der Kontroll (Natrosol-)-Gruppe, der SC 560-Gruppe, der Lumiracoxib-Gruppe, der Roflumilast-Gruppe, der Roflumilast + SC 560-Gruppe und der Roflumilast + Lumiracoxib-Gruppe (Statistik: One-way analysis of variance; ns = nicht signifikant; * = p< 0.05; ***= p < 0.001).

Zusammenfassend lässt sich sagen, dass die in der Roflumilast-Gruppe beobachteteten PDE4-Inhibitor-vermittelten Nebenwirkungen wie Körpergewichtsverlust (Abb. 5), Milzgewichtsverlust (Abb. 6), Neutrophilie (Abb. 7) und mesentere Vaskulitis (monozytäre / polymorphnukleäre perivaskuläre Infiltration im Mesenterium als Maß für eine Vaskulitis, Abb. 8) erheblich reduziert oder verhindert werden können (häufig sogar Reduktion fast auf den Wert der Kontroll-Gruppe), indem man gleichzeitig bzw. nur um einige Stunden versetzt, ein COX-2 selektives NSAID wie Lumiracoxib koappliziert (siehe Roflumilast + Lumiracoxib). Der COX-1 selektive NSAID SC-560 hat keinerlei protektive Wirkung auf Körpergewichtsverlust, Milzgewichtsverlust, Neutrophilie und die monozytäre / polymorphnukleäre perivaskuläre Infiltration als Maß für eine Vaskulitis. Die gemessenen Parameter nach Applikation von SC-560 oder von Lumiracoxib alleine zeigten sich sehr ähnlich zu den Kontrollgruppen.

Insgesamt lässt sich schlussfolgern, dass die protektive Wirkung eines NSAIDs auf die PDE4-Inhibitor-vermittelten Nebenwirkungen auf der Hemmung von COX-2 beruhen.

Einerseits hat bei einer Therapie von COPD- und Asthma-Patienten mit einem PDE4-Inhibitor die gleichzeitige Verabreichung eines COX-2-Inhibitors wie beispielsweise Lumiracoxib folglich große Vorteile, da durch das NSAID die PDE4-Rezeptor-vermittelten Nebenwirkungen erheblich reduziert oder sogar verhindert werden können und andererseits keine starken gastrointestinalen Nebenwirkungen durch den COX-2-Inhibitor zu erwarten sind (wie dies bei NSAIDs der Fall ist). Andererseits, ist jedoch seit langem bekannt, dass COX-2-Inhibitoren wie Lumiracoxib bei regelmäßiger Einnahme kardiovaskuläre Nebenwirkungen (Herzinfarkt, Thrombosen, Schlaganfall) zeigen (siehe Clark et al; The Journal of Pharmacology and Experimental Therapeutics; 325; p. 425-434). Eine regelmäßige Einnahme dieser NSAIDs wären jedoch notwendig zur Reduktion der PDE4-vermittelten Nebenwirkungen, da die Therapie von COPD- und Asthma-Patienten mit PDE4-Inhibitoren in der Regel eine Dauertherapie darstellt. Folglich stellt sich also die Frage nach Alternativen zur PDE4-Inhibitor/COX-2-Inhibitor-Kombinationstherapie, die ein geringeres Nebenwirkungsprofil aufweisen.

### Versuch 3: Der EP4-Rezeptor-selektive Inhibitor MF498 (N-{[4-(5.9-Diethoxy-6-oxo-6,8-dihydro-7H-pyrrolo(3.4-g)quinolin-7-yl)-3-methylbenzyl)sulfonyl}-2-(2-metoxyphenyl) acetamide, auch bezeichnet als Verbindung 2.1) schützt vor Roflumilast-vermittelten Effekten wie Körpergewichtsverlust, Milzgewichtsverlust, Neutrophilie und mesentere Vaskulitis:

Jeweils sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zum Zeitpunkt 13 Uhr.
Gruppe 2 ("MF-498-Gruppe"): Sechs männliche Wistar-Ratten erhielten eine tägliche Applikation von 20 mg/kg MF-498 (EP4 Antagonist) jeweils zum Zeitpunkt 13 Uhr.
Gruppe 3 ("Roflumilast-Gruppe"): Sechs männliche Wistar-Ratten erhielten eine tägliche Applikation von 10 mg/kg Roflumilast (PDE4-Inhibitor) um 13 Uhr.
Gruppe 4 ("Roflumilast + MF-498-Gruppe"): Sechs männliche Wistar-Ratten erhielten eine tägliche Applikation von 20 mg/kg MF-498 (EP4 Antagonist) und von 10 mg/kg Roflumilast (PDE4-Inhibitor) zum Zeitpunkt 13 Uhr.
Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.
Während des Versuchs wurden die Körpergewichte der Tiere bestimmt und die Unterschiede der Körpergewichte der Ratten aus den verschiedenen Gruppen zum Versuchsende als Veränderung in % bezogen auf den 1. Applikationszeitpunkts (= Tag 1, 8 Uhr (= Zeitpunkt t₀)) dargestellt. Der Mittelwert ± Standardabweichung der Körpergewichte zum Zeitpunkt t₀ betrug 283 ± 6 g.
Am Versuchsende (Tag 5, 95 Stunden nach t₀ (t₀ = Zeitpunkt der ersten Applikation am Tag 1, 8 Uhr)) wurden aus dem Blut von 4 bzw. 5 der Ratten aus den einzelnen Gruppen der Anteil der Neutrophilen (in % der Weißen Blutzellen, Abbildung 2) bestimmt. Desweiteren wurden die Milzgewichte der Tiere gemessen und die Mesenterien für eine histopathologische Untersuchung auf Vaskulitis (multifokale perivaskuläre mononukleäre/ polymorphnukleäre Infiltration) präpariert.

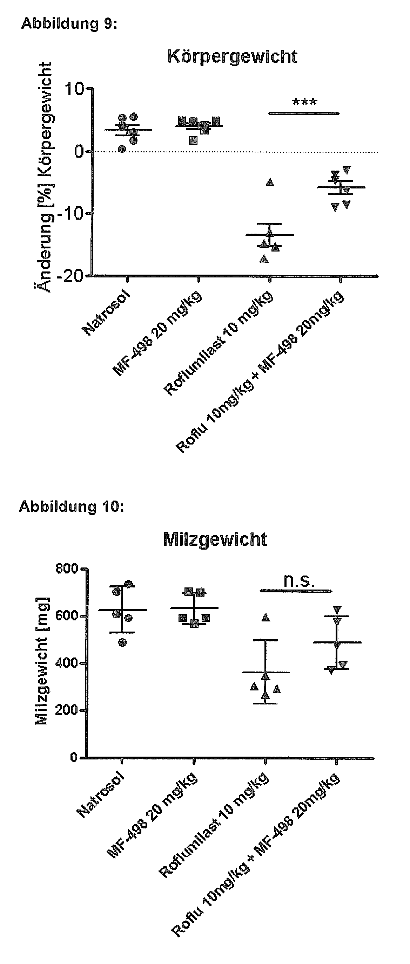

- Abbildung 9: zeigt die Änderung des gemessenen Körpergewichts nach den jeweiligen Applikationen in der Kontroll (Natrosol-)-Gruppe, der MF-498-Gruppe, der Roflumilast-Gruppe und der Roflumilast + MF-498-Gruppe (Statistik: Two-way analysis of variance; ns = nicht signifikant; ** = p < 0.01, *** = p < 0.001).
- Abbildung 10: zeigt die Änderung des gemessenen Milzgewichts nach den jeweiligen Applikationen in der Kontroll (Natrosol-)-Gruppe, der MF-498-Gruppe, der Roflumilast-Gruppe und der Roflumilast + MF-498-Gruppe (Statistik: Two-way analysis of variance; ns = nicht signifikant; ** = p < 0.01, *** = p < 0.001).

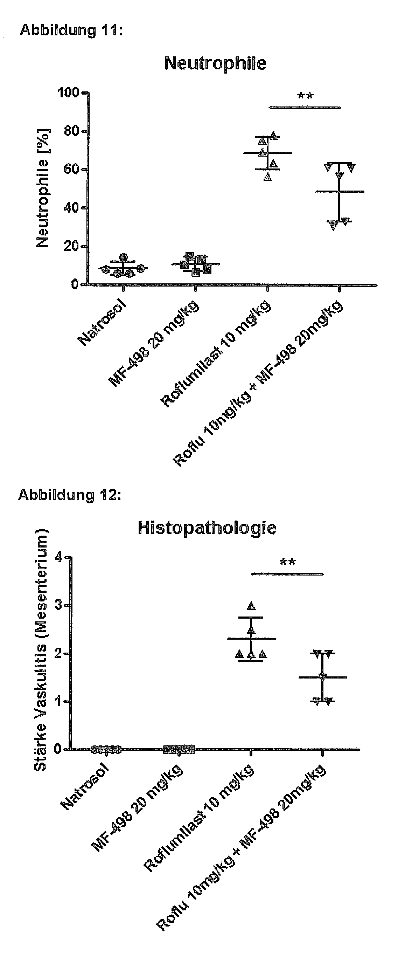

- Abbildung 11: zeigt den Prozentsatz der Neutrophilen im Blut nach den jeweiligen Applikationen in der Kontroll (Natrosol-)-Gruppe, der MF-498-Gruppe, der Roflumilast-Gruppe und der Roflumilast + MF-498-Gruppe (Statistik: Two-way analysis of variance; ns = nicht signifikant; ** = p < 0.01, *** = p < 0.001).
- Abbildung 12: zeigt die Erhöhung der beobachteten Vaskulitis im Mesenterium nach den jeweiligen Applikationen in der Kontroll (Natrosol-)-Gruppe, der MF-498-Gruppe, der Roflumilast-Gruppe und der Roflumilast + MF-498-Gruppe (Statistik: Two-way analysis of variance; ns = nicht signifikant; ** = p < 0.01, *** = p < 0.001).

Zusammenfassend lässt sich sagen, dass die in der Roflumilast-Gruppe beobachteteten PDE4-Inhibitor-vermittelten Nebenwirkungen wie Körpergewichtsverlust (Abb. 9), Milzgewichtsverlust (Abb. 10), Neutrophilie (Abb. 11) und mesentere Vaskulitis (monozytäre / polymorphnukleäre perivaskuläre Infiltration im Mesenterium als Maß für eine Vaskulitis, Abb. 12) erheblich reduziert werden können, indem man gleichzeitig einen selektiven EP4 Antagonisten koappliziert (siehe Roflumilast + MF-498). Die Unterschiede im Milzgewicht zwischen der "Roflumilast-" und der "Roflumilast + MF498-Gruppe" erreichen keine statistische Signifikanz, da in diesem Experiment ein Tier einen ungewöhnlich geringen Verlust an Milzgewicht in der "Roflumilast-Gruppe" zeigte. Die gemessenen Parameter nach Applikation von MF-498 alleine zeigten sich sehr ähnlich zur Kontrollgruppe.
Insgesamt lässt sich schlussfolgern, dass die beobachtete protektive Wirkung eines NSAIDs bzw. eines COX-2-Inhibitors auf die PDE4-Inhibitor-vermittelten Nebenwirkungen, zumindest teilweise auf die Verringerung der Prostaglandin E2 Synthese des COX-2 Enzyms beruhen, welches im weiteren Verlauf der Signalkette die Nebenwirkungen über den EP4 Rezeptor vermittelt. Daher lassen sich die Nebenwirkungen ebenso durch eine Blockade des EP4 Rezeptors verringern. Wichtig ist in diesem Zusammenhang, daß der EP4 Rezeptor selbst wieder über eine Steigerung des Botenmoleküls cAMP in die Zelle signalisiert und cAMP das Substrat für die PDE4 Enzyme sind, welche durch Roflumilast inhibiert werden. Daher kommt es zu einem doppelten Einfluss des PDE4-Inhibitors Roflumilast einmal durch die Steigerung der Expression der Cyclooxygenase-2, damit einhergehend einer stärkeren Produktion von Prostaglandin E2 (PGE2) sowie einer gleichzeitigen Verstärkung des PGE2/EP4-vermittelten Signals (cAMP) in den betroffenen Zellen durch eine Verhinderung des cAMP Abbaus.

### Versuch 4: Der EP2-Rezeptor-präferentielle Inhibitor AH6809 (6-isopropoxy-9-oxoxanthene-2-carboxylic acid), schützt nicht vor Roflumilast-vermittelten Effekten wie Körperaewichtsverlust, Milzgewichtsverlust, Neutrophilie und mesentere Vaskulitis:

Jeweils sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zum Zeitpunkt 13 Uhr.
Gruppe 2 ("AH-6809-Gruppe"): Sechs männliche Wistar-Ratten erhielten eine tägliche Applikation von 10 mg/kg AH-6809 (präferentieller EP2 Antagonist) zum Zeitpunkt 13 Uhr.
Gruppe 3 ("Roflumilast-Gruppe"): Sechs männliche Wistar-Ratten erhielten eine tägliche Applikation von 10 mg/kg Roflumilast (PDE4-Inhibitor) zum Zeitpunkt 13 Uhr.
Gruppe 4 ("Roflumilast + AH-6809-Gruppe"): Sechs männliche Wistar-Ratten erhielten eine tägliche Applikation von 10 mg/kg AH-6809 (präferentieller EP2 Antagonist) und von 10 mg/kg Roflumilast (PDE4-Inhibitor) zum Zeitpunkt 13 Uhr.
Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.
Während des Versuchs wurden die Körpergewichte der Tiere bestimmt und die Unterschiede der Körpergewichte der Ratten aus den verschiedenen Gruppen zum Versuchsende als Veränderung in % bezogen auf den 1. Applikationszeitpunkts (= Tag 1, 8 Uhr (= Zeitpunkt t₀)) dargestellt. Der Mittelwert ± Standardabweichung der Körpergewichte zum Zeitpunkt t₀ betrug 284 ± 9 g.
Am Versuchsende (Tag 5, 95 Stunden nach t₀ (t₀ = Zeitpunkt der ersten Applikation am Tag 1, 8 Uhr)) wurden aus dem Blut von 4 bzw. 5 der Ratten aus den einzelnen Gruppen der Anteil der Neutrophilen (in % der Weißen Blutzellen, Abbildung 2) bestimmt. Desweiteren wurden die Milzgewichte der Tiere gemessen und die Mesenterien für eine histopathologische Untersuchung auf Vaskulitis (multifokale perivaskuläre mononukleäre/ polymorphnukleäre Infiltration) präpariert.

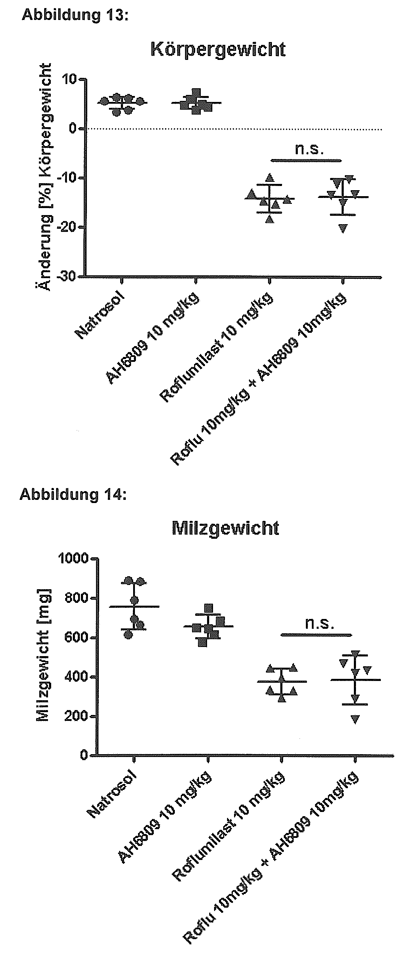

- Abbildung 13: zeigt die Änderung des gemessenen Körpergewichts nach den jeweiligen Applikationen in der Kontroll (Natrosol-)-Gruppe, der AH6809-Gruppe, der Roflumilast-Gruppe und der Roflumilast + AH6809-Gruppe (Statistik: Two-way analysis of variance; ns = nicht signifikant).
- Abbildung 14: zeigt die Änderung des gemessenen Milzgewichts nach den jeweiligen Applikationen in der Kontroll (Natrosol-)-Gruppe, der AH6809-Gruppe, der Roflumilast-Gruppe und der Roflumilast + AH6809-Gruppe (Statistik: Two-way analysis of variance; ns = nicht signifikant).

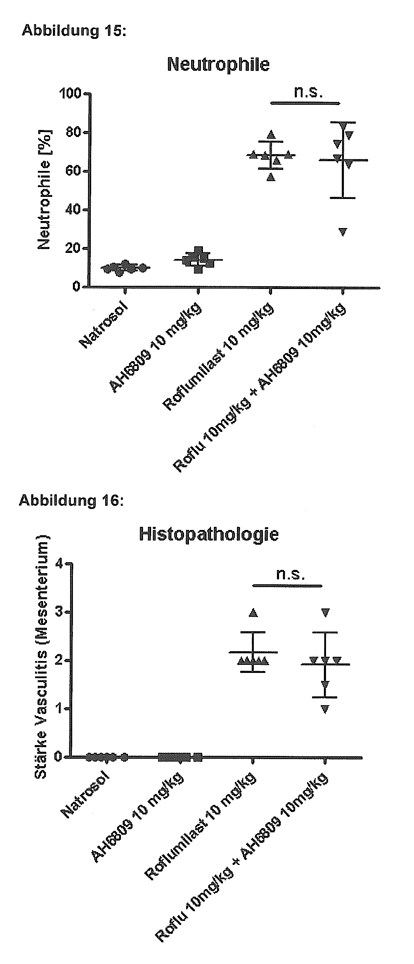

- Abbildung 15: zeigt den Prozentsatz der Neutrophilen im Blut nach den jeweiligen Applikationen in der Kontroll (Natrosol-)-Gruppe, der AH6809-Gruppe, der Roflumilast-Gruppe und der Roflumilast + AH6809-Gruppe (Statistik: Two-way analysis of variance; ns = nicht signifikant).
- Abbildung 16: zeigt die Erhöhung der beobachteten Vaskulitis im Mesenterium nach den jeweiligen Applikationen in der Kontroll (Natrosol-)-Gruppe, der AH6809-Gruppe, der Roflumilast-Gruppe und der Roflumilast + AH6809-Gruppe (Statistik: Two-way analysis of variance; ns = nicht signifikant).

Zusammenfassend lässt sich sagen, dass kein Einfluß des EP2 preferentiellen Rezeptor Antagonisten AH-6809 auf die in der Roflumilast-Gruppe beobachteteten PDE4-Inhibitor-vermittelten Nebenwirkungen wie Körpergewichtsverlust (Abb. 13), Milzgewichtsverlust (Abb. 14), Neutrophilie (Abb. 15) und mesentere Vaskulitis (monozytäre / polymorphnukleäre perivaskuläre Infiltration im Mesenterium als Maß für eine Vaskulitis, Abb. 16) zu beobachten war. Die gemessenen Parameter nach Applikation von AH-6809 alleine zeigten sich sehr ähnlich zur Kontrollgruppe.
Insgesamt lässt sich schlussfolgern, dass die beobachtete protektive Wirkung eines NSAIDs bzw. eines COX-2-Inhibitors auf die PDE4-Inhibitor-vermittelten Nebenwirkungen, zumindest teilweise auf die Verringerung der Prostaglandin E2 Synthese des COX-2 Enzyms beruhen, welches im weiteren Verlauf der Signalkette die Nebenwirkungen über den EP4 Rezeptor vermittelt. Daher lassen sich die Nebenwirkungen ebenso durch eine Blockade des EP4 Rezeptors verringern. Wichtig ist in diesem Zusammenhang, daß der EP4 Rezeptor selbst wieder über eine Steigerung des Botenmoleküls cAMP in die Zelle signalisiert und cAMP das Substrat für die PDE4 Enzyme sind, welche durch Roflumilast inhibiert werden. Daher kommt es zu einem doppelten Einfluss des PDE4-Inhibitors Roflumilast einmal durch die Steigerung der Expression der Cyclooxygenase-2, damit einhergehend einer stärkeren Produktion von Prostaglandin E2 (PGE2) sowie einer gleichzeitigen Verstärkung des PGE2/EP4-vermittelten Signals (cAMP) in den betroffenen Zellen durch eine Verhinderung des cAMP Abbaus. Eine Blockade des zweiten cAMP-gekoppelten Prostaglandin E2 Rezeptors EP2 durch den EP2 präferentiellen Antagonisten AH-6809 zeigt in diesem Zusammenhang keine Auswirkung auf die gemessenen Parameter.

Bei einer Therapie von COPD- und Asthma-Patienten mit einem PDE4-Inhibitor hat also die gleichzeitige Verabreichung eines EP4-Rezeptor-Antagonisten wie beispielsweise MP498 große Vorteile, da einerseits durch diesen EP4-Rezeptor-Antagonisten die PDE4-Rezeptor-vermittelten Nebenwirkungen erheblich reduziert oder sogar verhindert werden können und andererseits bei Gabe eines EP4-Rezeptor-Antagonisten, im Gegensatz zu NSAID und zu COX-2-Inhibitoren, keinerlei nennenswerte eigene Nebenwirkungen - auch bei einer Dauertherapie - zu erwarten sind.

### DARREICHUNGSFORMEN

Die Applikation der erfindungsgemäßen Wirkstoffkombinationen aus **1** und **2** erfolgt bevorzugt auf oralem Wege. Hierzu müssen die Bestandteile (**1**) und (**2**) in geeigneten oralen Darreichungsformen bereitgestellt werden.

Geeignete orale Darreichungsformen sind beispielsweise Tabletten, Kapseln, Lösungen, Säfte, Emulsionen. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen.

Besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat, mikrokristalliner Cellulose, Sorbitol, Mannitol, Isomaltose oder Milchzucker, Sprengmitteln, wie Maisstärke, quervernetztes Polyvinylpyrrolidon, quervernetzte Natrium Carboxymethylcellulose, Natriumstärkeglykolat oder Alginsäure, Bindemitteln, wie Stärke, Hydroxypropylmethylcellulose, Polyvinylpyrrolidon oder Gelatine, Schmiermitteln, wie Magnesiumstearat oder Talk, und/oder Mitteln zur Erzielung des Depoteffektes, wie Hydroxypropylcellulose, Hydroxypropylmethylcellulose, Ethylcellulose, Amminomethacrylat, Polyvinylpyrrolidon-Polyvinylacetat Copolymer, Carboxymethylcellulose oder Polyvinylacetat erhalten werden. Die Tabletten können auch aus mehreren Schichten bestehen.

Entsprechend können Dragees bzw. Filmtabletten durch Überziehen von analog den Tabletten hergestellten Kernen mit üblicherweise in Dragee- bzw. Filmüberzügen verwendeten Mitteln, beispielsweise Kollidon oder Schellack, Gummi arabicum, Talk, Titandioxid, Zucker, Hydroxypropylmethylcellulose, Ehthycellulose, Celluloseacetatphthalat, Polymethacrylate, Polyethylenglycol, Polyvinylalcohol, Polyvinylalkohol-Polyethylenglycol Copolymere oder Polyvinylacetat hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

### Formulierungsbeispiele:

Die folgenden Formulierungsbeispiele für Kombinationsformulierungen sollen der besseren Illustration der Erfindung dienen, ohne jedoch diese darauf zu beschränken. Insbesondere können die Wirkstoffe **1** und **2** auch in voneinander getrennten Formulierungen vorliegen und innerhalb eines Zeitfensters von maximal 6 Stunden versetzt voneinander getrennt verabreicht werden.

| | | | |
|---|---|---|---|
| 1) | 0,10 | mg | Wirkstoff **1** |
| | 5,00 | mg | Wirkstoff **2.1** |
| | 25,00 | mg | Milchzucker |
| | 202,40 | mg | Microkristalline Cellulose |
| | 7,50 | mg | Polyvinylpyrrolidon |
| | 7,50 | mg | quervernetztes Polyvinylpyrrolidon |
| | 2,50 | mg | Magnesiumstearat |
| | 250 mg | | |

| | | | |
|---|---|---|---|
| 2) | 0,10 | mg | Wirkstoff **1** |
| | 6,00 | mg | Wirkstoff **2.1** |
| | 25,00 | mg | Milchzucker |
| | 201,40 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 3) | 0,10 | mg | Wirkstoff **1** |
| | 7,00 | mg | Wirkstoff **2.1** |
| | 25,00 | mg | Milchzucker |
| | 200,40 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 4) | 0,10 | mg | Wirkstoff **1** |
| | 8,00 | mg | Wirkstoff **2.1** |
| | 25,00 | mg | Milchzucker |
| | 199,40 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 5) | 0,10 | mg | Wirkstoff **1** |
| | 9,00 | mg | Wirkstoff **2.1** |
| | 25,00 | mg | Milchzucker |
| | 198,40 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |
| 6) | 0,50 | mg | Wirkstoff **1** |
| | 25,00 | mg | Wirkstoff **2.1** |
| | 25,00 | mg | Milchzucker |
| | 182,00 | mg | Microkristalline Cellulose |
| | 7,50 | mg | Polyvinylpyrrolidon |
| | 7,50 | mg | quervernetztes Polyvinylpyrrolidon |
| | 2,50 | mg | Magnesiumstearat |
| | 250 mg | | |

| | | | |
|---|---|---|---|
| 7) | 0,50 | mg | Wirkstoff **1** |
| | 30,00 | mg | Wirkstoff **2.1** |
| | 25,00 | mg | Milchzucker |
| | 177,00 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 8) | 0,50 | mg | Wirkstoff **1** |
| | 35,00 | mg | Wirkstoff **2.1** |
| | 25,00 | mg | Milchzucker |
| | 172,00 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 9) | 0,50 | mg | Wirkstoff **1** |
| | 40,00 | mg | Wirkstoff **2.1** |
| | 25,00 | mg | Milchzucker |
| | 167,00 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 10) | 0,50 | mg | Wirkstoff **1** |
| | 45,00 | mg | Wirkstoff **2.1** |
| | 25,00 | mg | Milchzucker |
| | 162,00 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 11) | 1,00 | mg | Wirkstoff **1** |
| | 50,00 | mg | Wirkstoff **2.1** |
| | 25,00 | mg | Milchzucker |
| | 156,50 | mg | Microkristalline Cellulose |
| | 7,50 | mg | Polyvinylpyrrolidon |
| | 7,50 | mg | quervernetztes Polyvinylpyrrolidon |
| | 2,50 | mg | Magnesiumstearat |
| | 250 mg | | |

| | | | |
|---|---|---|---|
| 12) | 1,00 | mg | Wirkstoff **1** |
| | 60,00 | mg | Wirkstoff **2.1** |
| | 25,00 | mg | Milchzucker |
| | 146,50 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 13) | 1,00 | mg | Wirkstoff **1** |
| | 70,00 | mg | Wirkstoff **2.1** |
| | 25,00 | mg | Milchzucker |
| | 136,50 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 14) | 1,00 | mg | Wirkstoff **1** |
| | 80,00 | mg | Wirkstoff **2.1** |
| | 25,00 | mg | Milchzucker |
| | 126,50 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 15) | 1,00 | mg | Wirkstoff **1** |
| | 90,00 | mg | Wirkstoff **2.1** |
| | 25,00 | mg | Milchzucker |
| | 116,50 | mg | Microkristalline Cellulose |
| | 7,50 | mg | quervernetztes Polyvinylpyrrolidon |
| | 7,50 | mg | Polyvinylpyrrolidon |
| | 2,50 | mg | Magnesiumstearat |
| | 250 mg | | |

| | | | |
|---|---|---|---|
| 16) | 5,00 | mg | Wirkstoff **1** |
| | 250,00 | mg | Wirkstoff **2.1** |
| | 50,00 | mg | Milchzucker |
| | 160,00 | mg | Microkristalline Cellulose |
| | 15,00 | mg | Polyvinylpyrrolidon |
| | 15,00 | mg | quervernetztes Polyvinylpyrrolidon |
| | 5,00 | mg | Magnesiumstearat |
| | 500 mg | | |

| | | | |
|---|---|---|---|
| 17) | 5,00 | mg | Wirkstoff **1** |
| | 300,00 | mg | Wirkstoff **2.1** |
| | 50,00 | mg | Milchzucker |
| | 110,00 | mg | Microkristalline Cellulose |
| | 15,00 | mg | quervernetztes Polyvinylpyrrolidon |
| | 15,00 | mg | Polyvinylpyrrolidon |
| | 5,00 | mg | Magnesiumstearat |
| | 500 mg | | |

| | | | |
|---|---|---|---|
| 18) | 5,00 | mg | Wirkstoff **1** |
| | 350,00 | mg | Wirkstoff **2.1** |
| | 50,00 | mg | Milchzucker |
| | 60,00 | mg | Microkristalline Cellulose |
| | 15,00 | mg | quervernetztes Polyvinylpyrrolidon |
| | 15,00 | mg | Polyvinylpyrrolidon |
| | 5,00 | mg | Magnesiumstearat |
| | 500 mg | | |

| | | | |
|---|---|---|---|
| 19) | 5,00 | mg | Wirkstoff **1** |
| | 400,00 | mg | Wirkstoff **2.1** |
| | 60,00 | mg | Milchzucker |
| | 93,00 | mg | Microkristalline Cellulose |
| | 18,00 | mg | quervernetztes Polyvinylpyrrolidon |
| | 18,00 | mg | Polyvinylpyrrolidon |
| | 6,00 | mg | Magnesiumstearat |
| | 600 mg | | |

| | | | |
|---|---|---|---|
| 20) | 5,00 | mg | Wirkstoff **1** |
| | 450,00 | mg | Wirkstoff **2.1** |
| | 60,00 | mg | Milchzucker |
| | 43,00 | mg | Microkristalline Cellulose |
| | 18,00 | mg | quervernetztes Polyvinylpyrrolidon |
| | 18,00 | mg | Polyvinylpyrrolidon |
| | 6,00 | mg | Magnesiumstearat |
| | 600 mg | | |

## Patentansprüche

1. Arzneimittelkombination, **dadurch gekennzeichnet, dass** sie neben einem oder mehreren PDE4-Inhibitoren (**1**) der allgemeinen Formel **1** 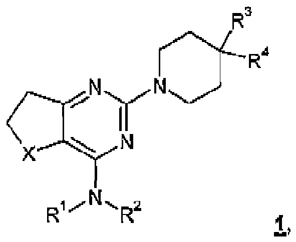 worin
**X** SO oder SO₂,
**R¹** H oder C₁₋₆-Alkyl,
**R²** H ist oder ein Rest ausgewählt aus der Gruppe bestehend aus C₁₋₁₀-Alkyl und C₂-₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR²¹, COOR^{2.1},CONR^{2.2}R^{2.3}, SR^{2.1},SO-R^{2.1}, SO₂R^{2.1}, C₆₋₁₀-Aryl, -Het, Hetaryl, einem mono- oder bicyclischem -C₃₋₁₀Cycloalkyl, CH₂-NR^{2.2}R^{2.3} und MR^{2.2}R^{2.3} substituiert sein kann,
welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
**Het** ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
**Hetaryl** ein fünf- bis zehngliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀-Aryl-C₁₋₆-alkylen, Hetaryl-C₁₋₆-alkylen-, Het-₁₋₆-alkylen-, C₃₋₁₀-Cycloalkyl-C₁₋₆-alkylen-, ein mono- oder bicyclisches C₆₋₁₀-Aryl, Heteroaryl und ein -Het,
der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann,
wobei **R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃, - CO-N(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
oder
**R²** ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-R^{2.1}, OR¹¹, COOR^{2.1}, -SO₂-NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -NH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl), -N(C₁-₃-Alkyl)-CO-(C₁₋₀-Alkyl), -N(C₁₋₃-Alkyl)-CO-O-(C₆₋₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀-Aryl), - N(C₁₋₃Alkyl)-CO-(C₁₋₃-Alkylen)-(C₆₋₁₀-Aryl), C₆₋₁₀-Aryl, C₁₋₆-Alkyl, C₆₋₁0-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, C₃₋₁₀-Cycloalkyl, Het, C₁₋₆-Alkyl, C₁₋₃-Fluoroalkyl, CF₃, CHF₂, CH₂F, C₆₋₁₀-Aryl-C₁₋₆-alkylen, Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
**NR¹R²** gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁-₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
**R³** ein C₆₋₁₀-Aryl ist,
welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}, SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het, - CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁₀-Aryl-C₁₋₁₂alkylen, Het-C₁₋₂-alkylen, -Het, -CO-Het, , CO-N(CH₃)-C₃₋₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist,
wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
**R³** ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO2-(CH₂-CH₃), C₆₋₁₀-Aryl, Het, C₃₋₇-Cycloalkyl und Hetaryl substituiert sein kann,
der wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁-₃-Alkyl) und O-(C₁-₃-Alkyl) substituiert sein kann,
oder worin
**R³** -O-R^{3.1},
wobei **R^{3.1}** ein Rest ausgewählt aus der Gruppe bestehend aus -C₁₋₆-Alkyl, -C₆₋₁₀-Aryl, - C₁₋₃-Alkylen-C₆₋₁₀Aryl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁₋₆-Alkylen)-Hetaryl, -CO-N(C₁₋₃-Alkyl)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R²¹; SO-R^{2.1}, SO₂-R^{2.1}, COOH, COCO-(C₁₋₄-Alkyl), -O-C₁₋₃-Alkylen-N(C₁₋₃-Alkyl), CO-NR^{2.2}R^{2.3}, NR^{2.2}-CO-R^{2.1}, C₆₋₁₀-Aryl, C₆₋₁₀-Aryl-C₁₋₂-alkylen, Het-C₁₋₂-alkylen, -CO-Het, Het, -CO-C₃₋₇-Cycloalkyl, -CO-N(C₁₋₃-Alkyl)-C₃₋₇-Cycloalkyl C₃₋₇-Cycloalkyl, C₃₋₇-Cycloalkyl-C₁₋₂-alkylen, Helaryl-C₁₋₂-alkylen und Hetaryl, substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann.
und worin
**R⁴** H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(C₁₋₃-Alkyl)-SO₂-(C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl), -C₁₋₃-Alkylen-NH-CO-(C₁₋₃-Alkylen)-O-(C₁₋₃-Alkyl), -CO-(C₆₋₁₀-Aryl), -(C₁₋₂-Alkylen)-N(C₁₋₃-Alkyl)-CO-(C₁₋₂-Alkylen)-O-(C₁₋₃-Alkyl),
wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann,
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆-Alkyl, -O-R^{2.1}, - COOR^{2.1}, SO-R^{2.1}, SO₂-R^{2.1}, -C₁₋₃-alkylen-NR^{2.2}R^{2.3}, -NR^{2.2}R^{2.1}, C₆₋₁₀-Aryl, C₃₋₇-Cycloalkyl, Het und Hetaryl substituiert sein kann,
bedeuten,
wenigstens einen EP4-Rezeptor-Antagonisten (**2**) umfasst.

2. Arzneimittelkombination nach Anspruch 1, **dadurch gekennzeichnet, dass** sie neben einem oder mehreren, bevorzugt einem PDE4-Inhibitor (**1**) der allgemeinen Formel **1** 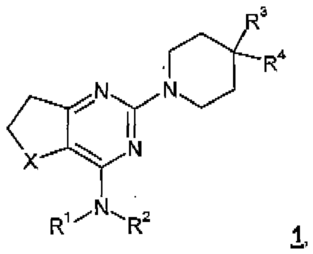 worin
**X** SO oder SO₂,
**R¹** H, C₁₋₆-Alkyl,
**R²** H ist oder ein Rest ausgewählt aus der Gruppe bestehend aus C₁-₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1}, CONR^{2.2}R^{2.3}, SR^{2.1}, SO-R^{2.1}, SO₂-R^{2.1}, C₆₋₁₀Aryl, -Het, Hetaryl, einem mono- oder bicyclischem -C₃₋₁₀cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
**Het** ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
**Hetaryl** ein fünf- bis zehngliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀-Aryl-C₁₋₆-alkylen, Hetaryl-C₁₋₆-alkylen-, Het-C₁₋₆-alkylen-, C₃₋₁₀-Cycloalkyl-C₁₋₆-alkylen-, ein mono- oder bicyclisches C₆₋₁₀-Aryl, Heteroaryl und ein -Het,
der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann,
wobei **R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃, - CO-N(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
oder
**R²** ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, -SO₂NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -NH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl), -N(C₁₋₃-Alkyl)-CO-(C₁₋₆-Alkyl), -N(C₁₋₃-Alkyl)-CO-O-(C₆₋₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀Aryl), - N(C₁₋₃-Alkyl)-CO-(C₁₋₃-Alkylen)-(C₆₋₁₀-Aryl), C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH_{2F}, C₁₋₆-Alkyl, C₆₋₁₀-Aryl und NR^{2.2}R^{2.3} substituiert sein kann,
oder
**R²** ein mono- oder polycyclischen C₆₋₁₀Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR-R^{2.3}, C₃₋₁₀-Cycloalkyl, Het, C₁₋₆-Alkyl, C₁₋₃-Fluoroalkyl, CF₂, CHF₂, CH₂F, C₆₋₁₀Aryl-C₁₋₆-alkylen, Het-C₁₋₆-alkylen, Hetaryl-C₁₋₆-alkylen, C₆₋₁₀-Aryl, SO₂-CH₃, SO₂CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
**NR¹R²** gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃₋Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
**R³** ein C₆₋₁₀-Aryl ist,
welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het, - CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁0-Aryl-C₁₋₂-alkylen, Het-C₁₋₂-alkylen, -Het, -CO-Het, , CO-N(CH₃)-C₃-₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist,
wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
**R³** ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-Aryl, Het, C₃₋₇Cycloalkyl und Hetaryl substituiert sein kann,
der wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
oder worin
**R³** -O-R^{3.1},
wobei **R^{3.1}** ein Rest ausgewählt aus der Gruppe bestehend aus -C₁₋₆-Alkyl, -C₆₋₁₀-Aryl, - C₁₋₃-Alkylen-C₆-₁₀-Aryl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁₋₆-Alkylen)-Hetaryl, -CO-N(C₁₋₃-Alkyl)-Het, -CO-N(C₃₋₇-Cycloalkyl)-Het, -C₁₋₃-alkylen-OR^{2.1}, -C₁₋₃-alkylen₋NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, C₆₋₁₀-Aryl, C₆₋₁₀-Aryl-C₁₋₂alkylen, Het-C₁₋₂-alkylen, -CO-Het, Het, -CO-C₃₋₇Cycloalkyl, -CO-N(C₁₋₃-Alkyl)-C₃₋₇-Cycloalkyl C₃₋₇-Cycloalkyl, C₃₋₇-Cycloalkyl-C₁₋₂-alkylen, Hetaryl-C₁₋₂-alkylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann.
und worin
**R⁴** H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(C₁₋₃-Alkyl)-SO₂₋C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl), -C₁₋₃-Alkylen-NO-CO-(C₁₋₃-Alkylen)-O-(C₁₋₃-Alkyl), -CO-(C₆₋₁₀-Aryl), -(C₁₋₂-Alkylen)-N(C₁₋₃-Alkyl)-CO-(C₁₋₂-Alkylen)-O-(C₁₋₃-Alkyl),
wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann,
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆Alkyl, -O-R^{2.1}, - COOR^{2.1}, SO-R^{2.1}, SO₂-R^{2.1}, -C₁₋₃-alkylen-NR^{2.2}R^{2.3}, -NR^{2.2}R^{2.3}, C₆₋₁₀-Aryl, C₃₋₇-Cycloalkyl, Het und Hetaryl substituiert sein kann,
bedeuten,
wenigstens einen EP4-Rezeptor-Antagonisten (**2**) umfasst.

3. Arzneimittelkombination nach einem der Ansprüche 1 bis 2, **dadurch gekennzeichnet, dass** sie neben einem oder mehreren, bevorzugte einem PDE4-Inhibitor der allgemeinen Formel **1**, worin
**X** SO,
**R¹** H
**R²** H ist oder C₁₋₆-Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1}, COOR^{2.1}, CONR^{2.2}R^{2.3}, SR^{2.1}, SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
**Het** ein drei- bis siebengliedriger, monocyclischer, gesättigter oder teilweise gesättigter Heterocyclus ist oder ein sieben- bis elfgliedriger, bicyclischer, gesättigter oder teilweise gesättigter Heterocyclus ist, der 1, 2 oder 3 Heteroatome unabhängig voneinander ausgewählt aus der Gruppe bestehend aus N, S oder O enthält, und wobei
**Hetaryl** ein fünf- bis sechsgliedriges, monocyclisches, aromatisches Heteroaryl ist, oder ein sieben- bis elfgliedriges, bicyclisches, aromatisches Heteroaryl ist, das 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
wobei **R².1** H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂alkylen, -Het-C₁₋₂-alkylen, C₃₋₇-Cycloalkyl-C₁₋₂-alkylen, Phenyl, Hetaryl und ein Het, der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Alkyl), CO-R^{2.1} und COOR^{2.1},
der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
**R²** ein monocyclisches C₃₋₇ Cycloalkyl, das gegebenenfalls mit einem Rest ausgewählt aus der Gruppe bestehend aus C₁₋₂-Alkanol, C₁₋₃-Fluoroalkyl, C₁₋₃-alkylen-OR^{2.1}, OR^{2.1} COOR^{2.1}, SO₂-NR^{2.2}R^{2.3}, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
**R²** ein Phenyl), das gegebenenfalls durch OH, SH, F, Cl oder Br oder durch einen oder mehrere Reste ausgewählt aus der Gruppe bestehend aus OR^{2.1}, COOR^{2.1},NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
**R²** ein Rest ausgewählt aus einer Gruppe bestehend aus Het und Hetaryl, der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂-alkylen, -Het, -Hetaryl und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Butyl, Isobutyl, Phenyl und NR^{2.2}R^{2.3} substituiert sein kann,
und worin
**R³** ein Naphthalin oder Phenyl ist,
welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃; SO₂-CH₃, SO-CH₃, COOCH₃, COOCH₂CH₃ , -CO-NH-(Methylen)-Hetaryl, -CO-NH-(Ethylen)-Hetaryl, -CO-NH-Hetaryl, -CO-N(CH₃)-Het, - CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het, -NH-CO-Methyl, NCH₃-CO-Methyl, -NH-CO-Ethyl, NCH₃-CO-Ethyl, -NH-CO-Propyl, NCH₃-CO-Propyl, -NH-CO-Isopropyl, NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO-N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₃) und -N(CH₃)₂ substituiert sein kann,
wobei dieser Rest gegebenenfalls durch einen oder mehrere Reste ausgewählt aus der Gruppe bestehend aus OH, F, Cl, -CF₃, CHF₂, CH₂F, Oxo, Methyl und Phenyl substituiert sein kann
oder worin
**R³** ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl, - COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₃)₂, -NH₂, - NH(CH₃), -N(CH₃)₂, Het und Hetaryl substituiert sein kann,
der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
oder worin
**R³** -O-R^{3.1},
wobei **R^{3.1}** ein Rest ausgewählt aus der Gruppe bestehend aus -C₁₋₃-Alkyl, -Phenyl, - C₁₋₃-Alkylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N(C₅₋₇-Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂, -Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl, , SO₂-Methyl, -SO₂-Ethyl, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇-Cycloalkyl, -CO-N(CH₃)-Cyclopropyl, C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
**R⁴** H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl) -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl) -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇-Cycloalkyl, Het und Hetaryl substituiert sein kann,
bedeuten,
wenigstens einen EP4-Rezeptor-Antagonisten (**2**) umfasst.

4. Arzneimittelkombination nach einem der Ansprüche 1 bis 3, **dadurch gekennzeichnet, dass** sie neben einem oder mehreren, bevorzugt einem PDE4-Inhibitor der allgemeinen Formel **1**, worin
**R²** ein Rest nach Formel **3** 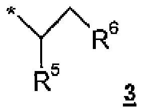
ist,
worin **R⁶** OH oder NH₂ ist und
worin **R⁵** ein Rest ausgewählt aus der Gruppe bestehend aus C₁₋₄-Alkyl, einem fünf- bis sechsgliedrigen Heteroaryl mit 1, 2 oder 3 Heteroatomen aus der Gruppe S, O und N und Phenyl ist, wobei dieser Rest gegebenenfalls mit einem oder mehreren Resten ausgewählt aus der Gruppe bestehend aus OH, F, Br, OR^{2.1}, Oxo, Methyl, Methyl, Methanol, Ethanol, Phenyl, COOR^{2.1}, CH₂-NR^{2.2}R^{2.3} und NR^{2.2}R^{2.3} substituiert sein kann,
bedeuten,
wenigstens einen EP4-Rezeptor-Antagonisten (**2**) umfasst.

5. Arzneimittelkombination nach Anspruch 4, **dadurch gekennzeichnet, dass** sie neben einem oder mehreren, bevorzugt einem PDE4-Inhibitor der allgemeinen Formel **1**, worin
**R²** ein Rest ist nach Formel **3** 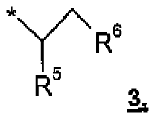
worin **R⁶** OH oder NH₂ ist und
worin **R⁵** Methyl, Ethyl, Propyl oder Isopropyl
bedeuten,
wenigstens einen EP4-Rezeptor-Antagonisten (**2**) umfasst.

6. Arzneimittelkombination nach einem der Ansprüche 1 bis 3, **dadurch gekennzeichnet, dass** sie neben einem oder mehreren, bevorzugt einem PDE4-Inhibitor der allgemeinen Formen **1**, worin
**R²** ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, und wobei
**R^{2.1}** ausgewählt ist aus der Gruppe bestehend aus Methyl, Ethyl, Propyl, Isopropyl, Butyl, und Isobutyl,
wenigstens einen EP4-Rezeptor-Antagonisten (**2**) umfasst.

7. Arzneimittelkombination nach einem der Ansprüche 1 bis 3, **dadurch gekennzeichnet, dass** sie neben einem oder mehreren, bevorzugt einem PDE4-Inhibitor der allgemeinen Formel **1**, worin
**R²** ein Cyclopropyl ist, das gegebenenfalls mit einem weiteren Rest ausgewählt aus der Gruppe bestehend aus -NH₂, CH2- NH₂, -NH(CH₃), -N(CH₃)₂, Methyl, Ethyl, Propyl, Isopropyl, -NH-CO-(tert-Butyl), -NH-CO-O-(tert-Butyl), -N(CH₃)-CO-(tert-Butyl), -N(CH₃)-CO-O-(tert-Butyl), -CF₃, -CHF₂, CH₂F, F, Cl und Br substituiert sein,
wenigstens einen EP4-Rezeptor-Antagonisten (**2**) umfasst.

8. Arzneimittelkombination nach einem der Ansprüche 1 bis 3, **dadurch gekennzeichnet, dass** sie neben einem oder mehreren, bevorzugt einem PDE4-Inhibitor der allgemeinen Formel **1**, worin
**R²** ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei R^{2.1} H, Methyl oder Ethyl, sein kann,
wenigstens einen EP4-Rezeptor-Antagonisten (**2**) umfasst.

9. Arzneimittelkombination nach einem der Ansprüche 1 bis 3, **dadurch gekennzeichnet, dass** sie neben einem oder mehreren, bevorzugt einem PDE4-Inhibitor der allegemeinen Formel **1**, worin
**R²** ein monocyclischer, gesättigter drei-, vier-, fünf-, sechs- oder siebengliedriger Heterocyclus mit 1, 2 oder 3 Heteroatomen jeweils ausgewählt aus der Gruppe bestehend aus N, O und S ist, der gegebenenfalls durch einen oder mehrere Reste ausgewählt aus der Gruppe Fluor, Chlor, Brom, CF₃, CHF₂, CH₂F, OH und Oxo oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, wobei die letztgenannte Gruppe von Resten gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
wobei **R^{2.1}**, **R^{2.2}** und **R^{2.3}** wie in Anspruch 1 definiert sind,
wenigstens einen EP4-Rezeptor-Antagonisten (**2**) umfasst.

10. Arzneimittelkombination nach Anspruch 9, **dadurch gekennzeichnet, dass** sie neben einem oder mehreren, bevorzugt einem PDE4-Inhibitor der allgemeinen Formel **1**, worin
**R²** ein monocyclischer, gesättigter sechsgliedriger Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃ und N(CH₃)₂, Methyl, Ethyl, Propyl, Isopropyl, Cyclopropyl, Methoxy und Ethoxy substituiert sein kann,
wenigstens einen EP4-Rezeptor-Antagonisten (**2**) umfasst.

11. Arzneimittelkombination nach einem der Ansprüche 1 bis 3, **dadurch gekennzeichnet, dass** sie neben einem oder mehreren, bevorzugt einem PDE4-Inhibitor der allgemeinen Formel **1**, worin
**R²** einen Rest ausgewählt aus einer Gruppe bestehend aus Piperidin oder Tetrahydropyran bedeutet, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
wenigstens einen EP4-Rezeptor-Antagonisten (**2**) umfasst.

12. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, worin
**R³** ein Naphthalin oder Phenyl ist,
welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, - OCH₃, OCH₂CH₃; SO₂-CH₃, SO₂-CH₂CH₃, COOCH₃ und CO-O-CH₂CH₃ substituiert sein kann,
wenigstens einen EP4-Rezeptor-Antagonisten (**2**) umfasst.

13. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, worin
**R³** ein Rest ausgewählt aus der Gruppe bestehend aus Het und Hetaryl ist, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₅₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃. N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl, -COO-Propyl, -COO-Isopropyl und O-Methyl, O-Ethyl, O-Propyl und O-Isopropyl substituiert sein kann,
und worin
**R⁴** H, CN, OH, CF₃, CHF₂. CH₂F, F, Methyl, Ethyl, O-Methyl oder O-Ethyl, und
wobei
**Het** ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder 0 enthält,
und wobei
**Cycloalkyl** gesättigt oder teilweise gesättigt sein kann,
bedeuten,
wenigstens einen EP4-Rezeptor-Antagonisten (**2**) umfasst.

14. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, worin
**R³** ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl, - COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann,
der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, - COO-Ethyl und O-Methyl, O-Ethyl substituiert sein kann,
wenigstens einen EP4-Rezeptor-Antagonisten (**2**) umfasst.

15. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, wobei
**R³** ein Rest ausgewählt aus einem monocyclischen, gesättigten oder teilweise gesättigten, drei- bis siebengliedrigem Heterocyclus oder einem monocyclischen fünfbis sechsgliedrigem Heteroaryl ist,
der ausgewählt ist aus der Gruppe bestehend aus Imidazol, Dihydroimidazol, Oxadiazol, Oxadiazolidin, Pyrazol, Pyridin und Dihydropyrazol,
welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl, - COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
wenigstens einen EP4-Rezeptor-Antagonisten (**2**) umfasst.

16. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1,** wobei
**R³** und **R⁴** gemeinsam einen mono- oder bicyclischen, ungesättigten oder teilweise gesättigten, drei- bis elfgliedrigen Heterocyclus bilden, der 1, 2 oder 3 Heteroatome ausgewählt aus der Gruppe bestehend aus N, 0 und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
wenigstens einen EP4-Rezeptor-Antagonisten (**2**) umfasst.

17. Arzneimittelkombination nach Anspruch 16, **dadurch gekennzeichnet, dass** sie neben einem oder mehreren, bevorzugt einem PDE4-Inhibitor der allgemeinen Formel **1**, wobei
**R³** und **R⁴** gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigten, fünf- oder sechsgliedrigen Heterocyclus und einem fünf- oder sechsgliedrigem Heteroaryl substituiert sein kann,
wenigstens einen EP4-Rezeptor-Antagonisten (**2**) umfasst.

18. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, wobei
**R³** -O-R^{3.1} ist,
**R^{3.1}** ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist,
welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl,, -CF₃, CHF₂, CH₂F, CO-(Methyl), CO-(Ethyl), CO-(Propyl), CO-(Isopropyl), CO-(Butyl), CO-(Isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl. -CO-N(CH₃)-(Propylen)-Hetaryl, -CO-N(CH₃)-(Isopropylen)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂,-O-Methylen-N(Ethyl)₂, -O-Ethylen-N(Ethyl)₂, CO-NH₂, CO-NHCH₃, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇-Cycloalkyl, -CO-Cyclopropyl, -CO-N(CH₃)-Cyclopropyl, -CO-N(CH₃)-C₄₋₇Cycloalkyl, C₄₋₇-Cycloalkyl, Cyclopropyl, C₄₋₇-Cycloalkyl-Methylen-, Cyclopropyl-Methylen-, C₄₋₇Cycloalkyl-Ethylen-, Cyclopropyl-Ethylen-, Hetaryl-Methylen-, Hetaryl-Ethylen- und Hetaryl substituiert ist,
welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
wenigstens einen EP4-Rezeptor-Antagonisten (**2**) umfasst.

19. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, wobei
**R⁴** H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl, -Ethylen-O-Ethyl, -(Methylen)-N(CH₃)-CO-(Methyl), -(Methylen)-N(CH₃)-CO-(Ethyl) -(Ethylen)-N(CH₃)CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl bedeutet,
und wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -Propyl, -OH und CF₃ substituiert sein kann
wenigstens einen EP4-Rezeptor-Antagonisten (**2**) umfasst.

20. Arzneimittelkombination nach einem der Ansprüche 1 bis 11, **dadurch gekennzeichnet, dass** sie neben einem oder mehreren, bevorzugt einem PDE4-Inhibitor der allgemeinen Formel **1**, wobei
**R³** ein Rest ist ausgewählt aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl, O-Methyl, O-Ethyl, O-Propyl, O-Isopropyl, OH, F, Cl, Br, CF₃, Phenyl, Hetaryl und C₃₋₈-Cycloalkyl substituiert sein kann,
wenigstens einen EP4-Rezeptor-Antagonisten (**2**) umfasst.

21. Arzneimittelkombinationen nach einem der Ansprüche 1 bis 20; **dadurch gekennzeichnet, dass** sie neben einem oder mehreren, bevorzugt einem PDE4-Inhibitor der allgemeinen Formel **1** ausgewählt aus der Gruppe bestehend aus:
1.1. (R)-2-{2-[4-(4-Chlorphenyl)-piperidin-1-yl]-5-oxo-6,7-dihydro-5*H*-5λ⁴-thieno[3,2-*d*]pyrimidin -4-ylamino}-3-methylbutan-1-ol
1.2. (1-{2-[4-(4-Chlorphenyl)-piperidin-1-yl]-6-oxo-6,7-dihydro-6H-5*H*,5λ⁴-thieno[3,2-*d*]pyrimidin-4-ylamino}-cyclopropyl)-methanol
1.3. (R)-2-{2-[4-(4-Chlorphenyl)-piperidin-1-yl]-5-oxo-6,7-dihydro-5*H*-5λ⁴-thieno[3,2-*d*]pyrimidin-4-ylamino}-pentan-1-ol
1.4. (R)-1-{2-[4-(4-Chlorphenyl)-piperidin-1-yl]-5-oxo-6,7-dihydro-5*H*-5λ⁴-thieno[3,2-*d*]pyrirnidin-4-ylamino}-1-(4-fluorphenyl)-2-methylpropan-2-ol
1.5. (S)-5-{2-[4-(4-Chlorphenyl)-piperidin-1-yl]-5-oxo-6,7-dihydro-5*H*-5λ⁴-thieno[3,2-*d*]pyrimidin-4-ylamino}-1-methylpiperidin-2-on
1.6. {2-[4-(4-Chlorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.7. 1-(4-(1-Hydroxymethylcyclopropylamino)-5-oxo-6,7-dihydro-5*H*-5λ⁴-thieno[3,2-*d*]pyrimidin-2-yl)-3'-methyl-1'*H*-spiro[piperidin-4,4'-chinazolin]-2'(3'*H*)-on
1.8. {1-[2-(4-Benzo[*d*]isoxazol-3-yl-piperidin-1-yl)-5-oxo-6,7-dihydro-5*H*-5λ⁴-thieno[3,2-*d*]pyrimidin-4-ylamino]-cyclopropyl}-methanol
1.9. (1-{2-[4-(2-Ethyl-5-fluor-1*H-*indol-3-yl)-piperidin-1-yl]-5-oxo-6,7-dihydro-5*H*-5λ-thieno[3,2-*d*]pyrimidin-4-ylamino}-cyclopropyl)methanol
1.10. 1-[4-((S)-1-Methyl-6-oxopiperidin-3-ylamino)-5-oxo-6,7-dihydro-5*H*-5λ⁴-thieno[3,2-*d*]pyrimidin-2-yl]-4-phenylpiperidin-4-carbonitril
1.11. 3'-methyl-1-(4-(tetrahydro-2*H*-pyran-4-ylamino)-5-oxo-6,7-dihydro-5*H*-5λ⁴-thieno[3,2-*d*]pyrimidin-2-yl)-1'*H*-spiro[piperidin'4,4'-chinazolin]-2'(3'*H*)-on
1.12. (3-Fluorphenyl)-[5-oxo-2-(3,4,5,6-tetrahydro-2*H*-[4,4']bipyridinyl-1-yl)-6,7-dihydro-5*H-*5λ⁴-thieno[3,2-*d*]pyrimidin-4-yl]-amin
1.13. {2-[4-(2-Ethyl-5-fluor-1*H*-indo[-3-yl)-piperidin-1-yl]-5-oxo-6,7-dihydro-5*H*-5λ⁴-thieno[3,2-*d*]pyrimidin-4-yl}-(3-fluorphenyl)-amin
1.14. (1-{2-[4-(2,4-Difluorphenyl)-piperidin-1-yl]-5-oxo-6,7-dihydro-5*H*-5λ⁴-thieno[3,2-*d*]pyrimidin-4-ylamino]-cyclopropyl)-methanol
1.15. {2-[4-(2,4-Difluorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.16. (S)-5-[2-(4-Benzoxazol-2-yl-piperidin-1-yl)-5-oxo-6,7-dihydro-5*H*-5λ⁴-thieno[3,2-*d*]pyrimidin-4-ylamino]-1-methylpiperidin-2-on
1.17. (1-{2-[4-(6-Fluorbenzo[d]isoxazol-3-yl)-piperidin-1-yl]-5-oxo-6,7-dihydro-5*H*-5λ⁴-thieno[3,2-*d*]pyrimidin-4-ylamino}-cyclopropyl)-methanol
1.18. (1-{2-[4-(5-Fluorbenzo[*d*]isoxazol-3-yl)-piperidin-1-yl]-6-oxo-6,7-dihydro-6*H*-5λ⁴-thieno[3,2-*d*]pyrimidin-4-ylamino-cyclopropyl)-methanol
1.19. {2-[4-(5-Furan-2-yl-2*H*-pyrazol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.20. (3-Fluorphenyl)-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-yl}-amin
1.21. (R)-3-Methyl-2-{5-oxo-2-[4-(3-pyridin-4-yl-[1,2,4]oxadiazol-5-yl)-piperidin-1-yl]-6,7-dihydro-5*H*-5λ⁴-thieno[3,2-*d*]pyrimidin-4-ylamino}-butan-1-ol
1.22. (S)-5-{2-[4-(4-Fluorphenoxy)-piperidin-1-yl]-5-oxo-6,7-dihydro-5*H*-5λ-thieno[3,2-*d*]pyrimidin-4-ylamino}-1-methylpiperidin-2-on
1.23. (2-{4-[4-(4,5-Dihydrooxazal-2-yl)-phenoxy]-piperidin-1-yl}-5-oxo-6,7-dihydro-5H-5λ⁴-thieno[3,2-*d*]pyrimidin-4-yl)-(tetrahydropyran-4-yl)-amin
1.24. 4-{1-[5-Oxo-4-(tetrahydropyran-4-ylamino)-6,7-dihydro-5H-5λ⁴-thieno[3,2-*d*]pyrimidin-2-yl]-piperidin-4-yloxy}-benzoesäure
1.25. 2-(1-{2-[4-(4-Chlorphenyl)-piperidin-1-yl]-5-oxo,6,7-dihydro-5*H*-5λ⁴.thieno[3,2-*d*]pyrimidin-4-ylamino}-cyclopropyl)-propan-2-ol
1.26. {2-[4-(5-*tert*-Butyl-1-methyl-1*H*-indol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.27. 2-[4-(5-Furan-2-yl-1-methyl-1*H*-pyrazol-3-yl)-piperidin-1-yl]-5-oxo-6,7-dihydro-5*H-*5λ⁴-thieno[3,2-*d*]pyrimidin-4-yl}(tetrahydropyran-4-yl)-amin
1.28. (S)-5-(2-{4-[4-(4,5-Dihydrooxazol-2-yl)-phenoxy]-piperidin-1-yl}-5-oxo-6,7-dihydro-5H-6λ⁴-thieno[3,2-*d*]pyrimidin-4-ylamino)-1-methylpiperidin-2-on
1.29. {2-[4-(5-Furan-2-yl-2-methyl-2*H*-pyrazol-3-yl)-piperidin-1-yl]-5-oxo-6,7-dihydro-5H-5λ⁴-thieno[3,2-*d*]pyrimidin-4-yl}-(tetrahydropyran-4-yl)-amin
1.30. {2-[4-(1-Methyl-1*H-*imidazo[4,5-*c*]pyridin-2-yl)-piperidin-1-yl]-5-oxo-6,7-dihydro-5*H-*5λ⁴-thieno[3,2-*d*]pyrimidin-4-yl}-(tetrahydropyran-4-yl)-amin
1.31. 2-Methoxy-*N*-{1-[5-oxo-4-(tetrahydropyran-4-ylamino)-6,7-dihydro-5*H*-5λ⁴-thieno[3,2-*d*]pyrmidin-2-yl]-4-phenylpiperidin-4-ylmethyl}-acetamid
1.32. N-Cyclopropyl-N-methyl-4-{1-[5-oxo-4-(tetrahydropyran-4-ylamino)-6,7-dihydro-5*H-*5λ⁴-thieno[3,2-*d*]pyrimidin-2-yl]-piperidin-4-yl}-benzamid
1.33. N-Cyclopropyl-N-methyl-4-{1-[5-oxo-4-(tetrahydropyran-4-ylamino)-6,7-dihydro-5H-5λ⁴-thieno[3,2-*d*]primidin-2-yl]-piperidin-4-yloxy}-benzamid
1.34. {5-Oxo-2-[4'(pyridin-4-yloxy)-piperidin-1-yl]-6,7-dihydro-5*H*-5λ⁴-thieno[3,2-d]pyrimidin-4-yl}-(tetrahydropyran-4-yl)-amin
1.35. {2-[4-(4-Chlorphenoxy)-piperidin-1-yl]-5-oxo-6,7-dihydro-5*H*-5λ⁴-thieno[3,2-*d*]pyrimidin-4-yl}-(tetrahydropyran-4-yl)-amin
1.36. (S)-1-Methyl-5-{2-[4-(6-methyl-4-phenyloxazol-2-yl)-piperidin-1-yl]-6-oxo-6,7-dihydro-5*H*-5λ⁴-thieno[3,2-*d*]pyrimidin-4-ylamino}-piperidin-2-on
1.37. (1-{2-[4-(5-Methyl-4-phenyloxazol-2-yl)-piperidin-1-yl]-5-oxo-6,7-dihydro-5*H*-5λ⁴-thieno[3,2-*d*]pyrimidin-4-ylamino}-cyclopropyl)-methanol
1.38. (S)-5-{2-[4-(4,5-Diphenyloxazol-2-yl)-piperidin-1-yl]-5-oxo-6,7-dihydro-5H-5λ⁴-thieno[3,2-*d*]pyrimidin-4-ylamino}-1-methylpiperidin-2-on
1.39. {4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-yl}-methanol
1.40. [1-(2-{4-[5-{4-Chlorphenyl)-4-methyloxazol-2-yl]-piperidin-1-yl}-5-oxo-6,7-dihydro-5H-5λ⁴-thieno[3,2-*d*]pyrimidin-4-ylamino)-cyclopropyl]-methanol
1.41. 4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-ol
1.42. {2-[4-(4-Chlorphenyl)-4-methoxypiperidin-1-yl]-6-oxo-6,7-dihydro-5*H*-5λ⁴-thieno[3,2-*d*]pyrimidin-4-yl}-(tetrahydropyran-4-yl)-amin
1.43. 4-{1-[4-(1-Hydroxymethylcyclopropylamino)-5-oxo-6,7-dihydro-5*H*-5λ⁴-thieno[3,2-*d*]pyrimidin-2-yl]-piperidin-4-yloxy}-benzonitril
1.44. 5-Oxo-2-[4-(4,5,6,7-tetrahydrobenzoxazol-2-yl)-piperidin-1-yl]-6,7-dihydro-5H-5λ⁴-thieno[3,2-*d*]pyrimidin-4-yl}-(tetrahydropyran-4-yl)-amin
1.45. (S)-5-{2-[4-(4-Chlorphenyl)-piperidin-1-yl]-5,5-dioxo-6,7-dihydro-5*H*-5λ⁶-thieno[3,2-*d*]pyrimidin-4-ylamino}-1-methylpiperidin-2-on.
wenigstens einen EP4-Rezeptor-Antagonisten (**2**) umfasst.

22. Arzneimittelkombination nach einem der Ansprüche 1 bis 3, **dadurch gekennzeichnet, dass** der wenigstens eine EP4-Rezeptor-Antagonist ausgewählt ist aus der Gruppe bestehend aus
[N-{[4-(5,9-Diethoxy-6-oxo-6,8-dihydro-7H-pyrrolo[3,4-g]chinolin-7-yl)-3-methylbenzyl]sulfonyl}-2-(2-methoxyphenyl)acetamid] (**2.1**); 5-Butyl-2,4-dihydro-4-[[2'-[N-(3-methyl-2-thiophen-carbonyl)sulfamoyl]biphenyl-4-yl]methyl]-2-[(2-trifluoromethyl)phenyl]-1,2,4-triazol-3-on (**2.2**); (4-{(1S)-1[({5-chloro-2-[(4-fluorophenyl)oxy]phenyl}carbanyl)amino]ethyl}benzoesäure (**2.3**);
N-[({2-[4-(2-ethyl-4,6-dimethyl-1H-imidazo[4,5-c]pyridin-1-yl)phenyl]ethyl}amino)carbonyl]-4-methylbenzol sulfonamid (**2.4**); 4-[[4-(5-methoxy-2-pyridinyl)phenoxy]methyl]-5-methyl-N-[(2-methylphenyl)sulfonyl]-2-Furancarboxamid (**2.5**);
11 alpha, 15alpha-Dihydroxy-16-(3-methoxymethylphenyl)-9-oxo-17,18,19, 20-tetranor-5-thia-13(E) Prostansäuremethylester (**2.6**);
4-cyano-2-[[2-(4-fluoro-1naphthalenyl)-1-oxopropyl]amino]-Benzolbutansäure (**2.7**) und N-{2-[4-(4,9-diethoxy-1-oxo-1,3-dihydro-2H-benzo[f]isoindol-2-yl)phenyl]acetyl}benzene sulphonamide (**2.8**)**.**

23. Arzneimittelkombination nach einem der Ansprüche 1 bis 22, **dadurch gekennzeichnet, dass** der PDE4-Inhibitor (**1**) in einer Tagesdosis von 0,01 mg bis 50 mg, vorzugsweise von 0,1 mg bis 10 mg umfasst ist.

24. Arzneimittelkombination nach einem der Ansprüche 1 bis 23, **dadurch gekennzeichnet, dass** der EP4-Rezeptor-Antagonist (**2**) in einer Tagesdosis von 0,001 bis 100 mg/kg Körpergewicht, vorzugsweise von 0,01 bis 50 mg/kg Körpergewicht, stärker bevorzugt von 0,1 bis 10 mg/kg Körpergewicht eingesetzt wird.

25. Arzneimittelkombination nach einem der Ansprüche 1 bis 24, **dadurch gekennzeichnet, dass** das der EP4-Rezeptor-Antagonist (**2**) und der PDE4-Inhibitor (**1**) in einem Gewichtsverhältnis von 1:1 bis 200:1, vorzugsweise in einem Gewichtsverhältnis von 10:1 bis 150:1, besonders bevorzugt in einem Gewichtsverhältnis von 30:1 bis 100:1 eingesetzt werden.

26. Verwendung eines EP4-Rezeptor-Antagonisten (**2**) zur Herstellung eines Medikamentes zur Reduzierung der Nebenwirkungen eines oder mehrerer PDE4-Inhibitoren der allgemeinen Formel 1 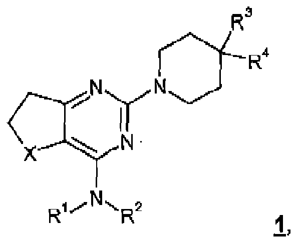 worin **X**, **R¹**, **R²**, **R³** und **R⁴** wie in einem der Ansprüche 1 bis 2 definiert sind,
bei der Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

27. Verwendung einer Kombination enthaltend einen oder mehrere PDE4-Inhibitoren (**1**) der allegemeinen Formel **1**, 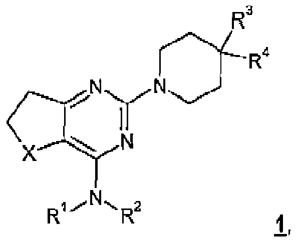 worin **X**, **R¹**, **R², R³** und **R⁴** wie in einem der Ansprüche 1 bis 20 definiert sind,
und wenigstens einen EP4-Rezeptor-Antagonisten (**2**) zur Herstellung eines Medikamentes zur Behandlung einer
Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

28. Verwendung nach einem der Ansprüche 26 bis 27, zur Behandlung einer Erkrankung ausgewählt aus COPD, chronischer Sinusitis, Asthma, Morbus Crohn und Colitis ulcerosa.

29. Verwendung nach einem der Ansprüche 26 bis 28, **dadurch gekennzeichnet, dass** der PDE4-Inhibitor (**1**) und der wenigstens eine EP4-Rezeptor-Antagonist (**2**) in einer einzigen gemeinsamen Formulierung gleichzeitig verabreicht werden.

30. Verwendung nach einem der Ansprüche 26 bis 38, **dadurch gekennzeichnet, dass** der PDE4-Inhibitor (**1**) und der wenigstens eine EP4-Rezeptor-Antagonist (**2**) in zwei separaten Formulierungen innerhalb eines zeitlichen Abstandes von 0 bis 6 Stunden voneinander verabreicht werden.

31. Verwendung nach Anspruch 30, wobei die Formulierng enthaltend den PDE4-Inhibitor (**1**) eine orale oder inhalative Formulierung, vorzugsweise eine orale Formulierung ist, und wobei die Formulierung enthaltend den wenigstens einen EP4-Rezeptor-Antagonist (**2**) eine orale Formulierung ist.

32. Verwendung nach einem der Ansprüche 30 oder 31, wobei die Formulierung enthaltend den PDE4-Inhibitor (**1**) einmal täglich und die Formulierung enthaltend den wenigstens einen EP4-Rezeptor-Antagonist (**2**) entweder einmal oder zweimal täglich verabreicht wird.

33. Verwendung nach einem der Ansprüche 26 bis 32, **dadurch gekennzeichnet, dass** der PDE4-Inhibitor eine Verbindung der Formel **1** ist ausgewählt aus der Gruppe bestehend aus
1.1. (R)-2-{2-[4-(4-Chlorphenyl)-piperidin-1-yl]-5-oxo-6,1-dihydro-5*H*-5λ⁴-thieno[3,2-*d*]pyrimidin-4-ylamino}-3-methylbutan-1-ol
1.2. (1-{2-[4-(4-Chlorphenyl)-piperidin-1-yl]-5-oxo-6,7-dihydro-5*H*-5λ⁴-thieno[3,2-*d*]pyrimidin-4-ylamino}-cyclopropyl)-methanol
1.3. (R)-2-{2-[4-(4-Chlorphenyl)-piperidin-1-yl]-5-oxo-6,7-dihydro-5*H*-5λ⁴-thieno[3,2-*d*]pyrimidin-4-ylamino}-pentan-1-ol
1.4. (R)-1-{2-[4-(4-Chlorphenyl)-piperidin-1-yl]-5-oxo-6,7-dihydro-5*H*-5λ⁴-thieno[3,2-*d*]pyrimidin-4-ylamino}-1-(4-fluorphenyl)-2-methylpropan-2-ol
1.5. (S)-5-{2-[4-(4-Chlorphenyl)-piperidin-1-yl]-5-oxo-6,7-dihydro-5*H*-5λ⁴-thieno[3,2-*d*]pyrimidin-4-ylamino}-1-methylpiperidin-2-on
1.6. {2-[4-(4-Chlorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.7. 1-(4-(1-Hydroxymethylcyclopropylamino)-5-oxo-6,7-dihydro-5*H-5*λ⁴-thieno[3,2-*d*]pyrimidin-2-yl)-3'-methyl-1'*H*-spiro[piperidin-4,4'-chinazolin]-2'(3'*H*)-on
1.8. {1-[2-(4-Benzo[*d*]isoxazol-3-yl-piperidin-1-yl)-5-oxo-6,7-dihydro-5H-5λ⁴-thieno[3,2-*d*]pyrimidin-4-ylamino]-cyclopropyl}-methanol
1.9. (1-{2-[4-(2-Ethyl-5-fluor-1*H*-indol-3-yl)-piperidin-1-yl]-5-oxo-6,7-dihydro-5*H*-5λ⁴-thieno[3,2-*d*]pyrimidin-4-ylamino}-cyclopropyl)-methanol
1.10. 1-[4-((S)-1-Methyl-6-oxopiperidin-3-ylamino)-5-oxo-6,7-dihydro-5*H*-5λ⁴-thieno[3,2-*d*]pyrimidin-2-yl]-4-phenylpiperidin-4-carbonitril
1.11. 3'-methyl-1-(4-(tetrahydro-2*H*-pyran-4-ylamino)-6-oxo-6,7-dihydro-5*H*-5λ⁴-thieno[3,2-*d*]pyrimidin-2-yl)-1'*H*-spiro[piperidin-4,4'-chinazolin]-2'(3'*H*)-on
1.12. (3-Fluorphenyl)-[5-oxo-2-(3,4,5,6-tetrahydro-2*H*-[4,4']bipyridinyl-1-yl)-6,7-dihydro-5*H-*5λ⁴-thieo[3,2-*d*]pyrimidin-4-yl]-amin
1.13. {2-[4-(2-Ethyl-5-fluor-1*H*-indol-3-yl)-piperidin-1-yl-5-oxo-6,7-dihydro-5*H*-5λ⁴-thieno[3,2-*d*]pyrimidin-4-yl}-(3-fluorphenyl)-amin
1.14. (1-{2-[4-(2,4-Difluorphenyl)-piperidin-1-yl]-5-oxo-6,7-dihydro-5*H*-5λ⁴-thieno[3,2-*d*]pyrimidin-4-ylamino}-cyclopropyl)-methanol
1.15. {2-[4-(2,4-Difluorphenyl)-piperidin-1-yl]-5-oxo-6,7-dihydro-5*H*-5λ⁴-thieno[3,2-*d*]pyrimidin-4-yl}-(tetrahydropyran-4-yl)-amin
1.16. (S)-5-[2-(4-Benzoxazol-2-yl-piperidin-1-yl)-5-oxo-6,7-dihydro-5*H*-5λ⁴-thieno[3,2-*d*]pyrimidin-4-ylamino]-1-methylpiperidin-2-on
1.17. (1-{2-[4-(6-Fluorbenzo[*d*]isoxazol-3-yl)-piperidin-1-yl]-5-oxo-6,7-dihydro-5*H*-5λ⁴-thieno[3.2-*d*]pyrimidin-4-ylamino}-cyclopropyl)-methanol
1.18. (1-{2-[4-(5-Fluorbenzo[*d*]isoxazol-3-yl)-piperidin-1-yl]-5-oxo-5,7-dihydro-5*H-*5λ⁴-thieno[3,2-*d*]pyrimidin-4-ylamino}-cyclopropyl)-methanol
1.19. {2-[4-(5-Furan-2-yl-2*H*-pyrazol-3-yl)-piperidin-1-yl]-5-oxo-6,7-dihydro-5*H*-5λ⁴-thieno[3,2-*d*]pyrimidin-4-yl}-(tetrahydropyran-4-yl)-amin
1.20. (3-Fluorphenyl)-{5-oxo-2-[4-(3-pyridin-4-yl-[1,2,3]oxadiazol-5-yl-piperidin-1-yl]-6,7-dihydro-5*H*-5λ⁴-thieno[3,2-*d*]pyrimidin-4-yl}-amin
1.21. (R)-3-Methyl-2-{5-oxo-2-[4-(3-pyridin-4-yl-[1,2,4]oxadiazol-5-yl)-piperidin-1-yl]-6,7-dihydro-5H-5λ⁴-thieno[3,2-*d*]pyrimidin-4-ylamino}-butan-1-ol
1.22. (S)-5-{2-[4-(4-Fluorphenoxy)piperidin-1-yl]-5-oxo-6,7-dihydro-5*H*-5λ⁴-thieno[3,2-*d*]pyrimidin-4-ylamino}-1-methylpiperidin-2-on
1.23. (2-{4-[4-(4,5-Dihydrooxazol-2-yl)-phenoxy]-piperidin-1-yl}-6-oxo-6,7-dihydro-5H-5λ⁴-thieno[3,2-*d*]pyrimidin-4-yl}-(tetrahydropyran-4-yl)-amin
1.24. 4-{1-[5-Oxo-4-(tetrahydropyran-4-ylamino)-6,7-dihydro-5H-5λ⁴-thieno[3,2-*d*]pyrimidin-2-yl]-piperidin-4-yloxy}-benzoesäure
1.25. 2-(1-{2-[4-(4-Chlorphenyl)-piperidin-1-yl]-5-oxo-6,7-dihydro-5*H*-5λ⁴-thieno[3,2-*d*]pyrimidin-4-ylamino}cyclopropyl)propan-2-ol
1.26. {2-[4-(5-*tert*-Butyl-1-methyl-1*H*-indol-3-yl)-piperidin-1-yl}-5-oxo-6,7-dihydro-5*H-*5λ⁴-thieno[3,2-*d*]pyrimidin-4-yl}-(tetrahydropyran-4-yl)-amin
1.27. 2-[4-(5-Furan-2-yl-1-methyl-1*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.28. (S)-5-(2-{4-[4-(4,5-Dihydrooxazol-2-yl)-phenoxy]-piperidin-1-yl}-5-oxo-6,7-dihydro-5*H*-5λ⁴-thieno[3,2-*d*]pyrimidin-4-ylamino)-1-methylpiperidin-2-on
1.29. {2-[4-(5-Furan-2-yl-2-methyl-2*H*-pyrazol-3-yl)-piperidin-1-yl]-5-oxo-6,7-dihydro-5*H-*5λ⁴-thieno[3,2-*d*]pyrimidin-4-yl}-(tetrahydropyran-4-yl)-amin
1.30. {2-[4-(1-Methyl-1*H*-imidazo[4,5-*c*]pyridin-2-yl)-piperidin-1-yl]-5-oxo-6,7-dihydro-5*H-*5λ⁴-thieno[3,2-*d*]pyrimidin-4-yl}-(tetrahydropyran-4-yl)-amin
1.31. 2-Methoxy-*N*-{1-[5-oxo-4-(tetrahydropyran-4-ylamino)-6,7-dihydro-5*H*-5λ⁴-thieno[3,2-*d*]pyrimidin-2-yl]-4-phenylpiperidin-4-ylmethyl}-acetamid
1.32. N-Cyclopropyl-N-methyl-4-{1-[5-oxo-4-(tetrahydropyran-4-ylamino)-6,7-dihydro-5*H-*5λ⁴-thieno[3,2-*d*]pyrimidin-2-yl]-piperidin-4-yl}-benzamid
1.33. N-Cyclopropyl-N-methyl-4-{1-[5-oxo-4-(tetrahydropyran-4-ylamino)-6,7-dihydro-5H-5λ⁴-thieno[3,2-*d*]pyrimidin-2-yl)-piperidin-4-yloxy}-benzamid
1.34. {5-Oxo-2-[4-(pyridin-4-yloxy)-piperidin-1-yl]-6,7-dihydro-5*H-*5λ⁴-thieno[3,2-*d*]pyrimidin-4-yl}-(tetrahydropyran-4-yl)-amin
1.35. {2-[4-(4-Chlorphenoxy)-piperidin-1-yl]-5-oxo-6,7-dihydro-5*H*-5λ⁴-thieno[3,2-*d*]pyrimidin-4-yl}-(tetrahydropyran-4-yl)-amin
1.36. (S)-1-Methyl-5-{2-[4-(5-methyl-4-phenyloxazol-2-yl)-piperidin-1-yl]-5-oxo-6,7-dihydro-5*H*-5λ⁴-thieno[3,2-*d*]pyrimidin-4-ylamino}-piperidin-2-on
1.37. (1-{2-[4-(5-Methyl-4-phenyloxazol-2-yl)-piperidin-1-yl]-5-oxo-6,7-dihydro-5*H*-5λ⁴-thieno[3,2-*d*]pyrimidin-4-ylamino}-cyclopropyl)-methanol
1.38. (S)-5-{2-[4-(4,5-Diphenyloxazol-2-yl)-piperidin-1-yl]-5-oxo-6,7-dihydro-5H-5λ⁴-thieno[3,2-*d*]pyrimidin-ylamino}-1-methylpiperidin-2-on
1.39. {4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-yl}-methanol
1.40. [1-(2-{4-[5-(4-Chlorphenyl)-4-methyloxazol-2-yl]-piperidin-1-yl)-5-oxo-6,7-dihydro-5H-5λ⁴-thieno[3,2-*d*]pyrimidin-4-ylamino)-cyclopropyl]-methanol
1.41. 4-(4-Chlorphenyl)-1-[5-oxo-4-(tetrahydropyran-4-ylamino)-6,7-dihydro-5*H*-5λ⁴-thieno[3,2-*d*]pyrimidin-2-yl]-piperidin-4-ol
1.42. {2-[4-(4-Chlorphenyl)-4-methoxypiperidin-1-yl]-5-oxo-6,7-dihydro-5*H*-5λ⁴-thieno[3,2-*d*]pyrimidin-4-yl}-(tetrahydropyran-4-yl)-amin
1.43. 4-{1-[4-(1-Hydroxymethylcyclopropylamino)-5-oxo-6,7-dihydro-5*H*-5λ⁴-thieno[3,2-*d*]pyrimidin-2-yl]-piperidin-4-yloxy}-benzonitril
1.44. 5-Oxo-2-[4-(4,5,6,7-tetrahydrobenzoxazol-2-yl)-piperidin-1-yl]-6,7-dihydro-5H-5λ⁴-thieno[3,2-*d*]pyrimidin-4-yl)-(tetrahydropyran-4-yl)-amin
1.45. (S)-5-{2-[4-(4-Chlorphenyl)-piperidin-1-yl]-5,5-dioxo-6.7-dihydro-5H-5λ⁶-thieno[3,2-*d*]pyrimidin-4-ylamino}-1-methylpiperidin-2-on.

34. Verwendung nach einem der Ansprüche 26 bis 33, **dadurch gekennzeichnet, dass** der wenigstens eine EP4-Rezeptor-Antagonist (**2**) ausgewählt ist aus der Gruppe bestehend aus:
[N-{[4-(5,9-Diethoxy-6-oxo-6,8-dihydro-7H-pyrrolo[3,4-g]chinolin-7yl)-3-methylbenzyl]sulfonyl}-2-(2-methoxyphenyl)acetamid] (**2.1**);
5-Butyl-2,4-dihydro-4-[[2'-[N-(3-methyl-2-thiophen-carbonyl)sulfamoyl]biphenyl-4-yl]methyl]-2-[(2-trifluoromethyl)phenyl]-1,2,4-triazol-3-on (**2.2**);
(4-{(1S)-1-[({5-chloro-2-[(4-fluorophenyl)oxy]phenyl}carbonyl)amino]ethyl}benzoesäure (**2.3**);
N-[({2-[4-(2-ethyl-4,6-dimethyl-1H-imidazo[4,5-c]pyridin-1-yl)phenyl]ethyl}amino)carbonyl]-4-methylbenzol sulfonamid (**2.4**);
4-[[4-(5-methoxy-2-pyridinyl)phenoxy]methyl]-5-methyl-N-[(2-methylphenyl)sulfonyl]-2-Furancarboxamid (**2.5**);
11alpha, 15alpha-Dihydroxy-16-(3-methoxymethylphenyl)-9-oxo-17,18,19,20-tetranor-6-thia-13(E) Prostansäuremethylester (**2.6**);
4-cyano-2-[[2-(4-fluoro-1-naphthalenyl)-1-oxopropyl]amino]-Benzolbutansäure (**2.7**) und N-{2-[4-(4,9-diethoxy-1-oxo-1,3-dihydro-2H-benzo[f]isoindol-2-yl)phenyl]acetyl)benzene sulphonamide (**2.8**).

35. Verwendung nach einem der Ansprüche 26 bis 34, **dadurch gekennzeichnet, dass** der PDE4-Inhibitor der allgemeinen Formel **1** in einer Tagesdosis von 0,01 mg bis 50 mg eingesetzt wird.

36. Verwendung nach einem der Ansprüche 26 bis 35, **dadurch gekennzeichnet, dass** der EP4-Rezeptor-Antagonist (**2**) in einer Tagesdosis von 0,001 bis 100 mg/kg Körpergewicht eingesetzt wird, vorzugsweise von 0,01 bis 50 mg/kg Körpergewicht, stärker bevorzugt von 0,1 bis 10 mg/kg Körpergewicht.

37. Verwendung nach einem der Ansprüche 26 bis 36, **dadurch gekennzeichnet, dass** das der EP4-Rezeptor-Antagonist (**2**) und der PDE4-Inhibitor (**1**) in einem Gewichtsverhältnis von 1:1 bis 200:1, vorzugsweise in einem Gewichtsverhältnis von 10:1 bis 150:1, besonders bevorzugt in einem Gewichtsverhältnis von 30:1 bis 100:1 eingesetzt werden.

## Claims

1. Medicament combination, **characterised in that**, in addition to one or more PDE4-inhibitors (**1**) of general formula **1** 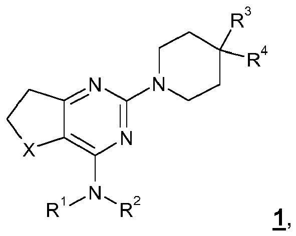 wherein
**X** denotes SO or SO₂,
**R¹** denotes H or C₁₋₆-alkyl,
**R²** is H or a group selected from among C₁₋₁₀-alkyl and C₂₋₆-alkenyl, which may optionally be substituted by one or more groups selected from halogen and C₁₋₃-fluoroalkyl or which is optionally substituted by one or more groups selected from among OR^{2.1}, COOR^{2.1}, CONR^{2.2}R^{2.3}, SR^{2.1}, SO-R^{2.1}, SO₂-R^{2.1}, C₆₋₁₀-aryl, het, hetaryl, a mono- or bicyclic C₃₋₁₀-cycloalkyl, CH₂-NR^{2.2}R^{2.3} and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, C₆₋₁₀-aryl, COOR^{2.1}, CH₂-NR^{2.2}R^{2.3} and NR^{2.2}R^{2.3},
wherein
**het** is a three- to eleven-membered, mono- or bicyclic, saturated or partly saturated, optionally annellated or optionally bridged heterocyclic group, which contains 1, 2, 3 or 4 heteroatoms selected independently of one another from among N, S or O, and wherein
**hetaryl** is a five- to ten-membered, mono- or bicyclic, optionally annellated heteroaryl, which contains 1, 2, 3 or 4 heteroatoms selected independently of one another from among N, S or O,
and wherein
**cycloalkyl** may be saturated or partly saturated,
wherein **R^{2.1}** is H or a group selected from among C₁₋₆-alkyl, C₁₋₆-alkanol, C₁₋₃-haloalkyl, mono- or bicyclic, -C₃₋₁₀-cycloalkyl, C₆₋₁₀-aryl-C₁₋₆-alkylene, hetaryl-C₁₋₆-alkylene, het-C₁₋₆-alkylene, C₃₋₁₀-cycloalkyl-C₁₋₆-alkylene, a mono- or bicyclic C₆₋₁₀-aryl, heteroaryl and a -het,
which may optionally be substituted by one or more groups selected from among OH, O-(C₁₋₃-alkyl), halogen, C₁₋₆-alkyl and C₆₋₁₀-aryl,
wherein **R^{2.2}** and **R^{2.3}** independently of one another denote H or a group selected from among C₁₋₆-alkyl, mono- or bicyclic C₃₋₁₀cycloalkyl, C₆₋₁₀-aryl-C₁₋₆-alkylene, hetaryl-C₁₋₆-alkylene, mono- or bicyclic C₆₋₁₀-aryl, het, hetaryl, CO-NH₂, CO-NHCH₃, -CO-N(CH₃)₂, SO₂-(C₁-C₂-alkyl), CO-R^{2.1} and COOR^{2.1},
which may optionally be substituted by one or more groups selected from among OH, halogen, C₁₋₆-alkyl, C₆₋₁₀-aryl and COOR^{2.1},
or
**R²** denotes a mono- or polycyclic C₃₋₁₀ cycloalkyl, which may optionally be singly or multiply bridged by C₁₋₃-alkyl groups and which may optionally be substituted by a group selected from among branched or unbranched C₁₋₆-alkanol, C₁₋₃-fluoroalkyl, C₁₋₃-alkylene-OR^{2.1}, OR^{2.1}, COOR^{2.1}, -SO₂-NR^{2.2}R^{2.3}, het, -NH-CO-O-(C₁₋₆-alkyl), -NH-CO-(C₁₋₆-alkyl), -NH-CO-O-(C₆₋₁₀-aryl), -NH-CO-(C₆₋₁₀-aryl), -NH-CO-O-hetaryl, -NH-CO-hetaryl, -NH-COO-(C₁₋₃-alkylene)-(C₆₋₁₀-aryl), -NH-CO-(C₁₋₃-alkylene)-(C₆₋₁₀-aryl), -N(C₁₋₃-alkyl)-CO-(C₁₋₆-alkyl), -N(C₁₋₃-alkyl)-CO-O-(C₆₋₁₀-aryl), -N(C₁₋₃-alkyl)-CO-(C₆₋₁₀-aryl), -N(C₁₋₃-alkyl)-CO-O-hetaryl, -N(C₁₋₃-alkyl)-CO-hetaryl, -N(C₁-₃-alkyl)-COO-(C₁₋₃-alkylene)-(C₆₋₁₀-aryl), -N(C₁₋₃-alkyl)-CO-(C₁-₃-alkylene)-(C₆₋₁₀-aryl), C₆₋₁₀-aryl, C₁₋₆-alkyl, C₆₋₁₀-aryl-C₁₋₆-alkylene, hetaryl-C₁₋₆-alkylene, mono- or bicyclic C₃₋₁₀ cycloalkyl and NR^{2.2}R^{2.3}, which may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
**R²** denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₃₋₁₀-cycloalkyl, het, C₁₋₆-alkyl, C₁₋₃-fluoroalkyl, CF₃, CHF₂, CH₂F, C₆₋₁₀-aryl-C₁₋₆-alkylene, het-C₁₋₆-alkylene, hetaryl-C₁₋₆-alkylene, C₆₋₁₀-aryl, SO₂-CH₃, SO₂-CH₂CH₃ and SO₂NR^{2.2}R^{2.3},
which in turn may optionally be substituted by one or more or several groups selected from among OH, OR^{2.1}, CF₃, CHF₂, CH₂F, oxo, halogen, CF₃, CHF₂, CH₂F, C₁₋₆-alkyl, C₆₋₁₀-aryl and NR^{2.2}R^{2.3},
or
**R²** denotes a group selected from among het and hetaryl, which may optionally be substituted by one or more groups selected from among halogen, OH, oxo, CF₃, CHF₂ and CH₂F or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, mono- or bicyclic C₃₋₁₀-cycloalkyl, C₆₋₁₀-aryl, C₁₋₆-alkyl, C₆₋₁₀-aryl-C₁₋₆-alkylene, hetaryl-C₁₋₆-alkylene, het, hetaryl, C₁₋₃-alkylene-OR^{2.1} and NR^{2.2}R^{2.3},
which may optionally in turn be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or wherein
**NR¹R²** together denotes a heterocyclic C₄₋₇ ring which may optionally be bridged, which contains 1, 2 or 3 heteroatoms selected from among N, O and S and which may optionally be substituted by one or more groups selected from among OH, OR^{2.1}, C₁₋₃-alkylene-O^{R.1}, oxo, halogen, C₁₋₆-alkyl, C₆₋₁₀-aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} and NR^{2.2}R^{2.3},
and wherein
**R³** is a C₆₋₁₀-aryl,
which is optionally substituted in the ortho, para or meta position by one, two or three groups selected independently of one another from among fluorine, chlorine, bromine, hydroxy, CN, C₁₋₆-alkyl, C₁₋₃-fluoroalkyl, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-alkylene)-hetaryl, -CO-NH-hetaryl, -CO-N(CH₃)-het, -CO-N(CH₃)-(C₁₋₃-alkylene)-het, -CO-N(CH₃)-(C₁₋₃-alkylene)-hetaryl, -CO-N(C₃₋₇-cycloalkyl)-het, -CO-NR^{2.2}R^{2.3}, -CO-NH-(C₁₋₆-alkylene)-het, NR^{2.2}-CO-R^{2.1}, C₆₋₁₀-aryl, C₆₋₁₀-aryl-C₁₋₂-alkylene, het-C₁₋₂-alkylene, -het, -CO-het, CO-N(CH₃)-C₃₋₇-cycloalkyl, C₃₋₇-cycloalkyl, C₃₋₇-cycloalkyl-C₁₋₂-alkylene, hetaryl-C₁₋₂-alkylene and hetaryl,
wherein this group may optionally be substituted by one or more groups selected from among OH, halogen, -C₁₋₃-fluoroalkyl, oxo, methyl and phenyl,
or wherein
**R³** is a group selected from among het and hetaryl, which may optionally be substituted by one or more groups selected from among halogen, C₁₋₃-fluoroalkyl, CN, OH, oxo, -C₁₋₆-alkyl, -C₁₋₃-alkylene-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-aryl, het, C₃₋₇-cycloalkyl and hetaryl,
which in turn may optionally be substituted by one or more groups selected from among OH, halogen, -C₁₋₃-fluoroalkyl, C₁₋₆-alkyl, C₆₋₁₀-aryl, -COO(C₁₋₃-alkyl) and O-(C₁₋₃-alkyl),
or wherein
**R³** denotes -O-R^{3.1},
wherein **R^{3.1}** is a group selected from among -C₁₋₆-alkyl, -C₆₋₁₀-aryl, -C₁₋₃-alkylene-C₆₋₁₀-aryl, hetaryl and het,
which may optionally be substituted in the ortho, para or meta position by one, two or three groups independently of one another selected from among fluorine, chlorine, bromine, hydroxy, CN, C₁₋₆- alkyl, C₁₋₃-fluoroalkyl, CO-(C₁₋₅-alkyl), -CO-(C₁₋₃-fluoroalkyl), -CO-NH-(C₁₋₆-alkylene)-hetaryl, -CO-N(C₁₋₃-alkyl)-(C₁₋₆-alkylene)-hetaryl, -CO-N(C₁₋₃-alkyl)-het, -CO-N(C₃₋₇-cycloalkyl)-het, -C₁₋₃-alkylene-OR^{2.1}, -C₁₋₃-alkylene-NR^{2.2}R^{2.3}, -NR^{2.2}R^{2.3}, O-R^{2.1}, SO-R^{2.1}, SO₂-R^{2.1}, COOH, COO-(C₁₋₄-alkyl), -O-C₁₋₃-alkylene-N(C₁₋₃-alkyl)₂, CO-NR^{2.2}R^{2.3}, NR^{2.2}-CO-R^{2.1}, C₆₋₁₀-aryl, C₆₋₁₀-aryl-C₁₋₂-alkylene, het-C₁₋₂-alkylene, -CO-het, het, -CO-C₃₋₇-cycloalkyl, -CO-N(C₁₋₃-alkyl)-C₃₋₇-cycloalkyl, C₃₋₇-cycloalkyl, C₃₋₇-cycloalkyl-C₁₋₂-alkylene, hetaryl-C₁₋₂-alkylene and hetaryl,
which in turn may optionally be substituted by 1, 2, 3 or 4 groups selected independently of one another from among F, Cl, Br, methyl, O-methyl, ethyl, O-ethyl, OH, oxo and CF₃.
and wherein
**R⁴** denotes H, CN, OH, CF₃, CHF₂, CH₂F, F, methyl, ethyl, -O-(C₁-₃-alkyl), -C₁₋₃-alkylene-OH, -COO(C₁₋₃-alkyl), -CO-het, -(C₁₋₂-alkylene)-NH-SO₂-(C₁₋₂-alkyl), -(C₁₋₂-alkylene)-N(C₁₋₃-alkyl)-SO₂-(C₁₋₂-alkyl), -(C₁₋₂-alkylene)-O-(C₁₋₂-alkylene)-C₆₋₁₀-aryl, -C₁₋₃-alkylene-O-C₁₋₃-alkyl, -(C₁₋₂-alkylene)-N(C₁₋₃-alkyl)-CO-(C₁₋₂-alkyl), -NH-CO-(C₁₋₃-alkylene)-O-(C₁₋₃-alkyl), -C₁₋₃-alkylene-NH-CO-(C₁₋₃-alkyl), -C₁₋₃-alkylene-NH-CO-(C₁₋₃-alkylene)-N(C₁₋₃-alkyl)₂, -O-(C₁₋₂-alkylene)-(C₆₋₁₀-aryl), -C₁₋₃-alkylene-NH-CO-(C₁₋₃-alkylene)-O-(C₁₋₃-alkyl), -CO-(C₆₋₁₀-aryl), -(C₁₋₂-alkylene)-N(C₁₋₃-alkyl)-CO-(C₁₋₂-alkylene)-O-(C₁₋₃-alkyl), wherein the aryl in the above groups may optionally in turn be substituted by one or more further groups selected from among F, Cl, Br, methyl, ethyl, propyl, isopropyl, cyclopropyl, -O-methyl, -O-ethyl,-O-propyl, -O-isopropyl, -O-cyclopropyl, -OH and CF₃,
or wherein
**R³** and **R⁴** together form a mono- or bicyclic, unsaturated, saturated or partly saturated heterocyclic group, which contains 1, 2 or 3 heteroatoms selected from among N, O and S and which may optionally be substituted by one or more groups selected from among halogen, OH, oxo, C₁₋₃-fluoroalkyl, CN, C₁₋₆-alkyl, -O-R^{2.1}, -COOR^{2.1}, SO-R^{2.1}, SO₂-R^{2.1}, -C₁₋₃-alkylene-NR^{2.2}R^{2.3}, -NR^{2.2}R^{2.3}, C₆₋₁₀-aryl, C₃₋₇-cycloalkyl, het and hetaryl,
it contains at least one EP4 receptor antagonist (**2**).

2. Medicament combination according to claim 1, **characterised in that**, in addition to one or more, preferably one, PDE4 inhibitor (**1**) of general formula **1**, 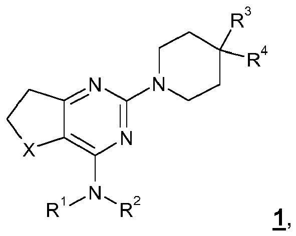 wherein
**X** denotes SO or SO₂,
**R¹** denotes H, C₁₋₆-alkyl,
**R²** is H or a group selected from among C₁₋₁₀-alkyl and C₂₋₆-alkenyl, which may optionally be substituted by one or more groups selected from halogen and C₁₋₃-fluoroalkyl or which is optionally substituted by one or more groups selected from among OR^{2.1}, COOR^{2.1}, CONR^{2.2}R^{2.3}, SR^{2.1}, SO-R^{2.1}, SO₂-R^{2.1}, C₆₋₁₀-aryl, het, hetaryl, a mono- or bicyclic C₃₋₁₀-cycloalkyl, CH₂-NR^{2.2}R^{2.3} and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, C₆₋₁₀-aryl, COOR^{2.1}, CH₂-NR²²R²³ and NR^{2.2}R^{2.3},
wherein
**het** is a three- to eleven-membered, mono- or bicyclic, saturated or partly saturated, optionally annellated or optionally bridged heterocyclic group, which contains 1, 2, 3 or 4 heteroatoms selected independently of one another from among N, S or O, and wherein
**hetaryl** is a five- to ten-membered, mono- or bicyclic, optionally annellated heteroaryl, which contains 1, 2, 3 or 4 heteroatoms selected independently of one another from among N, S or O,
and wherein
**cycloalkyl** may be saturated or partly saturated,
wherein **R^{2.1}** is H or a group selected from among C₁₋₆-alkyl, C₁₋₆-alkanol, C₁₋₃-haloalkyl, mono- or bicyclic, -C₃₋₁₀-cycloalkyl, C₆₋₁₀-aryl-C₁₋₆-alkylene, hetaryl-C₁₋₆-alkylene, het-C₁₋₆-alkylene, C₃₋₁₀-cycloalkyl-C₁₋₆-alkylene, a mono- or bicyclic C₆₋₁₀-aryl, heteroaryl and a -het,
which may optionally be substituted by one or more groups selected from among OH, O-(C₁₋₃-alkyl), halogen, C₁₋₆-alkyl and C₆₋₁₀-aryl,
wherein **R^{2.2}** and **R^{2.3}** independently of one another denote H or a group selected from among C₁₋₆-alkyl, mono- or bicyclic C₃₋₁₀cycloalkyl C₆₋₁₀-aryl-C₁₋₆-alkylene, hetaryl-C₁₋₆-alkylene, mono- or bicyclic C₆₋₁₀-aryl, het, hetaryl, CO-NH₂, CO-NHCH₃, -CO-N(CH₃)₂, SO₂-(C₁-C₂-alkyl), CO-R^{2.1} and COOR^{2.1},
which may optionally be substituted by one or more groups selected from among OH, halogen, C₁₋₆-alkyl, C₆₋₁₀-aryl and COOR^{2.1},
or
**R²** denotes a mono- or polycyclic C₃₋₁₀ cycloalkyl, which may optionally be singly or multiply bridged by C₁₋₃-alkyl groups and which may optionally be substituted by a group selected from among branched or unbranched C₁₋₆-alkanol, C₁₋₃-fluoroalkyl, C₁₋₃-alkylene-OR^{2.1}, OR^{2.1}, COOR^{2.1}, -SO₂-NR^{2.2}R^{2.3} het, -NH-CO-O-(C₁₋₆-alkyl), -NH-CO-(C₁₋₆-alkyl), -NH-CO-O-(C₆₋₁₀-aryl), -NH-CO-(C₆₋₁₀-aryl), -NH-CO-O-hetaryl, -NH-CO-hetaryl, -NH-CO-O-(C₁₋₃-alkylene)-(C₆₋₁₀-aryl), -NH-CO-(C₁₋₃-alkylene)-(C₆₋₁₀-aryl), -N(C₁₋₃-alkyl)-CO-(C₁₋₆-alkyl), -N(C₁₋₃-alkyl)-CO-O-(C₆₋₁₀-aryl), -N(C₁₋₃-alkyl)-CO-(C₆₋₁₀-aryl), -N(C₁₋₃-alkyl)-CO-O-hetaryl, -N(C₁₋₃-alkyl)-CO-hetaryl, -N(C₁₋₃-alkyl)-CO-O-(C₁₋₃-alkylene)-(C₆₋₁₀-aryl), -N(C₁₋₃-alkyl)-CO-(C₁₋₃-alkylene)-(C₆₋₁₀-aryl), C₆₋₁₀-aryl, C₁₋₆-alkyl, C₆₋₁₀-aryl-C₁₋₆-alkylene, hetaryl-C₁₋₆-alkylene, mono- or bicyclic C₃₋₁₀ cycloalkyl and NR^{2.2}R^{2.3},
which may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
**R²** denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₃₋₁₀-cycloalkyl, het, C₁₋₆-alkyl, C₁₋₃-fluoroalkyl, CF₃, CHF₂, CH₂F, C₆₋₁₀-aryl-C₁₋₆-alkylene, het-C₁₋₆-alkylene, hetaryl-C₁₋₆-alkylene, C₆₋₁₀-aryl, SO₂-CH₃, SO₂CH₂CH₃ and SO₂NR^{2.2}R^{2.3},
which in turn may optionally be substituted by one or more or several groups selected from among OH, OR^{2.1}, CF₃, CHF₂, CH₂F, oxo, halogen, CF₃, CHF₂, CH₂F, C₁₋₆-alkyl, C₆₋₁₀-aryl and NR^{2.2}R^{2.3},
or
**R²** denotes a group selected from among het and hetaryl, which may optionally be substituted by one or more groups selected from among halogen, OH, oxo, CF₃, CHF₂ and CH₂F or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, mono- or bicyclic C₃₋₁₀-cycloalkyl, C₆₋₁₀-aryl, C₁₋₆-alkyl, C₆₋₁₀-aryl-C₁₋₆-alkylene, hetaryl-C₁₋₆-alkylene, het, hetaryl, C₁₋₃-alkylene-OR^{2.1} and NR^{2.2}R^{2.3},
which may optionally in turn be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or wherein
**NR¹R²** together denotes a heterocyclic C₄₋₇ ring which may optionally be bridged, which contains 1, 2 or 3 heteroatoms selected from among N, O and S and which may optionally be substituted by one or more groups selected from among OH, OR^{2.1}, C₁₋₃-alkylene-O^{R.1}, oxo, halogen, C₁₋₆-alkyl, C₆₋₁₀-aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} and NR^{2.2}R^{2.3},
and wherein
**R³** is a C₆₋₁₀-aryl,
which is optionally substituted in the ortho, para or meta position by one, two or three groups selected independently of one another from among fluorine, chlorine, bromine, hydroxy, CN, C₁₋₆-alkyl, C₁₋₃-fluoroalkyl, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-alkylene)-hetaryl, -CO-NH-hetaryl, -CO-N(CH₃)-het, -CO-N(CH₃)-(C₁₋₃-alkylene)-het, -CO-N(CH₃)-(C₁₋₃-alkylene)-hetaryl, -CO-N(C₃₋₇cycloalkyl)-het, -CO-NR^{2.2}R^{2.3}, -CO-NH-(C₁₋₆-alkylene)-het, NR^{2.2}-CO-R^{2.1}, C₆₋₁₀-aryl, C₆₋₁₀-aryl-C₁₋₂-alkylene, het-C₁₋₂-alkylene, -het, -CO-het, CO-N(CH₃)-C₃₋₇-cycloalkyl, C₃₋₇-cycloalkyl, C₃₋₇-cycloalkyl-C₁₋₂-alkylene, hetaryl-C₁₋₂-alkylene and hetaryl,
wherein this group may optionally be substituted by one or more groups selected from among OH, halogen, -C₁₋₃-fluoroalkyl, oxo, methyl and phenyl,
or wherein
**R³** is a group selected from among het and hetaryl, which may optionally be substituted by one or more groups selected from among halogen, C₁₋₃-fluoroalkyl, CN, OH, oxo, -C₁₋₆-alkyl, -C₁₋₃-alkylene-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-aryl, het, C₃₋₇-cycloalkyl and hetaryl,
which in turn may optionally be substituted by one or more groups selected from among OH, halogen, -C₁₋₃-fluoroalkyl, C₁₋₆-alkyl, C₆₋₁₀-aryl, -COO(C₁₋₃-alkyl) and O-(C₁₋₃-alkyl),
or wherein
**R³** denotes -O-R^{3.1},
wherein **R^{3.1}** is a group selected from among -C₁₋₆-alkyl, -C₆₋₁₀-aryl, -C₁₋₃-alkylene-C₆₋₁₀-aryl, hetaryl and het,
which may optionally be substituted in the ortho, para or meta position by one, two or three groups independently of one another selected from among fluorine, chlorine, bromine, hydroxy, CN, C₁₋₆- alkyl, C₁₋₃-fluoroalkyl, CO-(C₁₋₅-alkyl), -CO-(C₁₋₃-fluoroalkyl), -CO-NH-(C₁₋₆-alkylene)-hetaryl, -CO-N(C₁₋₃-alkyl)-(C₁₋₆-alkylene)-hetaryl, -CO-N(C₁₋₃-alkyl)-het, -CO-N(C₃₋₇-cycloalkyl)-het, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOH, COO-(C₁₋₄-alkyl), -O-C₁₋₃-alkylene-N(C₁₋₃-alkyl)₂, CO-NR^{2.2}R^{2.3}, NR^{2.2}-CO-R^{2.1}, C₆₋₁₀-aryl, C₆₋₁₀-aryl-C₁₋₂-alkylene, het-C₁₋₂-alkylene, -CO-het, het, -CO-C₃₋₇-cycloalkyl, -CO-N(C₁₋₃-alkyl)-C₃₋₇-cycloalkyl, C₃₋₇-cycloalkyl, C₃₋₇-cycloalkyl-C₁₋₂-alkylene, hetaryl-C₁₋₂-alkylene and hetaryl,
which in turn may optionally be substituted by 1, 2, 3 or 4 groups selected independently of one another from among F, Cl, Br, methyl, O-methyl, ethyl, O-ethyl, OH, oxo and CF₃.
and wherein
**R⁴** denotes H, CN, OH, CF₃, CHF₂, CH₂F, F, methyl, ethyl, -O-(C₁₋₃-alkyl), -C₁₋₃-alkylene-OH, -COO(C₁₋₃-alkyl), -CO-het, -(C₁₋₂-alkylene)-NH-SO₂-(C₁₋₂-alkyl), -(C₁₋₂-alkylene)-N(C₁₋₃-alkyl)-SO₂-(C₁₋₂-alkyl), -(C₁₋₂-alkylene)-O-(C₁₋₂-alkylene)-C₆₋₁₀-aryl, -C₁₋₃-alkylene-O-C₁₋₃-alkyl, -(C₁₋₂-alkylene)-N(C₁₋₃-alkyl)-CO-(C₁₋₂-alkyl), -NH-CO-(C₁₋₃-alkylene)-O-(C₁₋₃-alkyl), -C₁₋₃-alkylene-NH-CO-(C₁₋₃-alkyl), -C₁₋₃-alkylene-NH-CO-(C₁₋₃-alkylene)-N(C₁₋₃-alkyl)₂, -O-(C₁₋₂-alkylene)-(C₆₋₁₀-aryl), -C₁₋₃-alkylene-NH-CO-(C₁₋₃-alkylene)-O-(C₁₋₃-alkyl), -CO-(C₆₋₁₀-aryl), -(C₁₋₂-alkylene)-N(C₁₋₃-alkyl)-CO-(C₁₋₂alkylene)-O-(C₁₋₃-alkyl),
wherein the aryl in the above groups may optionally in turn be substituted by one or more further groups selected from among F, Cl, Br, methyl, ethyl, propyl, isopropyl, cyclopropyl, -O-methyl, -O-ethyl, -O-propyl, -O-isopropyl, -O-cyclopropyl, -OH and CF₃,
or wherein
**R³** and **R⁴** together form a mono- or bicyclic, unsaturated, saturated or partly saturated heterocyclic group, which contains 1, 2 or 3 heteroatoms selected from among N, O and S and which may optionally be substituted by one or more groups selected from among halogen, OH, oxo, C₁₋₃-fluoroalkyl, CN, C₁₋₆-alkyl, -O-R^{2.1}, -COOR^{2.1}, SO-R^{2.1}, SO₂-R^{2.1}, -C₁₋₃-alkylene-NR^{2.2}R^{2.3}, -NR^{2.2}R^{2.3}, C₆₋₁₀-aryl, C₃₋₇-cycloalkyl, het and hetaryl,
it contains at least one EP4 receptor antagonist (**2**).

3. Medicament combination according to one of claims 1 to 2, **characterised in that**, in addition to one or more, preferably one, PDE4 inhibitor (**1**) of general formula **1**,
wherein
**X** is SO,
**R¹** is H
**R²** is H or C₁₋₆-alkyl, which may optionally be substituted by one or more groups selected from F, Cl, CF₃, CHF₂ or CH₂F or which may optionally be substituted by one or more groups selected from among OR^{2.1}, COOR^{2.1}, CONR^{2.2}R^{2.3}, SR^{2.1}, SO-R^{2.1}, SO2-R^{2.1}, phenyl, het, hetaryl, a monocyclic C₃₋₇-cycloalkyl, CH₂-NR^{2.2}R^{2.3} and NR^{2.2}R^{2.3},
which in turn may optionally be substituted by one or more groups selected from among OH, F, Cl, Br, CF₃, CHF₂, CH₂F, OR^{2.1}, oxo, methyl, ethyl, propyl, isopropyl, methanol, ethanol, phenyl, COOR^{2.1}, CH₂-NR^{2.2}R^{2.3} and NR^{2.2}R^{2.3},
wherein
**het** is a three- to seven-membered, monocyclic, saturated or partly saturated heterocyclic group or a seven- to eleven-membered, bicyclic, saturated or partly saturated heterocyclic group which contains 1, 2 or 3 heteroatoms selected independently of one another from among N, S or O, and wherein
**hetaryl** is a five- to six-membered, monocyclic, aromatic heteroaryl, or a seven- to eleven-membered, bicyclic, aromatic heteroaryl which contains 1, 2 or 3 heteroatoms selected independently of one another from among N, S or O,
and wherein
**cycloalkyl** may be saturated or partly saturated,
wherein **R^{2.1}** is H or a group selected from among methyl, ethyl, propyl, isopropyl, methanol, ethanol, monocyclic C₃₋₇-cycloalkyl, phenyl-C₁₋₂-alkylene, -hetaryl-C₁₋₂-alkylene, -het-C₁₋₂-alkylene, C₃₋₇-cycloalkyl-C₁₋₂-alkylene, phenyl, hetaryl and a het,
which may optionally be substituted by one or more groups selected from among OH, F, Cl, methyl, ethyl, propyl, isopropyl, O-methyl, O-ethyl, O-propyl, O-isopropyl and phenyl,
wherein **R^{2.2}** and **R^{2.3}** independently of one another denote H or a group selected from among methyl, ethyl, propyl, isopropyl, monocyclic C₃₋₇-cycloalkyl, phenyl-C₁₋₃-alkylene, hetaryl-C₁₋₃-alkylene, phenyl, -het, -hetaryl, CO-NH₂, CO-NHCH₃, CON(CH₃)₂, SO₂-(C₁₋₂-alkyl), CO-R^{2.1} and COOR^{2.1},
which may optionally be substituted by one or more groups selected from among OH, F, Cl, methyl, ethyl, propyl, isopropyl, phenyl and COOR^{2.1},
or
**R²** denotes a monocyclic C₃₋₇ cycloalkyl, which may optionally be substituted by a group selected from among C₁₋₂-alkanol, C₁₋₃-fluoroalkyl, C₁₋₃-alkylene-OR^{2.1}, OR^{2.1}, COOR^{2.1}, SO₂-NR^{2.2}R^{2.3}, -het, -NH-CO-O-(phenyl), methyl, ethyl, propyl, isopropyl, phenyl, phenyl-C₁₋₂-alkylene, -hetaryl-C₁₋₂-alkylene, monocyclic C₃₋₇ cycloalkyl and NR^{2.2}R^{2.3},
which may optionally be substituted by one or more groups selected from among OH, OR^{2.1}, oxo, F, Cl, CF₃, CHF₂, CH₂F, methyl, ethyl, propyl, isopropyl, phenyl and NR^{2.2}R^{2.3},
or
**R²** denotes a phenyl which may optionally be substituted by OH, SH, F, Cl or Br or by one or more groups selected from among OR^{2.1}, COOR^{2.2}, NR^{2.2}R^{2.3}, CH₂-NR^{2.2}R^{2.3}, monocyclic C₃₋₇-cycloalkyl, -het, methyl, ethyl, propyl, isopropyl, CF₃, CHF₂, CH₂F, phenyl-C₁₋₂-alkylene, het-C₁₋₂-alkylene, hetaryl-C₁₋₂-alkylene, phenyl, SO₂-CH₃, SO₂-CH₂CH₃ and SO₂-NR^{2.2}R^{2.3}, which in turn may optionally be substituted by one or more or several groups selected from among OH, OR^{2.1}, oxo, F, Cl, CF₃, CHF₂, CH₂F, methyl, ethyl, propyl, isopropyl, phenyl and NR^{2.2}R^{2.3},
or
**R²** denotes a group selected from among het and hetaryl,
which may optionally be substituted by one or more groups selected from among F, Cl, OH, oxo, CF₃, CHF₂ and CH₂F or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, methanol, ethanol, monocyclic C₃₋₇-cycloalkyl, phenyl, methyl, ethyl, propyl, isopropyl, phenyl-C₁₋₂-alkylene, hetaryl-C₁₋₂-alkylene, -het, -hetaryl and NR^{2.2}R^{2.3},
which may in turn optionally be substituted by one or more groups selected from among OH, OR².¹, oxo, F, Cl, CF₃, CHF₂, CH₂F, methyl, ethyl, propyl, isopropyl, butyl, isobutyl, phenyl and NR^{2.2}R^{2.3},
and wherein
**R³** is a naphthalene or phenyl,
which may optionally be substituted in the ortho, para or meta position by one or two groups independently of one another selected from among fluorine, chlorine, bromine, hydroxy, CN, methyl, ethyl, propyl, isopropyl, cyclopropyl, CF₃, CHF₂, CH₂F, -OCH₃, OCH₂CH₃; SO₂-CH₃, SO-CH₃, COOCH₃, COOCH₂CH₃, -CO-NH-(methylene)-hetaryl, -CO-NH-(ethylene)-hetaryl, -CO-NH-hetaryl, -CO-N(CH₃)-het, -CO-N(CH₃)-(methylene)-het, -CO-N(CH₃)-(ethylene)-het, -CO-N(CH₃)-(methylene)-hetaryl, -CO-N(CH₃)-(ethylene)-hetaryl, -CO-N(cyclopropyl)-het, CO-NH₂, CONH(CH₃), CON(CH₃)₂, -CO-NH-(methylene)-het, -CO-NH-(ethylene)-het, -NH-CO-methyl, NCH₃-CO-methyl,-NH-CO-ethyl, NCH₃-CO-ethyl, -NH-CO-propyl, NCH₃-CO-propyl, -NH-CO-isopropyl, NCH₃-CO-isopropyl, phenyl, phenyl-methylene, phenyl-ethylene, het-methylene, het-ethylene, -het, -CO-het, -CO-N(CH₃)-het, CO-N(CH₃)-cyclopropyl, C₃₋₇-cycloalkyl, C₃₋₇-cycloalkyl-methylene, C₃₋₇-cycloalkyl-ethylene, hetaryl-methylene, hetaryl-ethylene, -hetaryl, CH₂-NH₂, CH₂-NH(CH₃), CH₂-N(CH₃)₂, -NH₂, -NH(CH₃) and -N(CH₃)₂, wherein this group may optionally be substituted by one or more groups selected from among OH, F, Cl, -CF₃, CHF₂, CH₂F, oxo, methyl and phenyl,
or wherein
**R³** denotes a group selected from among a het and hetaryl which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO-(CH₃), SO-(CH₂-CH₃), SO₂-(CH₃), SO₂-(CH₂-CH₃), phenyl, CH₂-NH₂, CH₂-NH(CH₃), CH₂-(CH₃)₂, -NH₂, -NH(CH₃), -N(CH₃)₂, het and hetaryl,
which in turn may optionally be substituted by one or more groups selected from among OH, F, Cl, CF₃, CHF₂, CH₂F, methyl, ethyl, propyl, isopropyl, phenyl, -COO-methyl, -COO-ethyl and O-methyl, O-ethyl,
or wherein
**R³** denotes -O-R^{3.1},
wherein **R^{3.1}** is a group selected from among -C₁₋₃-alkyl, -phenyl, -C₁₋₃-alkylene-phenyl, hetaryl and het,
which may optionally be substituted in the ortho, para or meta position by one, two or three groups independently of one another selected from among fluorine, chlorine, bromine, hydroxy, CN, methyl, ethyl, propyl, isopropyl, butyl, isobutyl, CF₃, CHF₂, CH₂F, CO-(methyl), CO-(ethyl), CO-(propyl), CO-(isopropyl), -CO-(CF₃), -CO-NH-(methylene)-hetaryl, -CO-NH-(ethylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl, -CO-N(CH₃)-het, -CO-N(cyclopropyl)-het, -CO-N(C₅₋₇-cycloalkyl)-het, -methylene-O-methyl, -ethylene-O-methyl, -propylene-O-methyl, -methylene-O-ethyl, -ethylene-O-ethyl, -propylene-O-ethyl, -methylene-NH₂, -methylene-NHCH₃, -methylene-N(CH₃)₂, -ethylene-NH₂, -ethylene-NHCH₃, -ethylene-N(CH₃)₂, NH₂, N(CH₃)₂, NHCH₃, -O-methyl, O-ethyl, O-propyl, O-isopropyl, O-butyl, O-isobutyl, -SO-CH₃, SO-ethyl, -SO-propyl, -SO-isopropyl, SO₂-methyl, -SO₂-ethyl, SO₂-propyl, SO₂-isopropyl, COOH, COO-(methyl), COO-(ethyl), COO-(propyl), COO-(isopropyl), -O-methylene-N(methyl)₂, -O-ethylene-N(methyl)₂, -O-methylene-N(ethyl)₂, -O-ethylene-N(ethyl)₂, CO-NH₂, CO-NH(CH₃), CO-N(CH₃)₂, -NH-CO-methyl, -NCH₃-CO-methyl, -NH-CO-ethyl, NCH₃-CO-ethyl, phenyl, phenyl-methylene, phenyl-ethylene, het-methylene, het-ethylene, -CO-het, het, -CO-C₅₋₇-cycloalkyl, -CO-cyclopropyl, -CO-N(CH₃)-C₅₋₇-cycloalkyl, -CO-N(CH₃)-cyclopropyl, C₅₋₇-cycloalkyl, cyclopropyl, C₅₋₇-cycloalkyl-methylene, C₅₋₇-cycloalkyl-ethylene, cyclopropyl-methylene, cyclopropyl-ethylene, hetaryl-methylene, hetaryl-ethylene and hetaryl,
which in turn may optionally be substituted by 1, 2, 3 or 4 groups selected independently of one another from among F, Cl, Br, methyl, O-methyl, ethyl, O-ethyl, OH, oxo and CF₃,
and wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl), -(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl -ethylene-O-ethyl, -(methylene)-N(CH₃)-CO-(methyl), -(methylene)-N(CH₃)-CO-(ethyl) -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-CO-(ethylene)-N(methyl)₂, -methylene-N H-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(methyl), -(ethylene)-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(methylene)-O-(ethyl), -O-(methylene)-phenyl, -O-(ethylene)-phenyl, -CO-phenyl,
wherein the phenyl in the above groups may optionally be substituted by one or more other groups selected from among F, Cl, Br, methyl, ethyl, propyl, -O-methyl, -O-ethyl, -O-propyl, -OH and CF₃
or wherein
**R³** and **R⁴** together form a mono- or bicyclic, unsaturated, saturated or partly saturated heterocyclic group, which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, phenyl, C₅₋₇-cycloalkyl, het and hetaryl,
it contains at least one EP4 receptor antagonist (**2**).

4. Medicament combination according to one of claims 1 to 3, **characterised in that**, in addition to one or more, preferably one PDE4 inhibitor of general formula **1**, wherein
**R²** denotes a group of formula **3** 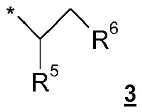
wherein **R⁶** is OH or NH₂ and
wherein **R⁵** denotes a group selected from among C₁₋₄-alkyl, a five- to six-membered heteroaryl with 1, 2 or 3 heteroatoms selected from among S, O and N and phenyl, while this group may optionally be substituted by one or more groups selected from among OH, F, Br, OR^{2.1}, oxo, methyl, ethyl, methanol, ethanol, phenyl, COOR^{2.1}, CH₂-NR^{2.2}R^{2.3} and NR^{2.2}R^{2.3},
it contains at least one EP4 receptor antagonist (**2**).

5. Medicament combination according to claim 4, **characterised in that**, in addition to one or more, preferably one, PDE4 inhibitor of general formula **1**, wherein
**R²** is a group according to formula **3** 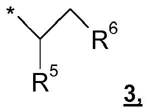
wherein **R⁶** is OH or NH₂ and
wherein **R⁵** denotes methyl, ethyl, propyl or isopropyl,
it contains at least one EP4 receptor antagonist (**2**).

6. Medicament combination according to one of claims 1 to 3, **characterised in that**, in addition to one or more, preferably one, PDE4 inhibitor of general formula **1**, wherein
**R²** is a monocyclic three-, four-, five-, six- or seven-membered cycloalkyl ring which may optionally be substituted in the spiro position by a group selected from among -CH₂-OR^{2.1}, branched or unbranched C₂₋₆-alkylene-OR^{2.1}, methyl, ethyl, propyl, isopropyl, butyl, isobutyl, cyclopropyl, -CF₃, CHF₂, CH₂F and C₂₋₄-fluoroalkyl, and wherein
R^{2.1} is selected from among methyl, ethyl, propyl, isopropyl, butyl, and isobutyl,
it contains at least one EP4 receptor antagonist (**2**).

7. Medicament combination according to one of claims 1 to 3, **characterised in that**, in addition to one or more, preferably one, PDE4 inhibitor of general formula **1**, wherein
**R²** is a cyclopropyl which may optionally be substituted by another group selected from among -NH₂, CH₂-NH₂, -NH(CH₃), -N(CH₃)₂, methyl, ethyl, propyl, isopropyl, -NH-CO-(tert-butyl), -NH-CO-O-(tert-butyl), -N(CH₃)-CO-(tert-butyl), -N(CH₃)-CO-O-(tert-butyl), -CF₃, -CHF₂, CH₂F, F, Cl and Br,
it contains at least one EP4 receptor antagonist (**2**).

8. Medicament combination according to one of claims 1 to 3, **characterised in that**, in addition to one or more, preferably one PDE4 inhibitor of general formula **1**, wherein
**R²** is a phenyl which may optionally be substituted in one or both meta positions by one or more groups selected from among methyl, ethyl, propyl, isopropyl, cyclopropyl, F, Cl, Br, OH, OR^{2.1}, COOR^{2.1}, CF₃, CHF₂, CH₂F, NH₂, NH(CH₃) and N(CH₃)₂, wherein R^{2.1} may be H, methyl or ethyl,
it contains at least one EP4 receptor antagonist (**2**).

9. Medicament combination according to one of claims 1 to 3, **characterised in that**, in addition to one or more, preferably one PDE4 inhibitor of general formula **1**, wherein
**R²** is a monocyclic, saturated three-, four, five, six- or seven-membered heterocyclic group with 1, 2 or 3 heteroatoms selected in each case from among N, O and S, which may optionally be substituted by one or more groups selected from among fluorine, chlorine, bromine, CF₃, CHF₂, CH₂F, OH and oxo or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, C₃₋₁₀-cycloalkyl, phenyl, C₁₋₆-alkyl, phenyl-C₁-₆-alkylene, hetaryl-C₁₋₆-alkylene, het, hetaryl and NR^{2.2}R^{2.3}, while the latter group may in turn optionally be substituted by one or more groups selected from among OH, OR^{2.1}, oxo, F, Cl, CF₃, CHF₂, CH₂F, C₁₋₆-alkyl, phenyl and NR^{2.2}R^{2.3},
wherein **R^{2.1}**, **R^{2.2}** and **R^{2.3}** are defined as in claim 1,
it contains at least one EP4 receptor antagonist (**2**)

10. Medicament combination according to claim 9, **characterised in that**, in addition to one or more, preferably one, PDE4 inhibitor of general formula **1**, wherein
**R²** denotes a monocyclic, saturated six-membered heterocyclic group with a heteroatom selected from among N, O and S, which may optionally be substituted by one or more groups selected from among F, Cl, Br, CF₃, CHF₂, CH₂F, OH, oxo, NH₂, NHCH₃, N(CH₃)₂, methyl, ethyl, propyl, isopropyl, cyclopropyl, methoxy and ethoxy,
it contains at least one EP4 receptor antagonist (**2**).

11. Medicament combination according to one of claims 1 to 3, **characterised in that**, in addition to one or more, preferably one, PDE4 inhibitor of general formula **1**, wherein
**R²** denotes a group selected from among piperidine or tetrahydropyran, which may optionally be substituted by one or more groups selected from among F, Cl, Br, OH, CF₃, CHF₂, CH₂F, NH₂, NHCH₃, N(CH₃)₂, oxo, methyl and methoxy,
it contains at least one EP4 receptor antagonist (**2**).

12. Medicament combination according to one of claims 1 to 11, **characterised in that**, in addition to one or more, preferably one, PDE4 inhibitor of general formula **1**, wherein
**R³** is a naphthalene or phenyl,
which may optionally be substituted in any desired position by one, two or three groups selected independently of one another from among fluorine, chlorine, bromine, hydroxy, CN, methyl, ethyl, propyl, isopropyl, cyclopropyl, CF₃, CHF₂, CH₂F, -OCH₃, OCH₂CH₃; SO₂-CH₃, SO₂-CH₂CH₃, COOCH₃ and CO-O-CH₂CH₃,
it contains at least one EP4 receptor antagonist (**2**).

13. Medicament combination according to one of claims 1 to 11, **characterised in that**, in addition to one or more, preferably one, PDE4 inhibitor of general formula **1**, wherein
**R³** is a group selected from among het and hetaryl, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, C₅₋₇-cycloalkyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, het and hetaryl, which may in turn optionally be substituted by one or more groups selected from among OH, F, Cl, Br, CF₃, CHF₂, CH₂F, methyl, ethyl, propyl, isopropyl, phenyl, -COO-methyl, -COO-ethyl, -COO-propyl, -COO-isopropyl and O-methyl, O-ethyl, O-propyl and O-isopropyl,
and wherein
**R⁴** denotes H, CN, OH, CF₃, CHF₂, CH₂F, F, methyl, ethyl, O-methyl or O-ethyl,
and
wherein
**het** is a three- to seven-membered, monocyclic, saturated or partly saturated heterocyclic group or a seven- to eleven-membered, bicyclic, annellated, saturated or partly saturated heterocyclic group, which contains 1, 2 or 3 heteroatoms selected independently of one another from among N, S or O,
and wherein
**hetaryl** is a five- to six-membered, monocyclic, aromatic heteroaryl or a seven-to eleven-membered, bicyclic, annellated, aromatic heteroaryl which contains in each case 1, 2 or 3 heteroatoms selected independently of one another from among N, S or O,
and wherein
**cycloalkyl** may be saturated or partly saturated,
it contains at least one EP4 receptor antagonist (**2**).

14. Medicament combination according to one of claims 1 to 11, **characterised in that**, in addition to one or more, preferably one, PDE4 inhibitor of general formula **1**, wherein
**R³** denotes a group selected from a bicyclic, seven- to eleven-membered, saturated or partly saturated heterocyclic group or a bicyclic, seven- to eleven-membered heteroaryl, which is selected from among indole, dihydroindole, quinazoline, dihydroquinazoline, tetrahydroquinazoline, benzisoxazole, dihydrobenzisoxazole, benzoxazine, dihydrobenzoxazine, benzothiazole, dihydrobenzothiazole, triazolopyridine, dihydrotriazolopyridine, benzofuran, dihydrobenzofuran, isobenzofuran and dihydroisobenzofuran,
which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl, which may in turn be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
it contains at least one EP4 receptor antagonist (**2**).

15. Medicament combination according to one of claims 1 to 11, **characterised in that**, in addition to one or more, preferably one, PDE4 inhibitor of general formula **1**, wherein
**R³** is a group selected from a monocyclic, saturated or partly saturated, three- to seven-membered heterocyclic group or a monocyclic five- to six-membered heteroaryl,
which is selected from among imidazole, dihydroimidazole, oxadiazole, oxadiazolidine, pyrazole, pyridine and dihydropyrazole,
which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl, which may in turn be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
it contains at least one EP4 receptor antagonist (**2**).

16. Medicament combination according to one of claims 1 to 11, **characterised in that**, in addition to one or more, preferably one, PDE4 inhibitor of general formula **1**, wherein
**R³** and **R⁴** together form a mono- or bicyclic, unsaturated or partly saturated, three- to eleven-membered heterocyclic group which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), SO-(CH₃), SO-(CH₂-CH₃), phenyl, -CH₂-NH₂, -CH₂NHCH₃, -CH₂-N(CH₃)₂, NH₂, NHCH₃, N(CH₃)₂, a saturated or partly saturated, five- to six-membered heterocyclic group and a five- to six-membered heteroaryl,
it contains at least one EP4 receptor antagonist (**2**).

17. Medicament combination according to claim 16, **characterised in that**, in addition to one or more, preferably one, PDE4 inhibitor of general formula **1**, wherein
**R³** and **R⁴** together form a bicyclic heterocyclic group selected from among tetrahydroquinazoline, tetrahydrobenzoxazine and dihydroindole, dihydroisobenzofuran, which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), phenyl, -CH₂-NH₂, -CH₂NHCH₃, -CH₂-N(CH₃)₂, NH₂, NHCH₃, N(CH₃)₂, a saturated or partly saturated, five- or six-membered heterocyclic group and a five- or six-membered heteroaryl,
it contains at least one EP4 receptor antagonist (**2**).

18. Medicament combination according to one of claims 1 to 11, **characterised in that**, in addition to one or more, preferably one, PDE4 inhibitor of general formula **1**, wherein
**R³** is -O-R³¹,
**R^{3.1}** is a group selected from among methyl, ethyl, propyl, isopropyl, butyl, isobutyl, pentyl, isopentyl, -phenyl, -methylene-phenyl, -ethylene-phenyl, -propylene-phenyl, -isopropylene-phenyl, hetaryl and het, which may optionally be substituted in the ortho, para or meta position by one, two or three groups selected independently of one another from among fluorine, chlorine, bromine, hydroxy, CN, methyl, ethyl, propyl, isopropyl, butyl, isobutyl, -CF₃, CHF₂, CH₂F, CO-(methyl), CO-(ethyl), CO-(propyl), CO-(isopropyl), CO-(butyl), CO-(isobutyl), -CO-(CF₃), -CO-(CH₂F), -CO-(CHF₂), -CO-NH-(methylene)-hetaryl, -CO-NH-(ethylene)-hetaryl, -CO-NH-(propylene)-hetaryl, -CO-NH-(isopropylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl, -CO-N(CH₃)-het, -CO-N(C₃₋₇-cycloalkyl)-het, -methylene-O-methyl, -ethylene-O-methyl, -methylene-O-ethyl, -ethylene-O-ethyl, -methylene-NH₂, -ethylene-NH₂, -methylene-NHCH₃, -ethylene-NHCH₃, -methylene-N(CH₃)₂, -ethylene-N(CH₃)₂, -NH₂, -NHCH₃, -N(CH₃)₂, -O-methyl, -O-ethyl, -O-propyl, -O-isopropyl, -SO-CH₃, -SO-(CH₂CH₃), -SO₂-CH₃, -SO₂-(CH₂CH₃), COOH, COO-(methyl), COO-(ethyl), COO-(propyl), COO-(isopropyl), -O-methylene-N(methyl)₂, -O-ethylene-N(methyl)₂, -O-methylene-N(ethyl)₂, -O-ethylene-N(ethyl)₂, CO-NH₂, CO-NHCH₃, CO-N(CH₃)₂, NH-CO-methyl, NCH₃-CO-methyl, NH-CO-ethyl, N(CH₃)-CO-ethyl, phenyl, phenyl-methylene, phenyl-ethylene, het-methylene, het-ethylene, -CO-het, het, -CO-C₄₋₇-cycloalkyl, -CO-cyclopropyl, -CO-N(CH₃)-cyclopropyl, -CO-N(CH₃)-C₄₋₇-cycloalkyl, C₄₋₇-cycloalkyl, cyclopropyl, C₄₋₇-cycloalkyl-methylene, cyclopropyl-methylene, C₄₋₇-cycloalkyl-ethylene, cyclopropyl-ethylene, hetaryl-methylene, hetaryl-ethylene and hetaryl,
which in turn may optionally be substituted by 1, 2, 3 or 4 groups selected independently of one another from among F, Cl, Br, methyl, O-methyl, ethyl, O-ethyl, OH, oxo and CF₃,
it contains at least one EP4 receptor antagonist (**2**).

19. Medicament combination according to one of claims 1 to 11, **characterised in that**, in addition to one or more, preferably one, PDE4 inhibitor of general formula **1**, wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl), -(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl, -ethylene-O-ethyl, -(methylene)-N(CH₃)-CO-(methyl), -(methylene)-N(CH₃)-CO-(ethy), -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-CO-(ethylene)-N(methyl)₂, -methylene-NH-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(methyl), -(ethylene)-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(methylene)-O-(ethyl), -O-(methylene)-phenyl, -O-(ethylene)-phenyl, -CO-phenyl,
and wherein the phenyl in the above groups may optionally be substituted by one or more other groups selected from among F, Cl, Br, methyl, ethyl, propyl, -O-methyl, -O-ethyl, -O-propyl, -OH and CF₃,
it contains at least one EP4 receptor antagonist (**2**).

20. Medicament combination according to one of claims 1 to 11, **characterised in that**, in addition to one or more, preferably one, PDE4 inhibitor of general formula **1**, wherein
**R³** is a group selected from among oxazole, imidazole and thiazole, wherein this group may optionally be substituted by one, two or three further groups selected independently of one another from among methyl, ethyl, propyl, isopropyl, O-methyl, O-ethyl, O-propyl, O-isopropyl, OH, F, Cl, Br, CF₃, phenyl, hetaryl and C₃₋₆-cycloalkyl,
it contains at least one EP4 receptor antagonist (**2**).

21. Medicament combination according to one of claims 1 to 20, **characterised in that**, in addition to one or more, preferably one, PDE4 inhibitor of general formula **1** selected from among:
1.1. (R)-2-{2-[4-(4-chlorophenyl)-piperidin-1-yl]-5-oxo-6,7-dihydro-5H-5λ⁴-thieno[3,2-*d]*pyrimidin-4-ylamino}-3-methylbutan-1-ol
1.2. (1-{2-[4-(4-chlorophenyl)-piperidin-1-yl]-5-oxo-6,7-dihydro-5*H*-5λ⁴-thieno[3,2-*d]*pyrimidin-4-ylamino}-cyclopropyl)-methanol
1.3. (R)-2-{2-[4-(4-chlorophenyl)-piperidin-1-yl]-5-oxo-6,7-dihydro-5*H*-5λ⁴-thieno[3,2-*d]*pyrimidin-4-ylamino}-pentan-1-ol
1.4. (R)-1-{2-[4-(4-chlorophenyl)-piperidin-1-yl]-5-oxo-6,7-dihydro-5*H*-5λ⁴-thieno[3,2-*d]*pyrimidin-4-ylamino}-1-(4-fluorophenyl)-2-methylpropan-2-ol
1.5. (S)-5-{2-[4-(4-chlorophenyl)-piperidin-1-yl]-5-oxo-6,7-dihydro-5*H*-5λ⁴-thieno[3,2-*d]*pyrimidin-4-ylamino}-1-methylpiperidin-2-one
1.6. {2-[4-(4-chlorophenyl)-piperidin-1-yl]-5-oxo-6,7-dihydro-5*H*-5λ⁴-thieno[3,2-*d]*pyrimidin-4-yl}-(tetrahydropyran-4-yl)-amine
1.7. 1-(4-(1-hydroxymethylcyclopropylamino)-5-oxo-6,7-dihydro-5*H*-5λ⁴-thieno[3,2-*d]*pyrimidin-2-yl)-3'-methyl-1'*H*-spiro[piperidin-4,4'-quinazolin]-2'(3'*H*)-one
1.8. {1-[2-(4-benzo[*d*]isoxazol-3-yl-piperidin-1-yl)-5-oxo-6,7-dihydro-5*H*-5λ⁴-thieno[3,2-*d]*pyrimidin-4-ylamino]-cyclopropyl}-methanol
1.9. (1-{2-[4-(2-ethyl-5-fluoro-1*H*-indol-3-yl)-piperidin-1-yl]-5-oxo-6,7-dihydro-5*H-*5λ⁴-thieno[3,2-*d]*pyrimidin-4-ylamino}-cyclopropyl)-methanol
1.10. 1-[4-((S)-1-methyl-6-oxopiperidin-3-ylamino)-5-oxo-6,7-dihydro-5*H*-5λ⁴-thieno[3,2-*d]*pyrimidin-2-yl]-4-phenylpiperidin-4-carbonitrile
1.11. 3'-methyl-1-(4-(tetrahydro-2*H*-pyran-4-ylamino)-5-oxo-6,7-dihydro-5*H*-5λ⁴-thieno[3,2-*d]*pyrimidin-2-yl)-1'*H*-spiro[piperidin-4,4'-quinazolin]-2'(3'*H*)-one
1.12. (3-fluorophenyl)-[5-oxo-2-(3,4,5,6-tetrahydro-2*H*-[4,4']bipyridinyl-1-yl)-6,7-dihydro-5*H*-5λ⁴-thieno[3,2-*d]*pyrimidin-4-yl]-amine
1.13. {2-[4-(2-ethyl-5-fluoro-1*H*-indol-3-yl)-piperidin-1-yl]-5-oxo-6,7-dihydro-5*H*-5λ⁴-thieno[3,2-*d]*pyrimidin-4-yl}-(3-fluorophenyl)-amine
1.14. (1-{2-[4-(2,4-difluorophenyl)-piperidin-1-yl]-5-oxo-6,7-dihydro-5*H*-5λ⁴-thieno[3,2-*d]*pyrimidin-4-ylamino}-cyclopropyl)-methanol
1.15. {2-[4-(2,4-difluorophenyl)-piperidin-1-yl]-5-oxo-6,7-dihydro-5*H*-5λ⁴-thieno[3,2-*d]*pyrimidin-4-yl}-(tetrahydropyran-4-yl)-amine
1.16. (S)-5-[2-(4-benzoxazol-2-yl-piperidin-1-yl)-5-oxo-6,7-dihydro-5*H*-5λ⁴-thieno[3,2-*d]*pyrimidin-4-ylamino]-1-methylpiperidin-2-one
1.17. (1-{2-[4-(6-fluorobenzo[*d*]isoxazol-3-yl)-piperidin-1-yl]-5-oxo-6,7-dihydro-5*H-*5λ⁴-thieno[3,2-*d]*pyrimidin-4-ylamino}-cyclopropyl)-methanol
1.18. (1-{2-[4-(5-fluorobenzo[*d*]isoxazol-3-yl)-piperidin-1-yl]-5-oxo-6,7-dihydro-5*H-*5λ⁴-thieno[3,2-*d]*pyrimidin-4-ylamino}-cyclopropyl)-methanol
1.19. {2-[4-(5-furan-2-yl-2*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.20. (3-fluorophenyl)-{5-oxo-2-[4-(3-pyridin-4-yl-[1,2,4]oxadiazol-5-yl)-piperidin-1-yl]-6,7-dihydro-5*H*-5λ⁴-thieno[3,2-*d]*pyrimidin-4-yl}-amine
1.21. (R)-3-methyl-2-{5-oxo-2-[4-(3-pyridin-4-yl-[1,2,4]oxadiazol-5-yl)-piperidin-1-yl]-6,7-dihydro-5*H*-5λ⁴-thieno[3,2-*d]*pyirimidin-4-ylamino}-butan-1-ol
1.22. (S)-5-{2-[4-(4-fluorophenoxy)-piperidin-1-yl]-5-oxo-6,7-dihydro-5*H*-5λ⁴-thieno[3,2-*d]*pyrimidin-4-ylamino}-1-methylpiperidin-2-one
1.23. (2-{4-[4-(4,5-dihydroxazol-2-yl)-phenoxy]-piperidin-1-yl}-5-oxo-6,7-dihydro-5H-5λ⁴-thieno[3,2-*d]*pyrimidin-4-yl)-(tetrahydropyran-4-yl)-amine
1.24. 4-{1-[5-oxo-4-(tetrahydropyran-4-ylamino)-6,7-dihydro-5H-5λ⁴-thieno[3,2-*d]*pyrimidin-2-yl]-piperidin-4-yloxy}-benzoic acid
1.25. 2-(1-{2-[4-(4-chlorophenyl)-piperidin-1-yl]-5-oxo-6,7-dihydro-5*H*-5λ⁴-thieno[3,2-*d]*pyrimidin-4-ylamino}-cyclopropyl)-propan-2-ol
1.26. {2-[4-(5-tert-butyl-1-methyl-1*H*-indol-3-yl)-piperidin-1-yl]-5-oxo-6,7-dihydro-*5H*-5λ⁴-thieno[3,2-*d]*pyrimidin-4-yl}-(tetrahydropyran-4-yl)-amine
1.27. 2-[4-(5-furan-2-yl-1-methyl-1*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.28. (S)-5-(2-{4-[4-(4,5-dihydroxazol-2-yl)-phenoxy]-piperidin-1-yl}-5-oxo-6,7-dihydro-5*H*-5λ⁴-thieno[3,2-*d]*pyrimidin-4-ylamino)-1-methylpiperidin-2-one
1.29. {2-[4-(5-furan-2-yl-2-methyl-2*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.30. {2-[4-(1-methyl-1*H*-imidazo[4,5-*c*]pyridin-2-yl)-piperidin-1-yl]-5-oxo-6,7-dihydro-5*H*-5λ⁴-thieno[3,2-*d]*pyrimidin-4-yl}-(tetrahydropyran-4-yl)-amine
1.31. 2-methoxy-N-{1-[5-oxo-4-(tetrahydropyran-4-ylamino)-6,7-dihydro-5*H*-5λ⁴-thieno[3,2-*d]*pyrimidin-2-yl]-4-phenylpiperidin-4-ylmethyl}-acetamide
1.32. N-cyclopropyl-N-methyl-4-{1-[5-oxo-4-(tetrahydropyran-4-ylamino)-6,7-dihydro-5*H*-5λ⁴-thieno[3,2-*d]*pyrimidin-2-yl]-piperidin-4-yl}-benzamide
1.33. N-cyclopropyl-N-methyl-4-{1-[5-oxo-4-(tetrahydropyran-4-ylamino)-6,7-dihydro-5H-5λ⁴-thieno[3,2-*d]*pyrimidin-2-yl]-piperidin-4-yloxy}-benzamide
1.34. {5-oxo-2-[4-(pyridin-4-yloxy)-piperidin-1-yl]-6,7-dihydro-5*H*-5λ⁴-thieno[3,2-*d]*pyrimidin-4-yl}-(tetrahydropyran-4-yl)-amine
1.35. {2-[4-(4-chlorophenoxy)-piperidin-1-yl]-5-oxo-6,7-dihydro-5*H*-5λ⁴-thieno[3,2-*d]*pyrimidin-4-yl}-(tetrahydropyran-4-yl)-amine
1.36. (S)-1-methyl-5-{2-[4-(5-methyl-4-phenyloxazol-2-yl)-piperidin-1-yl]-5-oxo-6,7-dihydro-5*H*-5λ⁴-thieno[3,2-*d]*pyrimidin-4-ylamino}-piperidin-2-one
1.37. (1-{2-[4-(5-methyl-4-phenyloxazol-2-yl)-piperidin-1-yl]-5-oxo-6,7-dihydro-5*H-*5λ⁴-thieno[3,2-*d]*pyrimidin-4-ylamino}-cyclopropyl)-methanol
1.38. (S)-5-{2-[4-(4,5-diphenyloxazol-2-yl)-piperidin-1-yl]-5-oxo-6,7-dihydro-5H-5λ⁴-thieno[3,2-*d]*pyrimidin-4-ylamino}-1-methylpiperidin-2-one
1.39. {4-(4-chlorophenyl)-1-[5-oxo-4-(tetrahydropyran-4-ylamino)-6,7-dihydro-5*H-*5λ⁴-thieno[3,2-*d]*pyrimidin-2-yl]-piperidin-4-yl}-methanol
1.40. [1-(2-{4-[5-(4-chlorophenyl)-4-methyloxazol-2-yl]-piperidin-1-yl}-5-oxo-6,7-dihydro-5H-5λ⁴-thieno[3,2-*d]*pyrimidin-4-ylamino)-cyclopropyl]-methanol
1.41. 4-(4-chlorophenyl)-1-[5-oxo-4-(tetrahydropyran-4-ylamino)-6,7-dihydro-5*H-*5λ⁴-thieno[3,2-*d]*pyrimidin-2-yl]-piperidin-4-ol
1.42. {2-[4-(4-ch!orophenyl)-4-methoxypiperidin-1-yl]-5-oxo-6,7-dihydro-5*H*-5λ⁴-thieno[3,2-*d]*pyrimidin-4-yl}-(tetrahydropyran-4-yl)-amine
1.43. 4-{1-[4-(1-hydroxymethylcyclopropylamino)-5-oxo-6,7-dihydro-5*H*-5λ⁴-thieno[3,2-*d]*pynmidin-2-yl]-piperidin-4-yloxy}-benzonitrile
1.44. 5-oxo-2-[4-(4,5,6,7-tetrahydrobenzoxazol-2-yl)-piperidin-1-yl]-6,7-dihydro-5*H-*5λ⁴-thieno[3,2-*d]*pyrimidin-4-yl}-(tetrahydropyran-4-yl)-amine
1.45. (S)-5-{2-[4-(4-chlorophenyl)-piperidin-1-yl]-5,5-dioxo-6,7-dihydro-5*H*-5⁶-thieno[3,2-*d]*pyrimidin-4-ylamino}-1-methylpiperidin-2-one,
it contains at least one EP4 receptor antagonist (**2**).

22. Medicament combination according to one of claims 1 to 3, **characterised in that** the at least one EP4 receptor antagonist is selected from among
[N-{[4-(5,9-diethoxy-6-oxo-6,8-dihydro-7H-pyrrolo[3,4-*g*]quinolin-7yl)-3-methylbenzyl]sulphonyl}-2-(2-methoxyphenyl)acetamide] (**2.1**);
5-butyl-2,4-dihydro-4-[[2'-[N-(3-methyl-2-thiophene-carbonyl)sulphamoyl]biphenyl-4-yl]methyl]-2-[(2-trifluoromethyl)phenyl]-1, 2, 4-triazol-3-one (**2.2**);
(4-{(1S)-1-[({5-chloro-2-[(4-fluorophenyl)oxy]phenyl}carbonyl)-amino]ethyl}benzoic acid (**2.3**);
N-[({2-[4-(2-ethyl-4,6-dimethyl-1H-imidazo [4,5-c]pyridin-1-yl)phenyl]ethyl}amino)carbonyl]-4-methylbenzene sulphonamide (**2.4**);
4-[[4-(5-methoxy-2-pyridinyl)phenoxy]methyl]-5-methyl-N-[(2-methylphenyl)sulphonyl]-2-furancarboxamide (**2.5**);
methyl 11 alpha, 15alpha-dihydroxy-16-(3-methoxymethylphenyl)-9-oxo-17,18,19,20-tetranor-5-thia-13(E) prostanoate (**2.6**);
4-cyano-2-[[2-(4-fluoro-1-naphthalenyl)-1-oxopropyl]amino]-benzenebutanoic acid (**2.7**) and
N-{2-[4-(4,9-diethoxy-1-oxo-1,3-dihydro-2H-benzo[f]isoindol-2-yl)phenyl]acetyl}benzene sulphonamide (**2.8**).

23. Medicament combination according to one of claims 1 to 22, **characterised in that** the PDE4 inhibitor (**1**) is contained in a daily dose of 0.01 mg to 50 mg, preferably 0.1 mg to 10 mg.

24. Medicament combination according to one of claims 1 to 23, **characterised in that** the EP4-receptor antagonist (**2**) is used in a daily dose of 0.001 to 100 mg/kg body weight, preferably 0.01 to 50 mg/kg body weight, more preferably 0.1 to 10 mg/kg body weight.

25. Medicament combination according to one of claims 1 to 24, **characterised in that** the EP4-receptor antagonist (**2**) and the PDE4 inhibitor (**1**) are used in a ratio by weight of 1:1 to 200:1, preferably in a ratio by weight of 10:1 to 150:1, particularly preferably in a ratio by weight of 30:1 to 100:1.

26. Use of an EP4-receptor-antagonist (**2**) for the preparation of a medicament for reducing the side effects of one or more PDE4-inhibitors of general formula 1 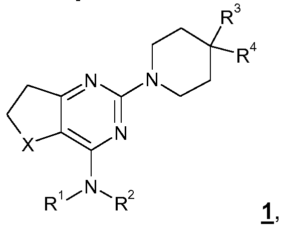 wherein **X**, **R¹**, **R²**, **R³** and **R⁴** are defined as in one of claims 1 to 2,
in the treatment of a disease selected from among respiratory complaints, pulmonary diseases, gastrointestinal complaints, diseases and also inflammatory diseases of the joints, skin or eyes, cancers and diseases of the peripheral or central nervous system.

27. Use of a combination containing one or more PDE4-inhibitors (**1**) of general formula **1** 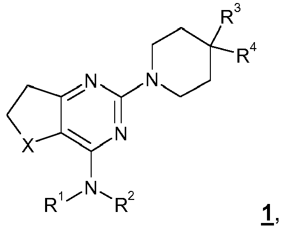 wherein **X**, **R¹**, **R²**, **R³** and **R⁴** are defined as in one of claims 1 to 20,
and at least one EP4 receptor antagonist (2) for the preparation of a medicament for treating a disease selected from among respiratory complaints, pulmonary diseases, gastrointestinal complaints, diseases and also inflammatory diseases of the joints, skin or eyes, cancers and diseases of the peripheral or central nervous system.

28. Use according to one of claims 26 to 27, for the treatment of a disease selected from COPD, chronic sinusitis, asthma, Crohn's disease and ulcerative colitis.

29. Use according to one of claims 26 to 28, **characterised in that** the PDE4 inhibitor (**1**) and the at least one EP4-receptor antagonist (**2**) are administered simultaneously in a single, combined formulation.

30. Use according to one of claims 26 to 38, **characterised in that** the PDE4 inhibitor (**1**) and the at least one EP4-receptor antagonist (**2**) are administered in two separate formulations within a time interval of 0 to 6 hours of one another.

31. Use according to claim 30, wherein the formulation containing the PDE4 inhibitor (**1**) is an oral or inhalative formulation, preferably an oral formulation, and wherein the formulation containing the at least one EP4 receptor antagonist (**2**) is an oral formulation.

32. Use according to one of claims 30 or 31, wherein the formulation containing the PDE4 inhibitor (**1**) is administered once a day and the formulation containing the at least one EP4-receptor antagonist (**2**) is administered either once or twice a day.

33. Use according to one of claims 26 to 32, **characterised in that** the PDE4 inhibitor is a compound of formula **1** selected from among:
1.1. (R)-2-{2-[4-(4-chlorophenyl)-piperidin-1-yl]-5-oxo-6,7-dihydro-5*H*-5λ⁴-thieno[3,2-*d]*pyrimidin -4-ylamino}-3-methylbutan-1-ol
1.2. (1-{2-[4-(4-chlorophenyl)-piperidin-1-yl]-5-oxo-6,7-dihydro-5*H*-5λ⁴-thieno[3,2-*d]*pyrimidin-4-ylamino}-cyclopropyl)-methanol
1.3. (R)-2-{2-[4-(4-chlorophenyl)-piperidin-1-yl]-5-oxo-6,7-dihydro-5*H*-5λ⁴-thieno[3,2-*d]*pyrimidin-4-ylamino}-pentan-1-ol
1.4. (R)-1-{2-[4-(4-chlorophenyl)-piperidin-1-yl]-5-oxo-6,7-dihydro-5*H*-5λ⁴-thieno[3,2-*d]*pyrimidin-4-ylamino}-1-(4-fluorophenyl)-2-methylpropan-2-ol
1.5. (S)-5-{2-[4-(4-chlorophenyl)-piperidin-1-yl]-5-oxo-6,7-dihydro-5*H*-5λ⁴-thieno[3,2-*d]*pyrimidin-4-ylamino}-1-methylpiperidin-2-one
1.6. {2-[4-(4-chlorophenyl)-piperidin-1-yl]-5-oxo-6,7-dihydro-5*H*-5λ⁴-thieno[3,2-*d]*pyrimidin-4-yl}-(tetrahydropyran-4-yl)-amine
1.7. 1-(4-(1-hydroxymethylcyclopropylamino)-5-oxo-6,7-dihydro-5*H*-5λ⁴-thieno[3,2-*d]*pyrimidin-2-yl)-3'-methyl-1'*H*-spiro[piperidin-4,4'-quinazolin]-2'(3'*H*)-one
1.8. {1-[2-(4-benzo[*d*]isoxazol-3-yl-piperidin-1-yl)-5-oxo-6,7-dihydro-5*H*-5λ⁴-thieno[3,2-*d]*pyrimidin-4-ylamino]-cyclopropyl}-methanol
1.9. (1-{2-[4-(2-ethyl-5-fluoro-1*H*-indol-3-yl)-piperidin-1-yl]-5-oxo-6,7-dihydro-5*H-*5λ⁴-thieno[3,2-*d]*pyrimidin-4-ylamino}-cyclopropyl)-methanol
1.10. 1-[4-((S)-1-methyl-6-oxopiperidin-3-ylamino)-5-oxo-6,7-dihydro-5*H*-5λ⁴-thieno[3,2-*d]*pyrimidin-2-yl]-4-phenylpiperidin-4-carbonitrile
1.11. 3'-methyl-1-(4-(tetrahydro-2*H*-pyran-4-ylamino)-5-oxo-6,7-dihydro-5*H*-5λ⁴-thieno[3,2-*d]*pyrimidin-2-yl)-1'*H*-spiro[piperidin-4,4'-quinazolin]-2'(3'*H*)-one
1.12. (3-fluorophenyl)-[5-oxo-2-(3,4,5,6-tetrahydro-2*H*-[4,4']bipyridinyl-1-yl)-6,7-dihydro-5*H*-5λ⁴-thieno[3,2-*d]*pyrimidin-4-yl]-amine
1.13. {2-[4-(2-ethyl-5-fluoro-1*H*-indol-3-yl)-piperidin-1-yl]-5-oxo-6,7-dihydro-5*H*-5λ⁴-thieno[3,2-*d]*pyrimidin-4-yl}-(3-fluorophenyl)-amine
1.14. (1-{2-[4-(2,4-difluorophenyl)-piperidin-1-yl]-5-oxo-6,7-dihydro-5*H*-5λ⁴-thieno[3,2-*d]*pyrimidin-4-ylamino}-cyclopropyl)-methanol
1.15. {2-[4-(2,4-difluorophenyl)-piperidin-1-yl]-5-oxo-6,7-dihydro-5*H*-5λ⁴-thieno[3,2-*d]*pyrimidin-4-yl}-(tetrahydropyran-4-yl)-amine
1.16. (S)-5-[2-(4-benzoxazol-2-yl-piperidin-1-yl)-5-oxo-6,7-dihydro-5*H*-5λ⁴-thieno[3,2-*d]*pyrimidin-4-ylamino]-1-methylpiperidin-2-one
1.17. (1-{2-[4-(6-fluorobenzo[*d*]isoxazol-3-yl)-piperidin-1-yl]-5-oxo-6,7-dihydro-5*H-*5λ⁴-thieno[3,2-*d]*pyrimidin-4-ylamino}-cyclopropyl)-methanol
1.18. (1-{2-[4-(5-fluorobenzo[*d*]isoxazol-3-yl)-piperidin-1-yl]-5-oxo-6,7-dihydro-5*H-*5λ⁴-thieno[3,2-*d]*pyrimidin-4-ylamino}-cyclopropyl)-methanol
1.19. {2-[4-(5-furan-2-yl-2*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.20. (3-fluorophenyl)-{5-oxo-2-[4-(3-pyridin-4-yl-[1,2,4]oxadiazol-5-yl)-piperidin-1-yl]-6,7-dihydro-5*H*-5λ⁴-thieno[3,2-*d]*pyrimidin-4-yl}-amine
1.21. (R)-3-methyl-2-{5-oxo-2-[4-(3-pyridin-4-yl-[1,2,4]oxadiazol-5-yl)-piperidin-1-yl]-6,7-dihydro-5*H*-5λ⁴-thieno[3,2-*d]*pyrimidin-4-ylamino}-butan-1-ol
1.22. (S)-5-{2-[4-(4-fluorophenoxy)-piperidin-1-yl]-5-oxo-6,7-dihydro-5*H*-5λ⁴-thieno[3,2-*d]*pyrimidin-4-ylamino}-1-methylpiperidin-2-one
1.23. (2-{4-[4-(4,5-dihydroxazol-2-yl)-phenoxy]-piperidin-1-yl}-5-oxo-6,7-dihydro-5H-5λ⁴-thieno[3,2-*d]*pyrimidin-4-yl)-(tetrahydropyran-4-yl)-amine
1.24. 4-{1-[5-oxo-4-(tetrahydropyran-4-ylamino)-6,7-dihydro-5H-5λ⁴-thieno[3,2-*d]*pyrimidin-2-yl]-piperidin-4-yloxy}-benzoic acid
1.25. 2-(1-{2-[4-(4-chlorophenyl)-piperidin-1-yl]-5-oxo-6,7-dihydro-5*H*-5λ⁴-thieno[3,2-*d]*pyrimidin-4-ylamino}-cyclopropyl)-propan-2-ol
1.26. {2-[4-(5-*tert*-butyl-1-methyl-1*H*-indol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.27. 2-[4-(5-furan-2-yl-1-methyl-1*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.28. (S)-5-(2-{4-[4-(4,5-dihydroxazol-2-yl)-phenoxy]-piperidin-1-yl}-5-oxo-6,7-dihydro-5*H*-5λ⁴-thieno[3,2-*d]*pyrimidin-4-ylamino)-1-methylpiperidin-2-one
1.29. {2-[4-(5-furan-2-yl-2-methyl-2*H*-pyrazol-3-yl)-piperidin-1-yl]-5-oxo-6,7-dihydro-5*H*-5λ⁴-thieno[3,2-*d]*pyrimidin-4-yl}-(tetrahydropyran-4-yl)-amine
1.30. {2-[4-(1-methyl-1*H*-imidazo[4,5-*c*]pyridin-2-yl)-piperidin-1-yl]-5-oxo-6,7-dihydro-5*H*-5λ⁴-thieno[3,2-*d]*pyrimidin-4-yl}-(tetrahydropyran-4-yl)-amine
1.31. 2-methoxy-*N*-{1-[5-oxo-4-(tetrahydropyran-4-ylamino)-6,7-dihydro-5*H*-5λ⁴-thieno[3,2-*d]*pyrimidin-2-yl]-4-phenylpiperidin-4-ylmethyl}-acetamide
1.32. N-cyclopropyl-N-methyl-4-{1-[5-oxo-4-(tetrahydropyran-4-ylamino)-6,7-dihydro-5*H*-5λ⁴-thieno[3,2-*d]*pyrimidin-2-yl]-piperidin-4-yl}-benzamide
1.33. N-cyclopropyl-N-methyl-4-{1-[5-oxo-4-(tetrahydropyran-4-ylamino)-6,7-dihydro-5H-5λ⁴-thieno[3,2-*d]*pyrimidin-2-yl]-piperidin-4-yloxy}-benzamide
1.34. {5-oxo-2-[4-(pyridin-4-yloxy)-piperidin-1-yl]-6,7-dihydro-5*H*-5λ⁴-thieno[3,2-*d]*pyrimidin-4-yl}-(tetrahydropyran-4-yl)-amine
1.35. {2-[4-(4-chlorophenoxy)-piperidin-1-yl]-5-oxo-6,7-dihydro-5*H*-5λ⁴-thieno[3,2-*d]*pyrimidin-4-yl}-(tetrahydropyran-4-yl)-amine
1.36. (S)-1-methyl-5-{2-[4-(5-methyl-4-phenyloxazol-2-yl)-piperidin-1-yl]-5-oxo-6,7-dihydro-5*H*-5λ⁴-thieno[3,2-*d]*pyrimidin-4-ylamino}-piperidin-2-one
1.37. (1-{2-[4-(5-methyl-4-phenyloxazol-2-yl)-piperidin-1-yl]-5-oxo-6,7-dihydro-5*H-*5λ⁴-thieno[3,2-*d]*pyrimidin-4-ylamino}-cyclopropyl)-methanol
1.38. (S)-5-{2-[4-(4,5-diphenyloxazol-2-yl)-piperidin-1-yl]-5-oxo-6,7-dihydro-5H-5λ⁴-thieno[3,2-*d*]pyrimidin-4-ylamino}-1-methylpiperidin-2-one
1.39. {4-(4-chlorophenyl)-1-[5-oxo-4-(tetrahydropyran-4-ylamino)-6,7-dihydro-5*H-*5λ⁴-thieno[3,2-*d]*pyrimidin-2-yl]-piperidin-4-yl}-methanol
1.40. [1-(2-{4-[5-(4-chlorophenyl)-4-methyloxazol-2-yl]-piperidin-1-yl}-5-oxo-6,7-dihydro-5H-5λ⁴-thieno[3,2-*d]*pyrimidin-4-ylamino)-cyclopropyl]-methanol
1.41. 4-(4-chlorophenyl)-1-[5-oxo-4-(tetrahydropyran-4-ylamino)-6,7-dihydro-5*H-*5λ⁴-thieno[3,2-*d]*pyrimidin-2-yl]-piperidin-4-ol
1.42. {2-[4-(4-chlorophenyl)-4-methoxypiperidin-1-yl]-5-oxo-6,7-dihydro-5*H*-5λ⁴-thieno[3,2-*d]*pyrimidin-4-yl}-(tetrahydropyran-4-yl)-amine
1.43. 4-{1-[4-(1-hydroxymethylcyclopropylamino)-5-oxo-6,7-dihydro-5*H*-5λ⁴-thieno[3,2-*d]*pyrimidin-2-yl]-piperidin-4-yloxy}-benzonitrile
1.44. 5-oxo-2-[4-(4,5,6,7-tetrahydrobenzoxazol-2-yl)-piperidin-1-yl]-6,7-dihydro-5H-5λ⁴-thieno[3,2-*d]*pyrimidin-4-yl}-(tetrahydropyran-4-yl)-amine
1.45. (S)-5-{2-[4-(4-chlorophenyl)-piperidin-1-yl]-5,5-dioxo-6,7-dihydro-5H-5λ⁶-thieno[3,2-*d]*pyrimidin-4-ylamino}-1-methylpiperidin-2-one.

34. Use according to one of claims 26 to 33, **characterised in that** the at least one EP4-receptor antagonist (2) is selected from among:
[N-{[4-(5,9-diethoxy-6-oxo-6,8-dihydro-7H-pyrrolo[3,4-*g*] quinolin-7yl)-3-methylbenzyl]sulphonyl}-2-(2-methoxyphenyl)acetamide] (**2.1**);
5-butyl-2,4-dihydro-4-[[2'-[N-(3-methyl-2-thiophene-carbonyl)sulphamoyl]biphenyl-4-yl]methyl]-2-[(2-trifluoromethyl)phenyl]-1, 2, 4-triazol-3-one (**2.2**);
(4-{(1S)-1-[({5-chloro-2-[(4-fluorophenyl)oxy]phenyl}carbonyl)-amino]ethyl}benzoic acid (**2.3**);
N-[({2-[4-(2-ethyl-4,6-dimethyl-1H-imidazo [4,5-c]pyridin-1-yl)phenyl]ethyl}amino)carbonyl]-4-methylbenzene sulphonamide (**2.4**);
4-[[4-(5-methoxy-2-pyridinyl)phenoxy]methyl]-5-methyl-N-[(2-methylphenyl)sulphonyl]-2-furancarboxamide (**2.5**);
methyl 11 alpha, 15alpha-dihydroxy-16-(3-methoxymethylphenyl)-9-oxo-17,18,19,20-tetranor-5-thia-13(E) prostanoate (**2.6**);
4-cyano-2-[[2-(4-fluoro-1-naphthalenyl)-1-oxopropyl]amino]-benzenebutanoic acid (**2.7**) and
N-{2-[4-(4,9-diethoxy-1-oxo-1,3-dihydro-2H-benzo[f]isoindol-2-yl)phenyl]acetyl}benzene sulphonamide (**2.8**).

35. Use according to one of claims 26 to 34, **characterised in that** the PDE4 inhibitor of general formula **1** is used in a daily dose of 0.01 mg to 50 mg.

36. Use according to one of claims 26 to 35, **characterised in that** the EP4-receptor antagonist (**2**) is used in a daily dose of 0.001 to 100 mg/kg body weight, preferably 0.01 to 50 mg/kg body weight, more preferably 0.1 to 10 mg/kg body weight.

37. Use according to one of claims 26 to 36, **characterised in that** the EP4-receptor antagonist (**2**) and the PDE4 inhibitor (**1**) are used in a ratio by weight of 1:1 to 200:1, preferably in a ratio by weight of 10:1 to 150:1, particularly preferably in a ratio by weight of 30:1 to 100:1.

## Revendications

1. Combinaison de médicaments, **caractérisée en ce qu'**elle comprend, en plus d'un ou plusieurs inhibiteurs de PDE4 (1) de formule générale 1 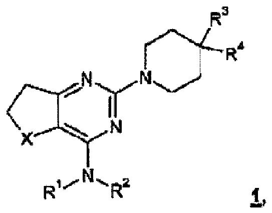 dans laquelle
X représente SO ou SO₂,
R¹ représente H ou un groupe C₁₋₆-alkyle,
R² représente H ou un radical choisi dans le groupe consistant en un groupe C₁₋₁₀-alkyle et C₂₋₆-alcényle qui peut être substitué éventuellement par un ou plusieurs radicaux choisis parmi un atome d'halogène et un groupe C₁₋₃-fluoroalkyle ou qui peut être substitué éventuellement par un ou plusieurs radicaux choisis dans le groupe consistant en OR^{2.1}, COOR^{2.1}, CONR^{2.2}R^{2.3}, SR^{2.1}, SO-R^{2.1}, SO₂R^{2.1}, C₆₋₁₀-aryle, -het, hétaryle, un groupe -C₃₋₁₀-cycloalkyle monocyclique ou bicyclique, CH₂-NR^{2.2}R^{2.3} et NR^{2.2}R^{2.3},
qui peut lui-même être de nouveau substitué éventuellement par un ou plusieurs radicaux choisis dans le groupe consistant en OH, un atome d'halogène, OR^{2.1}, un groupe oxo, CF₃, CHF₂, CH₂F, C₁₋₆-alkyle, C₁₋₈-alcanol, C₆₋₁₀-aryle, COOR^{2.1}, CH₂-NR^{2.2}R^{2.3} et NR^{2.2}R^{2.3},
où
het représente un hétérocycle de trois à onze chaînons, monocyclique ou bicyclique, saturé ou partiellement saturé, éventuellement condensé ou éventuellement ponté, qui contient 1, 2, 3 ou 4 hétéroatomes choisis indépendamment les uns des autres dans le groupe consistant en N, S ou O,
et où
hétaryle représente un groupe hétéroaryle de cinq à dix chaînons, monocyclique ou bicyclique, éventuellement condensé qui contient 1, 2, 3 ou 4 hétéroatomes, choisis indépendamment les uns des autres dans le groupe consistant en N, S ou O,
et où
le groupe cycloalkyle peut être saturé ou partiellement saturé,
où R^{2.1} représente H ou un radical choisi dans le groupe consistant en un groupe C₁₋₆-alkyle, C₁₋₆-alcanol, C₁₋₃-halogénoalkyle, monocyclique ou bicyclique, C₃₋₁₀-cycloalkyle, C₆₋₁₀-aryl-C₁₋₆-alkylène, hétaryl-C₁₋₆-alkylène, het-C₁₋₆-alkylène, C₃₋₁₀-cycloalkyl-C₁₋₆-alkylène, un groupe C₆₋₁₀-aryle monocyclique ou bicyclique, hétéroaryle et un groupe het,
qui peut éventuellement être substitué par un ou plusieurs radicaux choisis dans le groupe consistant en OH, O-(C₁₋₃-alkyle), un atome d'halogène, un groupe C₁₋₆-alkyle et C₆₋₁₀-aryle,
où R^{2.2} et R^{2.3} représentent, indépendamment l'un de l'autre, H ou un radical choisi dans le groupe consistant en un groupe C₁₋₆-alkyle, un groupe C₃₋₁₀-cycloalkyle monocyclique ou bicyclique, C₆₋₁₀-aryl-C₁₋₆-alkylène, hétaryl-C₁₋₆-alkylène, un groupe C₆₋₁₀-aryle mono ou bicyclique, het, hétaryle, CO-NH₂, CO-NHCH₃, -CO-N(CH₃)₂, SO₂-(C₁-C₂-alkyle), CO-R^{2.1} et COOR^{2.1},
qui peut éventuellement être substitué par un ou plusieurs radicaux choisis dans le groupe consistant en OH, un atome d'halogène, un groupe C₁₋₆-alkyle, C₆₋₁₀-aryle et COOR^{2.1},
ou
R² représente un groupe C₃₋₁₀-cycloalkyle monocyclique ou polycyclique qui peut éventuellement être ponté, une ou plusieurs fois, par un groupe C₁₋₃-alkyle et qui peut être substitué éventuellement par un radical choisi dans le groupe consistant en les groupes, ramifiés ou non ramifiés, C₁₋₆-alcanol, C₁₋₃-fluoroalkyle, C₁₋₃-alkylène-OR^{2.1}, OR^{2.1}, COOR^{2.1}, -SO₂-NR^{2.2}R^{2.3}, het, -NH-CO-O-(C₁₋₆-alkyle), -NH-CO-(C₁₋₆-alkyle), -NH-CO-O-(C₆₋₁₀-aryle), -NH-CO-(C₆₋₁₀-aryle), -NH-CO-O-hétaryle, -NH-CO-hétaryle, -NH-CO-O-(C₁₋₃-alkylène)-(C₆₋₁₀-aryle), -NH-CO-(C₁₋₃-alkylène) - (C₆-₁₀-aryle), -N(C₁₋₃-alkyl)-CO-(C₁₋₆-alkyle), -N(C₁₋₃-alkyl)-CO-O-(C₆₋₁₀-aryle), -N(C₁₋₃-alkyl)-CO-(C₆₋₁₀-aryle), -N(C₁₋₃-alkyl)-CO-O-hétaryle, N(C₁₋₃-alkyl)-CO-hétaryle, -N (C₁₋₃-alkyl) -CO-O-(C₁₋₃-alkylène)-(C₆₋₁₀-aryle), -N (C₁₋₃-alkyl)-CO-(C₁₋₃-alkylène)-(C₆₋₁₀-aryle), C₆₋₁₀-aryle, C₁₋₆-alkyle, C₆₋₁₀-aryle-C₁₋₆-alkylène, hétaryl-C₁₋₆-alkylène, un groupe C₃₋₁₀ cycloalkyle monocyclique ou bicyclique et NR^{2.2}R^{2.3},
qui peut éventuellement être substitué par un ou plusieurs radicaux choisis dans le groupe consistant en OH, OR^{2.1}, un groupe oxo, un atome d'halogène, CF₃, CHF₂, CH₂F, C₁₋₆-alkyle, C₆₋₁₀-aryle et NR^{2.2}R^{2.3},
ou
R² représente un groupe C₆₋₁₀-aryle monocyclique ou polycyclique qui peut être substitué éventuellement par OH, SH ou un atome d'halogène ou par un ou plusieurs radicaux choisis dans le groupe consistant en OR^{2.1}, COOR^{2.1}, NR^{2.2}R^{2.3}, CH₂-NR^{2.2}R^{2.3}, C₃₋₁₀-cycloalkyle, het, C₁₋₆-alkyle, C₁₋₃-fluoroalkyle, CF₃, CHF₂, CH₂F, C₆₋₁₀-aryl-C₁₋₆-alkylène, het-C₁₋₆-alkylène, hétaryl-C₁₋₆-alkylène, C₆-₁₀-aryle, SO₂-CH₃, SO₂-CH₂CH₃ et SO₂-NR^{2.2}R^{2.3},
qui peut lui-même être substitué éventuellement par un ou plusieurs radicaux choisis dans le groupe consistant en OH, OR^{2.1}, CF₃, CHF₂, CH₂F, un groupe oxo, un atome d'halogène, CF₃, CHF₂, CH₂F, C₁₋₆-alkyle, C₆₋₁₀-aryle et NR^{2.2}R^{2.3},
ou
R² représente un radical choisi dans un groupe consistant en un groupe het et hétaryle qui peut être substitué éventuellement par un ou plusieurs radicaux choisis dans le groupe consistant en un atome d'halogène, OH, un groupe oxo, CF₃, CHF₂ et CH₂F ou par un ou plusieurs radicaux choisis dans le groupe OR^{2.1}, C₁₋₃-alkylène-OR^{2.1}, SR^{2.1}, SO-R^{2.1}, SO₂-R^{2.1}, COOR^{2.1}, COR^{2.1}, C₁₋₆-alcanol, un groupe C₃₋₁₀-cycloalkyle monocyclique ou bicyclique, C₆₋₁₀-aryle, C₁₋₆-alkyle, C₆₋₁₀-aryl-C₁₋₆-alkylène, hétaryl-C₁₋₆-alkylène, het, hétaryle, C₁₋₃-alkylène-OR^{2.1} et NR^{2.2}R^{2.3},
qui peut lui-même être substitué éventuellement par un ou plusieurs radicaux choisis dans le groupe consistant en OH, OR^{2.1}, un groupe oxo, un atome d'halogène, CF₃, CHF₂ , CH₂F, un groupe C₁₋₆-alkyle, C₆₋₁₀-aryle et NR^{2.2}R^{2.3},
ou dans laquelle
NR¹R² représente conj ointement un cycle C₄₋₇ hétérocyclique qui peut éventuellement être ponté, qui contient 1, 2 ou 3 hétéroatomes choisis dans le groupe consistant en N, O et S et qui peut être substitué éventuellement par un ou plusieurs radicaux choisis dans le groupe consistant en OH, OR^{2.1}, C₁₋₃-alkylène-O-^{R.1}, oxo,
un atome d'halogène, un groupe C₁₋₆-alkyle, C₆₋₁₀-aryle, COOR^{2.1}, CH₂-NR^{2.2}-COO-R^{2.1}, CH₂-NR^{2.2}-COR^{2.1}, CH₂-NR^{2.2}-CO-CH₂-NR^{2.2}R^{2.3}, CH₂-NR^{2.2}-SO₂-C₁₋₃-alkyle, CH₂-NR^{2.2}-SO₂-NR^{2.2}R^{2.3}, CH₂-NR^{2.2}-CO-NR^{2.2}-R^{2.3}, CO-NR^{2.2}R^{2.3}, CH₂-NR^{2.2}R^{2.3} et NR^{2.2}R^{2.3},
et dans laquelle
R³ représente un groupe C₆₋₁₀-aryle, qui est éventuellement substitué en position ortho, para et méta par un, deux ou trois radicaux choisis indépendamment les uns des autres dans le groupe consistant en un atome de fluor, de chlore, de brome, un groupe hydroxy, CN, C₁₋₆-alkyle, C₁₋₃-fluoroalkyle, -C₁₋₃-alkylène-OR^{2.1}, -C₁₋₃-alkylène-NR^{2.2}R^{2.3}, -NR^{2.2}R^{2.3}, O-R^{2.1}, SO-R^{2.1}, SO₂-R^{2.1}, COOR^{2.1}, -CO-NH-(C₁₋₆-alkylène)-hétaryle, -CO-NH-hétaryle, - CO-N(CH₃)-het, -CO-N(CH₃)-(C₁₋₃-alkylène)-het, -CO-N (CH₃) - (C₁₋₃-alkylène) -hétaryle, -CO-N(C₃₋₇-cycloalkyl)-het, -CO-NR^{2.2}R^{2.3}, -CO-NH-(C₁₋₆-alkylène)-het, NR^{2.2}-CO-R^{2.1}, C₆₋₁₀-aryle, C₆₋₁₀-aryl-C₁₋₂-alkylène, het-C₁₋₂-alkylène, -het, -CO-het, CO-N-(CH₃) -C₃₋₇-cycloalkyle, C₃₋₇-cycloalkyle, C₃₋₇-cycloalkyl-C₁₋₂-alkylène, hétaryl-C₁₋₂-alkylène et hétaryle,
où ce radical peut éventuellement être substitué par un ou plusieurs radicaux choisis dans le groupe consistant en OH, un atome d'halogène, un groupe -C₁₋₃-fluoroalkyle, oxo, méthyle et phényle,
ou dans laquelle
R³ représente un radical choisi dans le groupe consistant en un groupe het et hétaryle qui peut être substitué éventuellement par un ou plusieurs radicaux choisis dans le groupe consistant en un atome d'halogène, un groupe C₁₋₃-fluoroalkyle, CN, OH, oxo, -C₁₋₆-alkyle, -C₁₋₃-alkylène-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-aryle, het, C₃₋₇-cycloalkyle et hétaryle,
qui peut lui-même être substitué éventuellement par un ou plusieurs radicaux choisis dans le groupe consistant en OH, un atome d'halogène, un groupe -C₁₋₃-fluoroalkyle, C₁₋₆-alkyle, C₆₋₁₀-aryle, -COO(C₁₋₃-alkyle) et O-(C₁₋₃-alkyle),
ou dans laquelle
R³ représente -O-R^{3.1},
où R^{3.1} est un radical choisi dans le groupe consistant en un groupe -C₁₋₆-alkyle, -C₆₋₁₀-aryle, -C₁₋₃-alkylène-C₆₋₁₀-aryle, hétaryle et het,
qui est substitué éventuellement en position ortho, para ou méta, par un, deux ou trois radicaux choisis indépendamment les uns des autres dans le groupe consistant en un atome de fluor, de chlore, de brome, un groupe hydroxy, CN, C₁₋₆-alkyle, C₁₋₃-fluoroalkyle, CO-(C₁₋₅-alkyle), -CO-(C₁₋₃-fluoroalkyle), -CO-NH-(C₁₋₆-alkylène)-hétaryle, -CO-N(C₁₋₃-alkyl)-(C₁₋₆-alkylène)-hétaryle, -CO-N(C₁₋₃-alkyl)-het, -CO-N(C₃₋₇-cycloalkyl)-het, -C₁₋₃-alkylène-OR^{2.1}, -C₁₋₃-alkylène-NR^{2.2}R^{2.3}, -NR^{2.2}R^{2.3}, O-R^{2.1}, SO-R^{2.1}, SO₂-R^{2.1}, COOH, COO-(C₁₋₄-alkyle), -O-C₁₋₃-alkylène-N(C₁₋₃-alkyle)₂, CO-NR^{2.2}R^{2.3}, NR^{2.2}-CO-R^{2.1}, C₆₋₁₀-aryle, C₆₋₁₀-aryl-C₁₋₂-alkylène, het-C₁₋₂-alkylène, -CO-het, het, -CO-C₃₋₇-cycloalkyle, -CO-N(C₁₋₃-alkyl)-C₃₋₇-cycloalkyle, C₃₋₇-cycloalkyl, C₃₋₇-cycloalkyl-C₁₋₂-alkylène, hétaryl-C₁₋₂-alkylène et hétaryle,
qui peut lui-même être substitué éventuellement par 1, 2, 3 ou 4 radicaux choisis indépendamment les uns des autres dans le groupe consistant en F, Cl, Br, un groupe méthyle, O-méthyle, éthyle, O-éthyle, OH, oxo et CF₃,
et dans laquelle
R⁴ représente H, CN, OH, CF₃, CHF₂, CH₂F, F, un groupe méthyle, éthyle, -O-(C₁₋₃-alkyle), -C₁₋₃-alkylène-OH, -COO(C₁₋₃-alkyle), -CO-het, -(C₁₋₂-alkylène)-NH-SO₂-(C₁₋₂-alkyle), -(C₁₋₂-alkylène)-N(C₁₋₃-alkyl)-SO₂-(C₁₋₂-alkyle), -(C₁₋₂-alkylène)-O-(C₁₋₂-alkylène) -C₆₋₁₀-aryle, -C₁₋₃-alkylène-O-C₁₋₃-alkyle, -(C₁₋₂-alkylène)-N(C₁₋₃-alkyl)-CO-(C₁₋₂-alkyle), -NH-CO(C₁₋₃-alkylène)-O-(C₁₋₃-alkyle), -C₁₋₃-alkylène-NH-CO-(C₁₋₃-alkyle), -C₁₋₃-alkylène-NH-CO-(C₁₋₃-alkylène)-N(C₁₋₃-alkyle)₂, -O-(C₁₋₂-alkylène)-(C₆₋₁₀-aryle), -C₁₋₃-alkylène-NH-CO-(C₁₋₃-alkylène)-O- (C₁₋₃-alkyle), -CO-(C₆₋₁₀-aryle), -(C₁₋₂-alkylène)-N(C₁₋₃-alkyl)-CO-(C₁₋₂-alkylène)-O-(C₁₋₃-alkyle),
le groupe aryle dans les radicaux ci-dessus pouvant être substitué éventuellement lui-même par un ou plusieurs autres radicaux choisis dans le groupe F, Cl, Br, un groupe méthyle, éthyle, propyle, isopropyle, cyclopropyle, -O-méthyle, -O-éthyle, -O-propyle, -O-isopropyle, -O-cyclopropyle, -OH et CF₃,
ou dans laquelle
R³ et R⁴ forment conjointement un hétérocycle mono-cyclique ou bicyclique insaturé, saturé ou partiellement saturé qui contient 1, 2 ou 3 hétéroatomes choisis dans le groupe consistant en N, 0 et S et qui peut éventuellement être substitué par un ou plusieurs radicaux choisis dans le groupe consistant en un atome d'halogène, un groupe OH, oxo, C₁₋₃-fluoroalkyle, CN, C₁₋₆-alkyle, -O-R^{2.1}, -COOR^{2.1}, SO-R^{2.1}, SO₂-R^{2.1}, -C₁₋₃-alkylène-NR^{2.2}R^{2.3}, -NR^{2.2}-R^{2.3}, C₆₋₁₀-aryle, C₃₋₇-cycloalkyle, het et hétaryle,
au moins un antagoniste (2) du récepteur EP4.

2. Combinaison de médicaments selon la revendication 1, **caractérisée en ce qu'**elle comprend, en plus d' un ou plusieurs, de préférence un inhibiteur de PDE4 (1) de formule générale 1 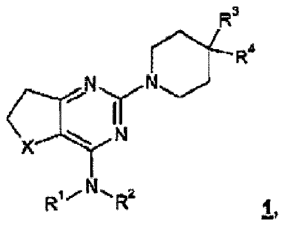 dans laquelle
X représente SO ou SO₂,
R¹ représente H ou un groupe C₁₋₆-alkyle,
R² représente H ou un radical choisi dans le groupe consistant en un groupe C₁₋₁₀-alkyle et C₂₋₆-alcényle qui peut être substitué éventuellement par un ou plusieurs radicaux choisis parmi un atome d'halogène et un groupe C₁₋₃-fluoroalkyle ou qui peut être substitué éventuellement par un ou plusieurs radicaux choisis dans le groupe consistant en OR^{2.1}, COOR^{2.1}, CONR^{2.2}R^{2.3}, SR^{2.1}, SO-R^{2.1}, SO₂R^{2.1}, C₆₋₁₀-aryle, -het, hétaryle, un groupe C₃₋₁₀ cycloalkyle monocyclique ou bicyclique, CH₂-NR^{2.2}R^{2.3} et NR^{2.2}R^{2.3},
qui peut lui-même être substitué éventuellement par un ou plusieurs radicaux choisis dans le groupe consistant en OH, un atome d'halogène, OR^{2.1}, un groupe oxo, CF₃, CHF₂, CH₂F, C₁₋₆-alkyle, C₁₋₆-alcanol, C₆₋₁₀-aryle, COOR^{2.1}, CH₂-NR^{2.2}R^{2.3} et NR^{2.2}R^{2.3},
où
het représente un hétérocycle de trois à onze chaînons, monocyclique ou bicyclique, saturé ou partiellement saturé, éventuellement condensé ou éventuellement ponté, qui contient 1, 2, 3 ou 4 hétéroatomes choisis indépendamment les uns des autres dans le groupe consistant en N, S ou O,
et où
hétaryle représente un groupe hétéroaryle de cinq à dix chaînons, monocyclique ou bicyclique, éventuellement condensé, qui contient 1, 2, 3 ou 4 hétéroatomes choisis indépendamment les uns des autres dans le groupe consistant en N, S ou 0,
et où
le groupe cycloalkyle peut être saturé ou partiellement saturé,
où R^{2.1} représente H ou un radical choisi dans le groupe consistant en un groupe C₁₋₆-alkyle, C₁₋₆-alcanol, C₁₋₃-halogénoalkyle, monocyclique ou bicyclique, C₃₋₁₀-cycloalkyle, C₆₋₁₀-aryl-C₁₋₆-alkylène, hétaryl-C₁₋₆-alkylène, het-C₁₋₆-alkylène, C₃₋₁₀-cycloalkyl-C₁₋₆-alkylène, un groupe C₆₋₁₀-aryle monocyclique ou bicyclique, hétéroaryle et un groupe het,
qui peut éventuellement être substitué par un ou plusieurs radicaux choisis dans le groupe consistant en OH, O-(C₁₋₃-alkyle), un atome d'halogène, un groupe C₁₋₆-alkyle et C₆₋₁₀-aryle,
où R^{2.2} et R^{2.3} représentent, indépendamment l'un de l'autre, H ou un radical choisi dans le groupe consistant en un groupe C₁₋₆-alkyle, un groupe C₃₋₁₀-cycloalkyle monocyclique ou bicyclique, C₆₋₁₀-aryle-C₁₋₆-alkylène, hétaryl-C₁₋₆-alkylène, un groupe C₆₋₁₀-aryle monocyclique ou bicyclique, het, hétaryle, CO-NH₂, CO-NHCH₃, -CO-N(CH₃)₂, SO₂-(C₁-C₂-alkyle), CO-R^{2.1} et COOR^{2.1},
qui peut éventuellement être substitué par un ou plusieurs radicaux choisis dans le groupe consistant en OH, un atome d'halogène, un groupe C₁₋₆-alkyle, C₆₋₁₀-aryle et COOR^{2.1},
ou
R² représente un groupe C₃₋₁₀-cycloalkyle monocyclique ou polycyclique qui peut éventuellement être ponté, une ou plusieurs fois, par un groupe C₁₋₃-alkyle et qui peut être substitué éventuellement par un radical choisi dans le groupe consistant en les groupes, ramifiés ou non ramifiés, C₁₋₆-alcanol, C₁₋₃-fluoroalkyle, C₁₋₃-alkylène-OR^{2.1}, OR^{2.1}, COOR^{2.1}, -SO₂-NR^{2.2}R^{2.3}, het, -NH-CO-O-(C₁₋₆-alkyle), -NH-CO-(C₁₋₆-alkyle), -NH-CO-O-(C₆₋₁₀-aryle), -NH-CO-(C₆₋₁₀-aryle), -NH-CO-O-hétaryle, - NH-CO-hétaryle, -NH-CO-O-(C₁₋₃-alkylène)-(C₆₋₁₀-aryle), -NH-CO-(C₁₋₃-alkylène)-(C₆₋₁₀-aryle), -N(C₁₋₃-alkyl)-CO-(C₁₋₆-alkyle), -N(C₁₋₃-alkyl)-CO-O-(C₆₋₁₀-aryle), -N (C₁₋₃-alkyl)-CO-(C₆₋₁₀-aryle) , -N (C₁₋₃-alkyl)-CO-O-hétaryle, N(C₁₋₃-alkyl)-CO-hétaryle, -N (C₁₋₃-alkyl)-CO-O-(C₁₋₃-alkylène)-(C₆₋₁₀-aryle), -N(C₁₋₃-alkyl)-CO-(C₁₋₃-alkylène)-(C₆₋₁₀-aryle), C₆₋₁₀-aryle, C₁₋₆-alkyle, C₆₋₁₀-aryle-C₁₋₆-alkylène, hétaryl-C₁₋₆-alkylène, un groupe C₃₋₁₀ cycloalkyle monocyclique ou bicyclique et NR^{2.2}R^{2.3},
qui peut éventuellement être substitué par un ou plusieurs radicaux choisis dans le groupe consistant en OH, OR^{2.1}, un groupe oxo, un atome d'halogène, CF₃, CHF₂, CH₂F, C₁₋₆-alkyle, C₆₋₁₀-aryle et NR^{2.2}R^{2.3},
ou
R² représente un groupe C₆₋₁₀-aryle monocyclique ou polycyclique qui peut être substitué éventuellement par OH, SH ou un atome d'halogène ou par un ou plusieurs radicaux choisis dans le groupe consistant en OR^{2.1}, COOR^{2.1}, NR^{2.2}R^{2.3}, CH₂-NR^{2.2}R^{2.3}, C₃₋₁₀-cycloalkyle, het, C₁₋₆-alkyle, C₁₋₃-fluoroalkyle, CF₃, CHF₂, CH₂F, C₆₋₁₀-aryl-C₁₋₆-alkylène, het-C₁₋₆-alkylène, hétaryl-C₁₋₆-alkylène, C₆₋₁₀-aryle, SO₂-CH₃, SO₂-CH₂CH₃ et SO₂-NR^{2.2}R^{2.3},
qui peut lui-même être substitué éventuellement par un ou plusieurs radicaux choisis dans le groupe consistant en OH, OR^{2.1}, CF₃, CHF₂, CH₂F, un groupe oxo, un atome d'halogène, CF₃, CHF₂, CH₂F, C₁₋₆-alkyle, C₆₋₁₀-aryle et NR^{2.2}R^{2.3},
ou
R² représente un radical choisi dans un groupe consistant en les groupes het et hétaryle qui peut être substitué éventuellement par un ou plusieurs radicaux choisis dans le groupe consistant en un atome d'halogène, OH, un groupe oxo, CF₃, CHF₂ et CH₂F ou par un ou plusieurs radicaux choisis dans le groupe OR^{2.1}, C₁₋₃-alkylène-OR^{2.1}, SR^{2.1}, SO-R^{2.1}, SO₂-R^{2.1}, COOR^{2.1}COR^{2.1}, C₁₋₆-alcanol, un groupe C₃₋₁₀-cycloalkyle monocyclique ou bicyclique, C₆₋₁₀-aryle, C₁₋₆-alkyle, C₆₋₁₀-aryl-C₁₋₆-alkylène, hétaryl-C₁₋₆-alkylène, het, hétaryle, C₁₋₃-alkylène-OR^{2.1} et NR^{2.2}R^{2.3},
qui peut éventuellement être substitué par un ou plusieurs radicaux choisis dans le groupe consistant en OH, OR^{2.1}, un groupe oxo, un atome d'halogène, CF₃, CHF₂ , CH₂F, un groupe C₁₋₆-alkyle, C₆₋₁₀-aryle et NR^{2.2}R^{2.3},
ou dans laquelle
NR¹R² représente conjointement un cycle C₄₋₇ hétérocyclique qui peut éventuellement être ponté, qui contient 1, 2 ou 3 hétéroatomes choisis dans le groupe consistant en N, O et S et qui peut être substitué éventuellement par un ou plusieurs radicaux choisis dans le groupe consistant en OH, OR^{2.1}, C₁₋₃-alkylène-O-^{R.1}, oxo, un atome d'halogène, un groupe C₁₋₆-alkyle, C₆₋₁₀-aryle, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyle, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} et NR^{2.2}R^{2.3},
et dans laquelle
R³ représente un groupe C₆₋₁₀-aryle,
qui est éventuellement substitué en position ortho, para et méta par un, deux ou trois radicaux choisis indépendamment les uns des autres dans le groupe consistant en un atome de fluor, de chlore, de brome, un groupe hydroxy, CN, C₁₋₆-alkyle, C₁₋₃-fluoroalkyle, -C₁₋₃-alkylène-OR^{2.1}, -C₁₋₃-alkylène-NR^{2.2}R^{2.3}, -NR^{2.2}R^{2.3}, O-R^{2.1}SO-R^{2.1}, SO₂-R^{2.1}, COOR^{2.1}, -CO-NH-(C₁₋₆-alkylène)-hétaryle, -CO-NH-hétaryle, - CO-N(CH₃)-het, -CO-N(CH₃)-(C₁₋₃-alkylène)-het, -CO-N(CH₃)-(C₁₋₃-alkylène)-hétaryle, -CO-N(C₃₋₇-cycloalkyl)-het, -CO-NR^{2.2}R^{2.3}, -CO-NH-(C₁₋₆-alkylène)-het, NR^{2.2}-CO-R^{2.1}, C₆₋₁₀-aryle, C₆₋₁₀-aryl-C₁₋₂-alkylène, het-C₁₋₂-alkylène, -het, -CO-het, CO-N (CH₃) -C₃₋₇-cycloalkyle, C₃₋₇-cycloalkyle, C₃₋₇-cycloalkyl-C₁₋₂-alkylène, hétaryl-C₁₋₂-alkylène et hétaryle,
où ce radical peut éventuellement être substitué par un ou plusieurs radicaux choisis dans le groupe consistant en OH, un atome d'halogène, un groupe -C₁₋₃-fluoroalkyle, oxo, méthyle et phényle,
ou dans laquelle
R³ représente un radical choisi dans le groupe consistant en un groupe het et hétaryle qui peut être substitué éventuellement par un ou plusieurs radicaux choisis dans le groupe consistant en un atome d'halogène, un groupe C₁₋₃-fluoroalkyle, CN, OH, oxo, -C₁₋₆-alkyle, -C₁₋₃-alkylène-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-aryle, het, C₃₋₇-cycloalkyle et hétaryle,
qui peut lui-même être substitué éventuellement par un ou plusieurs radicaux choisis dans le groupe consistant en OH, un atome d'halogène, un groupe -C₁₋₃-fluoroalkyle, C₁₋₆-alkyle, C₆₋₁₀-aryle, -COO(C₁₋₃-alkyle) et O-(C₁₋₃-alkyle),
ou dans laquelle
R³ représente -O-R^{3.1},
où R^{3.1} est un radical choisi dans le groupe consistant en un groupe -C₁₋₆-alkyle, -C₆₋₁₀-aryle, -C₁₋₃-alkylène-C₆₋₁₀-aryle, hétaryle et het,
qui est substitué éventuellement en position ortho, para ou méta, par un, deux ou trois radicaux choisis indépendamment les uns des autres dans le groupe consistant en un atome de fluor, de chlore, de brome, un groupe hydroxy, CN, C₁₋₆-alkyle, C₁₋₃-fluoroalkyle, CO-(C₁₋₅-alkyle), -CO-(C₁₋₃-fluoroalkyle), -CO-NH-(C₁₋₆-alkylène)-hétaryle, -CO-N(C₁₋₃-alkyl)-(C₁₋₆-alkylène)-hétaryle, -CO-N(C₁₋₃-alkyl)-het, -CO-N(C₃₋₇-cycloalkyl)-het, -C₁₋₃-alkylène-OR^{2.1}, -C₁₋₃-alkylène-NR^{2.2}R^{2.3}, -NR^{2.2}R^{2.3}, O-R^{2.1}, SO-R^{2.1}, SO₂-R^{2.1}, COOH, COO-(C₁₋₄-alkyle), -O-C₁₋₃-alkylène-N(C₁₋₃-alkyle)₂, CO-NR^{2.2}R^{2.3}, NR^{2.2}-CO-R^{2.1}, C₆₋₁₀-aryle, C₆₋₁₀-aryl-C₁₋₂-alkylène, het-C₁₋₂-alkylène, -CO-het, het, -CO-C₃₋₇-cycloalkyle, -CO-N(C₁₋₃-alkyl)-C₃₋₇-cycloalkyle, C₃₋₇-cyclo-alkyle, C₃₋₇-cycloalkyl-C₁₋₂-alkylène, hétaryl-C₁₋₂-alkylène et hétaryle,
qui peut lui-même être substitué éventuellement par 1, 2, 3 ou 4 radicaux choisis indépendamment les uns des autres dans le groupe consistant en F, Cl, Br, un groupe méthyle, O-méthyle, éthyle, 0-éthyle, OH, oxo et CF₃,
et dans laquelle
R⁴ représente H, CN, OH, CF₃, CHF₂, CH₂F, F, un groupe méthyle, éthyle, -O-(C₁₋₃-alkyle), -C₁₋₃-alkylène-OH, -COO(C₁₋₃-alkyle), -CO-het, -(C₁₋₂-alkylène) -NH-SO₂-(C₁₋₂-alkyle), -(C₁₋₂-alkylène)-N(C₁₋₃-alkyl)-SO₂-(C₁₋₂-alkyle), - (C₁₋₂-alkylène)-O-(C₁₋₂-alkylène)-C₆₋₁₀-aryle, -C₁₋₃-alkylène-O-C₁₋₃-alkyle, -(C₁₋₂-alkylène)-N(C₁₋₃-alkyl)-CO-(C₁₋₂-alkyle), -NH-CO(C₁₋₃-alkylène)-O-(C₁₋₃-alkyle), -C₁₋₃-alkylène-NH-CO-(C₁₋₃-alkyle), -C₁₋₃-alkylène-NH-CO- (C₁₋₃-alkylène)-N(C₁₋₃-alkyle)₂, -O-(C₁₋₂-alkylène)-(C₆₋₁₀-aryle), -C₁₋₃-alkylène-NH-CO- (C₁₋₃-alkylène) -O- (C₁₋₃-alkyle), -CO-(C₆₋₁₀-aryle), -(C₁₋₂-alkylène) -N(C₁₋₃-alkyl) -CO-(C₁₋₂-alkylène) -O-(C₁₋₃-alkyle),
où le groupe aryle dans les radicaux ci-dessus peut lui-même être substitué éventuellement par un ou plusieurs autres radicaux choisis dans le groupe consistant en F, Cl, Br, un groupe méthyle, éthyle, propyle, isopropyle, cyclopropyle, -O-méthyle, -0-éthyle, -O-propyle, -O-isopropyle, -O-cyclopropyle, -OH et CF₃,
ou dans laquelle
R³ et R⁴ forment conjointement un hétérocycle mono-cyclique ou bicyclique, insaturé, saturé ou partiellement saturé, qui contient 1, 2 ou 3 hétéroatomes choisis dans le groupe consistant en N, 0 et S et qui peut éventuellement être substitué par un ou plusieurs radicaux choisis dans le groupe consistant en un atome d'halogène, un groupe OH, oxo, C₁₋₃-fluoroalkyle, CN, C₁₋₆-alkyle, -O-R^{2.1}, -CO-OR^{2.1}, SO-R^{2.1}, SO₂-R^{2.1}, -C₁₋₃-alkylène-NR^{2.2}-R^{2.3}, -NR^{2.2}R^{2.3}, C₆₋₁₀-aryle, C₃₋₇-cycloalkyle, het et hétaryle,
au moins un antagoniste (2) du récepteur EP4.

3. Combinaison de médicaments selon l'une des revendications 1 à 2, **caractérisée en ce qu'**elle comprend, en plus d'un ou plusieurs, de préférence un inhibiteur de PDE4 de formule générale 1, dans laquelle
X représente SO,
R¹ représente H,
R² représente H ou un groupe C₁₋₆-alkyle qui peut être substitué éventuellement par un ou plusieurs radicaux choisis F, Cl, CF₃, CHF₂ ou CH₂F ou qui peut être substitué éventuellement par un ou plusieurs radicaux choisis dans le groupe consistant en OR^{2.1}, COOR^{2.1}, CONR^{2.2}R^{2.3}, SR^{2.1}, SO-R^{2.1}, SO₂R^{2.1}, un groupe phényle, het, hétaryle, un groupe C₃₋₇-cycloalkyle monocyclique, CH₂-NR^{2.2}R^{2.3} et NR^{2.2}R^{2.3},
qui peut lui-même être substitué éventuellement par un ou plusieurs radicaux choisis dans le groupe consistant en OH, F, Cl, Br, CF₃, CHF₂, CH₂F, OR^{2.1}, un groupe oxo, méthyle, éthyle, propyle, isopropyle, méthanol, éthanol, phényle, COOR^{2.1}, CH₂-NR^{2.2}R^{2.3} et NR^{2.2}R^{2.3},
où
het représente un hétérocycle de trois à sept chaînons, monocyclique, saturé ou partiellement saturé, ou un hétérocycle de sept à onze chaînons, bicyclique, saturé ou partiellement saturé, qui contient 1, 2, ou 3 hétéroatomes choisis indépendamment les uns des autres dans le groupe consistant en N, S ou 0,
et où
hétaryle représente un groupe hétéroaryle de cinq à six chaînons, monocyclique, aromatique ou un groupe hétéroaryle de sept à onze chaînons, bicyclique, aromatique, qui contient 1, 2 ou 3 hétéroatomes, choisis indépendamment les uns des autres dans le groupe consistant en N, S ou 0,
et où
le groupe cycloalkyle peut être saturé ou partiellement saturé,
où R^{2.1} représente H ou un radical choisi dans le groupe consistant en un groupe méthyle, éthyle, propyle, isopropyle, méthanol, éthanol, C₃₋₇-cyclo-alkyle monocyclique, phényl-C₁₋₂-alkylène, -hétaryl-C₁₋₂-alkylène, -het-C₁₋₂-alkylène, C₃₋₇-cyclo-alkyl-C₁₋₂-alkylène, phényle, hétaryle et un groupe het, qui peut éventuellement être substitué par un ou plusieurs radicaux choisis dans le groupe consistant en OH, F, Cl, un groupe méthyle, éthyle, propyle, isopropyle, O-méthyle, O-éthyle, O-propyle, O-isopropyle et phényle,
où R^{2.2} et R^{2.3} représentent, indépendamment l'un de l'autre, H ou un radical choisi dans le groupe consistant en un groupe méthyle, éthyle, propyle, isopropyle, C₃₋₇-cycloalkyle monocyclique, phényl-C₁₋₃-alkylène, hétaryl-C₁₋₃-alkylène, phényle, -het, -hétaryle, CO-NH₂, CO-NHCH₃, -CON(CH₃)₂, SO₂-(C₁₋₂-alkyle), CO-R^{2.1} et COOR^{2.1},
qui peut éventuellement être substitué par un ou plusieurs radicaux choisis dans le groupe consistant en OH, F, Cl, un groupe méthyle, éthyle, propyle, isopropyle, phényle et COOR^{2.1},
ou
R² représente un groupe C₃₋₇-cycloalkyle monocyclique qui peut éventuellement être substitué par un radical choisi dans le groupe consistant en un groupe C₁₋₂-alcanol, C₁₋₃-fluoroalkyle, C₁₋₃-alkylène-OR^{2.1}, OR^{2.1}, COOR^{2.1}, -SO₂-NR^{2.2}R^{2.3}, -het, -NH-CO-O-(phényle), méthyle, éthyle, propyle, isopropyle, phényle, phényl-C₁₋₂-alkylène, -hétaryl-C₁₋₂-alkylène, C₃₋₇-cycloalkyle monocyclique et NR^{2.2}R^{2.3},
qui peut éventuellement être substitué par un ou plusieurs radicaux choisis dans le groupe consistant en OH, OR^{2.1}, un groupe oxo, F, Cl, CF₃, CHF₂, CH₂F, méthyle, éthyle, propyle, isopropyle, phényle et NR^{2.2}R^{2.3},
ou
R² représente un groupe phényle qui peut être substitué éventuellement par OH, SH, F, Cl ou Br ou par un ou plusieurs radicaux choisis dans le groupe consistant en OR^{2.1}, COOR^{2.1}, NR^{2.2}R^{2.3}, CH₂-NR^{2.2}R^{2.3}, un groupe C₃₋₇-cycloalkyle monocyclique, -het, méthyle, éthyle, propyle, isopropyle, CF₃, CHF₂, CH₂F, un groupe phényl-C₁₋₂-alkylène, het-C₁₋₂-alkylène, hétaryl-C₁₋₂-alkylène, phényle, SO₂-CH₃, SO₂-CH₂CH₃ et SO₂-NR^{2.2}R^{2.3},
qui peut lui-même être substitué éventuellement par un ou plusieurs radicaux choisis dans le groupe consistant en OH, OR^{2.1}, un groupe oxo, F, Cl, CF₃, CHF₂, CH₂F, méthyle, éthyle, propyle, isopropyle, phényle et NR^{2.2}R^{2.3},
ou
R² représente un radical choisi dans un groupe consistant en les groupes het et hétaryle qui peut être substitué éventuellement par un ou plusieurs radicaux choisis dans le groupe consistant en F, Cl, OH, un groupe oxo, CF₃, CHF₂ et CH₂F ou par un ou plusieurs radicaux choisis dans le groupe OR^{2.1}, C₁₋₃-alkylène-OR^{2.1}, SR^{2.1}, SO-R^{2.1}, SO₂-R^{2.1}, COOR^{2.1}, COR^{2.1}, un groupe méthanol, éthanol, C₃₋₇-cycloalkyle monocyclique, phényle, méthyle, éthyle, propyle, isopropyle, phényl-C₁₋₂-alkylène, hétaryl-C₁₋₂-alkylène, -het, hétaryle et NR^{2.2}R^{2.3},
qui peut lui-même être substitué éventuellement par un ou plusieurs radicaux choisis dans le groupe consistant en OH, OR^{2.1}, un groupe oxo, F, Cl, CF₃, CHF₂, CH₂F, méthyle, éthyle, propyle, isopropyle, butyle, isobutyle, phényle et NR^{2.2}R^{2.3},
et dans laquelle
R³ représente un groupe naphtaline ou phényle, qui est éventuellement substitué en position ortho, para et méta par un ou deux radicaux choisis indépendamment les uns des autres dans le groupe consistant en un atome de fluor, de chlore, de brome, un groupe hydroxy, CN, méthyle, éthyle, propyle, isopropyle, cyclopropyle, CF₃, CHF₂, CH₂F, -OCH₃, OCH₂CH₃, SO₂-CH₃, SO-CH₃, COOCH₃, COOCH₂CH₃, -CO-NH-(méthylène)-hétaryle, -CO-NH-(éthylène)-hétaryle, -CO-NH-hétaryle, -CO-N(CH₃)-het, CO-N(CH₃)-(méthylène)-het, -CO-N(CH₃)-(éthylène)-het, -CO-N(CH₃)-(méthylène)-hétaryle, -CO-N(CH₃)-(éthylène)-hétaryle, -CO-N(cyclopropyl)-het, CO-NH₂, CONH(CH₃) , CON(CH₃)₂, -CO-NH-(méthylène) -het, -CO-NH-(éthylène)-het, -NH-CO-méthyle, NCH₃-CO-méthyle, -NH-CO-éthyle, NCH₃-CO-éthyle, -NH-CO-propyle, NCH₃-CO-propyle, -NH-CO-isopropyle, NCH₃-CO-isopropyle, phényle, phényl-méthylène, phényl-éthylène, het-méthylène, het-éthylène-het, -CO-het, -CO-N(CH₃)-het, CO-N(CH₃)-cyclopropyle, C₃₋₇-cycloalkyle, C₃₋₇-cycloalkyle-méthylène, C₃₋₇-cyclo-alkyle-éthylène, hétaryl-méthylène, hétaryl-éthylène, -hétaryle, CH₂-NH₂, CH₂-NH(CH₃), CH₂-N(CH₃)₂, -NH₂, -NH(CH₃) et -N(CH₃)₂,
où ce radical peut être substitué éventuellement par un ou plusieurs radicaux choisis dans le groupe consistant en OH, F, Cl, -CF₃, CHF₂, CH₂F, un groupe oxo, méthyle et phényle,
ou dans laquelle
R³ représente un radical choisi dans le groupe consistant en un groupe het et hétaryle qui peut être substitué éventuellement par un ou plusieurs radicaux choisis dans le groupe consistant en F, Cl, Br, CF₃, CHF₂, CH₂F, CN, OH, un groupe oxo, méthyle, éthyle, propyle, isopropyle, butyle, isobutyle, cyclopropyle, -0-méthyle, -0-éthyle, -O-propyle, -O-isopropyle, COO-méthyle, -COO-éthyle, -COO-propyle, -COO-isopropyle, SO-(CH₃), SO-(CH₂-CH₃), SO₂-(CH₃), SO₂-(CH₂-CH₃), phényle, CH₂-NH₂, CH₂-NH(CH₃), CH₂-N(CH₃)₂, -NH₂, -NH(CH₃), -N(CH₃)₂, het et hétaryle,
qui peut lui-même être substitué éventuellement par un ou plusieurs radicaux choisis dans le groupe consistant en OH, F, Cl, CF₃, CHF₂, CH₂F, un groupe méthyle, éthyle, propyle, isopropyle, phényle, -COO-méthyle, -COO-éthyle et 0-méthyle, 0-éthyle,
ou dans laquelle
R³ représente -O-R^{3.1},
où R³.¹ est un radical choisi dans le groupe consistant en un groupe -C₁₋₃-alkyle, phényle, -C₁₋₃-alkylène-phényle, hétaryle et het,
qui est substitué éventuellement en position ortho, para ou méta, par un, deux ou trois radicaux choisis indépendamment les uns des autres dans le groupe consistant en un atome de fluor, de chlore, de brome, un groupe hydroxy, CN, méthyle, éthyle, propyle, isopropyle, butyle, isobutyle, CF₃, CHF₂, CH₂F, CO-(méthyle), CO-(éthyle), -CO-(propyle), CO-(isopropyle), -CO-(CF₃), -CO-NH-(méthylène)-hétaryle, -CO-NH-(éthylène)-hétaryle, -CO-N(CH₃)-(méthylène)-hétaryle, -CO-N(CH₃)-(éthylène)-hétaryle, -CO-N(CH₃)-(propylène)-hétaryle, -CO-N(CH₃)-(isopropylène)-hétaryle, -CO-N(CH₃)-het, -CO-N (cyclopropyl) -het, -CO-N(C₅₋₇-cycloalkyl)-het, -méthylène-O-méthyle, -éthylène-0-méthyle, -propylène-O-méthyle, -méthylène-O-éthyle, -éthylène-O-éthyle, -propylène-O-éthyle,-méthylène-NH₂, -méthylène-NHCH₃, -méthylène-N-(CH₃)₂, -éthylène-NH₂, -éthylène-NHCH₃, -éthylène-N(CH₃)₂, NH₂, N(CH₃)₂, NHCH₃, -0-méthyle, 0-éthyle, O-propyle, O-isopropyle, 0-butyle, O-isobutyle,-SO-CH₃, SO-éthyle, -SO-propyle, -SO-isopropyle, SO₂-méthyle, -SO₂-éthyle, SO₂-propyle, SO₂-isopropyle, COOH, COO-(méthyle), COO-(éthyle), COO-(propyle), COO-(isopropyle), -O-méthylène-N-(méthyle)₂, -O-éthylène-N(méthyle)₂, -O-méthylène-N(éthyle)₂, -O-éthylène-N(éthyle)₂, CO-NH₂, CO-NH(CH₃), CO-N(CH₃)₂, -NH-CO-méthyle, -NCH₃-CO-méthyle, -NH-CO-éthyle, NCH₃-CO-éthyle, phényle, phényl-méthylène, phényl-éthylène, het-méthylène, het-éthylène, -CO-het, het, -CO-C₅₋₇-cycloalkyle,-CO-cyclopropyle, -CO-N(CH₃)-C₅₋₇-cycloalkyle, -CO-N(CH₃)-cyclopropyle, C₅₋₇-cycloalkyle, cyclopropyle, C₅₋₇-cycloalkyl-méthylène, C₅₋₇-cycloalkyl-éthylène, cyclopropyl-méthylène, cyclopropyl-éthylène, hétaryl-méthylène, hétaryl-éthylène et hétaryle,
qui peut lui-même être substitué éventuellement par 1, 2, 3 ou 4 radicaux choisis indépendamment les uns des autres dans le groupe consistant en F, Cl, Br, un groupe méthyle, 0-méthyle, éthyle, 0-éthyle, OH, oxo et CF₃,
et dans laquelle
R⁴ représente H, CN, OH, CF₃, CHF₂, CH₂F, F, un groupe méthyle, éthyle, 0-méthyle ou O-éthyle,-méthylène-OH, -éthylène-OH, -propylène-OH, iso-propylène-OH, -COO(méthyle), -COO(éthyle), -COO-(propyle), -COO(isopropyle), -CO-het, -(méthylène)-NH-SO₂-(méthyle), -(méthylène)-NH-SO₂-(éthyle), -(éthylène)-NH-SO₂-(méthyle), -(éthy-lène)-NH-SO₂-(éthyle), -(méthylène)-N(CH₃)-SO₂-(méthyle), -(méthylène)-N(CH₃)-SO₂-(éthyle), -(éthylène)-N-(CH₃)-SO₂-(méthyle)-(éthylène)-N(CH₃)-SO₂-(éthyle), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle, -éthylène-O-éthyle, -(méthylène)-N-(CH₃)-CO-(méthyle), -(méthylène)-N(CH₃)-CO-(éthyle), -(éthylène)-N(CH₃)-CO-(méthyle), -(éthylène)-N (CH₃) -CO- (éthyle), -NH-CO- (méthylène)-O-(méthyle), -NH-CO-(méthylène)-O-(éthyle), -NH-CO-(éthylène)-O-(méthyle), -NH-CO-(éthylène)-O-(éthyle), -méthylène-NH-CO-(méthyle), -méthylène-NH-CO-(éthyle), -éthylène-NH-CO-(méthyle), -éthylène-NH-CO-(éthyle), -méthylène-NH-CO-(méthylène)-N(méthyle)₂, -méthylène-NH-CO-(éthylène)-N(méthyle)₂, -éthylène-NH-CO-(méthylène)-N(méthyle)₂, -éthylène-NH-CO-(éthylène)-N(méthyle)₂, -méthylène-NH-CO-(méthylène)-O-(méthyle), -méthylène-NH-CO-(éthylène)-O-(méthyle), -éthylène-NH-CO-(méthylène)-O-(méthyle), -méthylène-NH-CO-(méthylène)-O-(éthyle), -méthylène-NH-CO-(éthylène)-O-(éthyle), -éthylène-NH-CO-(méthylène)-O-(éthyle), -méthylène-N(CH₃)-CO-(méthylène)-O-(méthyle), -méthylène-N(CH₃)-CO-(éthylène)-0-(méthyle), -(éthylène)-N(CH₃)-CO-(méthylène)-O-(méthyle), -(méthylène)-N(CH₃)-CO-(méthylène)-O-(éthyle), -(méthylène)-N(CH₃)-CO-(éthylène)-O-(éthyle), -(éthylène)-N(CH₃)-CO-(méthylène)-O-(éthyle), -O-(méthylène)-phényle, -O-(éthylène)-phényle, -CO-phényle,
où le groupe phényle dans les radicaux ci-dessus peut être substitué éventuellement par un ou plusieurs autres radicaux choisis dans le groupe F, Cl, Br, un groupe méthyle, éthyle, propyle, -0-méthyle, -0-éthyle, -O-propyle, -OH et CF₃,
ou dans laquelle
R³ et R⁴ forment conjointement, un hétérocycle mono-cyclique ou bicyclique, insaturé, saturé ou partiellement saturé, qui contient 1, 2 ou 3 hétéroatomes choisis dans le groupe consistant en N, 0 et S et qui peut éventuellement être substitué par un ou plusieurs radicaux choisis dans le groupe consistant en F, Cl, Br, OH, oxo, CF₃, CHF₂, CH₂F, CN, un groupe méthyle, éthyle, propyle, isopropyle, cyclopropyle, COO-méthyle, -COO-éthyle, O-méthyle, 0-éthyle, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, phényle, C₅₋₇-cycloalkyle, het et hétaryle,
au moins un antagoniste (2) du récepteur EP4.

4. Combinaison de médicaments selon l'une des revendications 1 à 3, **caractérisée en ce qu'**elle comprend, en plus d'un ou plusieurs, de préférence à un inhibiteur de PDE4 de formule générale 1, dans laquelle
R² est un radical selon la formule 3 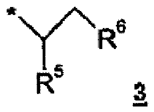 dans laquelle R⁶ est OH ou NH₂ et
dans laquelle R⁵ est un radical choisi dans le groupe consistant en un groupe C₁₋₄-alkyle, un groupe hétéroaryle de cinq à six chaînons comportant 1, 2 ou 3 hétéroatomes du groupe S, 0 et N et un groupe phényle, où ce radical peut être substitué éventuellement par un ou plusieurs radicaux choisis dans le groupe consistant en OH, F, Br, OR^{2.1}, un groupe oxo, méthyle, éthyle, méthanol, éthanol, phényle, COOR^{2.1}, CH₂-NR^{2.2}R^{2.3} et NR^{2.2}R^{2.3},
au moins un antagoniste (2) du récepteur EP4.

5. Combinaison de médicaments selon la revendication 4, **caractérisée en ce qu'**elle comprend, en plus d'un ou plusieurs, de préférence un inhibiteur de PDE4 de formule générale 1, dans laquelle
R² est un radical selon la formule 3 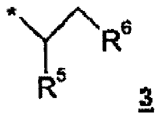
dans laquelle R⁶ est OH ou NH₂ et
dans laquelle R⁵ est un groupe méthyle, éthyle, propyle ou isopropyle,
au moins un antagoniste (2) du récepteur EP4.

6. Combinaison de médicaments selon l'une des revendications 1 à 3, **caractérisée en ce qu'**elle comprend, en plus d'un ou plusieurs, de préférence un inhibiteur de PDE4 de formule générale 1, dans laquelle
R² est un cycle cycloalkyle monocyclique de trois, quatre, cinq, six ou sept chaînons, qui peut éventuellement être substitué en position spiro par un radical choisi dans le groupe consistant en -CH₂-OR^{2.1}, un groupe C₂₋₆-alkylène-OR^{2.1} ramifié ou non ramifié, méthyle, éthyle, propyle, isopropyle, butyle, isobutyle, cyclopropyle, -CF₃, CHF₂, CH₂F et C₂₋₄-fluoroalkyle, et où
R^{2.1} est choisi dans le groupe consistant en les groupes méthyle, éthyle, propyle, isopropyle, butyle et isobutyle,
au moins un antagoniste (2) du récepteur EP4.

7. Combinaison de médicaments selon l'une des revendications 1 à 3, **caractérisée en ce qu'**elle comprend, en plus d'un ou plusieurs, de préférence un inhibiteur de PDE4 de formule générale 1, dans laquelle
R² est un groupe cyclopropyle qui peut être substitué éventuellement par un autre radical choisi dans le groupe consistant en -NH₂, CH₂- NH₂, -NH(CH₃), -N-(CH₃)₂, un groupe méthyle, éthyle, propyle, isopropyle, -NH-CO-(tert-butyle), -NH-CO-O-(tert-butyle), -N(CH₃)-CO-(tert-butyle)-, -N(CH₃)-CO-O-(tert-butyle), -CF₃, -CHF₂, CH₂F, F, Cl et Br,
au moins un antagoniste (2) du récepteur EP4.

8. Combinaison de médicaments selon l'une des revendications 1 à 3, **caractérisée en ce qu'**elle comprend, en plus d'un ou plusieurs, de préférence un inhibiteur de PDE4 de formule générale 1, dans laquelle
R² est un groupe phényle qui peut être substitué éventuellement dans une ou dans les deux positions méta, par un ou plusieurs radicaux choisis dans le groupe consistant en un groupe méthyle, éthyle, propyle, isopropyle, cyclopropyle, F, Cl, Br, OH, OR^{2.1}, COOR^{2.1}, CF₃, CHF₂, CH₂F, NH₂, NH(CH₃) et N(CH₃)₂, où R^{2.1} peut être H, un groupe méthyle ou éthyle,
au moins un antagoniste (2) du récepteur EP4.

9. Combinaison de médicaments selon l'une des revendications 1 à 3, **caractérisée en ce qu'**elle comprend, en plus d'un ou plusieurs, de préférence un inhibiteur de PDE4 de formule générale 1, dans laquelle
R² est un hétérocycle monocyclique, saturé, de trois, quatre, cinq, six ou sept chaînons, ayant 1, 2 ou 3 hétéroatomes choisis respectivement dans le groupe consistant en N, 0 et S, qui peut être substitué éventuellement par un ou plusieurs radicaux choisis dans le groupe atome de fluor, de chlore, de brome, CF₃, CHF₂, CH₂F, OH et oxo, ou par un ou plusieurs radicaux choisis dans le groupe OR^{2.1}, un groupe C₁₋₃-alkylène-OR^{2.1}, SR^{2.1}, SO-R^{2.1}, SO₂-R^{2.1}, COOR^{2.1}, COR^{2.1}, C₁₋₆-alcanol, C₃₋₁₀-cycloalkyle, phényle, C₁₋₆-alkyle, phényl-C₁₋₆-alkylène, hétaryl-C₁₋₆-alkylène, het, hétaryle et NR^{2.2}R^{2.3}, où le dernier groupe de radicaux cité peut être lui-même substitué éventuellement par un ou plusieurs radicaux choisis dans le groupe consistant en OH, OR^{2.1}, oxo, F, Cl, CF₃, CHF₂, CH₂F, un groupe C₁₋₆-alkyle, phényle et NR^{2.2}R^{2.3},
où R^{2.1}, R^{2.2} et R^{2.3} sont tels que définis dans la revendication 1,
au moins un antagoniste (2) du récepteur EP4.

10. Combinaison de médicaments selon la revendication 9, **caractérisée en ce qu'**elle comprend, en plus d'un ou plusieurs, de préférence un inhibiteur de PDE4 de formule générale 1, dans laquelle
R² est un hétérocycle monocyclique, saturé, de six chaînons, ayant un hétéroatome choisi dans le groupe consistant en N, 0 et S, qui peut être substitué éventuellement par un ou plusieurs radicaux choisis dans le groupe consistant en F, Cl, Br, CF₃, CHF₂, CH₂F, OH, oxo, NH₂, NHCH₃ et N(CH₃)₂, un groupe méthyle, éthyle, propyle, isopropyle, cyclopropyle, méthoxy et éthoxy,
au moins un antagoniste (2) du récepteur EP4.

11. Combinaison de médicaments selon l'une des revendications 1 à 3, **caractérisée en ce qu'**elle comprend, parallèlement un ou plusieurs, de préférence à un inhibiteur de PDE4 de formule générale 1, dans laquelle
R² représente un radical choisi dans le groupe consistant en un groupe pipéridine ou tétrahydro-pyrane qui peut être substitué éventuellement par un ou plusieurs radicaux choisis dans le groupe F, Cl, Br, OH, CF₃, CHF₂, CH₂F, NH₂, NHCH₃, N(CH₃)₂, un groupe oxo, méthyle et méthoxy,
au moins un antagoniste (2) du récepteur EP4.

12. Combinaison de médicaments selon l'une des revendications 1 à 11, **caractérisée en ce qu'**elle comprend, en plus d'un ou plusieurs, de préférence un inhibiteur de PDE4 de formule générale 1, dans laquelle
R³ est un groupe naphtaline ou phényle,
qui peut éventuellement être substitué dans une position quelconque par un, deux ou trois radicaux choisis indépendamment les uns des autres dans le groupe consistant en un atome de fluor, de chlore, de brome, un groupe hydroxy, CN, méthyle, éthyle, propyle, isopropyle, cyclopropyle, CF₃, CHF₂, CH₂F, OCH₃, OCH₂CH₃, SO₂-CH₃ , SO₂-CH₂CH₃, COOCH₃ et CO-O-CH₂CH₃,
au moins un antagoniste (2) du récepteur EP4.

13. Combinaison de médicaments selon l'une des revendications 1 à 11, **caractérisée en ce qu'**elle comprend, en plus d'un ou plusieurs, de préférence un inhibiteur de PDE4 de formule générale 1, dans laquelle
R³ représente un radical choisi dans le groupe consistant en un groupe het et hétaryle qui peut être substitué éventuellement par un ou plusieurs radicaux choisis dans le groupe consistant en F, Cl, Br, CF₃, CHF₂, CH₂F, CN, OH, oxo, un groupe méthyle, éthyle, propyle, isopropyle, butyle, isobutyle, cyclopropyle, C₅₋₇-cycloalkyle, 0-méthyle, -0-éthyle, -O-propyle, -0-isopropyle, -COO-méthyle, -COO-éthyle, -COO-propyle, -COO-isopropyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, het et hétaryle, qui peut lui-même être substitué par un ou plusieurs radicaux choisis dans le groupe OH, F, Cl, Br, CF₃, CHF₂, CH₂F, un groupe méthyle, éthyle, propyle, isopropyle, phényle, -COO-méthyle, -COO-éthyle, -COO-phényle, -COO-isopropyle et 0-méthyle, 0-éthyle, O-propyle et O-isopropyle,
et dans laquelle
R⁴ représente H, CN, OH, CF₃, CHF₂, CH₂F, F, un groupe méthyle, éthyle, -0-méthyle ou 0-éthyle et
où
het représente un hétérocycle de trois à sept chaînons, monocyclique, saturé ou partiellement saturé, ou un hétérocycle de sept à onze chaînons, bicyclique, condensé, saturé ou partiellement saturé, qui contient 1, 2 ou 3 hétéroatomes choisis indépendamment les uns des autres dans le groupe consistant en N, S ou 0,
et où
hétaryle représente un groupe hétéroaryle de cinq à six chaînons, monocyclique, aromatique ou un groupe hétéroaryle de sept à onze chaînons, bicyclique, condensé, aromatique, qui contient respectivement 1, 2 ou 3 hétéroatomes, choisis indépendamment les uns des autres dans le groupe consistant en N, S ou 0,
et où
le groupe cycloalkyle peut être saturé ou partiellement saturé,
au moins un antagoniste (2) du récepteur EP4.

14. Combinaison de médicaments selon l'une des revendications 1 à 11, **caractérisée en ce qu'**elle comprend, en plus d'un ou plusieurs, de préférence un inhibiteur de PDE4 de formule générale 1, dans laquelle
R³ représente un radical choisi parmi un hétérocycle bicyclique, de sept à onze chaînons, saturé ou partiellement saturé ou un groupe hétéroaryle bicyclique de sept à onze chaînons qui est choisi dans le groupe consistant en les groupes indole, dihydro-indole, quinazoline, dihydroquinazoline, tétrahydroquinazoline, benzo-isoxazole, dihydro-benzo-isoxazole, benzo-oxazine, dihydrobenzo-oxazine, benzothiazole, dihydrobenzothiazole, triazolopyridine, dihydrotriazolopyridine, benzo-furane, dihydrobenzofurane, isobenzofurane et dihydro-isobenzofurane,
qui peut être substitué éventuellement par un ou plusieurs radicaux choisis dans le groupe consistant en F, Cl, Br, CF₃, CHF₂, CH₂F, CN, OH, oxo, un groupe méthyle, éthyle, propyle, isopropyle, butyle, isobutyle, cyclopropyle, -0-méthyle, -0-éthyle, -O-propyle, -0-isopropyle, COO-méthyle, -COO-éthyle, -COO-propyle, -COO-isopropyle, SO₂-(CH₃), SO₂-(CH₂CH₃) , SO-(CH₃), SO-(CH₂-CH₃), phényle, -CH₂-NH₂, -CH₂-NHCH₃, -CH₂-N(CH₃)₂, NH₂, NHCH₃, N(CH₃)₂, un groupe furanyle et pyridinyle,
qui peut lui-même être substitué par un ou plusieurs radicaux choisis dans le groupe OH, F, Cl, Br, CF₃, CHF₂, CH₂F, un groupe méthyle, éthyle, propyle, isopropyle, phényle, -COO-méthyle, -COO-éthyle et 0-méthyle, 0-éthyle,
au moins un antagoniste (2) du récepteur EP4.

15. Combinaison de médicaments selon l'une des revendications 1 à 11, **caractérisée en ce qu'**elle comprend, en plus d'un ou plusieurs, de préférence un inhibiteur de PDE4 de formule générale 1, où
R³ représente un radical choisi parmi un hétérocycle monocyclique, saturé ou partiellement saturé, de trois à sept chaînons, ou un groupe hétéroaryle monocyclique de cinq à six chaînons,
qui est choisi dans le groupe consistant en les groupes imidazole, dihydro-imidazole, oxadiazole, oxadiazolidine, pyrazole, pyridine et dihydro-pyrazole
qui peut être substitué éventuellement par un ou plusieurs radicaux choisis dans le groupe consistant en F, Cl, Br, CF₃, CHF₂, CH₂F, CN, OH, oxo, un groupe méthyle, éthyle, propyle, isopropyle, butyle, isobutyle, cyclopropyle, -0-méthyle, -0-éthyle, -O-propyle, -0-isopropyle, COO-méthyle, -COO-éthyle, -COO-propyle, -COO-isopropyle, SO₂-(CH₃), SO₂-(CH₂CH₃), SO-(CH₃), SO-(CH₂-CH₃), phényle, -CH₂-NH₂, -CH₂-NHCH₃, -CH₂-N (CH₃)₂, NH₂, NHCH₃, N(CH₃)₂, un groupe furanyle et pyridinyle,
qui peut lui-même être substitué par un ou plusieurs radicaux choisis dans le groupe OH, F, Cl, Br, CF₃, CHF₂, CH₂F, un groupe méthyle, éthyle, propyle, isopropyle, phényle, -COO-méthyle, -COO-éthyle et 0-méthyle, 0-éthyle,
au moins un antagoniste (2) du récepteur EP4.

16. Combinaison de médicaments selon l'une des revendications 1 à 11, **caractérisée en ce qu'**elle comprend, en plus d'un ou plusieurs, de préférence un inhibiteur de PDE4 de formule générale 1, où
R³ et R⁴ forment conjointement un hétérocycle de trois à onze chaînons, monocyclique ou bicyclique, insaturé ou partiellement saturé, qui contient 1, 2 ou 3 hétéroatomes choisis dans le groupe consistant en N, 0 et S et qui peut éventuellement être substitué par un ou plusieurs radicaux choisis dans le groupe consistant en F, Cl, Br, OH, oxo, CF₃, CHF₂, CH₂F, CN, un groupe méthyle, éthyle, propyle, isopropyle, cyclopropyle, COO-méthyle,-COO-éthyle, 0-méthyle, 0-éthyle, SO₂-(CH₃), SO₂-(CH₂CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, un hétérocycle saturé ou partiellement saturé, de cinq à six chaînons et un groupe hétéroaryle de cinq à six chaînons,
au moins un antagoniste (2) du récepteur EP4.

17. Combinaison de médicaments selon la revendication 16, **caractérisée en ce qu'**elle comprend, parallèlement un ou plusieurs, de préférence un inhibiteur de PDE4 de formule générale 1, où
R³ et R⁴ forment conjointement un hétérocycle bicyclique choisi dans le groupe consistant en un groupe tétrahydroquinazoline, tétrahydrobenzoxazine et dihydro-indole, dihydro-isobenzofurane qui peut éventuellement être substitué par un ou plusieurs radicaux choisis dans le groupe consistant en F, Cl, Br, OH, oxo, CF₃, CHF₂, CH₂F, CN, un groupe méthyle, éthyle, propyle, isopropyle, cyclopropyle, COO-méthyle, -COO-éthyle, 0-méthyle, 0-éthyle, SO₂-(CH₃), SO₂-(CH₂CH₃), phényle, -CH₂-NH₂, -CH₂-NHCH₃, -CH₂-N(CH₃)₂, NH₂, NHCH₃, N(CH₃)₂, un hétérocycle saturé ou partiellement saturé, de cinq ou six chaînons et un groupe hétéroaryle de cinq ou six chaînons,
au moins un antagoniste (2) du récepteur EP4.

18. Combinaison de médicaments selon l'une des revendications 1 à 11, **caractérisée en ce qu'**elle comprend, en plus d'un ou plusieurs, de préférence un inhibiteur de PDE4 de formule générale 1, où
R³ est -O-R^{3.1},
R^{3.1} est un radical choisi dans le groupe consistant en un groupe méthyle, éthyle, propyle, isopropyle, butyle, isobutyle, pentyle, isopentyle, -phényle, -méthylène-phényle, -éthylène-phényle, -propylène-phényle, -isopropylène-phényle, hétaryle et het,
qui est substitué éventuellement en position ortho, para ou méta, par un, deux ou trois radicaux choisis indépendamment les uns des autres dans le groupe consistant en un atome de fluor, de chlore, de brome, un groupe hydroxy, CN, méthyle, éthyle, propyle, isopropyle, butyle, isobutyle,-CF₃, CHF₂, CH₂F, CO-(méthyle), CO-(éthyle), -CO-(propyle), CO-(isopropyle), CO-(butyle), CO-(isobutyle), -CO-(CF₃), -CO-(CH₂F), -CO(CHF₂), -CO-NH-(méthylène)-hétaryle, -CO-NH-(éthylène)-hétaryle, -CO-NH-(propylène)-hétaryle, -CO-NH-(isopropylène)-hétaryle, -CO-N(CH₃)-(méthylène)-hétaryle, -CO-N(CH₃)-(éthylène)-hétaryle, -CO-N-(CH₃)-(propylène)-hétaryle, -CO-N(CH₃)-(isopropylène)-hétaryle, -CO-N(CH₃)-het, -CO-N(C₃₋₇-cycloalkyl)-het, -méthylène-O-méthyle, -éthylène-0-méthyle, -méthylène-O-éthyle, -éthylène-O-éthyle, -méthylène-NH₂, -éthylène-NH₂, -méthylène-NHCH₃, -éthylène-NHCH₃, -méthylène-N (CH₃)₂, -éthylène-N (CH₃)₂, -NH₂, -NHCH₃, -N(CH₃)₂, -O-méthyle, O-éthyle, O-propyle, O-isopropyle, -SO-CH₃, SO-(CH₂CH₃), -SO₂-(CH₃), -SO₂- (CH₂CH₃), COOH, COO-(méthyle), COO-(éthyle), COO-(propyle), COO-isopropyle), -O-méthylène-N(méthyle)₂, -O-éthylène-N(méthyle)₂, -O-méthylène-N(éthyle)₂, -O-éthylène-N(éthyle)₂, CO-NH₂, CO-NHCH₃, CO-N(CH₃)₂, NH-CO-méthyle, NCH₃-CO-méthyle, NH-CO-éthyle, N(CH₃)-CO-éthyle, phényle, phényl-méthylène, phényl-éthylène, het-méthylène, het-éthylène, -CO-het, het, -CO-C₄₋₇-cycloalkyle, -CO-cyclopropyle, -CO-N(CH₃)-cyclopropyle, -CO-N(CH₃)-C₄₋₇-cycloalkyle, C₄₋₇-cycloalkyle, cyclopropyle, C₄₋₇-cycloalkyl-méthylène, cyclopropyl-méthylène C₄₋₇-cycloalkyl-éthylène, cyclopropyl-éthylène, hétaryl-méthylène, hétaryl-éthylène et hétaryle,
qui peut lui-même être substitué éventuellement par 1, 2, 3 ou 4 radicaux choisis indépendamment les uns des autres dans le groupe consistant en F, Cl, Br, un groupe méthyle, 0-méthyle, éthyle, 0-éthyle, OH, oxo et CF₃,
au moins un antagoniste (2) du récepteur EP4.

19. Combinaison de médicaments selon l'une des revendications 1 à 11, **caractérisée en ce qu'**elle comprend, en plus d'un ou plusieurs, de préférence un inhibiteur de PDE4 de formule générale 1, où
R⁴ représente H, CN, OH, CF₃, CHF₂, CH₂F, F, un groupe méthyle, éthyle, 0-méthyle ou 0-éthyle, -méthylène-OH, -éthylène-OH, -propylène-OH, isopropylène-OH, -COO(méthyle), -COO(éthyle), -COO(propyle), -COO(isopropyle), -CO-het, -(méthylène)-NH-SO₂-(méthyle), -(méthylène)-NH-SO₂-(éthyle), -(éthylène)-NH-SO₂-(méthyle), -(éthylène)-NH-SO₂-(éthyle), - (méthylène)-N(CH₃)-SO₂-(méthyle), -(méthylène)-N(CH₃)-SO₂-(éthyle), -(éthylène)-N-(CH₃)-SO₂-(méthyle), -(éthylène)-N(CH₃)-SO₂-(éthyle), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle,-éthylène-O-éthyle, -(méthylène)-N-(CH₃)-CO-(méthyle), -(méthylène)-N(CH₃)-CO-(éthyle), -(éthylène)-N(CH₃)-CO-(méthyle), -(éthylène)-N(CH₃)-CO-(éthyle), -NH-CO-(méthylène)-O-(méthyle), -NH-CO-(méthylène)-O-(éthyle), -NH-CO-(éthylène)-O-(méthyle), -NH-CO-(éthylène)-O-(éthyle), -méthylène-NH-CO-(méthyle), -méthylène-NH-CO-(éthyle), -éthylène-NH-CO-(méthyle), -éthylène-NH-CO-(éthyle), -méthylène-NH-CO-(méthylène)-N(méthyle)₂,-méthylène-NH-CO-(éthylène)-N(méthyle)₂, -éthylène-NH-CO-(méthylène)-N(méthyle)₂, -éthylène-NH-CO-(éthylène)-N(méthyle)₂, -méthylène-NH-CO-(méthylène)-O-(méthyle), -méthylène-NH-CO-(éthylène)-0-(méthyle), -éthylène-NH-CO-(méthylène)-O-(méthyle), -méthylène-NH-CO-(méthylène)-O-(éthyle), -méthylène-NH-CO-(éthylène)-O-(éthyle), -éthylène-NH-CO-(méthylène)-O-(éthyle), -méthylène-N(CH₃)-CO-(méthylène)-O-(méthyle), -méthylène-N(CH₃)-CO-(éthylène)-O-(méthyle), -(éthylène)-N(CH₃)-CO-(méthylène)-O-(méthyle), -(méthylène)-N(CH₃)-CO-(méthylène)-O-(éthyle), (méthylène)-N(CH₃)-CO-(éthylène)-O-(éthyle), -(éthylène)-N(CH₃)-CO-(méthylène)-O-(éthyle), -O-(méthylène)-phényle, -O-(éthylène)-phényle, -CO-phényle,
et où groupe phényle dans les radicaux ci-dessus peut être substitué éventuellement par un ou plusieurs autres radicaux choisis dans le groupe consistant en F, Cl, Br, un groupe méthyle, éthyle, propyle, -O-méthyle, -O-éthyle, -O-propyle, -OH et CF₃, au moins un antagoniste (2) du récepteur EP4.

20. Combinaison de médicaments selon l'une des revendications 1 à 11, **caractérisée en ce qu'**elle comprend, en plus d'un ou plusieurs, de préférence un inhibiteur de PDE4 de formule générale 1, où
R³ est un radical choisi dans le groupe consistant en les groupes oxazole, imidazole et thiazole, où ce radical peut être substitué éventuellement par un, deux ou trois autres radicaux choisis indépendamment les uns des autres dans le groupe consistant en un groupe méthyle, éthyle, propyle, isopropyle, O-méthyle, O-éthyle, O-propyle, O-isopropyle, OH, F, Cl, Br, CF₃, phényle, hétaryle et C₃₋₆-cycloalkyle,
au moins un antagoniste (2) du récepteur EP4.

21. Combinaison de médicaments selon l'une des revendications 1 à 20, **caractérisée en ce qu'**elle est choisie, en plus d'un ou plusieurs, de préférence un inhibiteur de PDE4 de formule générale 1, dans le groupe consistant en :
1.1. le (R)-2-{2-[4-(4-chlorophényl)-pipéridin-1-yl]-5-oxo-6,7-dihydro-5H-5λ⁴-thiéno[3,2-d]pyrimidin-4-ylaminol-3-méthylbutan-1-ol
1.2. le (1-{2-[4-(4-chlorophényl)-pipéridin-1-yl]-5-oxo-6,7-dihydro-5H-5λ⁴-thiéno[3,2-d]pyrimidin-4-ylamino}-cyclopropyl)-méthanol
1.3. le (R)-2-{2-[4-(4-chlorophényl)-pipéridin-1-yl]-5-oxo-6,7-dihydro-5H-5λ⁴-thiéno[3,2-d]pyrimidin-4-ylamino}-pentan-1-ol
1.4. le (R)-1-{2-[4-(4-chlorophényl)-pipéridin-1-yl]-5-oxo-6,7-dihydro-5H-5λ⁴-thiéno[3,2-d]pyrimidin-4-ylaminol-1-(4-fluorophényl)-2-méthylpropan-2-ol
1.5. la (S)-5-{2-[4-(4-chlorophényl)-pipéridin-1-yl]-5-oxo-6,7-dihydro-5H-5λ⁴-thiéno[3,2-d]pyrimidin-4-ylamino}-1-méthylpipéridin-2-one
1.6. la {2-[4-(4-chlorophényl)-pipéridin-1-yl]-5-oxo-6,7-dihydro-5H-5λ⁴-thiéno[3,2-d]pyrimidin-4-yl}-(tétrahydropyran-4-yl)-amine
1.7. la 1-(4-(1-hydroxyméthylcyclopropylamino)-5-oxo-6,7-dihydro-5H-5λ⁴-thiéno[3,2-d]pyrimidin-2-yl)-3'-méthyl-1'H-spiro[pipéridin-4,4'-quinazolin]-2'(3'H)-one
1.8. le {1-[2-(4-benzo[d]isoxazol-3-yl-pipéridin-1-yl)-5-oxo-6,7-dihydro-5H-5λ⁴-thiéno[3,2-d]pyrimidin-4-ylaminol-cyclopropyl}-méthanol
1.9. le (1-{2-[4-(2-éthyl-5-fluoro-1H-indol-3-yl)-pipéridin-1-yl]-5-oxo-6,7-dihydro-5H-5λ⁴-thiéno[3,2-d]pyrimidin-4-ylamino}-cyclopropyl)-méthanol
1.10. le 1-[4-((S)-1-méthyl-6-oxopipéridin-3-ylamino)-5-oxo-6,7-dihydro-5H-5λ⁴-thiéno[3,2-d]pyrimidin-2-yl]-4-phénylpipéridin-4-carbonitrile
1.11. la 3'-méthyl-1-(4-(tétrahydro-2H-pyran-4-ylamino)-6-oxo-6,7-dihydro-5H-5λ⁴-thiéno[3,2-d]pyrimidin-2-yl)-1'H-spiro[pipéridin-4,4'-quinazolin]-2'(3'H)-one
1.12. la (3-fluorophényl)-[5-oxo-2-(3,4,5,6-tétrahydro-2H-[4,4']bipyridinyl-1-yl)-6,7-dihydro-5H-5λ⁴-thiéno[3,2-d]pyrimidin-4-yl]-amene
1.13. la {2-[4-(2-éthyl-5-fluoro-1H-indol-3-yl)-pipéridin-1-yl]-5-oxo-6,7-dihydro-5H-5λ⁴-thiéno[3,2-d]pyrimidin-4-yl}-(3-fluorophényl)-amine
1.14. le (1-{2-[4-(2,4-difluorophényl)-pipéridin-1-yl]-5-oxo-6,7-dihydro-5H-5λ⁴-thiéno[3,2-d]pyrimidin-4-ylamino}-cyclopropyl)-méthanol
1.15. la {2-[4-(2,4-difluorophényl)-pipéridin-1-yl]-5-oxo-6,7-dihydro-5H-5λ⁴-thiéno[3,2-d]pyrimidin-4-yl}-(tétrahydropyran-4-yl)-amine
1.16. la (S)-5-[2-(4-benzoxazol-2-yl-pipéridin-1-yl)-5-oxo-6,7-dihydro-5H-5λ⁴-thiéno[3,2-d]pyrimidin-4-ylamino]-1-méthylpipéridin-2-one
1.17. le (1-{2-[4-(6-fluorobenzène[d]isoxazol-3-yl)-pipéridin-1-yl]-5-oxo-6,7-dihydro-5H-5λ⁴-thiéno[3,2-d]pyrimidin-4-ylamino}-cyclopropyl)-méthanol
1.18. le (1-{2-[4-(5-fluorobenzène[d]isoxazol-3-yl)-pipéridin-1-yl]-5-oxo-6,7-dihydro-5H-5λ⁴-thiéno[3,2-d]pyrimidin-4-ylamino}-cyclopropyl)-méthanol
1.19. la {2-[4-(5-furan-2-yl-2H-pyrazol-3-yl)-pipéridin-l-yl]-5-oxo-6,7-dihydro-5H-5λ⁴-thiéno[3,2-d]pyrimidin-4-yl}-(tétrahydropyran-4-yl)-amine
1.20. la (3-fluorophényl)-{5-oxo-2-[4-(3-pyridin-4-yl-[1,2,4]oxadiazol-5-yl)-pipéridin-1-yl]-6,7-dihydro-5H-5λ⁴-thiéno[3,2-d]pyrimidin-4-yl}-amene
1.21. le (R)-3-méthyl-2-{5-oxo-2-[4-(3-pyridin-4-yl-[1,2,4]oxadiazol-5-yl)-pipéridin-1-yl]-6,7-dihydro-5H-5λ⁴-thiéno[3,2-d]pyrimidin-4-ylamino}-butan-1-ol
1.22. la (S)-5-{2-[4-(4-fluorophénoxy)-pipéridin-1-yl]-5-oxo-6,7-dihydro-5H-5λ⁴-thiéno[3,2-d]pyrimidin-4-ylamino}-1-méthylpipéridin-2-one
1.23. la (2-{4-[4-(4,5-dihydro-oxazol-2-yl)-phénoxy]-pipéridin-1-yl}-5-oxo-6,7-dihydro-5H-5λ⁴-thiéno[3,2-d]pyrimidin-4-yl)-(tétrahydropyran-4-yl)-amine
1.24. l'acide 4-{1-[5-oxo-4-(tétrahydropyran-4-ylamino)-6,7-dihydro-5H-5λ⁴-thiéno[3,2-dlpyrimidin-2-yl]-pipéridin-4-yloxyl-benzoïque
1.25. la 2-(1-{2-[4-(4-chlorophényl)-pipéridin-1-yl]-5-oxo-6,7-dihydro-5H-5λ⁴-thiéno[3,2-d]pyrimidin-4-ylaminol-cyclopropyl)propan-2-ol
1.26. la {2-[4-(5-tert-butyl-1-méthyl-1H-indol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.27. la 2-[4-(5-furan-2-yl-1-méthyl-1H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.28. la (S)-5-(2-{4-[4-(4,5-dihydrooxazol-2-yl)-phénoxy]-pipéridin-1-yl}-5-oxo-6,7-dihydro-5H-5λ⁴-thiéno[3,2-d]pyrimidin-4-ylamino)-1-méthylpipéridin-2-one
1.29. la {2-[4-(5-furan-2-yl-2-méthyl-2H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.30. la {2-[4-(1-méthyl-1H-imidazol[4,5,c]pyridin-2-yl)-pipéridin-1-yl]-5-oxo-6,7-dihydro-5H-5λ⁴-thiéno[3,2-d]pyrimidin-4-yl}-(tétrahydropyran-4-yl)-amine
1.31. la 2-méthoxy-N-{1-[5-oxo-4-(tétrahydropyran-4-ylamino)-6,7-dihydro-5H-5λ⁴-thiéno[3,2-d]pyrimidin-2-yl]-4-phénylpipéridin-4-ylméthyl}-acétamide
1.32. la N-cyclopropyl-N-méthyl-4-{1-[5-oxo-4-(tétrahydropyran-4-ylamino)-6,7-dihydro-5H-5λ⁴-thiéno[3,2-d]pyrimidin-2-yl]-pipéridin-4-yl}-benzamide
1.33. la N-cyclopropyl-N-méthyl-4-{1-[5-oxo-4-(tétrahydropyran-4-ylamino)-6,7-dihydro-5H-5λ⁴-thiéno[3,2-d]pyrimidin-2-yl]-pipéridin-4-yloxy}-benzamide
1.34. la {5-oxo-2-[4-(pyridin-4-yloxy)-pipéridin-1-yl]-6,7-dihydro-5H-5λ⁴-thiéno[3,2-d]pyrimidin-4-yl}-(tétrahydropyran-4-yl)-amine
1.35. la {2-[4-(4-chlorophénoxy)-pipéridin-1-yl]-5-oxo-6,7-dihydro-5H-5λ⁴-thiéno[3,2-d]pyrimidin-4-yl}-(tétrahydropyran-4-yl)-amine
1.36. la (S)-1-méthyl-5-{2-[4-(5-méthyl-4-phényloxazol-2-yl)-pipéridin-1-yl]-5-oxo-6,7-dihydro-5H-5λ⁴-thiéno[3,2-d]pyrimidin-4-ylamino}-pipéridin-2-one
1.37. le (1-{2-[4-(5-méthyl-4-phényloxazol-2-yl)-pipéridin-1-yl]-5-oxo-6,7-dihydro-5H-5λ⁴-thiéno[3,2-d]pyrimidin-4-ylamino}-cyclopropyl)-méthanol,
1.38. la (S)-5-{2-[4-(4,5-diphényloxazol-2-yl)-pipéridin-1-yl]-5-oxo-6,7-dihydro-5H-5λ⁴-thiéno[3,2-d]pyrimidin-4-ylamino}-1-méthylpipéridin-2-one
1.39. le {4-(4-chlorophényl)-1-[5-oxo-4-(tétrahydropyran-4-ylamino)-6,7-dihydro-5H-5λ⁴-thiéno[3,2-d]pyrimidin-2-yl]-pipéridin-4-yl}-méthanol
1.40. le [1-(2-{4-[5-(4-chlorophényl)-4-méthyloxazol-2-yl]-pipéridin-1-yl}-5-oxo-6,7-dihydro-5H-5λ⁴-thiéno[3,2-d]pyrimidin-4-ylamino)-cyclopropyl]-méthanol
1.41. le 4-(4-chlorophényl)-1-[5-oxo-4-(tétrahydropyran-4-ylamino)-6,7-dihydro-5H-5λ⁴-thiéno[3,2-d]pyrimidin-2-yl]pipéridin-4-ol
1.42. le {2-[4-(4-chlorophényl)-4-méthoxypipéridin-1-yl]-6-oxo-6,7-dihydro-5H-5λ⁴-thiéno[3,2-d]pyrimidin-4-yl]-(tétrahydropyran-4-yl)-amine
1.43. le 4-{1-[4-(1-hydroxyméthylcyclopropylamino)-5-oxo-6,7-dihydro-5H-5λ⁴-thiéno[3,2-d]pyrimidin-2-yl]-pipéridin-4-yloxy}benzonitrile
1.44. le 5-oxo-2-[4-(4,5,6,7-tétrahydrobenzoxazol-2-yl)-pipéridin-1-yl]-6,7-dihydro-5H-5λ⁴-thiéno[3,2-d]pyrimidin-4-yl-(tétrahydropyran-4-yl)amine
1.45. le (S)-5-{2-[4-(4-chlorophényl)pipéridin-1-yl]5,5-dioxo-6,7-dihydro-5H-5λ⁴-thiéno[3,2-d]pyrimidin-4-ylamino}-1-méthylpipéridin-2-one.
au moins un antagoniste (2) du récepteur EP4.

22. Combinaison de médicaments selon l'une des revendications 1 à 3, **caractérisée en ce que** l'au moins un antagoniste du récepteur EP4 est choisi dans le groupe consistant en :
le [N-{[4-(5,9-diéthoxy-6-oxo-6,8-dihydro-7H-pyrrolo[3,4-g]quinolin-7-yl)-3-méthylbenzyl] sulfonyl}-2-(2-méthoxyphényl)acétamide] (2.1) ;
la 5-butyl-2,4-dihydro-4-[[2'-[N-(3-méthyl-2-thiophène-carbonyl)sulfamoyl]biphényl-4-yl]méthyl]-2-[(2-trifluorométhyl)phényl]1,2,4-triazol-3-one (2.2) ;
l'acide (4-{(1S)-1-[({5-chloro-2-[(4-fluorophényl)oxy]phényl}carbonyl)amino]éthyl} benzoïque (2.3) ;
le N-[({2-[4-(2-éthyl-4,6-diméthyl-1H-imidazo[4,5-c]pyridin-1-yl)phényl]éthyl}amino)carbonyl]-4-méthylbenzolsulfonamide (2.4) ;
le 4-[[4-(5-méthoxy-2-pyridinyl)phénoxy]méthyl]-5-méthyl-N-[(2-méthylphényl)sulfonyl]-2-furane carboxamide (2.5) ;
l'ester méthylique de l'acide 11-alpha, 15-alpha-dihydroxy-16-(3-méthoxyméthylphényl)-9-oxo-17,18, 19,20-tétranor-5-thia-13(E)prostanoïque (2.6) ;
l'acide 4-cyano-2-[[2-(4-fluoro-1-naphtalényl)-1-oxopropyl]amino]benzènebutanoïque (2.7) et
le N-{2-[4-(4,9-diéthoxy-1-oxo-1,3-dihydro-2H-benzo[f]iso-indol-2-yl)phényl]acétyl}benzène-sulfonamide (2.8).

23. Combinaison de médicaments selon l'une des revendications 1 à 22, **caractérisée en ce que** l'inhibiteur de PDE4 (1) est compris dans une dose quotidienne de 0,01 mg à 50 mg, de préférence de 0,1 mg à 10 mg.

24. Combinaison de médicaments selon l'une des revendications 1 à 23, **caractérisée en ce que** l'antagoniste (2) du récepteur EP4 est utilisé en une dose quotidienne de 0,001 à 100 mg/kg de poids corporel, de préférence de 0,01 à 50 mg/kg de poids corporel, de manière davantage préférée de 0,1 à 10 mg/kg de poids corporel.

25. Combinaison de médicaments selon l'une des revendications 1 à 24, **caractérisée en ce que** l'antagoniste (2) du récepteur EP4 et l'inhibiteur de PDE4 (1) sont utilisés en un rapport en poids de 1 : 1 à 200 : 1, de préférence en un rapport en poids de 10 : 1 à 150 : 1, de manière particulièrement préférée en un rapport en poids de 30 :1 à 100 : 1.

26. Utilisation d'un antagoniste (2) du récepteur EP4 pour la préparation d'un médicament pour la réduction des effets secondaires d'un ou plusieurs inhibiteurs de PDE4 de formule générale 1 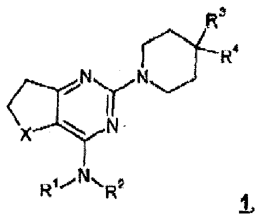 dans laquelle X, R¹, R², R³ et R⁴ sont tels que définis dans l'une des revendications 1 à 2,
pour le traitement d'une maladie choisie dans le groupe consistant en des maladies des voies respiratoires, des maladies pulmonaires, des troubles gastro-intestinaux, également des maladies telles que des maladies inflammatoires des articulations, de la peau ou des yeux, des cancers et des maladies du système nerveux périphérique ou central.

27. Utilisation d'une combinaison contenant un ou plusieurs inhibiteurs de PDE4 (1) de formule générale 1, 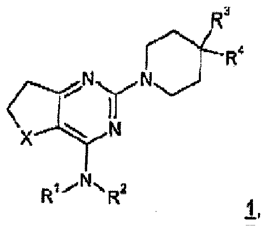 dans laquelle X, R¹, R², R³ et R⁴ sont tels que définis dans l'une des revendications 1 à 20,
et au moins un antagoniste (2) du récepteur EP4 pour la préparation d'un médicament pour le traitement d'une maladie choisie dans le groupe consistant en des maladies des voies respiratoires, des maladies pulmonaires, des troubles gastro-intestinaux, également des maladies telles que des maladies inflammatoires des articulations, de la peau ou des yeux, des cancers et des maladies du système nerveux périphérique ou central.

28. Utilisation selon l'une des revendications 26 à 27, pour le traitement d'une maladie choisie parmi la BPCO, la sinusite chronique, l'asthme, la maladie de Crohn et la rectocolite hémorragique.

29. Utilisation selon l'une des revendications 26 à 28, **caractérisée en ce que** l'inhibiteur de PDE4 (1) et l'au moins un antagoniste (2) du récepteur EP4 sont administrés de façon concomitante dans une formulation commune unique.

30. Utilisation selon l'une des revendications 26 à 38, **caractérisée en ce que** l'inhibiteur de PDE4 (1) et l'au moins un antagoniste (2) du récepteur EP4 sont administrés en deux formulations séparées l'une de l'autre dans un intervalle temporel de 0 à 6 heures.

31. Utilisation selon la revendication 30, dans laquelle la formulation contenant l'inhibiteur de PDE4 (1) est une formulation orale ou à inhaler, de préférence une formulation orale, et où la formulation contenant l'au moins un antagoniste (2) du récepteur EP4 est une formulation orale.

32. Utilisation selon l'une des revendications 30 ou 31, dans laquelle la formulation contenant l'inhibiteur de PDE4 (1) est administrée une fois par jour et la formulation contenant l'au moins un antagoniste (2) du récepteur EP4 est administrée une ou deux fois par jour.

33. Utilisation selon l'une des revendications 26 à 32, **caractérisée en ce que** l'inhibiteur de PDE4 est un composé de formule 1 choisi dans le groupe consistant en :
1.1. le (R)-2-{2-[4-(4-chlorophényl)-pipéridin-1-yl]-5-oxo-6,7-dihydro-5H-5λ⁴-thiéno[3,2-d]pyrimidin-4-ylamino}-3-méthylbutan-1-ol
1.2. le (1-{2-[4-(4-chlorophényl)-pipéridin-1-yl]-5-oxo-6,7-dihydro-5H-5λ⁴-thiéno[3,2-d]pyrimidin-4-ylamino}-cyclopropyl)-méthanol
1.3. le (R)-2-{2-[4-(4-chlorophényl)-pipéridin-1-yl]-5-oxo-6,7-dihydro-5H-5λ⁴-thiéno[3,2-d]pyrimidin-4-ylamino}-pentan-1-ol
1.4. le (R)-1-{2-[4-(4-chlorophényl)-pipéridin-1-yl]-5-oxo-6,7-dihydro-5H-5λ⁴-thiéno[3,2-d]pyrimidin-4-ylamino}-1-(4-fluorophényl)-2-méthylpropan-2-ol
1.5. la (S)-5-{2-[4-(4-chlorophényl)-pipéridin-1-yl]-5-oxo-6,7-dihydro-5H-5λ⁴-thiéno[3,2-d]pyrimidin-4-ylamino}-1-méthylpipéridin-2-one
1.6. la {2-[4-(4-chlorophényl)-pipéridin-1-yl]-5-oxo-6,7-dihydro-5H-5λ⁴-thiéno[3,2-d]pyrimidin-4-yl}-(tétrahydropyran-4-yl)-amine
1.7. la 1-(4-(1-hydroxyméthylcyclopropylamino)-5-oxo-6,7-dihydro-5H-5λ⁴-thiéno[3,2-d]pyrimidin-2-yl)-3'-méthyl-1'H-spiro[pipéridin-4,4'-quinazolin]-2'(3'H)-one
1.8. le {1-[2-(4-benzo[d]isoxazol-3-yl-pipéridin-1-yl)-5-oxo-6,7-dihydro-5H-5λ⁴-thiéno[3,2-d]-pyrimidin-4-ylamino]-cyclopropyl}-méthanol
1.9. le (1-{2-[4-(2-éthyl-5-fluoro-1H-indol-3-yl)-pipéridin-1-yl]-5-oxo-6,7-dihydro-5H-5λ⁴-thiéno-[3,2-d]pyrimidin-4-ylamino}-cyclopropyl)méthanol
1.10. le 1-[4-((S)-1-méthyl-6-oxopipéridin-3-ylamino)-5-oxo-6,7-dihydro-5H-5λ⁴-thiéno[3,2-d]pyrimidin-2-yl]-4-phénylpipéridin-4-carbonitrile
1.11. la 3'-méthyl-1-(4-(tétrahydro-2H-pyran-4-yl-amino)-6-oxo-6,7-dihydro-5H-5λ⁴-thiéno [3,2-d]-pyrimidin-2-yl)-1'H-spiro[pipéridin-4,4'-quinazolin]-2'(3' H) -one
1.12. la (3-fluorophényl)-[5-oxo-2-(3,4,5,6-tétrahydro-2H-[4,4']bipyridinyl-1-yl)-6,7-dihydro-5H-5λ⁴-thiéno[3,2-d]pyrimidin-4-yl]-amine
1.13. la {2-[4-(2-éthyl-5-fluoro-1H-indol-3-yl)-pipéridin-1-yl]-5-oxo-6,7-dihydro-5H-5λ⁴-thiéno[3,2-d]pyrimidin-4-yl}-(3-fluorophényl)-amine
1.14. le (1-{2-[4-(2,4-difluorophényl)-pipéridin-1-yl]-5-oxo-6,7-dihydro-5H-5λ⁴-thiéno[3,2-d]-pyrimidin-4-ylamino}-cyclopropyl)-méthanol
1.15. la {2-[4-(2,4-difluorophényl)-pipéridin-1-yl]-5-oxo-6,7-dihydro-5H-5λ⁴-thiéno[3,2-d]pyrimidin-4-yl}-(tétrahydropyran-4-yl)-amine
1.16. la (S)-5-[2-(4-benzoxazol-2-yl-pipéridin-1-yl)-5-oxo-6,7-dihydro-5H-5λ⁴-thiéno[3,2-d]pyrimidin-4-ylamino]-1-méthylpipéridin-2-one
1.17. le (1-{2-[4-(6-fluorobenzène[d]isoxazol-3-yl)-pipéridin-1-yl]-5-oxo-6,7-dihydro-5H-5λ⁴-thiéno-[3,2-d]pyrimidin-4-ylaminol-cyclopropyl)méthanol
1.18. le (1-{2-[4-(5-fluorobenzène[d]isoxazol-3-yl)-pipéridin-1-yl]-5-oxo-6,7-dihydro-5H-5λ⁴-thiéno-[3,2-d]pyrimidin-4-ylaminol-cyclopropyl)méthanol
1.19. la {2-[4-(5-furan-2-yl-2H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.20. la (3-fluorophényl)-{5-oxo-2-[4-(3-pyridin-4-yl-[1,2,4]oxadiazol-5-yl)-pipéridin-1-yl]-6,7-dihydro-5H-5λ⁴-thiéno[3,2-d]pyrimidin-4-yl}-amine
1.21. le (R)-3-méthyl-2-{5-oxo-2-[4-(3-pyridin-4-yl-[1, 2,4]oxadiazol-5-yl)-pipéridin-1-yl]-6,7-dihydro-5H-5λ⁴-thiéno[3,2-d]pyrimidin-4-yl}-butan-1-ol
1.22. la (S)-5-{2-[4-(4-fluorophénoxy)-pipéridin-1-yl]-5-oxo-6,7-dihydro-5H-5λ⁴-thiéno[3,2-d]-pyrimidin-4-ylamino}-1-méthylpipéridin-2-one
1.23. la (2-{4-[4-(4,5-dihydro-oxazol-2-yl)-phénoxy]-pipéridin-1-yl}-5-oxo-6,7-dihydro-5H-5λ⁴-thiéno[3,2-d]pyrimidin-4-yl)-(tétrahydropyran-4-yl)-amine
1.24. l'acide 4-{1-[5-oxo-4-(tétrahydropyran-4-ylamino)-6,7-dihydro-5H-5λ⁴-thiéno[3,2-d]-pyrimidin-2-yl]-pipéridin-4-yloxy}-benzoïque
1.25. la 2-(1-{2-[4-(4-chlorophényl)-pipéridin-1-yl]-5-oxo-6,7-dihydro-5H-5λ⁴-thiéno[3,2-d]pyrimidin-4-ylamino]-cyclopropyl)propan-2-ol
1.26. la {2-[4-(5-tert-butyl-1-méthyl-1H-indol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.27. la 2-[4-(5-furan-2-yl-1-méthyl-1H-pyrazol-3-yl)-pipéridin-1-yl]-5-oxo-6,7-dihydro-5H-5λ⁴-thiéno-[3,2-d]pyrimidin-4-yl-(tétrahydropyran-4-yl)-amine
1.28. la (S)-5-(2-{4-[4-(4,5-dihydrooxazol-2-yl)-phénoxy]-pipéridin-1-yl}-5-oxo-6,7-dihydro-5H-5λ⁴-thiéno[3,2-d]pyrimidin-4-ylamino)-1-méthyl-pipéridin-2-one
1.29. la {2-[4-(5-furan-2-yl-2-méthyl-2H-pyrazol-3-yl)-pipéridin-1-yl]-5-oxo-6,7-dihydro-5H-5λ⁴-thiéno[3,2-d]pyrimidin-4-yl}-(tétrahydropyran-4-yl)-amine
1.30. la {2-[4-(1-méthyl-1H-imidazol[4,5,c]pyridin-2-yl)-pipéridin-1-yl]-5-oxo-6,7-dihydro-5H-5λ⁴-thiéno[3,2-d]pyrimidin-4-yl}-(tétrahydropyran-4-yl)-amine
1.31. la 2-méthoxy-N-{1-[5-oxo-4-(tétrahydropyran-4-ylamino)-6,7-dihydro-5H-5λ⁴-thiéno[3,2-d]-pyrimidin-2-yl]-4-phénylpipéridin-4-ylméthyl}-acétamide
1.32. la N-cyclopropyl-N-méthyl-4-{1-[5-oxo-4-(tétrahydropyran-4-ylamino)-6,7-dihydro-5H-5λ⁴-thiéno-[3,2-d]pyrimidin-2-yl]-pipéridin-4-yl}-benzamide
1.33. la N-cyclopropyl-N-méthyl-4-{1-[5-oxo-4-(tétrahydropyran-4-ylamino)-6,7-dihydro-5H-5λ⁴-thiéno-[3,2-d]pyrimidin-2-yl]-pipéridin-4-yloxy}-benzamide
1.34. la {5-oxo-2-[4-(pyridin-4-yloxy)-pipéridin-1-yl]-6,7-dihydro-5H-5λ⁴-thiéno[3,2-d]pyrimidin-4-yl}-(tétrahydropyran-4-yl)-amine
1.35. la {2-[4-(4-chlorophénoxy)-pipéridin-1-yl]-5-oxo-6,7-dihydro-5H-5λ⁴-thiéno[3,2-d]pyrimidin-4-yl}-(tétrahydropyran-4-yl)-amine
1.36. la (S)-1-méthyl-5-{2-[4-(5-méthyl-4-phényl-oxazol-2-yl)-pipéridin-1-yl]-5-oxo-6,7-dihydro-5H-5λ⁴-thiéno[3,2-d]pyrimidin-4-ylamino}-pipéridin-2-one
1.37. le (1-{2-[4-(5-méthyl-4-phényloxazol-2-yl)-pipéridin-1-yl]-5-oxo-6,7-dihydro-5H-5λ⁴-thiéno[3,2-d]pyrimidin-4-ylamino}-cyclopropyl)-méthanol
1.38. la (S)-5-{2-[4-(4,5-diphényloxazol-2-yl)-pipéridin-1-yl]-5-oxo-6,7-dihydro-5H-5λ⁴-thiéno[3,2-d]pyrimidin-4-ylamino}-1-méthylpipéridin-2-one
1.39. le {4-(4-chlorophényl)-1-[5-oxo-4-(tétrahydropyran-4-ylamino)-6,7-dihydro-5H-5λ⁴-thiéno[3,2-d]pyrimidin-2-yl]-pipéridin-4-yl}-méthanol
1.40. le [1-(2-{4-[5-(4-chlorophényl)-4-méthyloxazol-2-yl]-pipéridin-1-yl}-5-oxo-6,7-dihydro-5H-5λ⁴-thiéno[3,2-d]pyrimidin-4-ylamino)-cyclopropyl]-méthanol
1.41. le 4-(4-chlorophényl)-1-[5-oxo-4-(tétrahydropyran-4-ylamino)-6,7-dihydro-5H-5λ⁴-thiéno[3,2-d]pyrimidin-2-yl]pipéridin-4-ol
1.42. le {2-[4-(4-chlorophényl)-4-méthoxypipéridin-1-yl]-5-oxo-6,7-dihydro-5H-5λ⁴-thiéno[3,2-d]pyrimidin-4-yl}-(tétrahydropyran-4-yl)-amine
1.43. le 4-{1-[4-(1-hydroxyméthylcyclopropylamino)-5-oxo-6,7-dihydro-5H-5λ⁴-thiéno[3,2-d]pyrimidin-2-yl]-pipéridin-4-yloxy}benzonitrile
1.44. le 5-oxo-2-[4-(4,5,6,7-tétrahydrobenzoxazol-2-yl)-pipéridin-1-yl]-6,7-dihydro-5H-5λ⁴-thiéno-[3,2-d]pyrimidin-4-yl-(tétrahydropyran-4-yl)-amine
1.45. le (S)-5-{2-[4-(4-chlorophényl)pipéridin-1-yl]-5,5-dioxo-6,7-dihydro-5H-5λ⁴-thiéno [3, 2-d]pyrimidin-4-ylamino}-1-méthylpipéridin-2-one.

34. Utilisation selon l'une des revendications 26 à 33, **caractérisée en ce que** l'au moins un antagoniste (2) du récepteur EP4 est choisi dans le groupe consistant en :
le [N-{[4-(5,9-diéthoxy-6-oxo-6,8-dihydro-7H-pyrrolo[3,4-g]quinolin-7-yl)-3-méthylbenzyl] sulfonyl}-2-(2-méthoxyphényl)acétamide] (2.1) ;
la 5-butyl-2,4-dihydro-4-[[2'-[N-(3-méthyl-2-thiophène-carbonyl)sulfamoyl]biphényl-4-yl]-méthyl]-2-[(2-trifluorométhyl)phényl]1,2,4-triazol-3-one (2.2) ;
l'acide (4-{(1S)-1-[({5-chloro-2-[(4-fluorophényl)oxy]phényl}carbonyl)amino]éthyl} benzoïque (2.3) ;
le N-[({2-[4-(2-éthyl-4,6-diméthyl-1H-imidazo[4,5-c]pyridin-1-yl)phényl]éthyl}amino)carbonyl]-4-méthylbenzolsulfonamide (2.4) ;
le 4-[[4-(5-méthoxy-2-pyridinyl)phénoxy]méthyl]-5-méthyl-N-[(2-méthylphényl)sulfonyl]-2-furane carboxamide (2.5) ;
l'ester méthylique de l'acide 11-alpha, 15-alpha-dihydroxy-16-(3-méthoxyméthylphényl)-9-oxo-17,18,19,20-tétranor-5-thia-13(E)prostanoïque (2.6) ;
l'acide 4-cyano-2-[[2-(4-fluoro-1-naphtalényl)-1-oxopropyl]amino]benzènebutanoïque (2.7) et
le N-{2-[4-(4,9-diéthoxy-1-oxo-1,3-dihydro-2H-benzo[f]iso-indol-2-yl)phényl]acétyl}benzène-sulfonamide (2.8).

35. Utilisation selon l'une des revendications 26 à 34, **caractérisée en ce que** l'inhibiteur de PDE4 de formule générale 1 est utilisé en une dose quotidienne de 0,01 mg à 50 mg.

36. Utilisation selon l'une des revendications 26 à 35, **caractérisée en ce que** l'antagoniste (2) du récepteur EP4 est utilisé en une dose quotidienne de 0,001 à 100 mg/kg de poids corporel, de préférence de 0,01 à 50 mg/kg de poids corporel, de manière davantage préférée de 0,1 à 10 mg/kg de poids corporel.

37. Utilisation selon l'une des revendications 26 à 36, **caractérisée en ce que** l'antagoniste (2) du récepteur EP4 et l'inhibiteur de PDE4 (1) sont utilisés en un rapport en poids de 1 : 1 à 200 : 1, de préférence en un rapport en poids de 10 : 1 à 150 : 1, de manière particulièrement préférée en un rapport en poids de 30 : 1 à 100 : 1.
